(19) Europäisches Patentamt
European Patent Office
Office européen des brevets

(11) EP 0 743 320 A2

(12) EUROPEAN PATENT APPLICATION

(43) Date of publication:
20.11.1996 Bulletin 1996/47

(51) Int. Cl.[6]: C07K 5/065, A61K 38/05

(21) Application number: 96107675.9

(22) Date of filing: 14.05.1996

(84) Designated Contracting States:
AT BE CH DE DK ES FI FR GB GR IE IT LI LU MC NL PT SE

(30) Priority: 18.05.1995 US 443940

(71) Applicant: BRISTOL-MYERS SQUIBB COMPANY
Princeton, NJ 08543-4000 (US)

(72) Inventors:
- Kimball, S. David
East Windsor, NJ 08520 (US)
- Das, Jagabandhu
Mercerville, NJ 08619 (US)
- Chen, Ping
Lawrenceville, NJ 08648 (US)
- Iwanowicz, Edwin J.
Cranbury, NJ 08512 (US)
- White, Ronald E.
Langhorne, PA 19047 (US)
- Zahler, Robert
Pennington, NJ 08534 (US)

(74) Representative: Josif, Albert, Dr.-Ing. et al
Baaderstrasse 3
80469 München (DE)

# (54) Acyl guanidine and amidine prodrugs

(57) Acyl guanidine, thioguanidine and amidine compounds are provided which have the structure

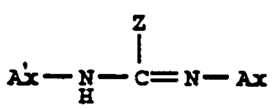

wherein Z is a substructure which when linked to

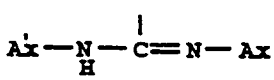

forms a prodrug of compounds with pharmaceutically active properties. In preferred embodiments, Z is a thrombin inhibitor substructure containing residues binding at the distal and proximal sites with the proviso that Z does not contain boron or a boron-containing moiety.

Ax and **A'x** may be the same or different and are independently selected from Acyl, H or alkyl, at least one of Ax and **A'x** being Acyl; and

including all stereoisomers thereof, and pharmaceutically acceptable salts thereof.

EP 0 743 320 A2

## Description

The present invention relates to prodrugs of guanidine, thioguanidine or amidine containing compounds which are pharmaceutically active and, for example, are useful in inhibiting formation of thrombin, or in inhibiting platelet aggregation, or as fibrinogen receptor antagonists, and the like.

In accordance with the present invention, compounds having the structure I

I

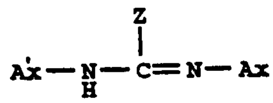

wherein Z is a substructure which when linked to

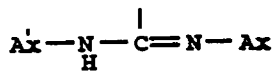

forms a prodrug of compounds with pharmaceutically active properties; with the proviso that Z does not contain boron or a boron-containing moiety. Thus, the Z substructure when linked to

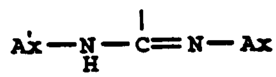

forms a prodrug of a thrombin inhibitor, a platelet aggregation inhibitor, or a fibrinogen receptor antagonist, a GPIIb/IIIa receptor blocker, an antihypertensive, an antidepressant, an antibiotic, a viricide, an immunostimulant, an antiinflammatory agent, a peptide hydrolase inhibitor, a Factor Xa inhibitor, an antianaphylactic, an antiulcer agent or can have other pharmaceutical activity as defined hereinafter.

Z is preferably a thrombin inhibitor substructure containing residues binding at the distal and proximal sites (which sites are described by Banner and Hadvary, J. Biol. Chem. (I99I), 266, 20085-20093);

Ax and **A'x** may be the same or different and are independently selected from Acyl, H or alkyl, at least one of Ax and **A'x** being Acyl, wherein Acyl includes the moiety

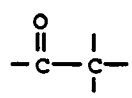

wherein

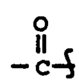

is linked to a nitrogen atom in formula I; and

including all stereoisomers thereof, and pharmaceutically acceptable salts thereof.

The term "Acyl" as employed in formula I more preferably refers to a moiety of the structure

(1)

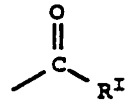,

(2)

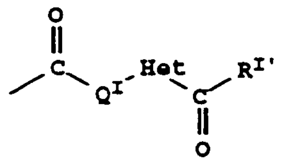,

(3)

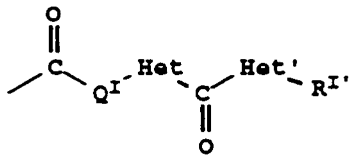

or

(4)

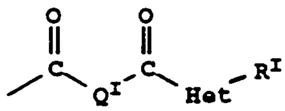

In formula I, Acyl is exclusive of a group which includes the moiety

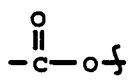

wherein

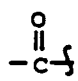

would be linked to a nitrogen atom in formula I; thus, formula I will not include the moiety

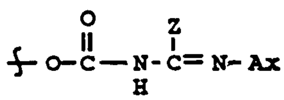 or 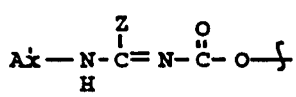.

In the above groups (I), (2), (3) and (4), $R^I$ is H, alkyl, cycloalkyl, heterocycloalkyl, heteroaryl, aryl, substituted alkyl or substituted aryl;

$R^{I'}$ is alkyl, cycloalkyl, heterocycloalkyl, heteroaryl, aryl, substituted alkyl or substituted aryl;

$Q^I$ is alkyl, cycloalkyl, aryl, heterocycloalkyl, substituted alkyl, substituted aryl or heteroaryl;

Het or Het' is independently O, NH, N-lower alkyl or S;

where in the definition of $R^I$, $R^{I'}$ and/or $Q^I$ substituted alkyl refers to alkyl, by itself or as part of another group, which includes linear or branched alkyl, substituted with from I to 4 substituents, which substituents include the following: OH, $NH_2$, SH, $OR^{II}$, $NHR^{II}$, $NR^{II}R^{III}$, $SR^{II}$, $SSR^{II}$, CHO, $COR^{II}$, alkyl, cycloalkyl, halogen and/or aryl; and substituted aryl refers to aryl, by itself or as part of another group, substituted with from I to 5 substituents which substituents include the following: OH, $NH_2$, SH, $OR^{II}$, $NHR^{II}$, $NR^{II}R^{III}$, $SR^{II}$, $SSR^{II}$, CHO, $COR^{II}$, alkyl, cycloalkyl, halogen and/or

aryl;

$R^{II}$ and $R^{III}$ are the same or different and are independently selected from H, alkyl, cycloalkyl, aryl, substituted alkyl or substituted aryl; and

the term "heterocyclyl" may include I to 3 rings and may be heteroaryl or cycloheteroalkyl.

In the Acyl moieties (2), (3) and (4), it is preferred that Het and Het' (where present) are each oxygen.

The preferred Acyl moiety will be Acyl moieties (I) and (2) where Het is O and $R^{I}$ is alkyl or arylalkyl and $R^{I'}$ is alkyl or arylalkyl, and $Q^{I}$ is alkyl or cycloalkyl.

In accordance with the present invention, the prodrugs of the guanidine, thioguanidine or amidine containing compounds of formula I of the invention have enhanced absorption and improved bioavailability properties.

The compounds of the invention include prodrugs of sulfonamido heterocyclic thrombin inhibitors having the Z substructure and which include a guanidine, thioguanidine or amidine moiety, and have the structure Ix

Ix 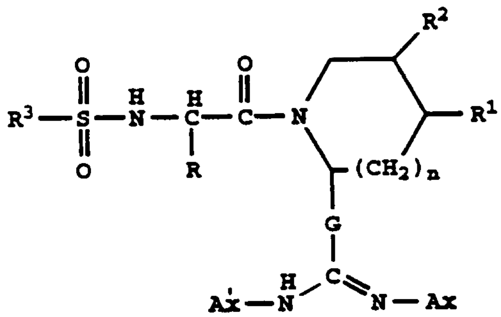

wherein

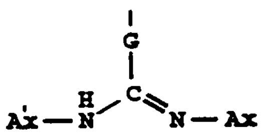

is an amido moiety which is

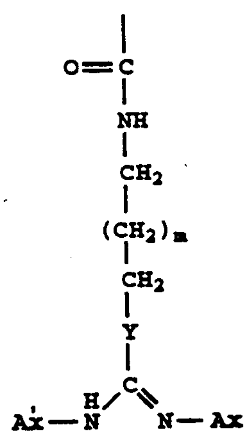

(G1)

or

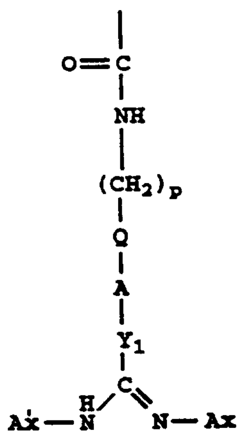

(G2)

including all stereoisomers thereof; and including all pharmaceutically acceptable salts thereof; wherein

R is hydrogen, hydroxyalkyl, aminoalkyl, amidoalkyl, alkyl, cycloalkyl, aryl, arylalkyl, cycloalkylalkyl, alkenyl, alkynyl, arylalkoxyalkyl, or an amino acid side chain, either protected or unprotected;

$R^1$ and $R^2$ are independently hydrogen, lower alkyl, cycloalkyl, aryl, hydroxy, alkoxy, oxo, thioxo, thioketal, thioalkyl, thioaryl, amino or alkylamino; or $R^1$ and $R^2$ together with the carbons to which they are attached form a cycloalkyl, aryl, or heteroaryl ring; and

$R^3$ is alkyl, aryl, arylalkyl, heteroaryl, quinolinyl, tetrahydroquinolinyl, I0-camphoryl, pentamethylchromanyl, pen-

taalkylphenyl, pentahalophenyl, trialkylphenyl, 3-carboxyphenyl, 3-trifluoromethylphenyl or 4-carboxyphenyl;

n is 0, l or 2;

m is 0, l, 2 or 3;

Y is NH or S;

p is 0, l or 2;

Q is a single bond or

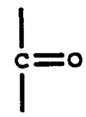

;

$Y_1$ is a bond or -NH-;

A is aryl or cycloalkyl, or an azacycloalkyl ring A of 4 to 8 ring members, or an azacycloalkenyl ring A of 5 to 9 ring members, or an azaheterocycloalkyl ring A of 6 to 8 ring members,

A

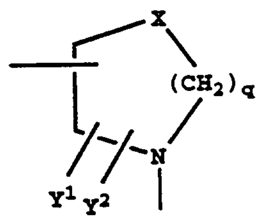

where X is $CH_2$, -CH=CH-, O, S or NH;

if A is an azacycloalkyl, azacycloalkenyl, or azaheterocycloalkyl ring A, then $Y_1$ is a bond and the acylamidine group

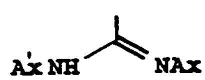

is attached to the nitrogen atom in the ring as indicated below

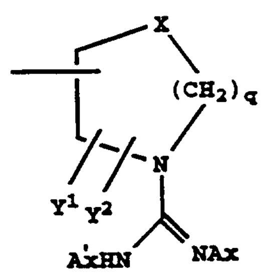

;

q is 0, l, 2, 3 or 4 if X is $CH_2$ or -CH=CH-;

q is 2, 3 or 4 if X is O, S or NH;

$Y^1$ and $Y^2$ are independently H, lower alkyl, oxo or halo;

provided that where X is a hetero atom (that is, A is azaheterocycloalkyl), then there must be at least a 2-carbon chain between X and any N atom in the ring A or outside ring A.

Examples of the A ring (azacycloalkyl, azacycloalkenyl or azaheterocycloalkyl) which may be employed herein include

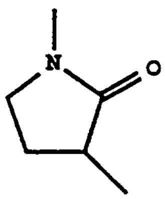, 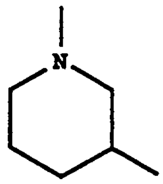, 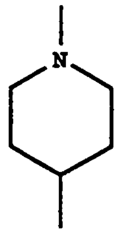, 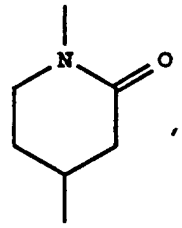, 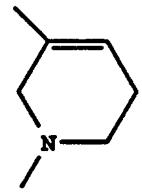,

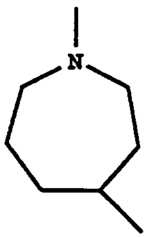, 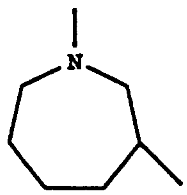, 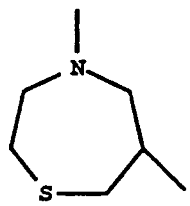, 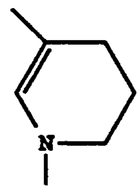,

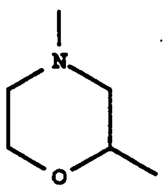, 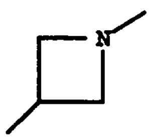, 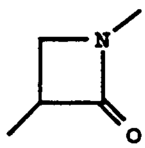, 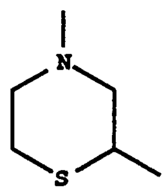, 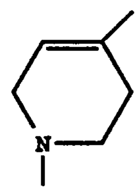,

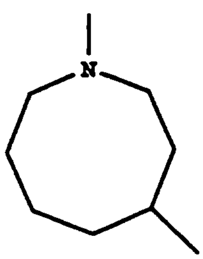, 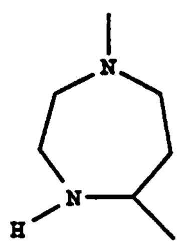, 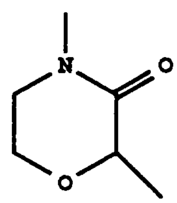, 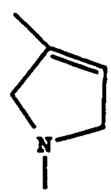,

and the like.

Preferred are compounds of formula $I_x$ wherein

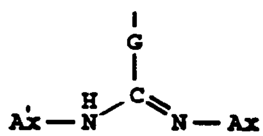 is

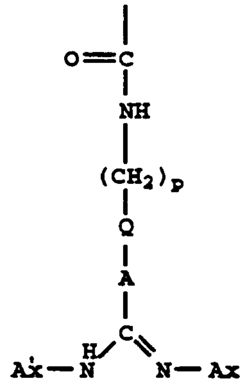

wherein Q is a single bond and A is an azacycloalkyl ring where

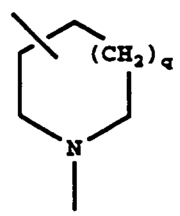

q is 0 or l; p is l or 2;
$R^3$ is lower alkyl or arylalkyl;
R is aralkyl or hydroxyalkyl;
$R^1$ and $R^2$ are each H;
n is 0 or l; and
Ax or **A'x** is

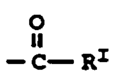

and $R^I$ is alkyl or arylalkyl and the other is H, or
Ax or **A'x** is

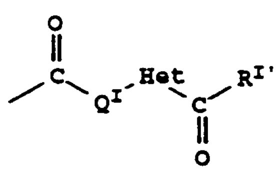

wherein $Q^I$ is alkyl or cycloalkyl;
Het is O and $R^{I'}$ is alkyl or arylalkyl, and the other is H.

A more preferred embodiment of the heterocyclic thrombin inhibitors of the invention has the structure IxA

IxA

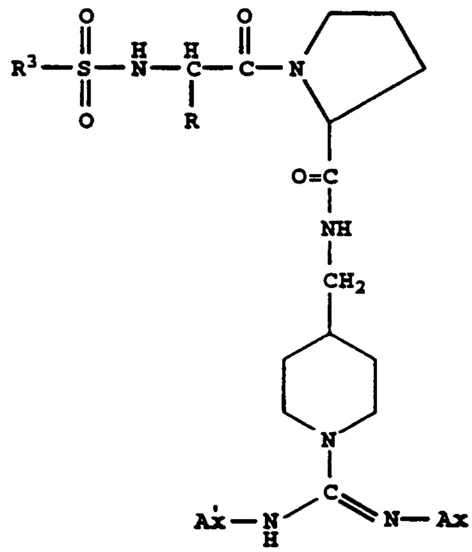

where R is aralkyl (preferably benzyl), aryl (preferably phenyl), substituted alkyl (preferably cyclohexylmethyl) or arylalkoxyalkyl (preferably benzyloxymethyl) and $R^3$ is preferably methyl, ethyl, trifluoroethyl or benzyl, and the preferred Ax and **A'x** are as set out above, including all stereoisomers thereof.

The compounds of the invention also include prodrugs of guanidinyl- or amidinyl-substituted heterocyclic thrombin inhibitors having the Z substructure and include a guanidine or amidine moiety, and have the structure 1.

1.

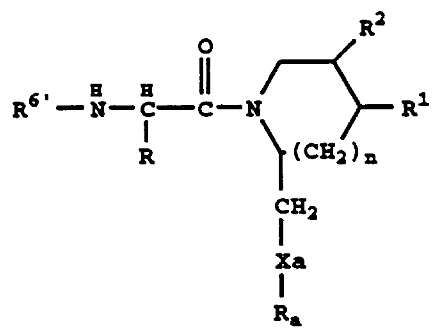

including all stereoisomers thereof, wherein n is 0, l or 2;

Xa is S, SO, $SO_2$ or O;

$R_a$ is $-A^1-R^{3a}$, where $A^1$ is an alkyl, alkenyl, or alkynyl chain, with the proviso that there is at least one carbon between any S or O and an alkenyl or alkynyl moiety, each of the $A^1$ radicals having from 2 to 6 carbon atoms, and $R^{3a}$ is

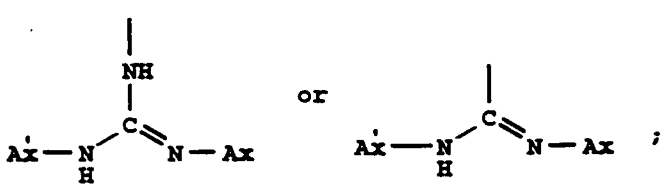

or

$R_a$ is $-(CH_2)_p-A^2-R^{2'}$ where $A^2$ is an azacycloalkyl ring A of 4 to 8 ring members, or a azacycloalkenyl ring A of 5 to 9 ring members, or an azaheterocycloalkyl ring A of 6 to 8 ring members,

A

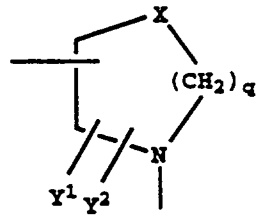

where X is $CH_2$, -CH=CH-, O, S or NH; p is 0, I or 2;

the

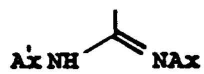

group is attached to the nitrogen atom in the ring as indicated below

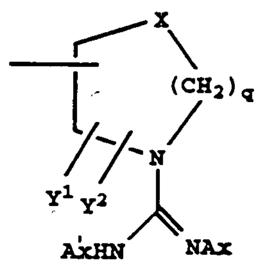

q is 0, I, 2, 3 or 4 if X is $CH_2$ or -CH=CH-;

q is 2, 3 or 4 if X is O, S or NH;

$Y^1$ and $Y^2$ are independently H, lower alkyl, oxo or halo;

provided that where X is a hetero atom (that is, A is azaheterocycloalkyl), then there must be at least a 2-carbon chain between X and any heteroatom atom in the ring A or outside ring A; and $R^{2'}$ is

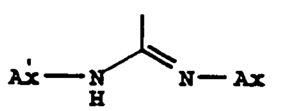 ;

or

$R_a$ is $-(CH_2)_p-A^3-R^4$,

wherein $R^4$ is

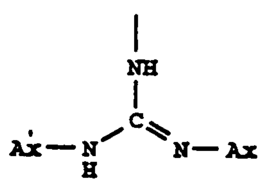 or 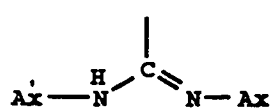

and

$A^3$ is aryl or cycloalkyl;

R, $R^1$ and $R^2$ are as defined hereinbefore; and

$R^{6'}$ is hydrogen,

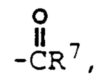

$-SO_2R^3$ or $-CO_2R^7$ (wherein $R^7$ is lower alkyl, aryl, arylalkyl, cycloheteroalkyl or heteroaryl; and $R^3$ is alkyl, arylalkyl, aryl, heteroaryl, quinolinyl, tetrahydrocuinolinyl, l0-camphoryl, pentamethylchromanyl, pentaalkylphenyl, pentahalophenyl, trialkylphenyl, 3-carboxyphenyl, 3-trifluoro-methylphenyl or 4-carboxyphenyl);

including pharmaceutically acceptable salts thereof, and all stereoisomers thereof.

Preferred are compounds of formula 1. wherein Xa is S or $SO_2$, n is 0, $R_a$ is $-(CH_2)_p-A^2R^{2'}$ or $-CH_2(CH_2)_z-R^{3a}$, z is l, 2, 3 or 4; Ax or **A'x** is

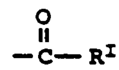

and $R^I$ is alkyl or arylalkyl, and the other is H;

or Ax or **A'x** is

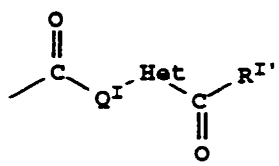

wherein $Q^I$ is alkyl or cycloalkyl,

Het is O and $R^{I'}$ is alkyl or arylalkyl, and the other is H; more preferably

$R_a$ is

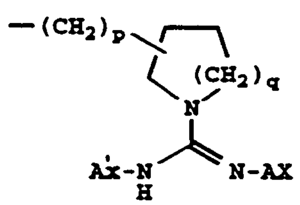,

q is l or 2, p is l or 2,

$R^1$ and $R^2$ are each H, R is H or $-CH_2OH$, Xa is S, and $R^{6'}$ is

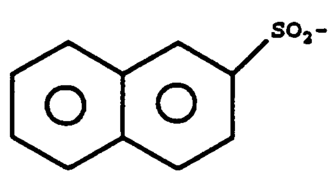

and Ax and **A'x** are as set out above.

The compounds of the invention also include prodrugs of guanidinyl- or amidinyl-substituted methylamino heterocyclic thrombin inhibitors having the Z substructure and include a guanidine or amidine moiety, and have the structure Iq

Iq

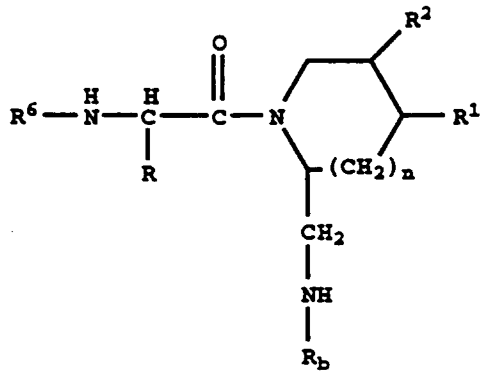

including all stereoisomers thereof
wherein n is 0, l or 2;

$R_b$ is $-A^1-R^{3a}$, $-CO-A^1-R^{3a}$ or $-SO_2-A^1-R^{3a}$;
wherein $R^{3a}$ is

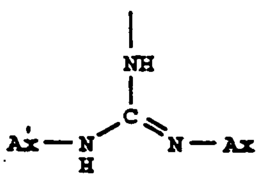 or 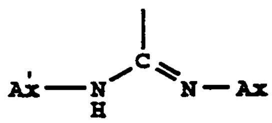 ;

and $A^1$ is an alkyl, alkenyl or alkynyl chain, each of the $A^1$ radicals preferably having 2 to 6 carbons, with the proviso that there is at least one carbon between any NH, S or O and an alkenyl or alkynyl moiety; or

$R_b$ is $-(CH_2)_p-A^2-R^{2'}$ or $-(CH_2)_p-CO-A^2-R^{2'}$ or $(CH_2)_p-SO_2-A^2-R^{2'}$ where p is 0, l or 2, $R^{2'}$ is

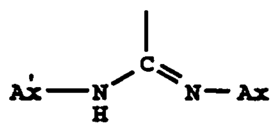

and $A^2$ is an azacycloalkyl ring A of 4 to 8 ring members, or an azacycloalkenyl ring A of 5 to 9 ring members, or an azaheterocycloalkyl ring A of 6 to 8 ring members),

A

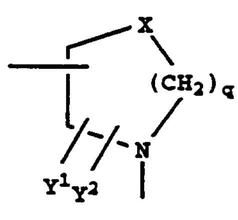

where X is $CH_2$, O, -CH=CH-, S or NH; and

the

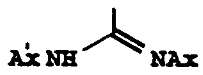

is attached to the nitrogen atom in the ring as indicated below

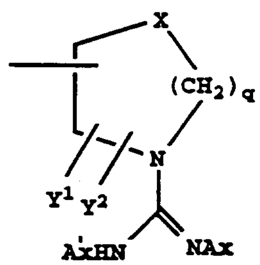

;

q is 0, l, 2, 3 or 4 if X is $CH_2$ or -CH=CH-;

q is 2, 3 or 4 if X is O, S, NH;

$Y^1$, $Y^2$ are independently H, lower alkyl, oxo or halo;

provided that where X is a hetero atom (that is, A is azaheterocycloalkyl), then there must be at least a 2-carbon chain between X and any N atom in the ring A or outside ring A or

$R_b$ is $-(CH_2)_p-A^3-R^4$, $-(CH_2)_p-CO-A^3-R^4$, or $-(CH_2)_p-SO_2-A^3-R^4$,

wherein $R^4$ is

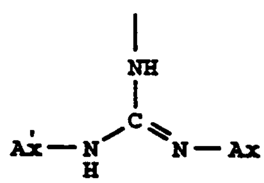

or

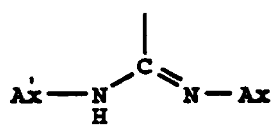

;

$A^3$ is aryl or cycloalkyl, and p is as defined above;

R, $R^1$ and $R^2$ are as defined hereinbefore;

$R^6$ is hydrogen,

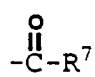

,

$-SO_2R^3$ or $-CO_2R^7$ (wherein $R^7$ is lower alkyl, aryl, arylalkyl, cycloheteroalkyl or heteroaryl; and $R^3$ is alkyl, arylalkyl, aryl, heteroaryl, quinolinyl, tetrahydroquinolinyl, l0-camphoryl, pentamethylchromanyl, pentaalkylphenyl, pentahalophenyl, trialkylphenyl, 3-carboxyphenyl, 3-trifluoromethylphenyl or 4-carboxyphenyl);

including pharmaceutically acceptable salts thereof.

Preferred are compounds of formula Iq wherein n is 0, $R_b$ is

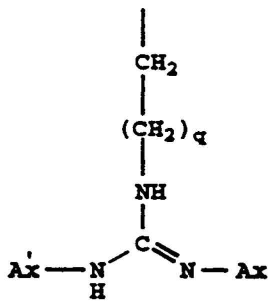

;

and q is 3, 4 or 5; and compounds of formula Iq wherein $R_b$ is

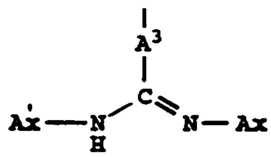

,

$A^3$ is phenyl and compounds of formula Iq wherein $R_b$ is

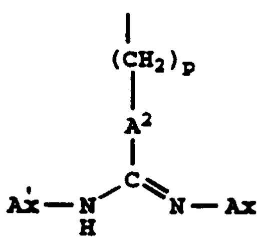

or

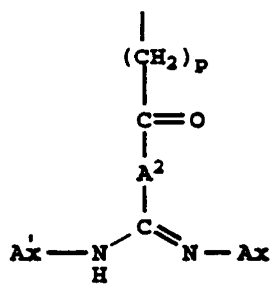

,

p is 0 or l, $A^2$ is azacycloalkyl or azacycloalkenyl;

$R^1$ and $R^2$ are each H, R is hydroxymethyl, $-CH_2COO$alkyl, or benzyl and $R^6$ is

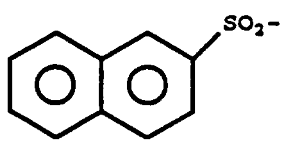

H, BOC or CBZ;

Ax or **A'x** is

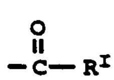

and $R^I$ is alkyl or arylalkyl, and the other is H,

or Ax or **A'x** is

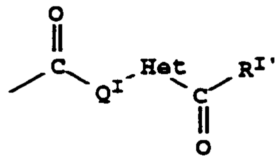

wherein $Q^I$ is alkyl or cycloalkyl;

Het is O and $R^{I'}$ is alkyl or arylalkyl, and the other is H.

Most preferred are compounds of formula Iq wherein n is 0, $R_b$ is

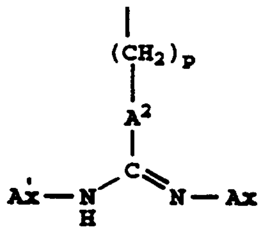

or

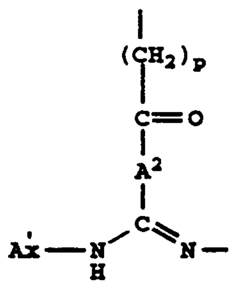

,

wherein p is 0 or l, $A^2$ is

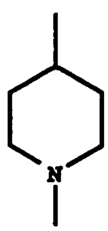

,

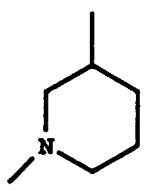

,

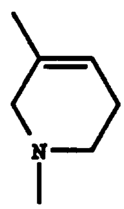

or

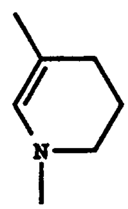

$R^1$ and $R^2$ are each H, R is hydroxymethyl, $-CH_2COOCH_3$, or benzyl, and $R^6$ is

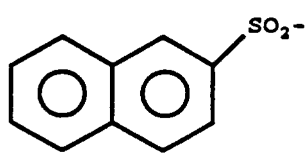

,

H, BOC or CBZ; and

Ax and **A'x** are as set out above.

The compounds of the invention also include prodrugs of heterocyclic thrombin inhibitors of the invention having the Z substructure and include a guanidine or amidine moiety, and have the structure A.

A. 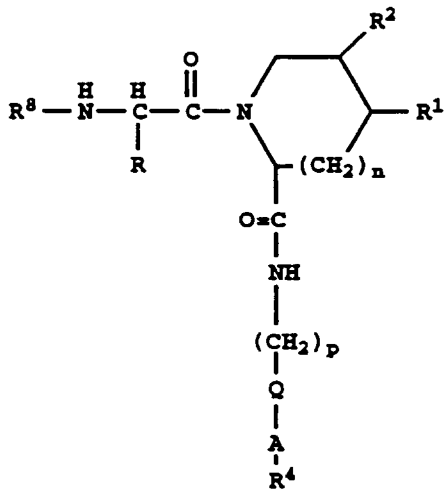

where $R^4$ is

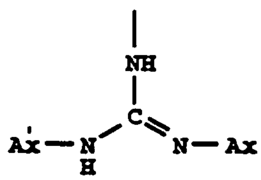 or 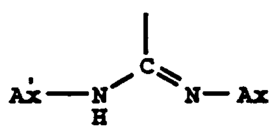

including all stereoisomers thereof, and including all pharmaceutically acceptable salts thereof;
wherein n is 0, l or 2;

p is 0, l or 2;

Q is a single bond or

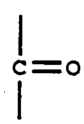 ;

A is aryl or cycloalkyl (in which case $R^4$ is either of the acylguanidine or acylamidine group set out above), or an azacycloalkyl ring A of 4 to 8 ring members; or an azacycloalkenyl ring A of 5 to 9 ring members, or an azaheterocycloalkyl ring A of 6 to 8 ring members,

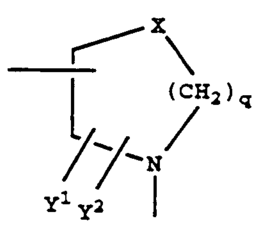

where X is $CH_2$, -CH=CH-, O, S or NH;

if A is an azacycloalkyl, azacycloalkenyl, or azaheterocycloalkyl ring A, then $R^4$ is the acylamidine group

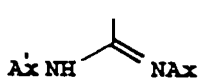

which is attached to the nitrogen atom in the ring as indicated below

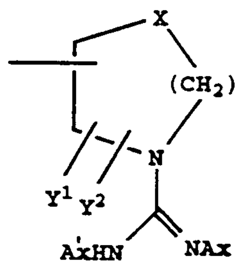

q is 0, I, 2, 3 or 4 if X is $CH_2$ or -CH=CH-;
q is 2, 3 or 4 if X is O, S or NH;
$Y^1$ and $Y^2$ are independently H, lower alkyl, oxo or halo;
provided that where X is a hetero atom (that is, A is azaheterocycloalkyl), then there must be at least a 2-carbon chain between X and any N atom in the ring A or outside ring A.

R, $R^1$ and $R^2$ are as defined hereinbefore; and
$R^8$ is hydrogen,

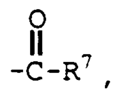

or $-CO_2R^7$ (wherein $R^7$ is lower alkyl, aryl, arylalkyl, cycloheteroalkyl or heteroaryl);
with the provisos that where A is aryl or cycloalkyl, $R^4$ includes guanidine or amidine;
where A is azacycloalkyl, azacycloalkenyl or azaheterocycloalkyl, $R^4$ includes amidine.

Preferred are compounds of formula A. wherein
n is 0 or I,
$R^8$ is H; R is aralkyl or hydroxyalkyl,
$R^1$ and $R^2$ are each H, p is 0 or I,
Q is a single bond, A is an azacycloalkyl ring

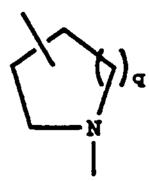

where q is I or 2; $R^4$ includes amidine; and
Ax or **A'x** is

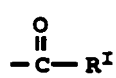

and $R^I$ is alkyl or arylalkyl, and the other is H,
or Ax or **A'x** is

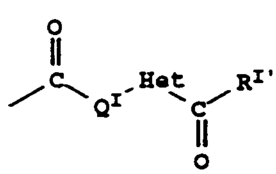

wherein $Q^I$ is alkyl or cycloalkyl,

Het is O and $R^{I'}$ is alkyl or arylalkyl, and the other is H.

Most preferred are compounds of formula A.

wherein $R^8$ is H, n is 0, $R^1$ and $R^2$ are each H,

R is aralkyl such as benzyl,

p is I, Q is a single bond, $AR^4$ is

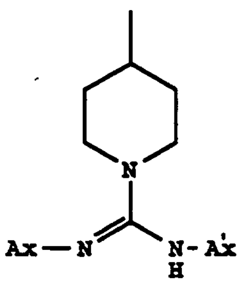

or

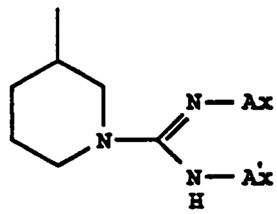

.

Ax and **A'x** are as set out above.

The compounds of the invention also include prodrugs of tripeptide thrombin inhibitors having the Z substructure and include a guanidine functionalized sidechain, and have the structure Iy

Iy

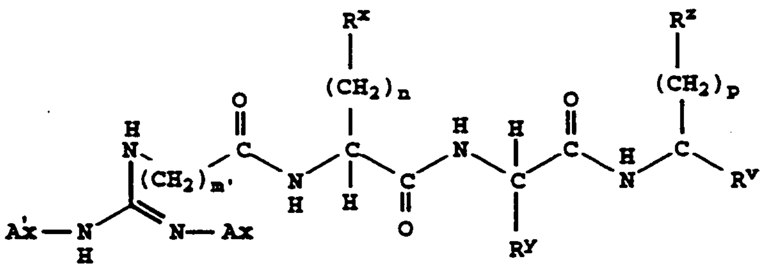

including all stereoisomers thereof, wherein Ax and A'x are as defined above, m' is 2, 3, 4 or 5; n is 0, I or 2; p is 0, I or 2;

$R^x$ is cycloalkyl, heteroaryl, $CO_2H$, $CONR^sR^t$ (where $R^s$ and $R^t$ are independently selected from H, alkyl, cycloalkyl, cycloheteroalkyl, aryl or heteroaryl), or aryl optionally substituted with $NO_2$, OH, alkoxy, acyloxy,

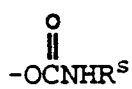

,

halogen, alkyl, aryl, $CO_2$alkyl, CONHalkyl, alkylthio, arylthio, NHalkyl or NHcycloalkyl;

$R^y$ is an amino acid sidechain (either protected or unprotected);

$R^z$ is H, alkyl, aryl, cycloalkyl, cycloheteroalkyl or heteroaryl;

$R^v$ is H, alkyl, $CO_2R^u$ or $CONR^sR^t$;

wherein $R^u$ is H or alkyl;

including pharmaceutically acceptable salts thereof;

The term "amino acid side chain" refers to any known alpha-amino acid, such as arginine, histidine, alanine, glycine, lysine, proline, leucine, valine, serine, threonine, allothreonine, homoserine, cyclohexylalanine, t-butylglycine, asparagine, glutamine, isoleucine, phenylalanine and the like.

Preferred are compounds of formula Iy wherein m' is 3, 4 or 5; n is 0, I or 2; p is 0 or I; $R^x$ is optionally substituted phenyl; $R^y$ is alkyl, hydroxyalkyl or aminocarbonylalkyl; $R^z$ is cycloalkyl, phenyl or H; and $R^v$ is alkoxycarbonyl or alkyl; and

Ax or **A'x** is

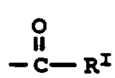

and $R^{I}$ is alkyl or arylalkyl, and the other is H,

or Ax or **A'x** is

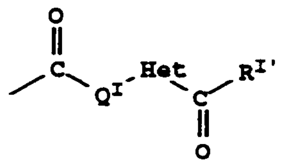

wherein $Q^{I}$ is alkyl or cycloalkyl,

Het is O and $R^{I'}$ is alkyl or arylalkyl, and the other is H.

Most preferred are compounds of formula Iy wherein m' is 3; n is l; and p is l;

$R^x$ is p-$NO_2C_6H_5$, -o-$FC_6H_5$, or $C_6H_5$;

$R^y$ is

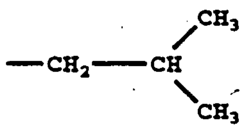 , 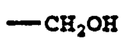 , 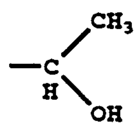 , 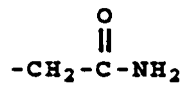 ;

$R^z$ is $C_6H_5$, or

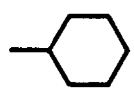 ;

$R^v$ is $HOCH_2$- or alkyl; Ax and **A'x** are as set out above.

The compounds of the invention also include prodrugs of disubstituted heterocyclic inhibitors having the Z substructure and include a guanidine, thioguanidine or amidine moiety, and have the structure

Iz 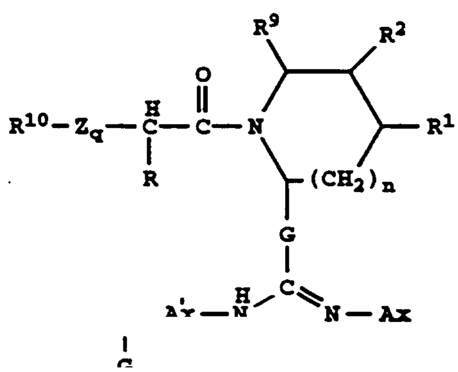

wherein

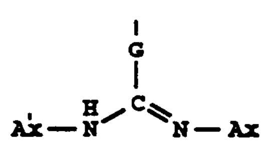

is an amido moiety which is

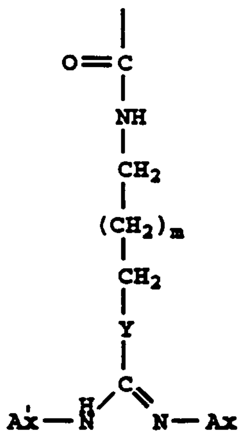

or

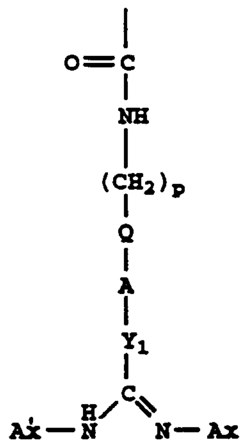

,

(G1) (G2)

including all stereoisomers thereof; and including all pharmaceutically acceptable salts thereof;
wherein R, $R^1$ and $R^2$ are as defined hereinbefore;

$R^9$ is lower alkyl, cycloalkyl, aryl, or arylalkyl; or $R^9$ and $R^2$ together with the carbons to which they are attached form a cycloalkyl, aryl or heteroaryl ring;

$R^{10}$ is hydrogen, lower alkyl, aryl, arylalkyl,

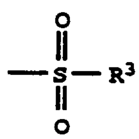

,

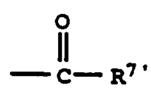

or $-CO_2R^{7'}$ wherein $R^{7'}$ is lower alkyl, aryl, arylalkyl, cycloheteroalkyl, heteroaryl, quinolinyl or tetrahydroquinolinyl; and $R^3$ is alkyl, arylalkyl, aryl, heteroaryl, quinolinyl, tetrahydroquinolinyl, l0-camphoryl, pentamethylchromanyl, pentaalkylphenyl, pentahalophenyl, trialkylphenyl, 3-carboxyphenyl, 3-trifluoromethylphenyl or 4-carboxyphenyl;

n is 0, l or 2;

m is 0, l, 2 or 3;

$Z_q$ is $NR^{11}$ or O (where $R^{11}$ is H, lower alkyl, aryl or arylalkyl);

Y is NH or S;

p is 0, l or 2;

Q is a single bond or

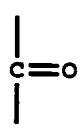

;

$Y_1$ is a bond or -NH-;

A is aryl or cycloalkyl, or an azacycloalkyl ring A of 4 to 8 ring members, or an azacycloalkenyl ring A of 5 to 9 ring members, or an azaheterocycloalkyl ring A of 6 to 8 ring members,

A

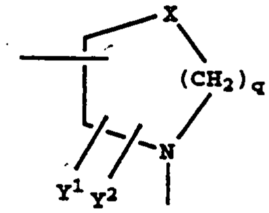

where X is $CH_2$, -CH=CH-, O, S or NH;

if A is an azacycloalkyl, azacycloalkenyl, or azaheterocycloalkyl ring A, then $Y_1$ is a bond and the acylamidine group

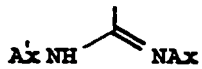

is attached to the nitrogen atom in the ring as indicated below

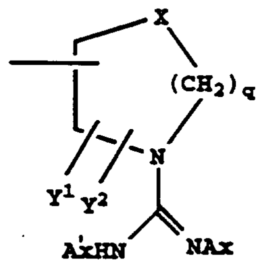

q is 0, I, 2, 3 or 4 if X is $CH_2$ or -CH=CH-;

q is 2, 3 or 4 if X is O, S or NH;

$Y^1$ and $Y^2$ are independently H, lower alkyl, oxo or halo;

provided that where X is a hetero atom (that is, A is azaheteroalkyl), then there must be at least a 2-carbon chain between X and any N atom in the ring A or outside ring A.

Preferred are compounds of formula Iz wherein

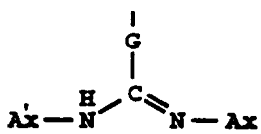 is

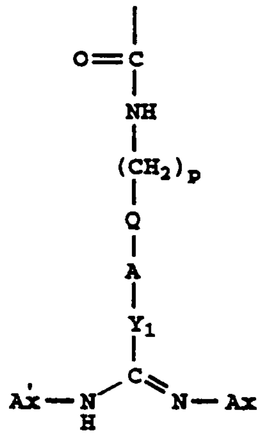

wherein R is H, p is I, Q is a single bond, A is an azacycloalkyl ring

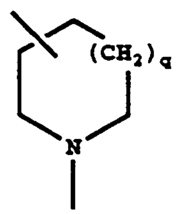

;

$Y_1$ is a bond;
where q is 0 or I; and

$R^9$ is arylalkyl such as benzyl, or alkyl;
$R^2$ and $R^1$ are independently H and/or alkyl;
$R^{10}$ is benzyloxycarbonyl, alkylsulfonyl, such as methylsulfonyl, or alkyl such as ethyl;
$Z_q$ is NH;
n is 0 or I; and
Ax or **A'x** is

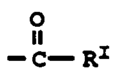

and $R^I$ is alkyl or arylalkyl, and the other is H,
or Ax or **A'x** is

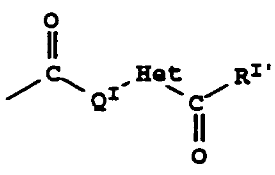

wherein $Q^I$ is alkyl or cycloalkyl,
Het is O and $R^{I'}$ is alkyl or arylalkyl, and the other is H.

Another preferred embodiment of the heterocyclic thrombin inhibitors of the invention of formula Iz wherein

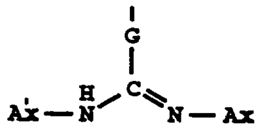 is

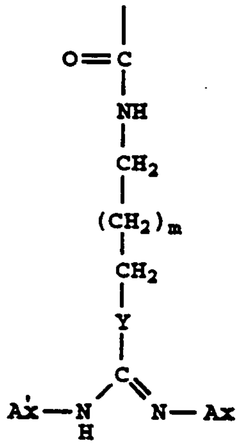

where $R^{10}$ is benzyloxycarbonyl, or alkylsulfonyl such as methyl sulfonyl, $Z_q$ is NH, R is H, $R^9$ is arylalkyl such as benzyl, $R^2$ and $R^1$ are independently H and/or alkyl, m is 2 and Y is NH; and

Ax or **A'x** is

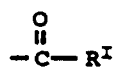

and $R^I$ is alkyl or arylalkyl, and the other is H,

or Ax or **A'x** is

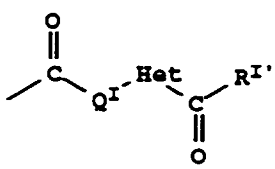

wherein $Q^I$ is alkyl or cycloalkyl,

Het is O and $R^{I'}$ is alkyl or arylalkyl, and the other is H.

It will be appreciated that the formula I structure includes all tautomers thereof, for example,

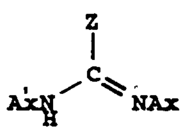 or 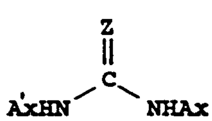 or 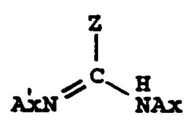

As seen above, examples of the Z thrombin inhibitor substructure include substructures Z(1) to Z(6) as set out below:

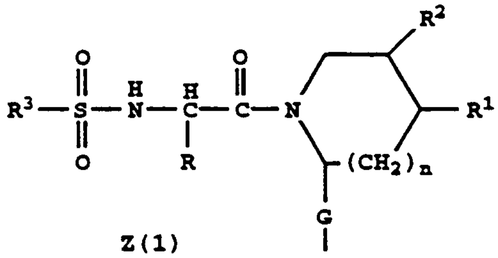

Z(1)

where G is

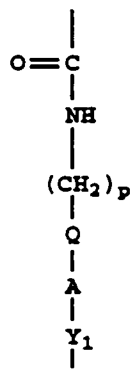

or

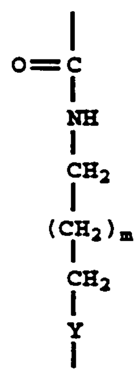

;

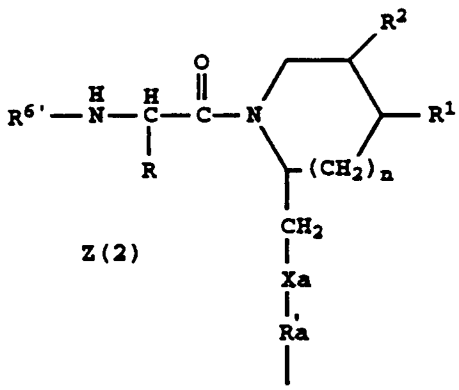

Z(2)

where **R'a** is

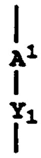 , 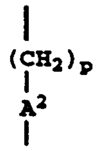 or 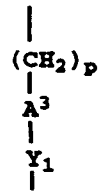 ;

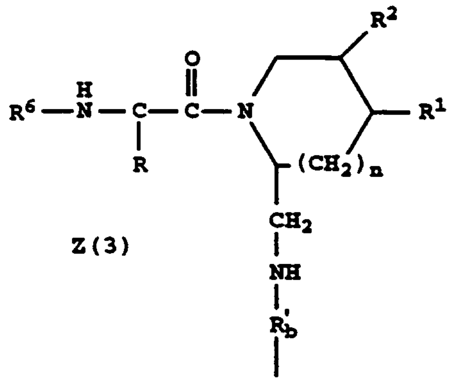

Z(3)

where $R_b'$ is

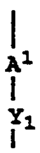 , 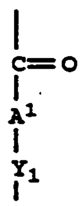 , 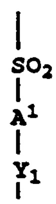 , 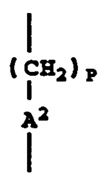 , 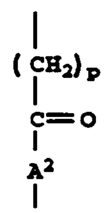 , 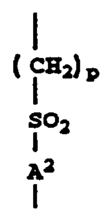 , 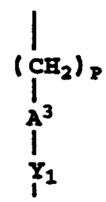 ,

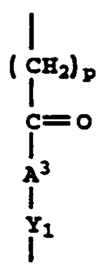 or 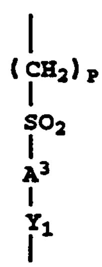 ;

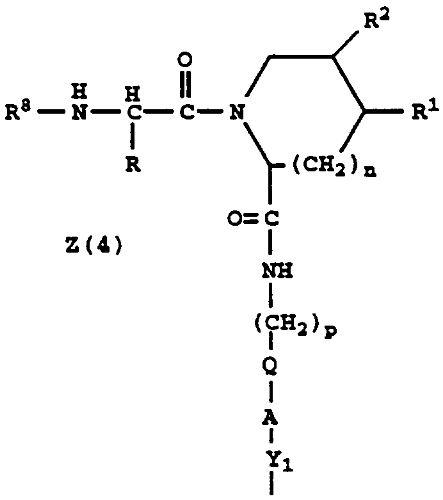

Z(4) ;

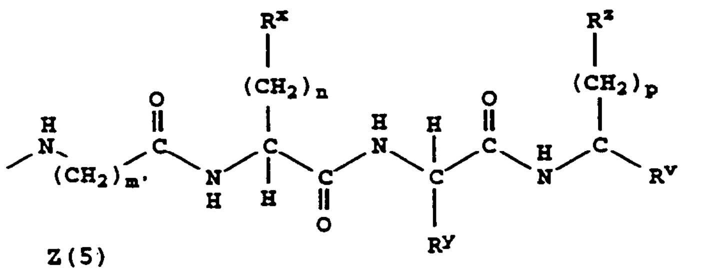

Z(5) ; and

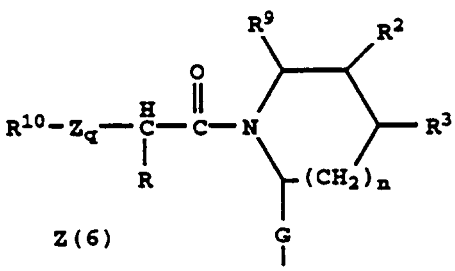

Z(6)

wherein G is an amido moiety which is

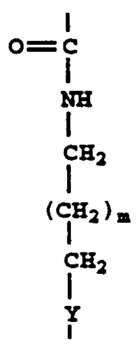

or

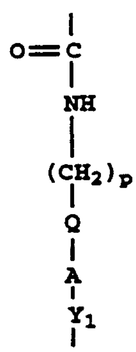

The thrombin inhibitor substructures Z(l) through Z(6)and methods for preparing same are disclosed in pending U.S. patent applications and published European applications as follows.

The Z(1) substructure is disclosed in U.S. application Serial No. l46,7l4, filed November l0, l993, and application Serial No. 373,334, filed January l7, 1995, and published European Application 060l459.

The Z(2) substructure is disclosed in U.S. application Serial No. 373,334, filed January 17, 1995, and published European application 0623595.

The Z(3) thrombin inhibitor substructure is disclosed in U.S. application Serial No. 373,334, filed January 17, 1995, and published European Application 0623596.

The Z(4) thrombin inhibitor substructure is disclosed in U.S. application Serial No. 373,334, filed January 17, 1995, and published European Application 0648780.

The Z(5) thrombin inhibitor substructure is disclosed in U.S. application Serial No. 396,320, filed February 28, 1995, which is incorporated herein by reference.

The Z(6) thrombin inhibitor substructure is disclosed in U.S. application Serial No. 215,433, filed March 21, 1994, which is incorporated herein by reference.

Compounds of the invention of formula I which include the moiety

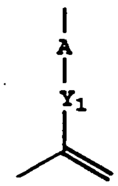

where $Y_1$ is NH or

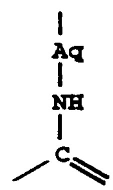

and Aq is any of the A, $A^1$ or $A^3$ moieties which are aryl or cycloalkyl, may be prepared employing the methods for preparing the Z substructures set out in the above-mentioned applications employing commercially available arylamines (such as aniline) and cycloalkylamines as starting materials.

In addition to the guanidine, thioguanidine or amidine-containing thrombin inhibitors (ZH) set out above employed to form the prodrugs of the invention of formula I, other guanidine, thioguanidine or amidine-containing compounds (ZH) which may be employed to form the prodrugs of formula I of the invention include, but are not limited to, the following:

$N^2$-arylsulfonyl-L-argininamides as disclosed in U.S. Patent Nos. 4,069,323; 4,066,758; 4,073,914; 4,055,651; 4,066,773; 4,117,127; 4,258,192; all to Okamoto et al;

ester derivatives of $N^{\alpha}$-(arylsulfonyl) L-arginine as disclosed in Okamoto et al, J. Med. Chem. 1980, 23, No. 8, 827-830;

amide derivatives of $N^{\alpha}$-substituted-L-arginine as disclosed in Kikumoto et al, J. Med. Chem. 1989, 23, No. 8, 830-836;

carboxyl-containing amide derivatives of $N^{\alpha}$-substituted L-arginine as disclosed in Kikumoto et al, J. Med. Chem. 1980, 23, No. 12, 1293-1299;

(2R,4R)-4-methyl-1-[$N^2$-[(3-methyl-1,2,3,4-tetrahydro-8-quinolinyl)sulfonyl]-L-arginyl)]-2-piperidinecarboxylic acid as disclosed in Kikumoto et al, Biochemistry 1984, 23, No. 1, 85-90;

N-arylsulphonyl-L-argininamide derivatives as disclosed in DE2655636-C2;

trisubstituted-2,3,4,5-tetrahydrobenzazepine derivatives as disclosed in Japanese Patent No. 2193971A;

derivatives of $N^{\alpha}$-substituted $N^{\alpha}$-arylsulfonylaminoacyl p-amidinophenylalaninamides as disclosed in published European Patent Application 0236163A1;

derivatives of $N^{\alpha}$-arylsulfonylaminoacyl p-amidino-phenylalaninamides as disclosed in European Patent Application 0236164A1;

glycopeptide derivatives as disclosed in published European Patent Application 558961A2;

amidinophenylalanine derivatives as disclosed in DE4115468A1 and published European Patent Application 508220A1;

2-[3-(4-amidinophenyl)]-propanoic acid derivatives as disclosed in DE4121947A1;

L- and D-phenylalanine derivatives as disclosed in WO92/08709;

piperazides of substituted phenyl derivatives as disclosed in WO94/18185-A1;

para-substituted phenylalanine derivatives as disclosed in WO92/16549;

cyclotheonamides A and B as disclosed in Maryanoff et al, Proc. Nat'l Acad. Sci. USA, Vol. 90, 8048-8052, September 1993, Biochemistry, and Maryanoff et al, J. Am. Chem. Soc. Vol. 117, No. 4, 1995, 1225-1239;

derivatives of the dipeptide of L-azetidine-2-carboxylic acid and L-arginine aldehyde as disclosed in U.S. Patent No. 5,252,566 to Shuman;

derivatives of the dipeptide L-proline-L-arginine aldehyde as disclosed in published European Patent Application 0479489A2;

polyfluorinated alkyl derivatives of tripeptides as disclosed in published European Patent Application 0504064A1;

peptide aldehydes as disclosed in WO94/17817 and WO93/15756;

guanidine derivatives as disclosed in WO94/08941 and guanidine derivatives as disclosed in published European Patent Application 84/118280;

guanidine derivatives as disclosed in published European Patent Application 0530167A1;

peptide aldehydes as disclosed in published European Patent Application 93/526877 and Balasubramanian et al, J. Med. Chem. (1993), 36, 300-303;

tripeptides as disclosed in published European Patent Application 0479489A2 or European Patent Application 0643073A1;

arginine aldehydes as disclosed in published European Patent Application 0526877A2;

guanidine derivatives as disclosed in WO93/18060A1;

tripeptides such as Boc-D-Phe-Pro-Arg-H, as disclosed in Shuman et al, J. Med. Chem., 1993, 36, 314-319;

tripeptides such as D-Phe-Pro-Arg-H as disclosed in Bajusz et al, J. Med. Chem., 1990, 33, 1729-1735;

agmatine derivatives as disclosed in U.S. Patent No. 4,346,078 to Bajusz et al;

peptidyl-arginine aldehyde derivatives as disclosed in U.S. Patent No. 4,316,889 to Bajusz et al;

peptide aldehydes as disclosed in U.S. Patent No. 4,703,036 to Bajusz et al;

guanidine derivatives as disclosed in WO 94/29335;

guanidine derivatives as disclosed in WO 93/11152A1;

isosteric peptides as disclosed in published European Patent Application 0530167A1;

peptide derivatives as disclosed in WO93/18060;

guanidine derivatives as disclosed in WO94/29336;

azetidinone derivatives as disclosed in U.S. Patent No. 5,326,863 to Han;

azetidin-2-one derivatives as disclosed in U.S. Patent No. 5,175,283 to Han;

arginine α-keto-amide derivatives as disclosed in WO94/08941A1;

N-aminopiperidinyl or N-amidino-1,4-oxazinyl substituted sulphonamide derivatives as disclosed in published European Patent Application 559046A1;

guanidine derivatives as disclosed in U.S. Patent Nos. 5,260,307, and 5,393,760 both to Ackermann et al;

guanidine derivatives as disclosed in published European Patent Application 0468231A2;

1-amidino piperidine and 4-amidino morpholine derivatives as disclosed in published European Patent Application 0641779A1;

fibrinogen and/or GPIIb/IIIa receptor antagonists and/or antiaggregants including, but not limited to, tetrapeptides as disclosed in Alig et al, J. Med. Chem. 1992, 35, 4393-4407, GR144053 and published European Application 542363;

substituted benzodiazepinediones as disclosed in WO93/08174 and WO94/14776;

BIBU-52 as disclosed in published European Application 93/567967;

SC-47643 as disclosed in U.S. Patent No. 4,879,313;

SDZ GPI 562 as disclosed in published European Patent Application 93/560730;

RO43-5054 as disclosed in published European Patent Application 91/445796A2;

RO44-9883 (LAMIFIBAN) as disclosed in published European Patent Application 92/505868 and U.S. Patent No. 5,378,712;

SKF107260 as disclosed in published European Patent Application 91/425212;

aromatic azacyclic compounds as disclosed in WO94/333051;

DMP-728 (Arg-Gly-Asp-Ser), disclosed in FASEB J. 1992, 6(4), Abs. 3827 and J. Org. Chem. (1995) 60, 946-952;

cyclic peptide derivatives as disclosed in WO94/26779;

SC-52012 and SC-57101 as disclosed in Nicholson et al, 67th Scientific Sessions of Amer. Heart Assn., Nov. 14-17, 1994, Poster, 0975 and J. Med. Chem. (1993) 36, 1811-1819;

SC54684 and SC54701 as disclosed in Drugs Fut., 1994, 19(5) 467 and WO93/07867, U.S. Patent No. 5,344,957 and U.S. Patent No. 5,239,113;

SC58053 and SC58052 disclosed in 1st Winter Confr. on Med. and Bioorg. Chem., Steamboat Springs, Co., 29 Jan-2 Feb. 1995, Poster I (Searle) and WO94/333038;

RO43-8857 as disclosed in U.S. Patent No. 5,084,466 and U.S. Patent No. 5,256,812;

GRI44053 as disclosed in published European Patent Application 542363A2 and in Thrombosis and Haemostasis (I993) <u>69</u>, Abstracts 64, I884, I885, I886;

antihypertensives and antidepressants related to guanethidine (as disclosed in U.S. Patent No. 2,928,829) and related to guanoxyfen (as disclosed in BE6I2362);

antibiotics and viricides related to

amidinomycin (as disclosed in JP 2I,4I8);

stallimycin (as disclosed in DE I,039,I98);

Arphamenine B (as disclosed in published European Patent Application 85/I33550A2);

chitinovorin-A (as disclosed in published European Patent Application 85/I50,378A2 and U.S. Patent No., 4,723,004);

streptomycin (as disclosed in U.S. Patent No. 2,868,779);

SB-59 (as disclosed in Justus Liebigs, Ann. Chem. (I973) 7, III2-II40);

TAN-I057-A (as disclosed in U.S. Patent No. 4,97I,965);

streptoniazid (as disclosed in J. Am. Chem. Soc. (I953) 75, 226I);

immunostimulants related to ST-789 (as disclosed in published European Patent Application 88/260588);

peptide hydrolase inhibitors related to nafamastat (as disclosed in U.S. Patent No. 4,454,338);

gabexate (as disclosed in U.S. Patent No. 3,75I,447);

sepimostat (as disclosed in U.S. Patent Nos. 4,777,I82 and 4,820,730);

Factor Xa inhibitors related to

DX-9065a (as disclosed in published European Patent Application 92/054005I);

anti-inflammatory agents related to paranyline as disclosed in U.S. Patent No. 2,877,269;

peptidyl aldehydes (as disclosed in WO94/I3693);

antianaphylactics related to GMCHA-TBP (Batebulast) (as disclosed in U.S. Patent No. 4,465,85I);

anti-ulcer agents related to

benexate (as disclosed in U.S. Patent No. 4,348,4I0);

deoxyspergualin (as disclosed in U.S. Patent Nos. 4,5I8,532, 4,658,058 and 4,983,328); and arginine.

The compounds of formula I of the invention may be prepared as shown in the following reaction schemes and as described below.

It will be appreciated that the term "guanidine prodrugs" as employed in conjunction with Reaction Scheme II encompasses guanidine prodrugs and thioguanidine prodrugs.

<u>Reaction Scheme I</u>

Reaction Scheme I depicts the synthesis of monoacyl **(Ia)** or bisacyl **(Ib)** guanidine prodrugs from an amino-containing substructure ZH wherein Z may be any of the Z substructures Z(I), Z(2), Z(3), Z(4), Z(5) and Z(6) as defined herein or any of the Z substructures set out in the various patent and literature references set out hereinbefore.

In the Scheme below, $-COR^{I}$ is used as the acyl precursor to Ax and A'x for illustrative purposes. In these equations, $-COR^{I}$ may be replaced by $-COQ^{I}HetCOR^{I'}$, $-COQ^{I}OCOHetR^{I'}$ or $-COQ^{I}COHetR^{I}$ as defined herein.

## Reaction Scheme I

**ZH is an amino-containing (e.g. thrombin inhibitor) or substructure as described hereinbefore**
**Z-C=NH($NH_2$) is a guanidine-containing thrombin inhibitor or other pharmaceutical**

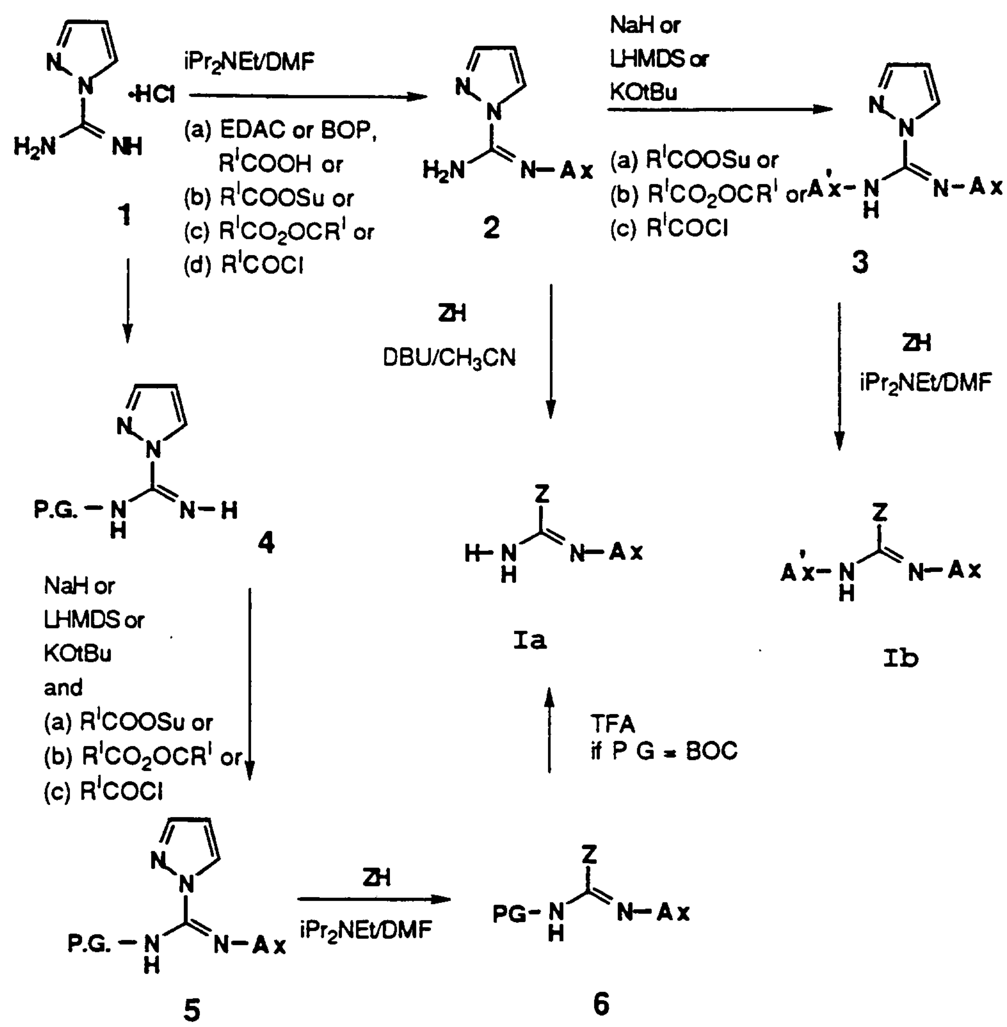


($R^I = R^I$)

$R^I$ as employed above and hereinafter may be any of the $R^I$ groups as defined herein.

Referring to Reaction Scheme I, monoacyl compounds of formula I may be prepared as follows.

1H-pyrazole-1-carboxamidine hydrochloride **1** is allowed to react with (a) an acid, such as $R^I$COOH and a carbodiimide such as ethyl dimethylaminopropyl carbodiimide (EDAC), diisopropylcarbodiimide (DIC) or BOP reagent [benzothiazol-l-yloxy-tris(dimethylamino)phosphonium hexafluorophosphate], or with (b) a succinimide ester, or with an (c) acid anhydride, or with an (d) acid chloride in the presence of a base, preferably diisopropylethylamine or N,N-dimethylaminopyridine in a solvent such as DMF or dichloromethane, to provide an N - monoacyl pyrazole carboxamidine **2**. Ax is an acyl group as defined previously, that is, Ax has the structure -COR$^I$, or -COQ$^I$HetCOR$^I$, -COQ$^I$OCOHetR$^{I'}$ or -COQ$^I$COHetR$^I$, depending on the reactant employed.

Monoacyl guanidine prodrugs **Ia** of the invention are prepared by reacting an amino containing (e.g. thrombin inhibitor) substructure **ZH** with **2** in a solvent such as acetonitrile or THF and in the presence of a base such as DBU or diisopropylethylamine. **ZH** is a previously defined substructure, such as a thrombin inhibitor substructure, more specifically, a substructure which contains a secondary or primary amine.

Alternatively, **Ia** can be prepared by protecting the pyrazole carboxamidine **1** with a protecting group (PG) such as BOC or CBZ to give the protected carboxamidine **4**, which is then acylated by reacting **4** with a base such as sodium hydride, or lithium hexamethyldisilazide, or potassium t-butoxide, preferably sodium hydride, in a solvent such as THF or DMF with an activated acid equivalent such as (a) a succinimide ester, or (b) an acid anhydride or (c) an acid chloride, to give compound **5**. Compound **5** may be reacted with **ZH**, where **ZH** is an amino-containing (e.g. thrombin inhibitor) substructure in the presence of a base, preferably diisopropylethylamine as well as N-methylmorpholine, triethylamine or DBU and an inert organic solvent, preferably DMF as well as dichloromethane, acetonitrile or tetrahydrofuran, to give **6**. Intermediate **6** may be deprotected using an appropriate reagent, e.g., TFA if PG = BOC , to give monoacyl compounds of formula **Ia**.

Bisacyl compounds of formula Ib where Ax is acyl and **A'x** is acyl are prepared as follows. The N,N'-bisacyl pyrazole carboxamidine **3** is prepared by reacting monoacyl carboxamidine **2** with a base such as sodium hydride, or lithium hexamethyldisilazide, or potassium t-butoxide, preferably NaH, in a solvent such as THF or DMF with an activated acid equivalent such as (a) a succinimide ester, or (b) an acid anhydride, or (c) an acid chloride. The bisacyl guanidine prodrugs **Ib** are prepared by reacting an amino containing (e.g. thrombin inhibitor) substructure **ZH** with **3** in a solvent such as DMF and in the presence of a base such as $iPr_2NEt$.

In carrying out the reactions set out in Reaction Scheme I, the (a) acid, (b) succinimide ester, (c) acid anhydride or (d) acid chloride is employed in a molar ratio to the IH-pyrazole-I-carboxamidine hydrochloride **1** within the range from about I0:I to about I:I, preferably from about 3:I to about I:I, and the carbodiimide (for example EDAC, DIC, DCC, or BOP reagent, with EDAC and DIC being preferred) is employed in a molar ratio to the (a) acid within the range from about 2:I to about I:I, preferably from about I.5:I to about I:I.

The reaction to form the monoacyl carboxamidine **2** is carried out at a temperature within the range from about -40 to about I00°C, preferably from about -20 to about 50°C, in the presence of a base, preferably diisopropylethylamine, as well as triethylamine, N-methylmorpholine or DBU, in an inert organic solvent such as chloroform, acetonitrile and tetrahydrofuran, in place of DMF (dimethylformamide) or dichloromethane (which are preferred).

The N-monoacyl pyrazole carboxamide **2** is reacted with substructure **ZH** employing a molar ratio of **2:ZH** within the range from about 5:I to about I:2, preferably from about 2:I to about I:I, in the presence of an inert organic solvent preferably acetonitrile, dichloromethane, dimethylformamide or tetrahydrofuran, and a base preferably DBU (I,8-diazabicyclo[5.4.0]undec-7-ene) as well as diisopropylethylamine, at a temperature within the range from about -40 to about I00°C, preferably from about -20 to about 50°C.

In the alternative method for preparing monoacyl **Ia**, the succinimide ester (a), acid anhydride (b) or acid chloride (c) will be employed in a molar ratio to the protected pyrazole carboxamide **4** within the range from about 3:I to about I:I, preferably from about 2:I to about I:I, and the reaction thereof will be carried out at a temperature within the range from about -40 to about 60°C, preferably from about -20 to about 30°C.

The protected monoacyl compound **5** will be employed in a molar ratio to ZH within the range from about 3:I to about I:2, preferably from about I.5:I to about I:I, and the reaction is carried out at a temperature within the range from about -40 to about 60°C, preferably from about -20 to about 40°C.

Compound **6** is deprotected using methods known in the art to give monoacyl compounds of formula **Ia**.

In preparing the bisacyl compounds of the invention **Ib**, the succinimide ester (a), acid anhydride (b) or acid chloride (c) is employed in a molar ratio to monoacyl carboxamidine **2** within the range from about 4:I to about I:I, preferably from about 2:I to about I:I, and the reaction is carried out at a temperature within the range from about -40 to about 50°C, preferably from about -I0 to about 30°C, to form the N,N'-bisacyl pyrazole carboxamidine **3**.

The N,N'-bisacyl pyrazole carboxamidine **3** is employed in a molar ratio to ZH within the range from about 4:I to about I:2, preferably from about 2:I to about I:I, and the reaction is carried out at a temperature within the range from about -20 to about 50°C, preferably from about 0 to about 30°C, in the presence of an inert organic solvent, preferably DMF as well as acetonitrile, dichloromethane or tetrahydrofuran, and a base preferably diisopropylethylamine as well as N-methylmorpholine, triethylamine or DBU.

<u>Reaction Scheme II</u>

**Synthesis of monoacyl (Ia) or bisacyl (Ib) guanidine prodrugs from guanidine- or thioguanidine-containing thrombin inhibitors or other pharmaceuticals**

In the scheme below, $-COR^I$ is used as the acyl precursor to Ax and A'x for illustrative purposes. In the equations, $-COR^I$ may be replaced by $-COQ^IHetCOR^{I'}$, $-COQ^IOCOHetR^{I'}$ or $-COQ^ICOHetR^I$ as defined herein.

**$Z\text{-}C{=}NH(NH_2)$ = guanidine- or thioguanidine-containing thrombin inhibitor or other pharmaceutical where Z may be any of the Z substructures Z(1), Z(2), Z(3), Z(4), Z(5) or Z(6) or other pharmaceutical substructure as defined herein**

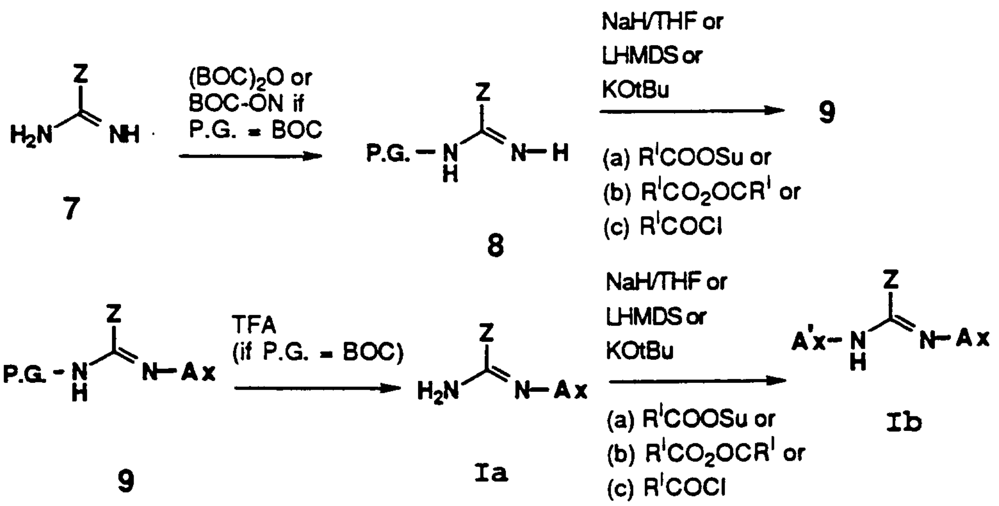


$(R^I = R^I)$

Referring to Reaction Scheme II, monoacyl compounds of formula **Ia** of the invention may be prepared as follows.

The guanidine- or thioguanidine-containing compound (e.g. thrombin inhibitor) **7** can be protected with a protecting group such as t-butoxycarbonyl (BOC) or benzyloxycarbonyl (CBZ), to give the protected guanidine **8**, and then acylated by allowing **8** to react with a base such as sodium hydride, or lithium hexamethyldisilazide, or potassium t-butoxide, in a solvent such as THF with activated acid equivalent such as a succinimide ester, or an acid anhydride, or an acid chloride, to give compound **9**. Compound **9** may be deprotected using an appropriate reagent, e.g., TFA if P.G. is BOC. Ax is an acyl group as defined previously, that is, Ax has the structure $-COR^I$, or $-COQ^IHetCOR^I$, $-COQ^IOCO$-Het'$R^{I'}$ or $-COQ^ICOHetR^I$.

The N,N'-bisacyl prodrug **Ib** is prepared by acylating monoacyl compound **Ia** as in the preparation of protected monoacyl guanidine **9**.

In carrying out the reactions set out in Reaction Scheme II, the protecting group (preferably BOC) is added by reacting compound **7** with a reagent such as BOC-ON or $(BOC)_2O$ in an inert organic solvent such as THF or DMF at a temperature within the range from about 0 to about l00°C.

The succinimide ester (a), acid anhydride (b) or acid chloride (c) is employed in a molar ratio to the protected guanidine- or thioguanidine-containing compound **8** within the range from about 4:l to about l:l, preferably from about 2:l to about l:l, and the reaction thereof will be carried out at a temperature within the range from about -40 to about 60°C, preferably from about -20 to about 30°C.

In preparing the bisacyl compounds of the invention **Ib**, the succinimide ester (a), acid anhydride (b) or acid chloride (c) is employed in a molar ratio to monoacyl guanidine **Ia** within the range from about 3:l to about l:l, preferably from about 2:l to about l:l, and the reaction is carried out at a temperature within the range from about -40 to about 60°C, preferably from about -20 to about 30°C.

<u>Reaction Scheme III</u>

**Synthesis of monoacyl (Ia) or bisacyl (Ib) amidine prodrugs from amidine-containing compounds, such as thrombin inhibitors**

In the scheme below, $-COR^I$ is used as the acyl precursor to Ax and A'x for illustrative purposes. In the equations, $-COR^I$ may be replaced by $-COQ^IHetCOR^{I'}$, $COQ^1OCOHetR^{I'}$ or $-COQ^ICOHetR^I$ as defined in the specifications.

**Z-C=NH($NH_2$) is an amidine-containing thrombin inhibitor or other pharmaceutical where Z may be any of the Z substructures (Z(1), Z(2), Z(3), Z(4), Z(5) or Z(6) or other pharmaceutical substructure as defined herein.**

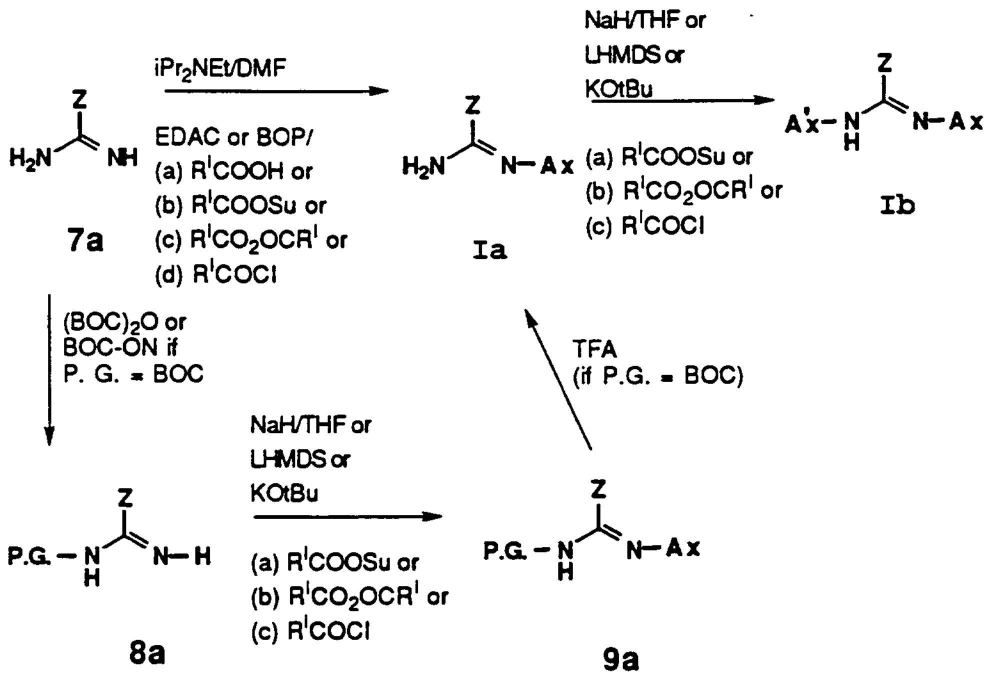


($R^I = R^I$)

Referring to Reaction Scheme III, monoacyl compounds of formula **Ia** of the invention may be prepared as follows.

The amidine-containing thrombin inhibitor **7a** is reacted with (a) an acid, such as $R^I$COOH and a carbodiimide preferably EDAC, DIC or BOP reagent, or with (b) a succinimide ester, or with (c) an acid anhydride, or with (d) an acid chloride in the presence of a base preferably diisopropylethylamine, in a solvent such as DMF or dichloromethane, to provide an N-monoacyl prodrug of formula **Ia**.

Alternatively, **Ia** can be prepared by protecting the amidine **7a** with a protecting group such as BOC or CBZ (as described in Reaction Scheme II) to give the protected amidine **8a**, and then acylating by allowing the protected amidine compound **8a** to react with a base such as sodium hydride, or lithium hexamethyldisilazide, or potassium t-butoxide in a solvent such as THF with activated acid equivalent such as a succinimide ester, or an acid anhydride, or an acid chloride to give compound **9a**. Compound **9a** may be deprotected using an appropriate reagent, e.g., TFA if P.G. is BOC. Ax is an acyl group as defined previously, that is, Ax has the structure -COR$^I$, or -COQ$^I$HetCOR$^{I'}$, -COQ$^I$OCOHetR$^{I'}$ or -COQ$^I$COHetR$^I$.

The N,N'-bisacyl prodrug **Ib** is prepared by acylating monoacyl compound **Ia** as in the preparation of monoacyl amidine **9a**.

In carrying out the reactions set out in Reaction Scheme III, the (a) acid, (b) succinimide ester, (c) acid anhydride or (d) acid chloride is employed in a molar ratio to the amidine-containing compound (such as thrombin inhibitor) **7a** within the range from about 3:l to about l:l, preferably from about 2:l to-about l:l, and the carbodiimide (for example EDAC, DIC, DCC or BOP reagent, with EDAC and BOP reagent being preferred) is employed in a molar ratio to the (a) acid within the range from about 2:l to about l:l, preferably from about l.5:l to about l:l.

The reaction to form monoacyl amidine **Ia** is carried out at a temperature within the range from about -20 to about 50°C, preferably from about 0 to about 30°C, in the presence of a base, preferably diisopropylethylamine, as well as N-methylmorpholine, potassium carbonate or DBU in an inert organic solvent such as acetonitrile, chloroform and tetrahydrofuran in addition to DMF (dimethylformamide) or dichloromethane (which are preferred).

In the preparation of the protected amidine **9a**, the succinimide ester (a), acid anhydride (b) or acid chloride (c) is employed in a molar ratio to the protected amidine-containing compound (such as thrombin inhibitor) **8a** within the range from about 4:l to about l:l, preferably from about 2:l to about l:l, and the reaction thereof will be carried out at a temperature within the range from about -40 to about 60°C, preferably from about -20 to about 30°C.

In preparing the bisacyl compounds of the invention **Id**, the succinimide ester (a), acid anhydride (b) or acid chloride (c) is employed in a molar ratio to monoacyl amidine **Ia** within the range from about 3:l to about l:l, preferably from about 2:l to about l:l, and the reaction is carried out at a temperature within the range from about -40 to about 60°C, preferably from about -l0 to about 30°C.

The starting materials ZH and

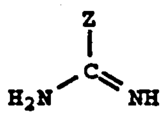

may be prepared as described in the aforementioned U.S. applications and published European and WO applications as well as in any of the aforementioned patent and/or literature references.

In General

The term "prodrug(s)" as used herein refers to a class of drugs the pharmacologic action of which results from conversion by processes within the body (biotransformation).

The phrase "pharmaceutically active properties" as employed herein in describing the compounds of the invention is used interchangeably with the phrase "pharmacologically active properties".

The term "lower alkyl" or "alkyl" as employed herein by itself or as part of another group includes both straight and branched chain radicals of up to l8 carbons, preferably l to 8 carbons, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, the various branched chain isomers thereof, and the like as well as such groups including l, 2 or 3 halo substituents (for example, to form $CF_3$ or $CF_3CH_2$) and/or l or 2 of the following substituents: an aryl substituent (for example, to form benzyl or phenethyl), a heteroaryl substituent, an alkyl-aryl substituent, a haloaryl substituent, a cycloalkyl substituent, an alkylcycloalkyl substituent, an alkenyl substituent, an alkynyl substituent, hydroxy or a carboxy substituent. It will be appreciated that the same "alkyl" group may be substituted with one or more of any of the above substituents.

The term "cycloalkyl" by itself or as part of another group includes saturated cyclic hydrocarbon groups containing 3 to l2 carbons, preferably 3 to 8 carbons, which include cyclopropyl, cyclobutyl, cyclopentyl, cyclohexyl, cycloheptyl, cyclooctyl, cyclodecyl and cyclododecyl, any of which groups may be substituted with substituents such as halogen, lower alkyl, alkoxy, and/or hydroxy groups.

The term "aryl" or "Ar" as employed herein by itself or as part of another group refers to monocyclic or bicyclic aromatic groups containing from 6 to l0 carbons in the ring portion, such as phenyl, or naphthyl. Aryl (or Ar), phenyl or naphthyl may include substituted aryl, substituted phenyl or substituted naphthyl, which may include l, 2, 3, 4 or 5 substituents on either the Ar, phenyl or naphthyl such as lower alkyl, cyano, amino, alkylamino, dialkylamino, nitro, carboxy, alkoxycarbonyl, trifluoromethyl, halogen (Cl, Br, I or F), lower alkoxy, aryl-alkoxy, hydroxy, alkylthio, alkylsulfinyl, alkylsulfonyl, arylthio, arylsulfinyl and/or arylsulfonyl.

In a separate embodiment of the invention, where $R^3$ in formula I is phenyl, the phenyl group may include 3, 4 or 5 substituents such as alkyl, for example, pentamethyl and 2,4,6-tri-isopropyl, and halo, for example, pentafluoro.

The term "aralkyl", "aryl-alkyl" or "aryl-lower alkyl" as used herein by itself or as part of another group refers to lower alkyl groups as discussed above having an aryl substituent, such as benzyl.

The term "lower alkoxy", "alkoxy" or aralkoxy" includes any of the above lower alkyl, alkyl or aralkyl groups linked to an oxygen atom.

The term "halogen" or "halo" as used herein by itself or as part of another group refers to chlorine, bromine, fluorine or iodine with chlorine being preferred.

The term "lower alkenyl" or "alkenyl" as employed herein by itself or as part of another group includes a carbon chain of up to l6 carbons, preferably 3 to l0 carbons, containing one double bond which will be separated from "N" by at least one saturated carbon moiety such as $-(CH_2)_q-$ where q can be l to l4, such as 2-propenyl, 2-butenyl, 3-butenyl, 2-pentenyl, 4-pentenyl and the like, and may include a halogen substituent such as I, Cl, or F.

The term "lower alkynyl" or "alkynyl" as employed herein by itself or as part of another group includes a carbon chain of up to l6 carbons, preferably 3 to l0 carbons, containing one triple bond which will be separated from "N" by at least one saturated carbon moiety such as $-(CH_2)_{q'}-$ where q' can be l to l4, such as 2-propynyl, 2-butynyl, 3-butynyl and the like.

The term "heteroaryl" or heteroaromatic by itself or as part of another group refers to a 5- to l0-membered monocyclic or bicyclic aromatic ring which includes l, 2, 3 or 4 hetero atoms such as nitrogen, oxygen or sulfur, such as

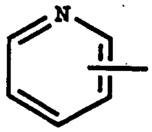 , 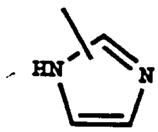 , 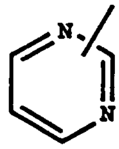 , 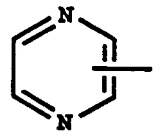 ,

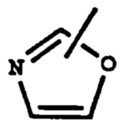 , 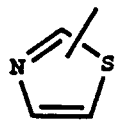 , 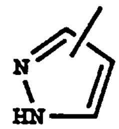 , 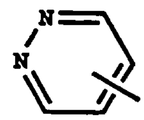 ,

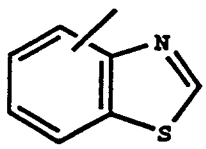 , 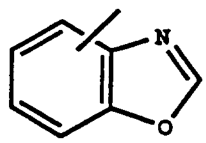 , 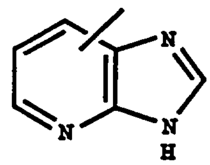 ,

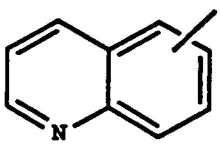 , 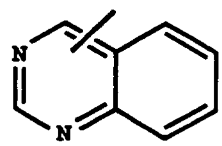 , 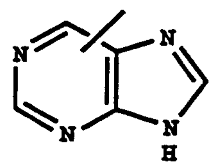

and the like. The heteroaryl rings may optionally be fused to aryl rings defined previously. The heteroaryl rings may optionally include I or 2 substituents such as halogen (Cl, Br, F or $CF_3$), lower alkyl, lower alkoxy, carboxy, amino, lower alkylamino and/or dilower alkylamino.

The term "cycloheteroalkyl" or "heterocycloalkyl" as used herein refers to a 5-, 6-or 7-membered saturated ring which includes I or 2 hetero atoms such as nitrogen, oxygen and/or sulfur, which may optionally include I to 4 substituents such as halo, alkyl or oxo, such as

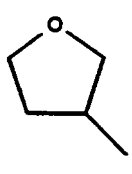 , 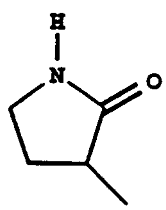 , 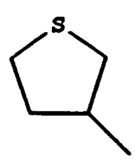 , 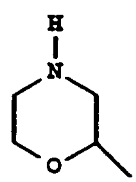 ,

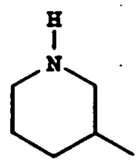 , 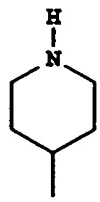 , 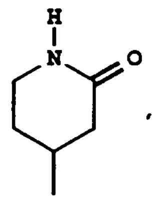 , 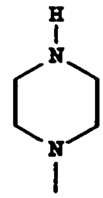 ,

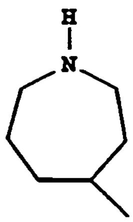 , 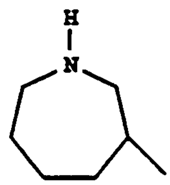 , 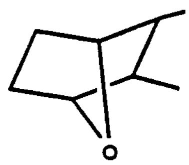 , 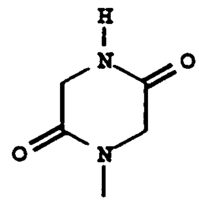 ,

and the like.

The term "azacycloalkenyl" as used herein refers to a 4- to 8-membered ring which includes a double bond, such as

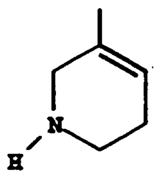 , 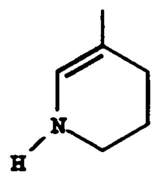 , 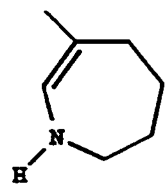 ,

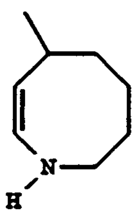 , 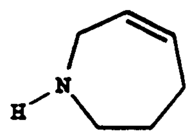

The term "amino acid side chain" refers to any of the known alpha-amino acids such as arginine, histidine, alanine, glycine, lysine, glutamine, cyclohexylalanine, t-butylglycine, leucine, valine, serine, homoserine, allothreonine, naphthylalanine, isoleucine, phenylalanine and the like.

The compounds of formulae I, I., Ia, A., Ix, Iq, Iy and Iz of the invention can be obtained as pharmaceutically acceptable acid addition salts by reacting a free base with an acid, such as hydrochloric, hydrobromic, hydroiodic, nitric, sulfuric, phosphoric, acetic, fumaric, citric, maleic, succinic, lactic, tartaric, gluconic, benzoic, methanesulfonic, ethanesulfonic, benzenesulfonic, p-toluenesulfonic acid or the like.

The prodrug compounds of formula I of the invention will have the same utility as the Z substructure employed therein linked to

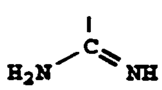 .

Thus, if the Z substructure linked to

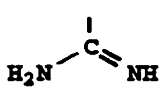

is a thrombin inhibitor, the prodrug of formula I will be useful as a thrombin inhibitor in dosages and dosage forms as described in the references set out above; if the Z substructure linked to

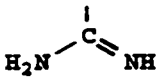

is an inhibitor of platelet aggregation, the prodrug of formula I will be useful as an inhibitor of platelet aggregation in dosages and dosage forms as described in the references set out above (and so on).

Depending on the Z substructure, the compounds of the present invention may be serine protease inhibitors, and in particular may inhibit thrombin, Factor Xa, and/or trypsin. Such compounds of the present invention are useful for the treatment or prophylaxis of those processes which involve the production and/or action of thrombin. This includes a number of thrombotic and prothrombotic states in which the coagulation cascade is activated which include, but are not limited to, deep vein thrombosis (DVT), disseminated intravascular coagulopathy (DIC), Kasabach-Merritt syndrome, pulmonary embolism, myocardial infarction, stroke, thromboembolic complications of surgery (such as hip replacement and endarterectomy) and peripheral arterial occlusion. In addition to its effects on the coagulation process, thrombin has been shown to activate a large number of cells (such as neutrophils, fibroblasts, endothelial cells, smooth muscle cells). Therefore, the compounds of the present invention may also be useful for the treatment or prophylaxis of adult respiratory distress syndrome, septic shock, septicemia, inflammatory responses which include, but are not limited to, edema, acute or chronic atherosclerosis, and reperfusion damage.

The compounds of the invention (as serine protease inhibitors) may also be useful in treating neoplasia/metastasis (in particular those which utilize fibrin) and neurodegenerative diseases such as Alzheimer's disease and Parkinson's disease. In addition, the compounds of the present invention may be useful to prevent restenosis following arterial injury induced by endogenous (rupture of an atherosclerotic plaque) or exogenous (invasive cardiological procedure) events.

The compounds of the present invention as thrombin inhibitors, inhibitors of platelet aggregation and/or fibrinogen receptor antagonists may also be used as an anticoagulant in extracorpeal blood circuits, such as those necessary in dialysis and surgery (such as coronary artery bypass surgery).

The compounds of the present invention as thrombin inhibitors, platelet aggregation inhibitors or fibrinogen receptor antagonists may also be used in combination with thrombolytic agents, such as tissue plasminogen activator (natural or recombinant), streptokinse, urokinase, prourokinase, anisolated streptokinase plasminogen activator complex (ASPAC), animal salivary gland plasminogen activators, and the like. The compounds of the present invention as thrombin inhibitors, platelet aggregation inhibitors or fibrinogen receptor antagonists may act in a synergistic fashion to prevent reocclusion following a successful thrombolytic therapy and/or reduce the time to reperfusion. The compounds of the present invention as thrombin inhibitors, platelet aggregation inhibitors or fibrinogen receptor antagonists may also allow for reduced doses of the thrombolytic agent to be used and therefore minimize potential hemorrhagic side-effects.

The compounds of the present invention as thrombin inhibitors, platelet aggregation inhibitors or fibrinogen receptor antagonists may also be used in combination with other antithrombotic or anticoagulant drugs such as thromboxane receptor antagonists, prostacyclin mimetics, phosphodiesterase inhibitors, fibrinogen antagonists, aspirin and the like.

Compounds of the present invention that inhibit trypsin may also be useful for the treatment of pancreatitis.

The compounds of the invention can be administered orally or parenterally such subcutaneously or intravenously, as well as by nasal application, rectally or sublingually to various mammalian species known to be subject to such maladies, e.g., humans, cats, dogs and the like in an effective amount within the dosage range of about 0.l to about l00 mg/kg, preferably about 0.2 to about 50 mg/kg and more preferably about 0.5 to about 25 mg/kg (or from about l to about 2500 mg, preferably from about 5 to about 2000 mg) on a regimen in single or 2 to 4 divided daily doses.

The active substance can be utilized in a composition such as tablet, capsule, solution or suspension or in other type carrier materials such as transdermal devices, iontophoretic devices, rectal suppositories, inhalant devices and the like. The composition or carrier will contain about 5 to about 500 mg per unit of dosage of a compound or mixture of compounds of formula I, I., Ia, Ix, Iq, Iy and Iz. They may be compounded in conventional matter with a physiologically acceptable vehicle or carrier, excipient, binder, preservative, stabilizer, flavor, etc., as called for by accepted pharmaceutical practice.

The following working Examples represent preferred embodiments of the present invention.

Example I

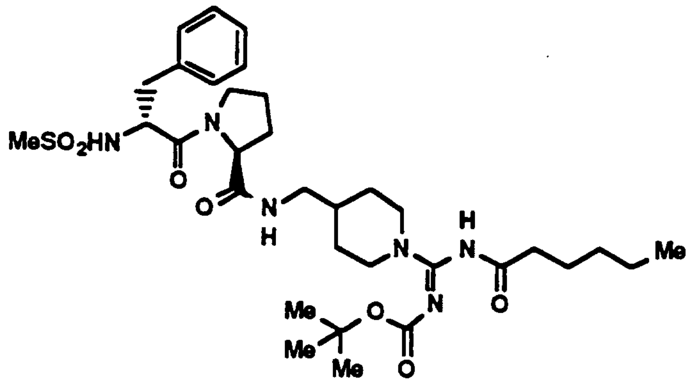

N-[[1-[[(1,1-Dimethylethoxy)carbonyl]imino]-[(1-oxo-hexyl)amino]methyl]-4-piperidinyl]methyl]-1-[N-(methylsulfonyl)-D-phenylalanyl]-L-prolinamide

A.

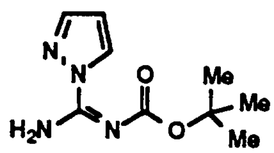

N-BOC-l-guanylpyrazole

Di-t-butylcarbonate (16.4 g, 75.2 mmol) was added in portions to a stirred solution of N-1-guanylpyrazole monohydrochloride (10 g, 67.8 mmol) and diisopropylethyl amine (10.02 g, 77.5 mmol) in DMF (21 mL) at 0-5°C. After 10 min, the cooling bath was removed and the solution was stirred at RT for 2 h. The mixture was diluted with EtOAc (225 mL) and water (100 mL). The aqueous layer was extracted with EtOAc (75 mL, 2x). The EtOAc extracts were combined, washed with 5% aq. $KHSO_4$ solution (100 mL, 2x), satd. $NaHCO_3$ solution (50 mL), water (50 mL), and brine (50 mL), dried ($MgSO_4$), filtered and concentrated *in vacuo* to obtain a white solid which was triturated with hexanes (80 mL). The precipitate was filtered, washed with hexanes (20 mL, 2x), and dried *in vacuo* to obtain N-BOC-1-guanylpyrazole (8 g). The filtrate was concentrated and the residue was chromatographed on a silica gel column. Elution with 5% EtOAc in hexanes, followed by 20% EtOAc in hexanes afforded additional N-BOC-1-guanylpyrazole (4 g; combined yield 84%).

B.

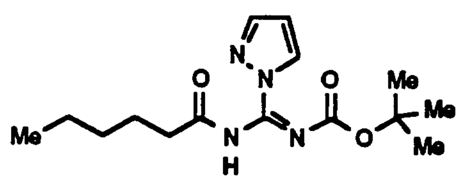

N-BOC-N'-hexanoyl-1-guanylpyrazole

N-BOC-1-guanylpyrazole (2.1g, 10 mmol) was added in portions to a stirred suspension of NaH (300 mg, 12.5 mmol) in THF (20 mL) at 0-5°C. After 10 min, a solution of hexanoic anhydride (2.56 g, 12 mmol) in THF (20 mL) was added over a period of 30 min. The suspension was stirred at 0-5°C for additional 30 min, diluted with water (20 mL) and extracted with EtOAc (50 mL). The aqueous layer was extracted with $CH_2Cl_2$ (25 mL, 3x) and the organic extracts combined, dried ($MgSO_4$), filtered and concentrated. The crude oil was chromatographed on a silica gel column and eluted with 10%, 20%, and 25% EtOAc in hexanes to obtain unreacted N-BOC-1-guanylpyrazole(1 g, 47%) as a colorless solid and N-BOC-N'-hexanoyl-1-guanylpyrazole (1.7 g, 53%) as a colorless oil.

C.

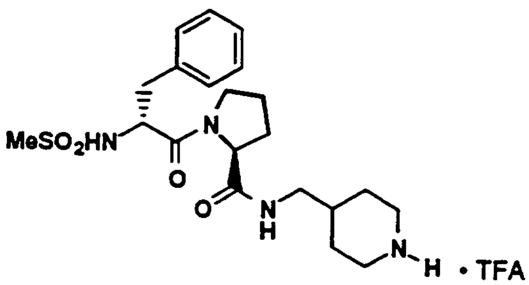

N-(4-piperidinylmethyl)-1-[N-(methylsulfonyl)-D-phenylalanyl]-L-prolinamide, trifluoroacetate

C(1).

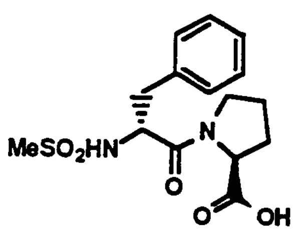

1-[N-(methylsulfonyl)-D-phenylalanyl]-L-proline

D-phenylalanine (50.12 g, 303.4 mmol) was weighed into a 5-necked flask with a thermocouple pH probe, overhead stirrer and two addition funnels. Sodium hydroxide (12.14 g, 303.4 mmol) in water (300 mL) was added with stirring and cooling to 0° C (pH = 11.6). Methanesulfonyl chloride (44.5 g, 388.4 mmol) and sodium hydroxide (6 N, 84 mL) were added dropwise through two addition funnels to maintain the pH at ca. 11 and the internal temperature at ca. 0°C. A second portion of methanesulfonyl chloride (7 mL) and sodium hydroxide (6N, 16 mL) was added dropwise, the reaction mixture brought to pH 13 with sodium hydroxide and allowed to warm to 15°C over one hour and washed with methyl isobutyl ketone (2 X 250 mL). The aqueous layer was acidified to pH = 1 and extracted with ethyl acetate 3 X 400 mL), the combined organic extracts washed with brine, dried over magnesium sulfate, and evaporated in vacuo to give a 10:1 ratio of N-methylsulfonyl-D-phenylalanine to N-methylsulfonyl-D-phenylalanyl-D-phenylalanine.

The above mixture of acids (71 g) was dissolved in dichloromethane (900 mL) at 0°C with DMF (0.8 mL) and treated with oxalyl chloride (2 M in dichloromethane, 160.5 mL, 321 mmol). The solution was stirred at room temperature for 1.5 hr, the solvents evaporated in vacuo and azeotroped with toluene (400 mL) to give the acid chloride as a solid (73 g).

The above acid chloride (73 g) in toluene (500 mL) was added to a stirred solution of L-proline (51.33 g, 445.8 mmol) in sodium hydroxide (10.51 g, 262.7 mmol in 300 mL water) solution at 0°C. The pH of the solution was maintained at ca. 11 and the temperature between 2 - 5°C during the addition. The orange biphasic mixture was allowed to stir for 0.5 hr, hexanes were added and the aqueous layer separated. The aqueous layer was washed with ethyl acetate (2 X 400 mL), acidified to pH 1 with hydrochloric acid, and extracted with ethyl acetate 1 X 1 L, 2 X 600 mL), the extracts combined, washed with brine, dried over magnesium sulfate, decolorized with charcoal, filtered and concentrated in

vacuo to give a solid which was washed with hexanes to give 77g of 1-[N-(methylsulfonyl)-D-phenylalanyl]-L-proline.

C(2).

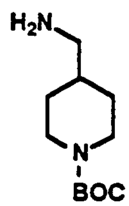

1-[(1,1-Dimethylethoxy)carbonyl]-4-aminomethyl piperidine

A mixture of 4-aminomethyl piperidine (100 g, 0.88 mol) and benzaldehyde (102 g, 0.96 mmol) in toluene (1.5 L) was heated to reflux with a Dean-Stark trap for 1 hr, cooled to 5°C, and treated with a solution of di-t-butyl-dicarbonate (200 g, 0.92 mol) in toluene (700 mL). The mixture was stirred at room temperature overnight, treated with 1 M sodium hydrogen sulfate (1 L) and stirrred for 3 hr. The layers were separated, and the aqueous layer was washed with ether (3 X 250 mL), made basic with sodium hydroxide (50% solution) and extracted with ether (3 X 500 mL). The combined organic layers were dried over sodium sulfate and evaporated in vacuo to give 218.7 g of 1-[(1,1-dimethylethoxy)carbonyl]-4-aminomethyl piperidine as an oil.

C(3).

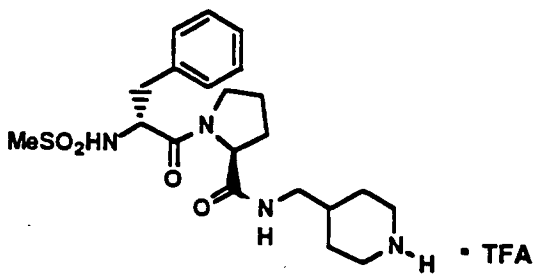

N-(4-piperidinylmethyl)-1-[N-(methylsulfonyl)-D-phenylalanyl]-L-prolinamide, trifluoroacetate

A mixture of Part C(l) 1-[N-(methylsulfonyl)-D-phenylalanyl]-L-proline (180.9 g, 0.53 mol) and Part C(2) 1-[(1,1-dimethylethoxy)carbonyl]-4-aminomethyl piperidine (129 g, 0.60 mol) in DMF (3 L) was treated with hydroxybenzotriazole (90 g, 0.59 mol) and N-methyl morpholine (55 g, 0.54 mol) at 5°C, and then with ethyl dimethylaminopropyl carbodiimide (105 g, 0.55 mol). The reaction mixture was stirred at room temperature overnight, poured into water (6 L), and extracted with ethyl acetate (2 X 3 L), washed with sodium hydrogen sulfate (2 X 1 L), water (2 X 1 L), sodium bicarbonate (2 X 1 L) and brine (1 X 1 L), and the solvents evaporated in vacuo to give a thick oil. The oil was taken up in ethyl acetate (1 L), diluted with heptane, and allowed to crystallize to give the t-BOC protected piperidine (89 g).

The above t-BOC-protected piperidine was dissolved in dichloromethane (200 mL) and treated with trifluoroacetic acid (200 mL) at room temperature. After 2 hr, the solvents were evaporated in vacuo, and the resulting foam triturated with ether (2 x 1L) to give a finely divided solid which was washed with ether and dried in vacuo to give N-(4-piperidinylmethyl)-1-[N-(methylsulfonyl)-D-phenylalanyl]-L-prolinamide, trifluoroacetate (82.5 g).

D.

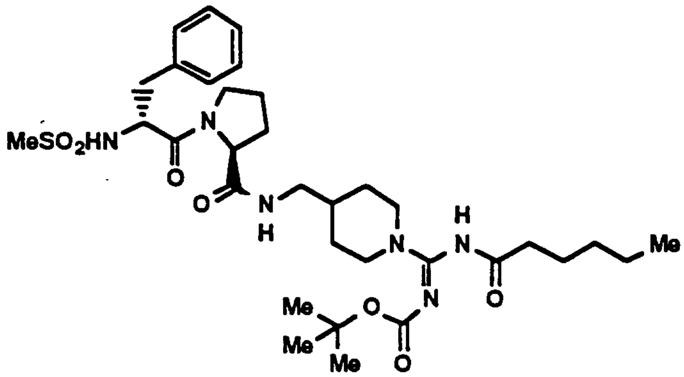

N-[[1-[[(1,1-Dimethylethoxy)carbonyl]imino]-[(1-oxo-hexyl)amino]methyl]-4-piperidinyl]methyl]-1-[N-(methylsulfonyl)-D-phenylalanyl]-L-prolinamide

Diisopropylethyl amine (1.5 mL) was added to a stirred solution of N-(4-piperidinylmethyl)-1-[N-(methylsulfonyl)-D-phenylalanyl]-L-prolinamide, trifluoroacetate prepared as described in Part C (1.62 g, 3 mmol) and N-BOC-N'-hexanoyl-1-guanylpyrazole (1.23 g, 4 mmol) in DMF (12 mL). The solution was stirred at RT overnight, concentrated under reduced pressure and *in vacuo*. The residue was chromatographed on a silica gel column and eluted with 50%, and 80% EtOAc in hexanes, followed by 1.5% methanol in EtOAc to obtain a foam which was stirred in hexanes-ether (75 mL, 4:1). The solid was filtered, washed with hexanes-ether (4:1) and dried *in vacuo* to obtain the title compound (1.25 g, 62%). $[\alpha]_D$ = - 69.6° (c = 0.25, MeOH).

| Analysis calcd for $C_{33}H_{52}N_6SO_7$: | | | | |
|---|---|---|---|---|
| | C, 58.56; | H, 7.74; | N, 12.42; | S, 4.74 |
| Found: | C, 58.49; | H, 8.03; | N, 12.38; | S, 4.52. |

Example 2

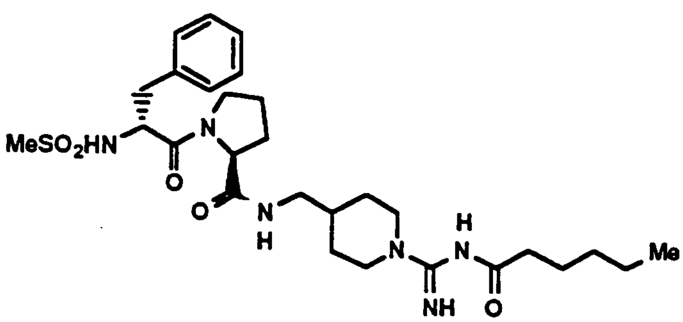

N-[[1-[Imino[(1-oxohexyl)amino]methyl]-4-piperidinyl]methyl]-1-[N-(methylsulfonyl)-D-phenylalanyl]-L-prolinamide

A solution of Example I compound, namely, N-[[1-[[(1,1-dimethylethoxy)carbonyl]imino]-[(1-oxo-hexyl)amino]methyl]-4-piperidinyl]methyl]-1-[N-(methylsulfonyl)-D-phenylalanyl]-L-prolinamide (750 mg, 1.11 mmol) in

trifluoroacetic acid (TFA) (3 mL) was stirred at RT for 2 h. Most of the TFA was removed by distillation under reduced pressure. The residual oil was coevaporated with $CH_2Cl_2$ (5 mL, 4x) and ether (5 ml, 5x) to obtain a foam which was suspended in ether (50 mL) and stirred at RT for 5 h. Precipitated solid was filtered, washed with ether (10 mL, 3x), dried in vacuo at 50°C overnight to obtain N-[[1-[imino[(1-oxohexyl)amino]methyl]-4-piperidinyl]methyl]-1-[N-(methylsulfonyl)-D-phenylalanyl]-L-prolinamide (650 mg, 84%). $[\alpha]_D$ = -57.8° (c = 0.5, MeOH).

| Analysis calcd for $C_{28}H_{44}N_6SO_5 \cdot 1.0\ CF_3COOH \cdot 0.6\ H_2O$: | | | | | |
|---|---|---|---|---|---|
| | C, 51.36; | H, 6.64; | N, 11.98; | S, 4.57; | F, 8.12 |
| Found: | C, 51.37; | H, 6.62; | N, 11.78; | S, 4.58; | F, 8.32. |

Example 3

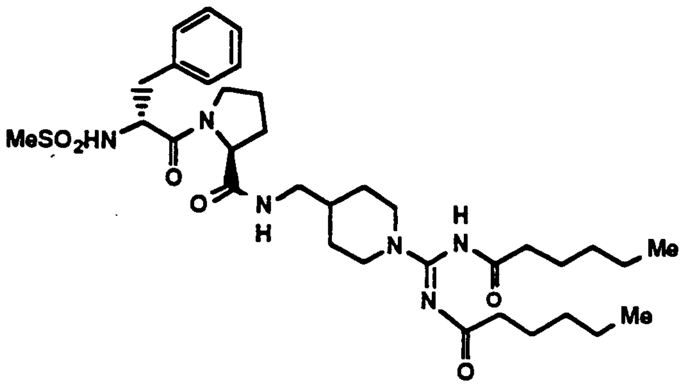

N-[[1-[[(1-Oxohexyl)amino][(1-oxohexyl)imino]methyl]-4-piperidinyl]methyl]-1-N-[(methylsulfonyl)-D-phenylalanyl]-L-prolinamide

A.

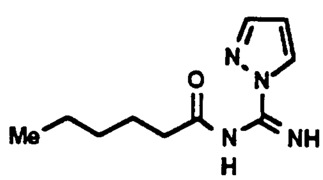

N-hexanoyl-1-guanylpyrazole

Hexanoic anhydride (3.42 g, 16 mmol) was added to a stirred solution of N-1-guanylpyrazole monohydrochloride (2.21 g, 15 mmol) and diisopropylethyl amine (3.23 g, 25 mmol) in DMF (50 mL). The solution was stirred at RT for 3 h, diluted with EtOAc (60 mL) and washed with 5% aqueous $KHSO_4$ solution (30 mL). The EtOAc extract was separated and the aqueous layer was extracted with $CH_2Cl_2$ (30 mL, 2x). The organic extracts were combined, washed with satd. $NaHCO_3$ solution (25 mL), dried ($MgSO_4$), filtered and concentrated. The crude oil was chromatographed on a silica gel column and eluted with 10%, and 20% EtOAc in hexanes to obtain the title compound (2.85 g, 91%) as a colorless oil.

B.

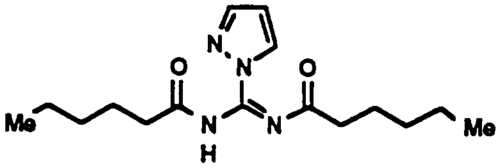

N,N'-bishexanoyl-1-guanylpyrazole

A solution of N-hexanoyl-1-guanylpyrazole (2.85 g, 13.57 mmol) in distilled THF (15 mL) was added dropwise to a stirred suspension of NaH (360 mg, 15 mmol) in THF (45 mL) at 0-5°C. After 10 min, a solution of hexanoic anhydride (3.21 g, 15 mmol) in THF (15 mL) was added over a period of 15 min. The suspension was stirred at 0-5°C for 1h, and diluted with water (50 mL). The THF layer was separated and the aqueous layer was extracted with $CH_2Cl_2$ (25 mL, 2x), the organic extracts combined, dried ($MgSO_4$), filtered and concentrated. The crude oil was chromatographed on a silica gel column and eluted with 10%, 15%, and 25% EtOAc in hexanes to obtain unreacted N-hexanoyl-1-guanylpyrazole (1.05 g, 37%) and N,N'-bishexanoyl-1-guanylpyrazole(2.27 g, 55%) as a colorless oil.

C.

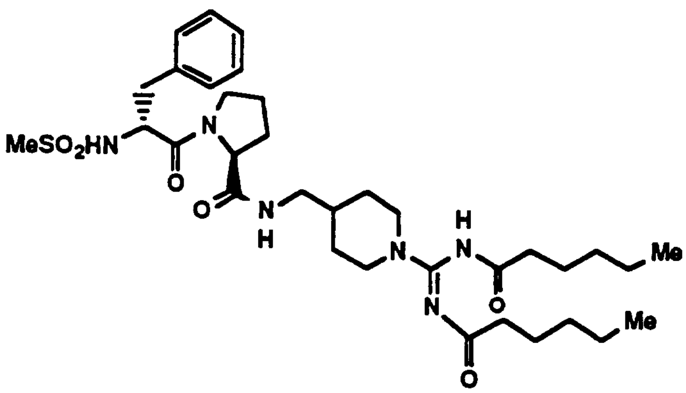

N-[[1-[[(1-Oxohexyl)amino][(1-oxohexyl)imino]methyl]-4-piperidinyl]methyl]-1-[N-(methylsulfonyl)-D-phenylalanyl]-L-prolinamide

Diisopropylethyl amine (1 mL) was added to a stirred solution of Example I Part C N-(4-piperidinylmethyl)-1-[N-(methylsulfonyl)-D-phenylalanyl]-L-prolinamide, trifluoroacetate (1.08 g, 2 mmol) and N,N'-bishexanoyl-1-guanylpyrazole(1.02 g, 2.5 mmol) in DMF (6 mL). The solution was stirred at RT overnight, concentrated under reduced pressure and *in vacuo*. The residue was chromatographed on a silica gel column and eluted with 70% EtOAc in hexanes, followed by 1%, and 1.5% methanol in EtOAc to obtain an oil which was coevaporated with $CH_2Cl_2$ (10 mL, 3x) and dried *in vacuo* to obtain the title compound as a foam (1.04 g, 77%). $[a]_D$ = - 61.8° (c = 0.275, MeOH).

| Analysis calcd for $C_{34}H_{54}N_6SO_6$: | | | | |
|---|---|---|---|---|
| | C, 60.51; | H, 8.06; | N, 12.45; | S, 4.75 |
| Found: | C, 60.27; | H, 8.38; | N, 12.53; | S, 4.66. |

Example 4

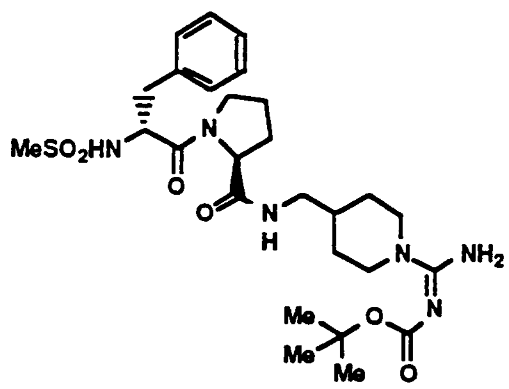

N-[[1-[Amino[[(1,1-dimethylethoxy)carbonyl]imino]methyl]-4-piperidinyl]methyl]-1-[N-(methylsulfonyl)-D-phenylalanyl]-L-prolinamide

N-[[1-(Aminoiminomethyl)-4-piperidinyl]-methyl]-1-N-(methylsulfonyl)-D-phenylalanyl]-L-prolinamide hydrochloride (prepared as described in Example 30 of U.S. application Serial No. l46,7l4 filed November l0, l993) (425 mg, 0.60 mmol) was dissolved in distilled THF (20 mL) and triethylamine (800 μL). BOC-ON (725 mg, 2.9 mmol) was added and the mixture sirred while heating in an oil bath maintained at 45-55°C for 40 hr. After cooling, the solvent was removed in vacuo and the residue purified by chromatography on silica gel, eluting with 5-10% methanol in dichloromethane to give the title compound. The material was triturated with ether, the solid harvested by filtration, washed again with ether and dried overnight at reduced pressure to give N-[[1-[amino-[[(1,1-dimethylethoxy)carbonyl]imino]methyl]-4-piperidinyl]methyl]-1-N-[(methylsulfonyl)-D-phenylalanyl]-L-prolinamide. mp 128-130° C (dec).

| Analysis calcd for $C_{27}H_{42}N_6O_6S \cdot 0.15\ Et_2O \cdot 0.4\ H_2O$: | | | | |
|---|---|---|---|---|
| | C, 55.52; | H, 7.48; | N, 14.08; | S, 5.37 |
| Found: | C, 55.42; | H, 7.51; | N, 14.35; | S, 5.32. |

Example 5

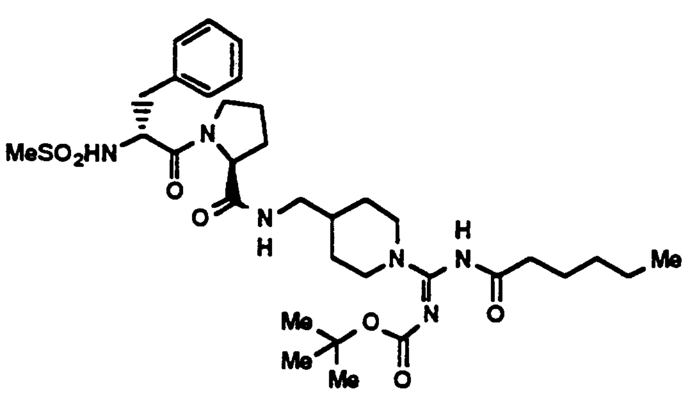

N-[[1-[[[(1,1-Dimethylethoxy)carbonyl)imino][(1-oxohexyl)amino]methyl]-4-piperidinyl]methyl]-1-[N-(methylsulfonyl)-D-phenylalanyl]-L-prolinamide

The title compound may be prepared by treating Example 4 compound, namely, N-[[1-[[[[(1,1-dimethylethoxy)carbonyl]-amino]imino]methyl]-4-piperidinyl]methyl]-1-N-[(methylsulfonyl)-D-phenylalanyl]-L-prolinamide with sodium hydride and hexanoic anhydride as described in the preparation of N-hexanoyl-1-guanylpyrazole (Example 2 Part A).

Example 6

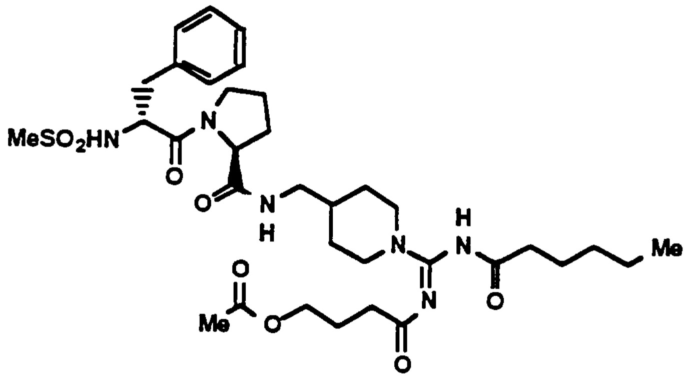

N-[[1-[[[4-(Acetyloxy)-1-oxobutyl]imino][(1-oxohexyl)amino]methyl]-4-piperidinyl]methyl]-1-[N-(methylsulfonyl)-D-phenylalanyl]-L-prolinamide

A.

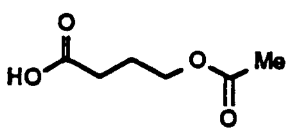

4-Acetoxybutyric acid

A solution of 4-hydroxybutyric acid, sodium salt (2.52 g, 20 mmol) and benzyl bromide (5.13 g, 30 mmol) in DMF (60 mL) was stirred at RT overnight. The mixture was diluted with water (100 mL) and extracted with ether (50 mL, 3x). The ether extract was dried ($MgSO_4$), filtered and concentrated to obtain crude 4-hydroxybutyric acid, benzyl ester as an oil.

A solution of crude benzyl ester and acetic anhydride (3.06 g, 30 mmol) in $CH_2Cl_2$ (60 mL) and triethyl amine (7 g, 50 mmol) was stirred at RT for 4h. The mixture was diluted with $CH_2Cl_2$ (40 mL) and washed with 1N HCl solution (40 mL, 2x), and brine (30 mL). The organic extract was dried ($MgSO_4$), filtered and concentrated. The crude oil was chromatographed on a silica gel column and eluted with 5%, 10%, and 25% EtOAc in hexanes to obtain 4-acetoxybutyric acid, benzyl ester (3.79 g, 81% overall yield) as an oil.

10% Palladium on charcoal (570 mg, 15% w/w) was added to a stirred solution of 4-acetoxybutyric acid, benzyl ester (3.79 g, 16.06 mmol) in methanol (80 mL). Air inside the flask was evacuated under reduced pressure and was then filled with hydrogen from a balloon (3x). Hydrogenolysis was continued overnight. The mixture was filtered through a pad of anhydrous $MgSO_4$ and the residue was washed with methanol. The filtrate was concentrated under reduced pressure and in vacuo to obtain 4-acetoxybutyric acid (1.39 g, 95%) as an oil.

B.

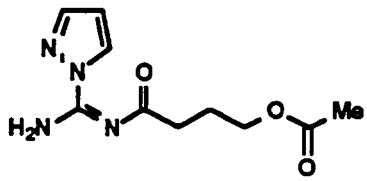

N-(4-Acetoxybutyryl)-1-guanylpyrazole

4-Methylmorpholine (2.2 mL, 20 mmol) was added to a stirred solution of N-1-guanylpyrazole monohydrochloride (1.4 g, 9.5 mmol) and 4-acetoxybutyric acid (1.39 g, 9.5 mmol) in DMF (30 mL). WSC (1.9 g, 9.5 mmol) was added. The solution was stirred at RT overnight, diluted with EtOAc (75 mL) and washed with 10% aq. $KHSO_4$ solution (50 mL), satd. $NaHCO_3$ solution (30 mL), 10% aqueous LiCl solution (20 mL, 2x), dried ($MgSO_4$), filtered and concentrated to obtain N-(4-acetoxybutyryl)-1-guanylpyrazole(1.88 g, 83%) as an oil.

C.

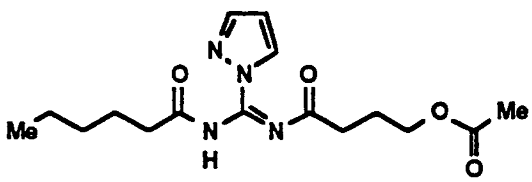

N-(4-Acetoxybutyryl)-N'-hexanoyl-1-guanylpyrazole

Sodium hydride (284 mg, 11.85 mmol) was added in portions to a solution of N-(4-acetoxybutyryl)-1-guanylpyrazole(1.88 g, 7.9 mmol) in distilled THF (45 mL) at 0-5°C. After a few min, a solution of hexanoic anhydride (2.03 g, 9.48 mmol) in THF (20 mL) was added. The suspension was stirred at 0-5°C for 1h, and then poured into satd. $NH_4Cl$ solution (50 mL). The THF layer was separated and the aqueous layer was extracted with EtOAc (50 mL). Organic extracts were combined, dried ($MgSO_4$), filtered and concentrated. The crude oil was chromatographed on a silica gel column and eluted with 10%, 15%, and 20% EtOAc in hexanes to obtain the title compound (1.36 g, 51%) as an oil.

D.

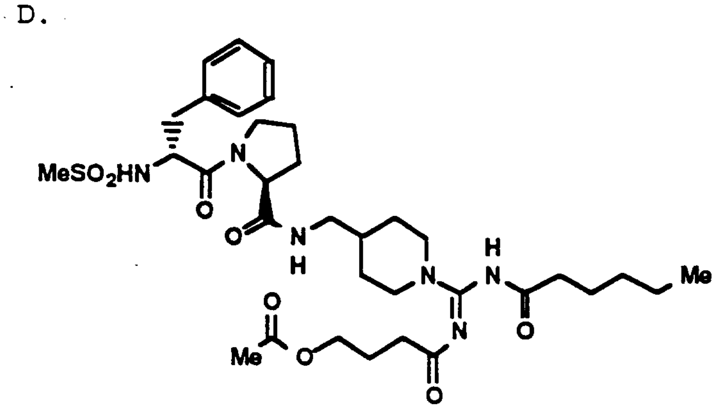

N-[[1-[[[4-(Acetyloxy)-1-oxobutyl]imino][(1-oxohexyl)amino]methyl]-4-piperidinyl]methyl]-1-[N-(methylsulfonyl)-D-phenylalanyl]-L-prolinamide

Diisopropylethyl amine (500 μL) was added to a stirred solution of [4-[piperidinyl]methyl]-1-[N-(methylsulfonyl)-D-phenylalanyl]-L-prolinamide (540 mg, 1 mmol) and N-(4-acetoxybutyryl)-N'-hexanoyl-1-guanylpyrazole(403 mg, 1.2 mmol) in DMF (6 mL). The solution was stirred at RT overnight, concentrated under reduced pressure and in vacuo. The residue was chromatographed on a silica gel column and eluted with 70% EtOAc in hexanes, followed by 0.5%, 1.5%, and 2% methanol in EtOAc to obtain an oil which was coevaporated with $CH_2Cl_2$ (5 mL, 3x) and dried *in vacuo* to obtain N-[[1-[[[4-(acetyloxy)-1-oxobutyl]imino][(1-oxohexyl)amino]methyl]-4-piperidinyl]methyl]-1-[N-(methylsulfonyl)-D-phenylalanyl]-L-prolinamide as a foam (557 mg, 81%).
$[\alpha]_D$ = - 54.3° (c = 0.245, MeOH).

| Analysis calcd for $C_{34}H_{52}N_6SO_8 \cdot$ 0.52 $H_2O$: | | | | |
|---|---|---|---|---|
| | C, 57.17; | H, 7.48; | N, 11.77; | S, 4.49 |
| Found: | C, 57.20; | H, 7.62; | N, 11.74; | S, 4.75. |

Example 7

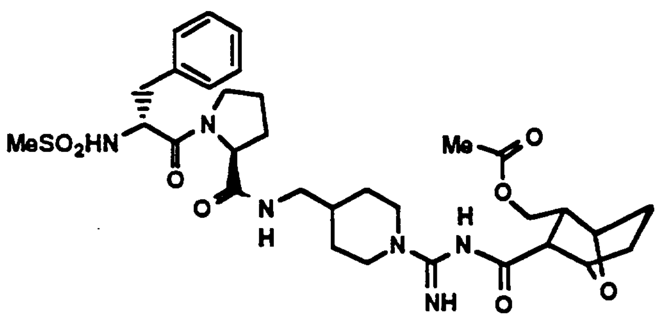

[1S-(exo,exo)]-N[[1-[[[[3-[(Acetyloxy)methyl]-7-oxabicyclo[2.2.1]hept-2-yl]carbonyl]imino]aminomethyl]-4-piperidinyl]methyl]-1-[N-(methylsulfonyl)-D-phenylalanyl]-L-prolinamide, trifluoroacetate

A.

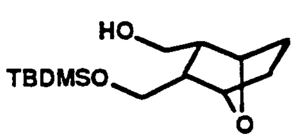

3-(Hydroxymethyl)-2-[(*t*-butyldimethylsiloxy)methyl]-7-oxabicyclo[2.2.1]heptane

To a solution of 60% sodium hydride (1.57 g, 39.3 mmol) in THF (40 mL) at 0°C was added a solution of 2,3-bis-[hydroxymethyl]-7-oxabicyclo[2.2.1]heptane (J. Org. Chem **1986**, *51*, 3388-90; 6.21 g, 39.3 mmol) in THF (20 mL) over 20 min. The reaction was stirred at RT for 3 hr, cooled to 0°C and a solution of *t*-butyldimethylsilylchloride (5.92 g) in THF (20 mL) was added over 10 min. The reaction was stirred an additional 20 hr, quenched with satd $NH_4Cl$ (100 mL), and extracted with ethyl acetate (4 X 100 mL). The combined organic layers were washed with brine (200 mL), dried ($MgSO_4$), filtered and concentrated *in vacuo*. The crude product was purified on silica gel using 1:1 ethyl acetate:hexanes to give the title compound (8.89 g, 83%).

B.

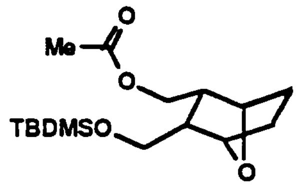

3-[(Acetyloxy)methyl]-2-[(*t*-butyldimethylsiloxy)methyl]-7-oxabicyclo[2.2.1]heptane

To a solution of 2-(hydroxymethyl)-3-[(*t*-butyldimethylsiloxy)methyl]-7-oxabicyclo[2.2.1]-heptane (4.51 g, 16.6 mmol) in dichloromethane (80 mL) was added triethylamine (1.72 mL, 18.3 mmol), followed by the dropwise addition of acetic anhydride (1.72 mL, 18.3 mmol) over 5 min, followed by DMAP (0.5 eq). After 2.5 hr the reaction was diluted with ethyl acetate (400 mL), washed with 0.5 N HCl (2 X 200 mL), satd $NaHCO_3$ (200 mL), dried ($MgSO_4$), filetered, and concentrated *in vacuo* to give the title compound (4.95 g, 95%).

C.

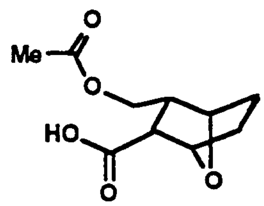

3-[(Acetyloxy)methyl]-2-carboxy-7-oxabicyclo-[2.2.1]heptane

To a solution of 2-[acetyloxy]methyl-3-[*t*-butyldimethylsiloxy]methyl-7-oxabicyclo[2.2.1]heptane (4.5 g) in acetone (90 mL) at 0°C was added Jones reagent (8.4 mL) until an orange-red color persisted. The reaction was stirred vigorously at RT for 3 hr, quenched with isopropyl alcohol, concentrated *in vacuo*, partitioned between ethyl acetate (200 mL) and 3 M $NaHSO_3$ (150 mL), and the aqueous layer extracted with ethyl acetate (2 X 200 mL). The combined organic layers were washed with water (2 X 80 mL), brine (100 mL), dried ($MgSO_4$), filtered, concentrated *in vacuo* and triturated with hexanes (3 X 20 mL) to give the title compound (1.85 g, 60%).

D.

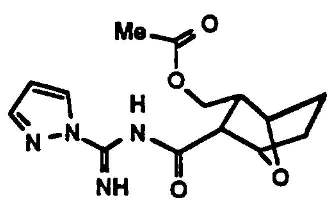

N-[3-[(Acetyloxy)methyl]-2-carboxy-7-oxabicyclo[2.2.1]hept-2-yl]-1-guanylpyrazole

To a suspension of 3-[(acetyloxy)methyl]-2-carboxy-7-oxabicyclo[2.2.1]heptane (1.80 g, 8.41 mmol), EDAC (3.22 g, 16.8 mmol) and guanylpyrazole hydrochloride (1.23 g, 8.41 mmol) in acetonitrile (40 mL) at RT was added diisopropylethylamine (4.40 mL, 25.2 mmol). The reaction was stirred for 20 hr, diluted with satd $NH_4Cl$ (40 mL), and concentrated *in vacuo* to remove organics. To the aqueous solution was added additional sat'd $NH_4Cl$ (40 mL), extracted with ethyl

acetate (4 X 70 mL), dried (MgSO4), filtered and concentrated *in vacuo* to give a crude product which was chromatographed on silica gel to afford the title compound (1.13 g, 44%).

E.

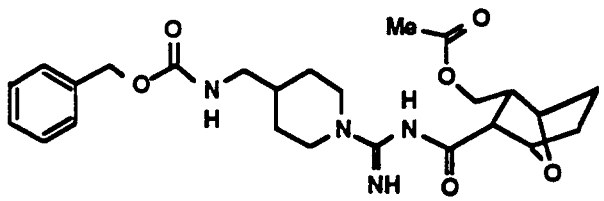

(exo,exo)-N1-[[[[3-[(Acetyloxy)methyl]-7-oxabicyclo[2.2.1]hept-2-yl]-carbonyl]imino]aminomethyl]-4-[(carbobenzyloxy)amino]methyl piperidine

To a solution of N-[3-[acetyloxy]methyl-2-carboxy-7-oxabicyclo-[2.2.1]hept-2-yl]-1-guanylpyrazole (1.12 g, 3.66 mmol) and 4-[(carbobenzyloxy)amino]methyl piperidine trifluoroacetate (1.33 g) in acetonitrile (28 mL) at RT was added DBU (1.10 mL, 7.32 mmol). The reaction was stirred for 20 hr, quenched with $NH_4Cl$ (50 mL) and the organic layer removed *in vacuo*. The aqueous phase was extracted with ethyl acetate (4 X 70 mL), dried ($MgSO_4$), filtered and concentrated *in vacuo* to give the crude product which was chromatographed on silica gel to give the title *cis* isomer (290 mg).

F.

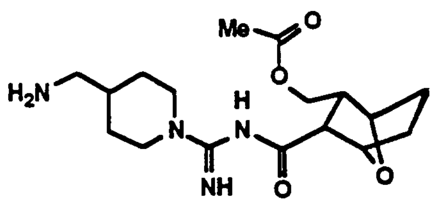

(exo,exo)-N1-[[[[3-[(Acetyloxy)methyl]-7-oxabicyclo[2.2.1]hept-2-yl]-carbonyl]imino]aminomethyl]-4-aminomethyl piperidine

To a solution of (exo,exo)-N1-[[[[3-[(acetyloxy)methyl]-7-oxabicyclo[2.2.1]hept-2-yl]carbonyl]imino]aminomethyl]-4-[(carbobenzyloxy)amino]methyl piperidine (342 mg) in isopropyl alcohol (*i*PA) (10 mL) was added $Pd(OH)_2$ on charcoal (68 mg), and the reaction hydrogenated for 16 hr at RT. The reaction was filtered, rinsed with *i*PA, and concentrated in vacuo to give the title compound (216 mg, 87%).

.G.

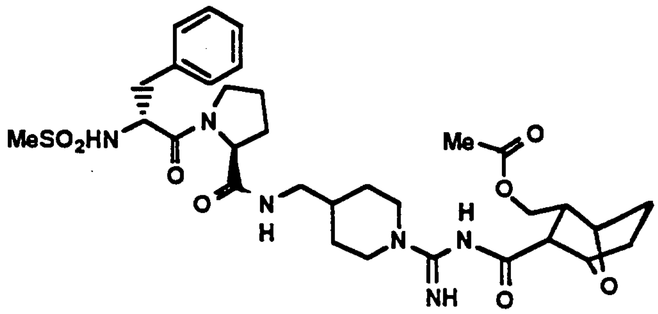

[1S-(exo,exo)]-N[[1-[[[[3-[(Acetyloxy)methyl]7-oxabicyclo[2.2.1]hept-2-yl]carbonyl]imino]aminomethyl]-4-piperidinyl]methyl]-1-[N-(methylsulfonyl)-D-phenylalanyl]-L-prolinamide

To a solution of (exo,exo)-N1-[[[[3-[(acetyloxy)methyl]-7-oxabicyclo[2.2.1]hept-2-yl]-carbonyl]imino]aminomethyl]-4-aminomethyl piperidine (296 mg, 0.84 mmol), N-(methylsulfonyl)-D-phenylalanyl-L-proline (286 mg, 0.84 mmol), HOBt (142 mg, 0.84 mmol) and NMM (0.18 mL, 1.68 mmol) in DMF (4 mL) was added EDAC (161 mg, 0.84 mmol). The reaction was stirred for 16 hr at RT, concentrated *in vacuo*, and partitioned between satd $NH_4Cl$ and ethyl acetate (3 X 60 mL). The combined organic layers were washed with satd $NH_4Cl$ (30 mL), brine (30 mL), dried ($MgSO_4$), filtered and concentrated *in vacuo* to give the crude product which was purified by chromatography on silica gel (methanol:dichloromethane) to give the title compound (157 mg, 27%).

| Anal. calc'd for $C_{32}H_{46}N_6O_8S \cdot 1.20\ CF_3COOH \cdot 1.20\ H_2O$: | | | | | |
|---|---|---|---|---|---|
| | C, 49.59; | H, 6.00; | N, 10.09; | S, 3.85; | F, 8.21 |
| Found: | C, 49.43; | H, 5.80; | N, 9.80; | S, 4.23; | F, 8.41. |

Example 8

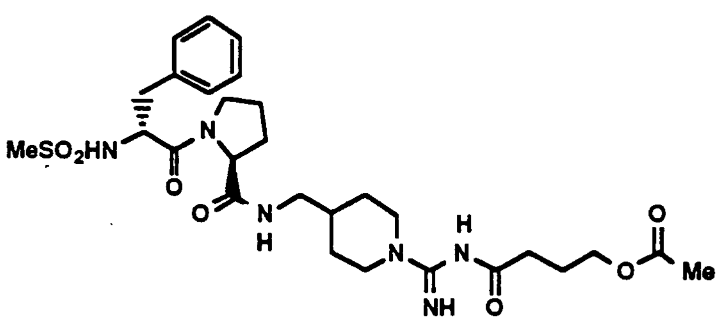

N-[[1-[[[4-(Acetyloxy)-1-oxobutyl]imino]aminomethyl]-4-piperidinyl]methyl]-1-[N-(methylsulfonyl)-D-phenylalanyl]-L-prolinamide

To a solution of Example I Part C N-(4-piperidinylmethyl)-1-[N-(methylsulfonyl)-D-phenylalanyl]-L-prolinamide trifluoroacetate (4.41 g, 8.02 mmol) in acetonitrile (30 mL) was added N-(4-acetoxybutyryl)-1-guanyl-pyrazole (2.10 g, 8.82 mmol) and DBU (2.64 mL, 17.6 mmol). The reaction was stirred for 16 hours, concentrated *in vacuo* and purified

by flash chromatography on silica gel to afford the title compound (1.67 g, 55%). $[\alpha]_D$ = - 67° (c = 1.38, MeOH).

| Analysis calcd for $C_{28}H_{42}N_6SO_7 \cdot 0.10\ H_2O$: | | | | |
|---|---|---|---|---|
| | C, 55.37; | H, 6.98; | N, 13.84; | S, 5.28. |
| Found: | C, 55.70; | H, 7.09; | N, 13.52; | S, 5.25. |

Example 9

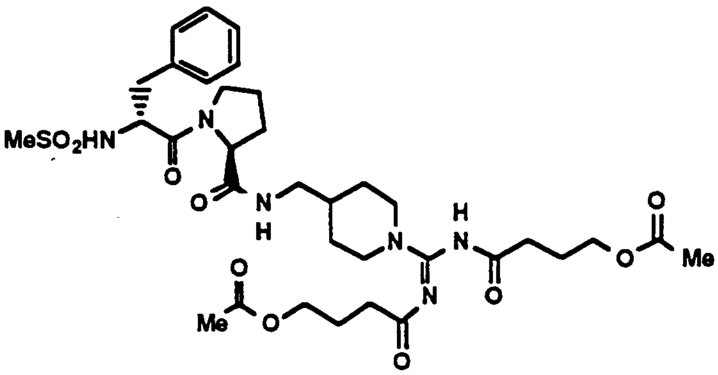

N-[[1-[[[4-(Acetyloxy)-1-oxobutyl]imino][4-(acetyloxy)-1-oxobutyl]amino]methyl]-4-piperidinyl]methyl]-1-[N-(methylsulfonyl)-D-phenylalanyl]-L-prolinamide

A.

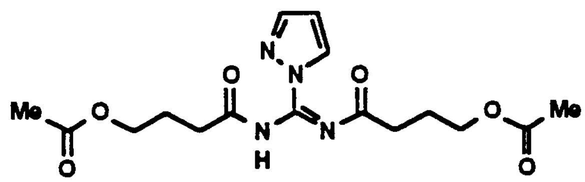

N,N'-Bis[4-(acetyloxy)-1-oxobutyl]-1-guanylpyrazole

To a solution of N-(4-acetyloxy-1-oxobutyl)-1-guanylpyrazole (1.82 g, 7.64 mmol) in distilled THF (60 mL) at 0°C was added NaH (460 mg, 19 mmol). After 40 min, a solution of 4-acetyloxy-1-oxobutanoic acid, succinimide ester (2.42 g, 9.93 mmol) in THF (30 mL) was added over a period of 20 min. The suspension was stirred at 0-5°C for 30 min, and then allowed to warm to RT over 2.5 hr. Additional 4-acetyloxy-1-oxobutanoic acid, succinimide ester (0.66 g) was added, and stirring was continued for an additional 2.5 hr. The reaction was quenched with satd $NH_4Cl$ (150 mL) and extracted with ethyl acetate. The organic layer was dried ($Na_2SO_4$), filtered and concentrated *in vacuo*. The crude oil was chromatographed on a silica gel column and eluted with 10%, 15%, and 25% EtOAc in hexanes to obtain N,N'-bis[4-acetyloxy-1-oxobutyl]-1-guanylpyrazole (1.45 g, 52%) as a colorless oil.

B.

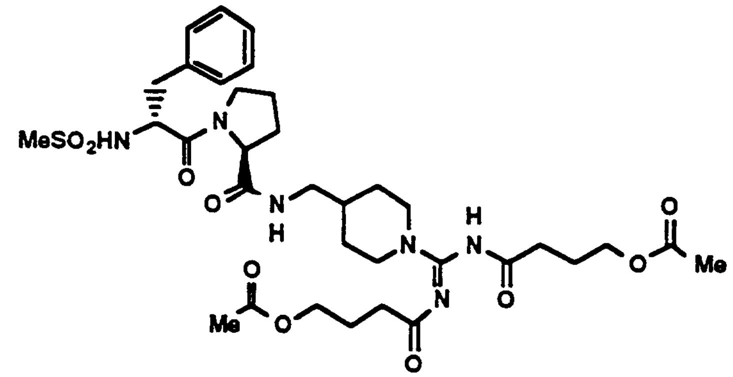

N-[[1-[[[4-(Acetyloxy)-1-oxobutyl]amino][4-(acetyloxy)-1-oxobutyl]imino]methyl]-4-piperidinyl]methyl]-1-[N-(methylsulfonyl)-D-phenylalanyl]-L-prolinamide

To a solution of N,N'-bis[4-acetyloxy-1-oxobutyl]-1-guanylpyrazole (0.41 g, 1.12 mmol) and [4-[piperidinyl]methyl]-1-[N-(methylsulfonyl)-D-phenylalanyl]-L-prolinamide trifluoroacetate (0.47 g, 0.86 mmol) in THF (50 mL) was added N,N-diisopropylethyl amine (0.43 mL, 2.5 mmol). The clear solution was stirred for 16 hr at RT, the solvent removed in vacuo and the product purified by chromatography on silica gel to give the title compound as a colorless solid (0.53 g, 74%).
$[\alpha]_D$ = - 56.2° (c = 0.5, MeOH).

| Analysis calcd for $C_{34}H_{50}N_6SO_{10}$. 0.61 $H_2O$: | | | | |
|---|---|---|---|---|
| | C, 54.75; | H, 6.92; | N, 11.27; | S, 4.36. |
| Found: | C, 54.82; | H, 6.98; | N, 11.20; | S, 4.30. |

Example I0

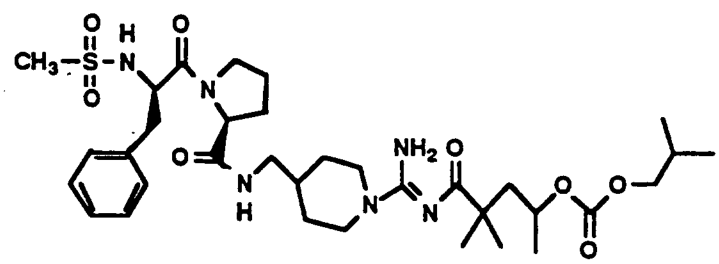

N-[[1-Amino[[[2,2-Dimethyl-4-[[(2-methylpropoxy)carbonyl]oxy]-1-oxopentyl]imino]methyl]-4-piperidinyl]-methyl]-1-[N-(methylsulfonyl)-D-phenylalanyl]-L-prolinamide, monohydrochloride

A.

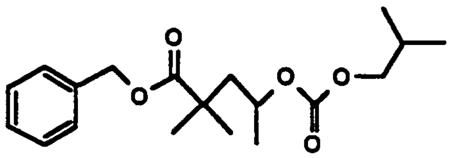

Benzyl-2,2-dimethyl-4-[[(2-methylpropoxy)carbonyl]oxy] pentanoate

Isobutylchloroformate (0.83 ml, 6.40 mmoles) was added to a rapidly stirring solution of benzyl-2,2-dimethyl-4-hydroxy pentanoate (1.26g, 5.3 mmol) and pyridine (0.52ml, 6.4 mmol) in dichloromethane (6 ml) at 0°C. After warming to room temperature overnight, the reaction was diluted with dichloromethane and washed with brine. The organic solvent dried over sodium sulfate and concentrated *in vacuo* to a yellow oil which was purified by flash chromatography (silica gel), eluting the column with ethyl acetate/hexane (2:98) to ethyl acetate/hexane (5:95). The desired fractions were combined and concentrated to obtain 1.46g of the title isobutyl carbonate.

B.

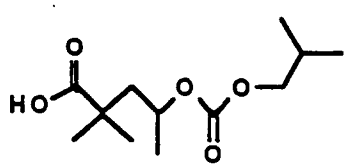

2,2-Dimethyl-4-[[(2-methylpropoxy)carbonyl]oxyl pentanoic acid

A mixture of the Part A compound benzyl-2,2-dimethyl-4-[3-methylpropyloxy[carbonyl[oxy]]] pentanoate (1.46g, 4.37 mmol) and Pearlman's catalyst (0.40g, 20% palladium on carbon) in ethyl acetate/ethanol (4:1) was aspirated and purged (3x) with hydrogen gas. After remaining at room temperature for 1 hour, the mixture was flushed with nitrogen gas and the catalyst removed by filtration. The filtrate was concentrated *in vacuo* to afford 0.89g of 2,2-dimethyl-4-[3-methylpropyloxy[carbonyl[oxy]]] pentanoic acid.

C.

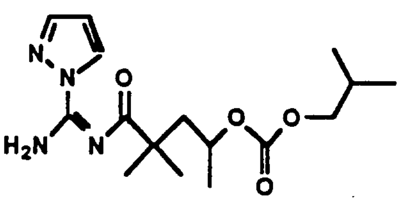

2,2-Dimethyl-4-[[(2-methylpropoxy)carbonyl]oxy] pentanoyl-1-guanylpyrazole

A mixture of Part B 2,2-dimethyl-4-[2-methylpropyl-oxy[carbonyl[oxy]]] pentanoic acid (0.89g, 3.62 mmoles) and 1-guanylpyrazole (0.64g, 4.34 mmoles) in dimethylformamide (8 ml) was cooled to 0°C before adding Benzotriazol-1-yloxy-tris(dimethylamino)phosphonium hexafluorophosphate (Bop reagent, 3.20g, 7.24 mmoles) followed by N-methylmorpholine (1.39 ml, 12.67 mmoles). After warming to room temperature overnight, the reaction was diluted with methylene chloride and washed with water and brine. The organic solvent was dried over sodium sulfate, concentrated *in vacuo* to title compound in the form of a yellow oil, which was purified by flash chromatography.

D.

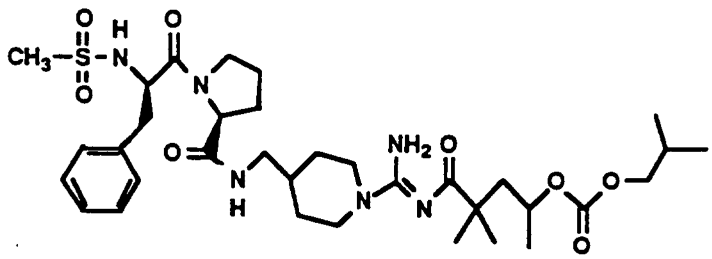

N-[[1-Amino[[[2,2-Dimethyl-4-[[(2-methylpropoxy)carbonyl]oxy]-1-oxopentyl]imino]methyl]-4-piperidinyl]-methyl]-1-[N-(methylsulfonyl)-D-phenylalanyl]-L-prolinamide, monohydrochloride

1,8-Diazabicyclo[5.4.0]undec-7-ene (0.36 ml, 1.20 mmoles) was added to a stirring solution of the above Part C compound (0.45g, 1.33 mmoles) and the Example 1, Part C compound(0.66g, 0.70 mmol) in acetonitrile (12 ml) at room temperature. After 48 hours, the reaction was concentrated *in vacuo* to a yellow oil and purified by flash chromatography, the appropriate fractions concentrated *in vacuo* and the resulting colorless oil triturated with methylene chloride and hexane to form a white soild. This material was dissolved in methanol, and cooled to 0°C before adding HCl (0.50 ml, 4N solution in dioxane). After stirring at 0°C for one hour, the solution was concetrated *in vacuo* to a colorless oil, evaporated with methylene chloride (3X), and triturated with methylene chloride and hexane to give 0.40 g of title compound in the form of a colorless solid.
$[\alpha]_D$ = -54° (c=0.22, $CH_3OH$)

| Elemental analysis for $C_{34}H_{54}O_8N_6S$ 1.10HCl | | | | | |
|---|---|---|---|---|---|
| | C | H | N | S | Cl |
| **calc'd** | 55.55 | 7.96 | 10.50 | 4.01 | 4.87 |
| **found** | 55.55 | 7.90 | 10.59 | 3.87 | 4.90 |

Example II

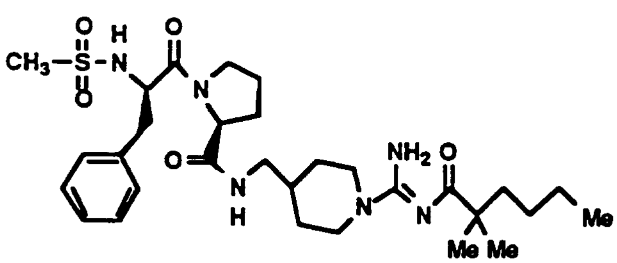

N-[[1-[Amino[(2,2-dimethyl-1-oxohexyl)imino]methyl]4-piperidinyl]methyl]-1-[N-(methylsulfonyl)-D-phenylalanyl]-L-prolinamide, monohydrochloride

A.

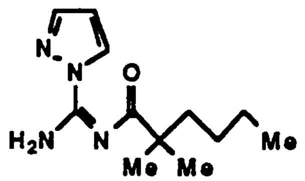

[2,2-Dimethyl-1-oxohexyl]-1-guanylpyrazole

To a solution of 1-guanylpyrazole (0.94 g, 6.4 mmol), 2,2-dimethyl hexanoic acid (0.77g, 5.34 mmol), and benzotriazol-1-yloxy-tris(dimethylamino)phosphonium hexafluorophosphate (2.84 g, 6.4 mmol, Bop reagent) in dichloromethane (27 mL) was added N-methyl morpholine (2.05 mL, 18.7 mmol), and the reaction allowed to stir for 24 hr. The solvent was evaporated, the product dissolved in ethyl acetate (100 mL) and the insoluble byproducts removed by filtration. The filtrate was washed with hydrochloric acid (3 X 15 mL), sat'd aqueous potassium bicarbonate (3 X 15 mL), brine, dried over sodium sulfate, evaporated in vacuo, and purified by chromatography to provide title compound (1.13 g).

B.

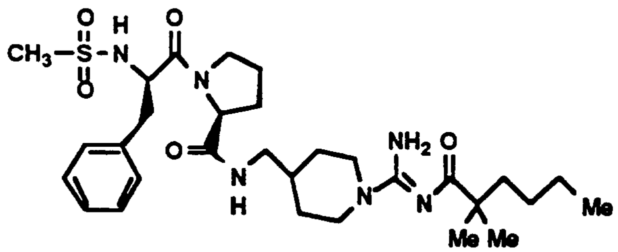

N-[[1-[Amino[(2,2-dimethyl-1-oxohexyl)imino]methyl]-4-piperidinyl]methyl]-1-[N-(methylsulfonyl)-D-phenylalanyl]-L-prolinamide, monohydrochloride

A solution of the Example 1, Part C compound (0.31 g, 0.56 mmol) and Part A [2,2-dimethyl-1-oxohexyl]-1-guanylpyrazole(0.16 g, 0.68 mmol) in acetonitrile (1.1 mL) was treated with DBU (0.21 mL, 1.4 mmol). The reaction was stirred for 16 hr at room temperature, the crude product absorbed on silica gel and purified by flash chromatography to give the free base, which was dissolved in methanol (3.5 mL), acidified with hydrochloric acid, and the solvents removed to provide the title monohydrochloride salt (0.31 g).
$[\alpha]_D$ = -62.4° (c=0.50, $CH_3OH$).

| Elemental analysis for $C_{30}H_{48}N_6O_5S$ • 1.26 H2O • 1.12 HCl | | | | | |
|---|---|---|---|---|---|
| | C | H | N | S | Cl |
| calc'd | 53.99 | 7.65 | 12.59 | 4.80m | 5.95 |
| found | 54.03 | 7.72 | 12.55 | 4.88 | 6.01 |

Following the procedures described above the following examples have been prepared:

Example I2

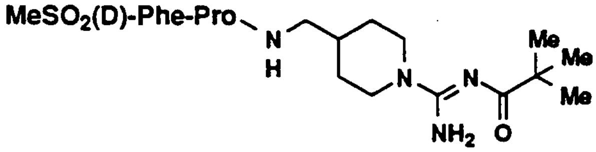

N-[[1-[Amino[(2,2-dimethyl-1-oxopropyl)imino]methyl]-4-piperidinyl]methyl]-1-[N-(methylsulfonyl)-D-phenylalanyl]-L-prolinamide

$[\alpha]_D$ = -66.6° (c=0.5, $CH_3OH$)

| Elemental analysis for $C_{27}H_{42}N_6O_5S \cdot 1.26\ H_2O \cdot 0.03\ (C_2H_5)_2O$: | | | | |
|---|---|---|---|---|
| | C | H | N | S |
| **calc'd** | 49.41 | 6.28 | 11.66 | 4.45 |
| **found** | 49.41 | 5.96 | 11.55 | 4.21 |

Example I3

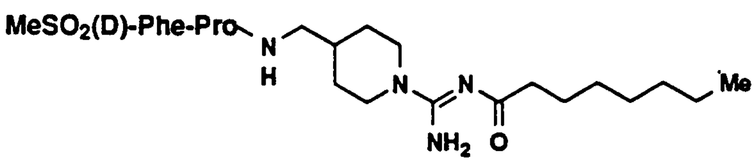

N-[[1-[Amino[(1-oxooctyl)imino]methyl]-4-piperidinyl]methyl]-1-[N-(methylsulfonyl)-D-phenylalanyl]-L-prolinamide

$[\alpha]_D$ = -67.8° (c=0.90, $CH_3OH$)

| Elemental analysis for $C_{30}H_{48}N_6O_5S \cdot 0.54\ H_2O$: | | | | |
|---|---|---|---|---|
| | C | H | N | S |
| **calc'd** | 57.30 | 8.12 | 13.37 | 5.10 |
| **found** | 57.58 | 7.78 | 13.09 | 4.84 |

Example I4

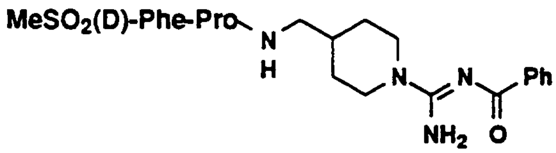

N-[[1-[Amino(benzoylimino)methyl]-4-piperidinyl]methyl]-1-[N-(methylsulfonyl)-D-phenylalanyl]-L-prolinamide

$[\alpha]_D$ = -55.5° (c=0.5, $CH_3OH$)

| Elemental analysis for $C_{29}H_{38}N_6O_5S \cdot 1.25\ H_2O \cdot 1.20\ CF_3COOH$: | | | | | |
|---|---|---|---|---|---|
| | C | H | N | S | F |
| calc'd | 50.82 | 5.66 | 11.33 | 4.32 | 9.22 |
| found | 50.75 | 5.53 | 11.28 | 4.10 | 9.01 |

Example I5

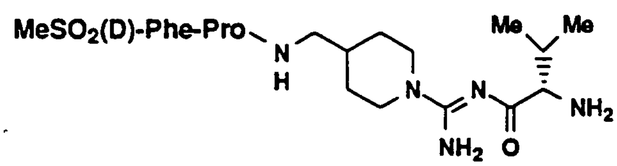

N-[[1-[Amino(L-valylimino)methyl]-4-piperidinyl]methyl]-1-[N-(methylsulfonyl)-D-phenylalanyl]-L-prolinamide

$[\alpha]_D$ = -51.5° (c=0.98, $CH_3OH$)

| Elemental analysis for $C_{27}H_{43}N_7O_5S \cdot 1.40\ H_2O \cdot 2.00\ CF_3COOH$: | | | | | |
|---|---|---|---|---|---|
| | C | H | N | S | F |
| calc'd | 44.81 | 5.80 | 11.80 | 3.86 | 13.72 |
| found | 44.80 | 5.81 | 11.79 | 3.98 | 13.39 |

Example I6

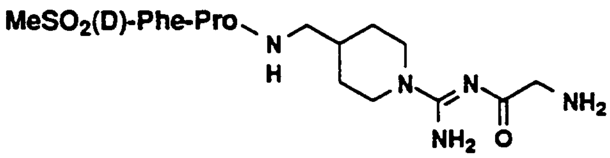

N-[[1-[Amino[(aminoacetyl)imino]methyl]-4-piperidinyl]methyl]-1-[N-(methylsulfonyl)-D-phenylalanyl]-L-prolinamide

$[\alpha]_D$ = -50.9° (c=0.70, $CH_3OH$)

| Elemental analysis for $C_{24}H_{37}N_7O_5S \cdot 1.90H_2O \cdot 2.00\ CF_3COOH$: | | | | | |
|---|---|---|---|---|---|
| | C | H | N | S | F |
| calc'd | 42.15 | 5.41 | 12.29 | 4.02 | 14.29 |
| found | 42.09 | 5.38 | 12.19 | 4.45 | 14.57 |

Example I7

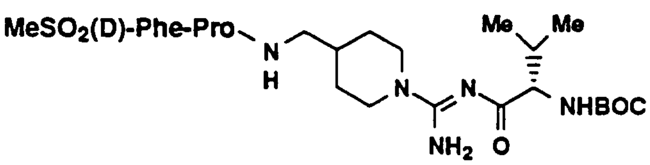

N-[[1-[Amino[[N-[[(1,1-dimethylethoxy)carbonyl]amino]-L-valyl]imino]methyl]-4-piperidinyl]methyl]-1-N-(methylsulfonyl)-D-phenylalanyl]-L-prolinamide

$[\alpha]_D$ = -76.0° (c=0.60, $CH_3OH$)

| Elemental analysis for $C_{32}H_{51}N_7O_7S \cdot 1.57\ H_2O$: | | | | |
|---|---|---|---|---|
| | C | H | N | S |
| calc'd | 54.43 | 7.73 | 13.89 | 4.54 |
| found | 54.29 | 7.47 | 13.81 | 4.39 |

Example I8

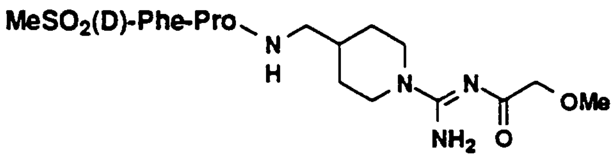

N-[[1-[Amino[(methoxyacetyl)imino]methyl]-4-piperidinyl]methyl]-1-N-(methylsulfonyl)-D-phenylalanyl]-L-prolinamide

$[\alpha]_D$ = -51.9° (c=0.54, $CH_3OH$)

| Elemental analysis for $C_{25}H_{38}N_6O_6S \cdot 0.25$ $H_2O \cdot 1.75$ $CF_3COOH$: | | | | | |
|---|---|---|---|---|---|
| | **C** | **H** | **N** | **S** | **F** |
| **calc'd** | 46.23 | 5.93 | 11.81 | 4.50 | 9.61 |
| **found** | 46.49 | 5.96 | 11.54 | 4.41 | 9.61 |

Example I9

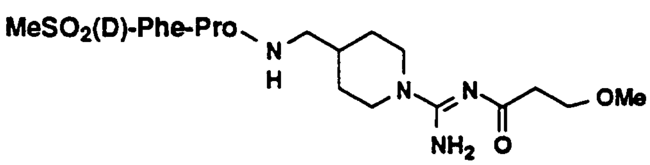

N-[[1-[Amino[(3-methoxy-1-oxopropyl)imino]methyl]-4-piperidinyl]methyl]-1-[N-(methylsulfonyl)-D-phenylalanyl]-L-prolinamide

$[\alpha]_D$ = -58.6° (c=0.81, $CH_3OH$)

| Elemental analysis for $C_{26}H_{40}N_6O_6S \cdot 1.65$ $H_2O \cdot 1.10$ $CF_3COOH$: | | | | | |
|---|---|---|---|---|---|
| | **C** | **H** | **N** | **S** | **F** |
| **calc'd** | 47.05 | 6.22 | 11.67 | 4.45 | 8.71 |
| **found** | 47.19 | 6.16 | 11.51 | 4.52 | 8.90 |

Example 20

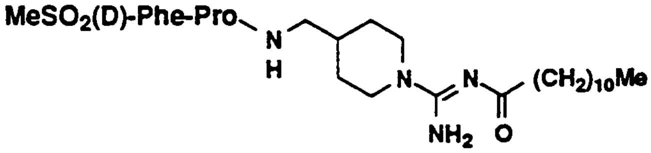

N-[[1-[Amino[(1-oxododecyl)imino]methyl]-4-piperidinyl]methyl]-1-[N-(methylsulfonyl)-D-phenylalanyl]-L-prolinamide

$[\alpha]_D$ = -46.2° (c=0.5, $CH_3OH$)

| Elemental analysis for $C_{34}H_{56}N_6O_5S \cdot 0.50$ $H_2O \cdot 1.7$ $CF_3COOH$: | | | | | |
|---|---|---|---|---|---|
| | C | H | N | S | F |
| calc'd | 52.12 | 6.84 | 9.75 | 3.72 | 11.24 |
| found | 52.11 | 6.95 | 9.73 | 3.68 | 11.11 |

Example 21

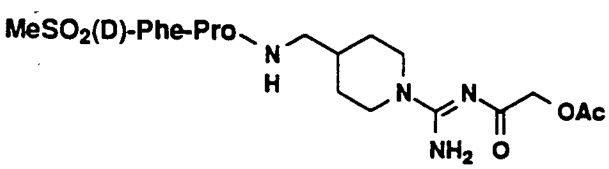

N-[[1-[[[(Acetyloxy)acetyl]imino]aminomethyl]-4-piperidinyl]methyl]-1-[N-(methylsulfonyl)-D-phenylalanyl]-L-prolinamide

$[\alpha]_D$ = -70.6° (c=0.34, $CH_3OH$)

| Elemental analysis for $C_{26}H_{38}N_6O_7S \cdot 1.23$ $H_2O$: | | | | |
|---|---|---|---|---|
| | C | H | N | S |
| calc'd | 51.97 | 6.79 | 13.99 | 5.34 |
| found | 52.09 | 6.69 | 13.87 | 5.35 |

Example 22

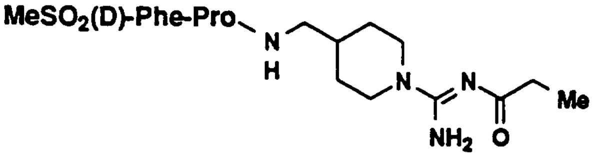

N-[[1-[Amino[(1-oxopropyl)imino]methyl]-4-piperidinyl]methyl]-1-[N-(methylsulfonyl)-D-phenylalanyl]-L-prolinamide

$[\alpha]_D$ = -61.5° (c=1.04, $CH_3OH$)

| Elemental analysis for $C_{25}H_{38}N_6O_5S \cdot 1.51$ $H_2O \cdot 1$ $CF_3COOH$: | | | | | |
|---|---|---|---|---|---|
| | C | H | N | S | F |
| calc'd | 47.98 | 6.27 | 12.43 | 8.43 | 4.74 |
| found | 48.07 | 6.41 | 12.34 | 8.88 | 4.70 |

Example 23

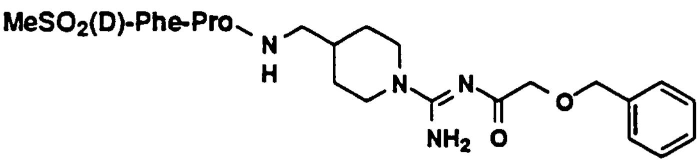

N-[[1-[Amino[[(phenylmethoxy)acetyl]imino]methyl]-4-piperidinyl]methyl]-1-[N-(methylsulfonyl)-D-phenylalanyl]-L-prolinamide

$[\alpha]_D$ = -46.3° (c=1.00, $CH_3OH$)

| Elemental analysis for $C_{31}H_{42}N_6O_6S \cdot 0.65$ $H_2O \cdot 1.40$ $CF_3COOH$: | | | | | |
|---|---|---|---|---|---|
| | C | H | N | S | F |
| calc'd | 50.87 | 5.65 | 10.53 | 4.02 | 10.00 |
| found | 50.88 | 5.59 | 10.51 | 4.13 | 9.90 |

Example 24

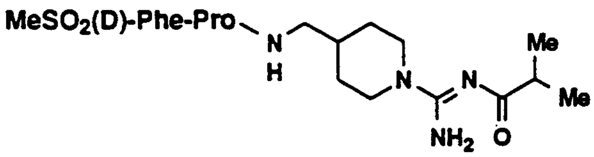

N-[[1-[Amino[(2-methyl-1-oxopropyl)imino]methyl]-4-piperidinyl]methyl]-1-[N-(methylsulfonyl)-D-phenylalanyl]-L-prolinamide

$[\alpha]_D$ = -58.0° (c=1.27, $CH_3OH$)

| Elemental analysis for $C_{26}H_{40}N_6O_5S \cdot 0.70\ H_2O \cdot 1.50\ CF_3COOH$: | | | | | |
|---|---|---|---|---|---|
| | C | H | N | S | F |
| calc'd | 47.56 | 5.90 | 11.48 | 4.38 | 11.67 |
| found | 47.67 | 5.82 | 11.33 | 4.47 | 11.57 |

Example 25

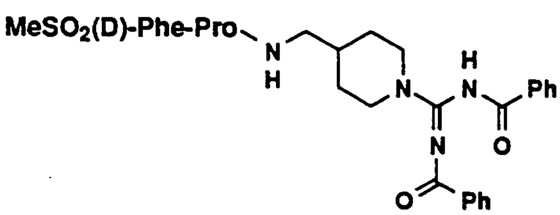

N-[[1-[(Benzoylamino)(benzoylimino)methyl]-4-piperidinyl]methyl]-1-[N-(methylsulfonyl)-D-phenylalanyl]-L-prolinamide

$[\alpha]_D$ = -59.4° (c=0.5, $CH_3OH$)

| Elemental analysis for $C_{36}H_{42}N_6O_6S \cdot 1.26\ H_2O$: | | | | |
|---|---|---|---|---|
| | C | H | N | S |
| calc'd | 60.94 | 6.32 | 11.85 | 4.52 |
| found | 60.83 | 6.23 | 11.96 | 4.19 |

Example 26

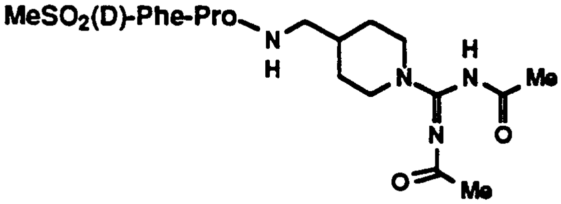

N-[[1-[(Acetylamino)(acetylimino)methyl]-4-piperidinyl]methyl]-1-[N-(methylsulfonyl)-D-phenylalanyl]-L-prolinamide

$[\alpha]_D$ = -70.4° (c=0.5, $CH_3OH$)

| Elemental analysis for $C_{26}H_{37}N_6O_6S \cdot 1.89\ H_2O \cdot 0.07\ CH_2Cl_2 \cdot 0.23\ CF_3COOH$: | | | | |
|---|---|---|---|---|
| | C | H | N | S |
| calc'd | 50.75 | 6.61 | 13.38 | 5.11 |
| found | 50.75 | 6.35 | 13.22 | 4.72 |

Example 27

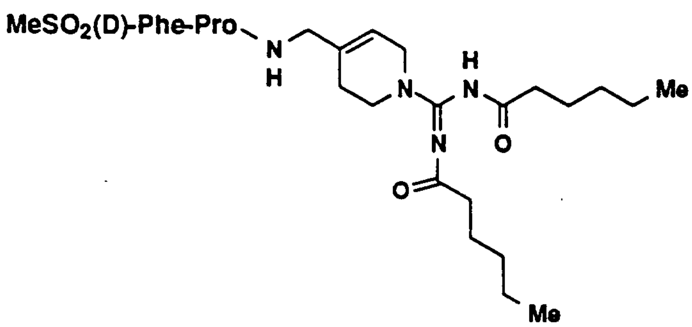

1-[N-(Methylsulfonyl)-D-phenylalanyl]-N-[[1,2,3,6-tetrahydro-1-[[(1-oxohexyl)amino][(1-oxohexyl)imino]methyl]-4-pyridinyl]methyl]-L-prolinamide

$[\alpha]_D$ = -53.6° (c=0.5, $CH_3OH$)

| Elemental analysis for $C_{34}H_{52}N_6O_6S \cdot 0.54\ H_2O$: | | | | |
|---|---|---|---|---|
| | C | H | N | S |
| calc'd | 59.82 | 7.84 | 12.31 | 4.70 |
| found | 59.77 | 7.76 | 12.37 | 4.64 |

Example 28

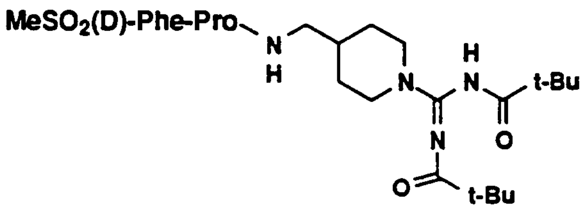

N-[[1-[[(2,2-Dimethyl-1-oxopropyl)amino][(2,2-dimethyl-1-oxopropyl)imino]methyl]-4-piperidinyl]methyl]-1-[N-(methylsulfonyl)-D-phenylalanyl]-L-prolinamide

$[\alpha]_D$ = -56.8° (c=0.5, $CH_3OH$)

| Elemental analysis for $C_{32}H_{50}N_6O_6S \cdot 0.60\ H_2O \cdot 0.50\ C_3H_7NO$: | | | | |
|---|---|---|---|---|
| | **C** | **H** | **N** | **S** |
| **calc'd** | 57.96 | 7.94 | 13.11 | 4.62 |
| **found** | 58.15 | 7.85 | 13.34 | 4.57 |

Example 29

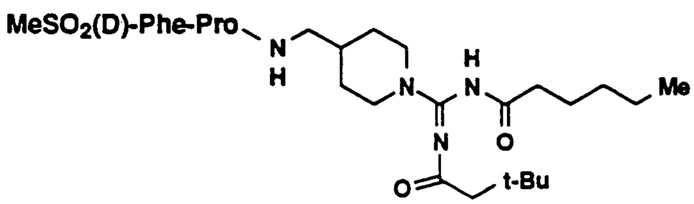

N-[[1-[[(3,3-Dimethyl-1-oxobutyl)imino][(1-oxohexyl)amino]methyl]-4-piperidinyl]methyl]-1-[N-(methylsulfonyl)-D-phenylalanyl]-L-prolinamide

$[\alpha]_D$ = -70.0° (c=0.28, $CH_3OH$)

| Elemental analysis for $C_{34}H_{54}N_6O_6S \cdot 0.69\ H_2O$: | | | | |
|---|---|---|---|---|
| | **C** | **H** | **N** | **S** |
| **calc'd** | 59.42 | 8.12 | 12.23 | 4.66 |
| **found** | 59.27 | 8.08 | 12.38 | 4.38 |

Example 30

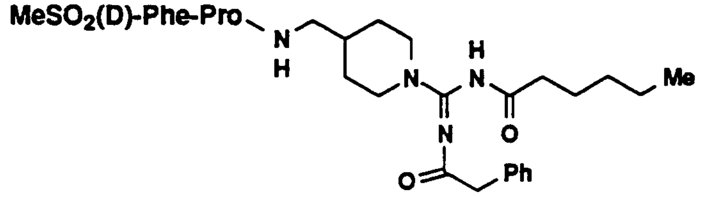

N-[[1-[[(1-oxohexyl)amino][(phenylacetyl)imino]methyl]-4-piperidinyl]methyl]-1-[N-(methylsulfonyl)-D-phenylalanyl]-L-prolinamide

$[\alpha]_D$ = -67.2° (c=0.25, $CH_3OH$)

| Elemental analysis for $C_{36}H_{50}N_6O_6S \cdot 0.64\ H_2O$: | | | | |
|---|---|---|---|---|
| | C | H | N | S |
| calc'd | 61.20 | 7.32 | 11.90 | 4.54 |
| found | 61.25 | 7.40 | 11.85 | 4.40 |

Example 31

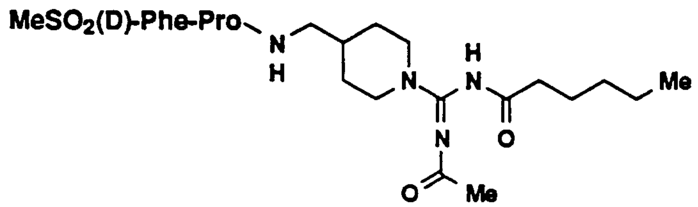

N-[[1-[[(Acetylimino)[(1-oxohexyl)amino]methyl]-4-piperidinyl]methyl]-1-[N-(methylsulfonyl)-D-phenylalanyl]-L-prolinamide

$[\alpha]_D$ = -67.7° (c=0.26, $CH_3OH$)

| Elemental analysis for $C_{30}H_{46}N_6O_6S \cdot 2.02\ H_2O$: | | | | |
|---|---|---|---|---|
| | C | H | N | S |
| calc'd | 55.00 | 7.70 | 12.83 | 4.89 |
| found | 55.00 | 7.26 | 12.74 | 5.03 |

Example 32

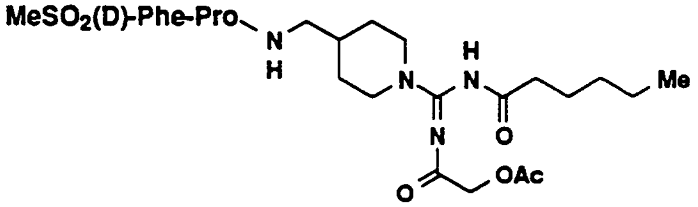

N-[[1-[[[(Acetyloxy)acetyl]imino][(1-oxohexyl)amino]methyl]-4-piperidinyl]methyl]-1-[N-(methylsulfonyl)-D-phenylalanyl]-L-prolinamide

$[\alpha]_D$ = -58.4° (c=0.5, $CH_3OH$)

| Elemental analysis for $C_{32}H_{48}N_6O_8S \cdot 0.93\ H_2O$: | | | | |
|---|---|---|---|---|
| | C | H | N | S |
| calc'd | 55.41 | 7.25 | 12.12 | 4.74 |
| found | 55.39 | 7.09 | 12.14 | 4.62 |

Example 33

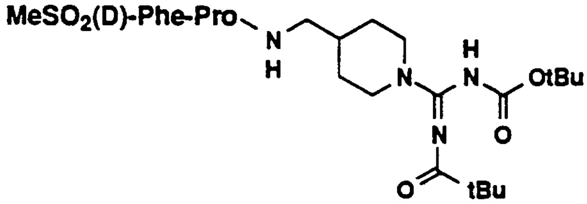

N-[[1-[[(1,1-Dimethylethoxy)carbonyl]amino][(1-oxo-2,2-dimethylpropyl)imino]methyl]-4-piperidinyl]methyl]-1-[N-(methylsulfonyl)-D-phenylalanyl]-L-prolinamide

$[\alpha]_D$ = -70.2° (c=0.5, $CH_3OH$)

| Elemental analysis for $C_{32}H_{50}N_6O_7S \cdot 1.30$ DMF: | | | | |
|---|---|---|---|---|
| | C | H | N | S |
| calc'd | 56.90 | 7.86 | 13.49 | 4.23 |
| found | 56.77 | 7.56 | 13.43 | 4.44 |

Example 34

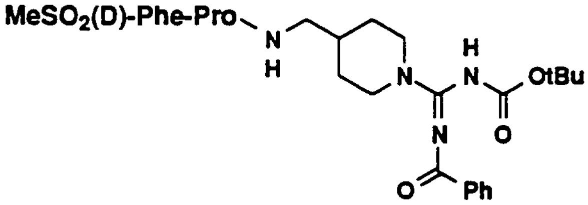

N-[[1-[(Benzoylimino)[[(1,1-dimethylethoxy)carbonyl]amino]methyl]-4-piperidinyl]methyl]-1-[N-(methylsulfonyl)-D-phenylalanyl]-L-prolinamide

$[\alpha]_D$ = -58.0° (c=0.5, $CH_3OH$)

| Elemental analysis for $C_{34}H_{46}N_6O_7S \cdot 0.71\ H_2O \cdot 0.23\ CH_2Cl_2$: | | | | | |
|---|---|---|---|---|---|
| | C | H | N | S | Cl |
| calc'd | 57.49 | 6.75 | 11.75 | 4.48 | 2.28 |
| found | 57.49 | 6.54 | 12.15 | 4.29 | 2.30 |

Example 35

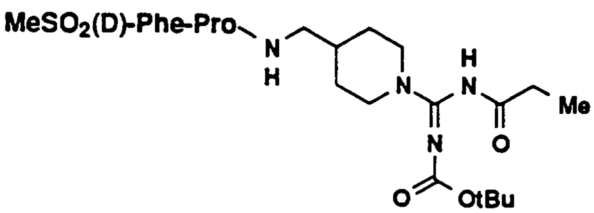

N-[[1-[[[(1,1-Dimethylethoxy)carbonyl]imino][(1-oxopropyl)amino]methyl]-4-piperidinyl]methyl]-1-[N-(methylsulfonyl)-D-phenylalanyl]-L-prolinamide

$[\alpha]_D$ = -65.1° (c=1.13, $CH_3OH$)

| Elemental analysis for $C_{30}H_{46}N_6O_7S \cdot 1.16\ H_2O$: | | | | |
|---|---|---|---|---|
| | C | H | N | S |
| calc'd | 54.95 | 7.43 | 12.82 | 4.89 |
| found | 55.05 | 7.46 | 12.37 | 4.71 |

Example 36

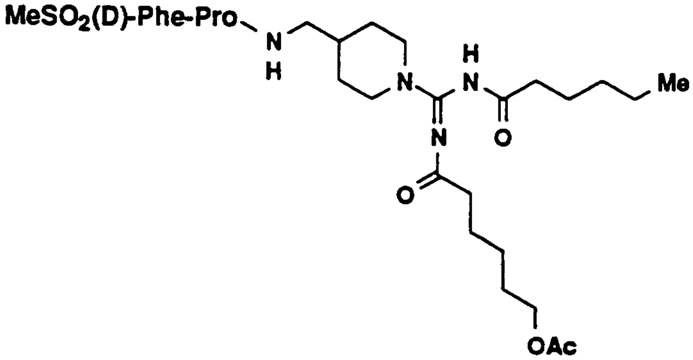

N-[[1-[[[6-(Acetyloxy)-1-oxohexyl]imino][(1-oxohexyl)amino]methyl]-4-piperidinyl]methyl]-1-[N-(methylsulfonyl)-D-phenylalanyl]-L-prolinamide

$[\alpha]_D$ = -71.9° (c=0.245, $CH_3OH$)

| Elemental analysis for $C_{36}H_{56}N_6O_8S \cdot 0.16\ H_2O$: | | | | |
|---|---|---|---|---|
| | C | H | N | S |
| calc'd | 58.76 | 7.71 | 11.42 | 4.36 |
| found | 58.44 | 7.88 | 11.42 | 5.34 |

Example 37

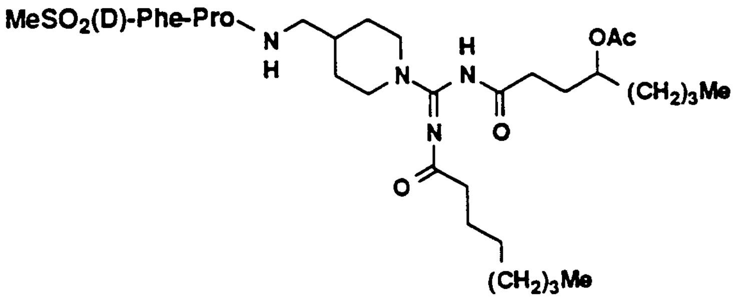

N-[[1-[[[(4-Acetyloxy-1-oxooctyl)amino][(1-oxooctyl)imino]methyl]-4-piperidinyl]methyl]-1-[N-(methylsulfonyl)-D-phenylalanyl]-L-prolinamide

$[\alpha]_D$ = -58.1° (c=0.215, $CH_3OH$)

| Elemental analysis for $C_{40}H_{64}N_6O_8S$: | | | | |
|---|---|---|---|---|
| | C | H | N | S |
| calc'd | 60.89 | 8.18 | 10.65 | 4.06 |
| found | 60.74 | 8.26 | 10.53 | 3.95 |

Example 38

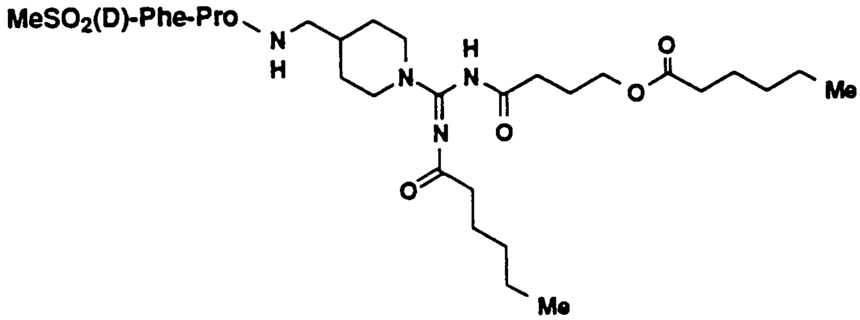

1-[N-(Methylsulfonyl)-D-phenylalanyl]-N-[[1-[[(1-oxohexyl)imino][[1-oxo-4-(1-oxohexyloxy)butyl]amino]methyl]-4-piperidinyl]methyl-L-prolinamide

$[\alpha]_D$ = -60.9° (c=0.45, $CH_3OH$)

| Elemental analysis for $C_{38}H_{60}N_6O_8S$: | | | | |
|---|---|---|---|---|
| | C | H | N | S |
| calc'd | 59.98 | 7.95 | 11.04 | 4.21 |
| found | 60.04 | 8.12 | 10.90 | 4.55 |

Example 39

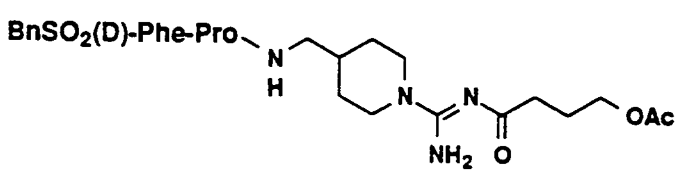

N-[[1-[[[4-(Acetyloxy)-1-oxobutyl]imino]aminomethyl]-4-piperidinyl]methyl]-1-[N-(benzylsulfonyl)-D-phenylalanyl]-L-prolinamide

$[\alpha]_D$ = -44.5° (c=0.5, $CH_3OH$)

| Elemental analysis for $C_{34}H_{46}N_6O_7S \cdot 1.07\ H_2O$: | | | | |
|---|---|---|---|---|
| | C | H | N | S |
| calc'd | 58.17 | 6.91 | 11.97 | 4.57 |
| found | 58.21 | 6.78 | 11.93 | 4.42 |

Example 40

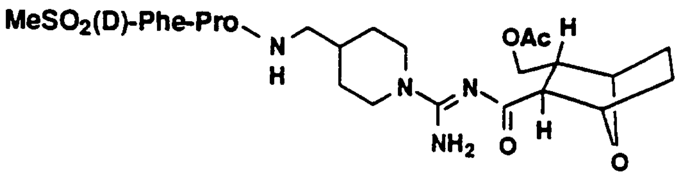

(*exo,exo*)-N-[[1-[[[[3-[(Acetyloxy)methyl]-7-oxabicyclo[2.2.1]hept-2-yl]carbonyl]amino]iminomethyl]-4-piperidinyl]methyl]-1-[N-(methylsulfonyl)-D-phenylalanyl]-L-prolinamide

$[\alpha]_D$ = -60.3° (c=0.67, $CH_3OH$)

| Elemental analysis for $C_{32}H_{46}N_6O_8S \cdot 0.69\ H_2O$: | | | | |
|---|---|---|---|---|
| | C | H | N | S |
| calc'd | 55.92 | 6.95 | 12.23 | 4.66 |
| found | 56.15 | 7.03 | 12.00 | 4.59 |

Example 41

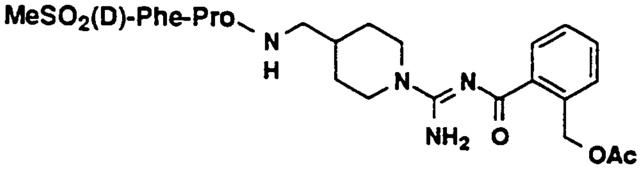

N-[[1-[[[2-[(Acetyloxy)methyl]benzoyl]imino]aminomethyl]-4-piperidinyl]methyl]-1-[N-(methylsulfonyl)-D-phenylalanyl]-L-prolinamide

$[\alpha]_D$ = -54.8° (c=0.34, $CH_3OH$)

| Elemental analysis for $C_{32}H_{42}N_6O_7S \cdot 0.50\ H_2O \cdot 1.35\ CF_3COOH$: | | | | | |
|---|---|---|---|---|---|
| | **C** | **H** | **N** | **S** | **F** |
| **calc'd** | 51.18 | 5.50 | 10.47 | 3.99 | 9.23 |
| **found** | 51.04 | 5.48 | 10.33 | 3.98 | 9.34 |

Example 42

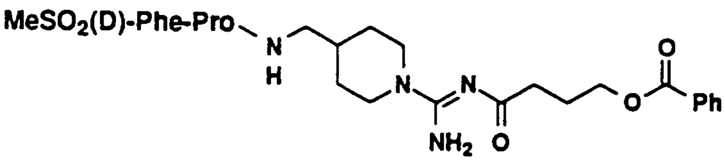

N-[[1-[Amino[[4-(benzoyloxy)-1-oxobutyl]imino]methyl]-4-piperidinyl]methyl]-1-[N-(methylsulfonyl)-D-phenylalanyl]-L-prolinamide

$[\alpha]_D$ = -64.8° (c=0.614, $CH_3OH$)

| Elemental analysis for $C_{33}H_{44}N_6O_7S \cdot 0.40\ H_2O \cdot 0.20$ hexane: | | | | |
|---|---|---|---|---|
| | **C** | **H** | **N** | **S** |
| **calc'd** | 59.25 | 6.92 | 12.12 | 4.62 |
| **found** | 59.19 | 6.92 | 11.96 | 4.65 |

Example 43

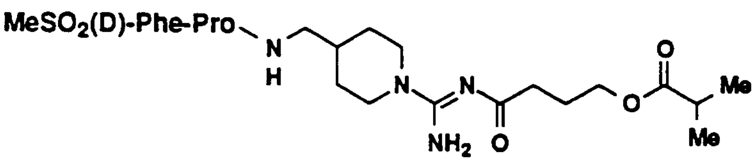

N-[[1-[Amino[[4-(2-methyl-1-oxopropoxy)-1-oxobutyl]imino]methyl]-4-piperidinyl]methyl]-1-[N-(methylsulfonyl)-D-phenylalanyl]-L-prolinamide

$[\alpha]_D$ = -73° (c=0.13, $CH_3OH$)

| Elemental analysis for $C_{30}H_{46}N_6O_7S \cdot 0.53\ H_2O$: | | | | |
|---|---|---|---|---|
| | **C** | **H** | **N** | **S** |
| **calc'd** | 55.93 | 7.36 | 13.04 | 4.98 |
| **found** | 56.13 | 7.42 | 12.84 | 4.88 |

Example 44

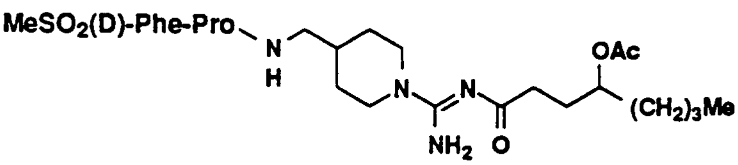

N-[[1-[[[4-(Acetyloxy)-1-oxooctyl]imino]aminomethyl]-4-piperidinyl]methyl]-1-[N-(methylsulfonyl)-D-phenylalanyl]-L-prolinamide

$[\alpha]_D$ = 43.0° (c=0.34, $CH_3OH$)

| Elemental analysis for $C_{32}H_{50}N_6O_7S \cdot 1.09\ H_2O \cdot 1.40\ CF_3COOH$: | | | | | |
|---|---|---|---|---|---|
| | **C** | **H** | **N** | **S** | **F** |
| **calc'd** | 49.60 | 6.41 | 9.97 | 3.80 | 9.53 |
| **found** | 49.60 | 6.23 | 9.67 | 3.63 | 9.53 |

Example 45

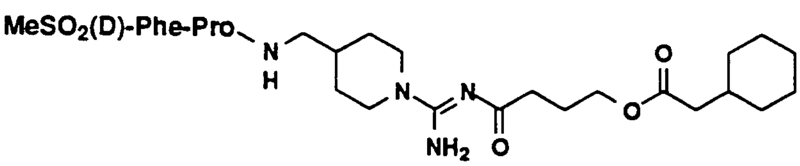

N-[[1-[Amino[[4-[(cyclohexylacetyl)oxy]-1-oxobutyl]imino]methyl]-4-piperidinyl]methyl]-1-[N-(methylsulfonyl)-D-phenylalanyl]-L-prolinamide

$[\alpha]_D$ = -60.9° (c=1.07, $CH_3OH$)

| Elemental analysis for $C_{34}H_{52}N_6O_7S \cdot 2.30\ H_2O \cdot 0.17$ hexane: | | | | |
|---|---|---|---|---|
| | C | H | N | S |
| calc'd | 56.46 | 7.98 | 11.28 | 4.30 |
| found | 56.74 | 7.64 | 10.93 | 4.27 |

Example 46

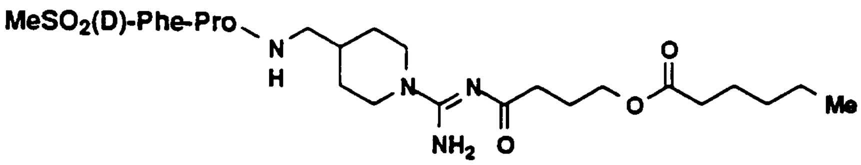

N-[[1-[Amino[[1-oxo-4-(1-oxohexyloxy)butyl]imino]methyl]-4-piperidinyl]methyl]-1-[N-(methylsulfonyl)-D-phenylalanyl]-L-prolinamide

$[\alpha]_D$ = -63° (c=0.58, $CH_3OH$)

| Elemental analysis for $C_{32}H_{50}N_6O_7S \cdot 1.19\ H_2O \cdot 0.13$ hexane: | | | | |
|---|---|---|---|---|
| | C | H | N | S |
| calc'd | 56.61 | 7.86 | 12.08 | 4.61 |
| found | 56.61 | 7.68 | 11.89 | 4.50 |

Example 47

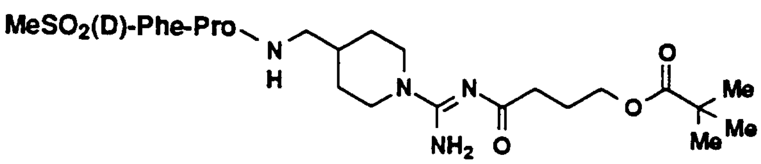

N-[[1-[Amino[[4-(2,2-dimethyl-1-oxopropoxy)-1-oxobutyl]imino]methyl]-4-piperidinyl]methyl]-1-[N-(methylsulfonyl)-D-phenylalanyl]-L-prolinamide

$[\alpha]_D$ = -75° (c=0.29, $CH_3OH$)

| Elemental analysis for $C_{31}H_{48}N_6O_7S \cdot 0.50\ H_2O \cdot 0.1$ hexane: | | | | |
|---|---|---|---|---|
| | **C** | **H** | **N** | **S** |
| **calc'd** | 56.89 | 7.63 | 12.60 | 4.81 |
| **found** | 56.89 | 7.68 | 12.39 | 4.84 |

Example 48

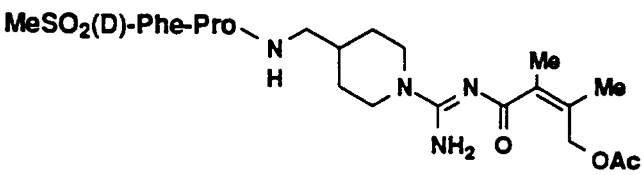

(2Z)-N-[[1-[[[4-(Acetyloxy)-2,3-dimethyl-1-oxo-2-butenyl]imino]aminomethyl]-4-piperidinyl]methyl]-1-[N-(methylsulfonyl)-D-phenylalanyl]-L-prolinamide

$[\alpha]_D$ = 47.2° (c=0.34, $CH_3OH$)

| Elemental analysis for $C_{30}H_{44}N_6O_6S \cdot 0.97\ H_2O \cdot 2.0\ CF_3COOH$: | | | | | |
|---|---|---|---|---|---|
| | **C** | **H** | **N** | **S** | **F** |
| **calc'd** | 46.50 | 5.50 | 9.57 | 3.66 | 12.98 |
| **found** | 46.56 | 5.43 | 9.51 | 3.80 | 12.71 |

Example 49

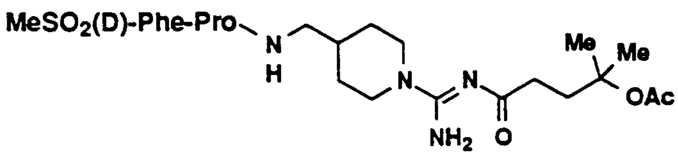

N-[[1-[[[4-(Acetyloxy)-4-methyl-1-oxopentyl]imino]aminomethyl]-4-piperidinyl]methyl]-1-[N-(methylsulfonyl)-D-phenylalanyl]-L-prolinamide

$[\alpha]_D$ = -64.9° (c=0.66, $CH_3OH$)

| Elemental analysis for $C_{30}H_{46}N_6O_7S$: | | | | |
|---|---|---|---|---|
| | C | H | N | S |
| calc'd | 56.70 | 7.31 | 13.22 | 5.04 |
| found | 56.73 | 7.45 | 13.19 | 4.97 |

Example 50

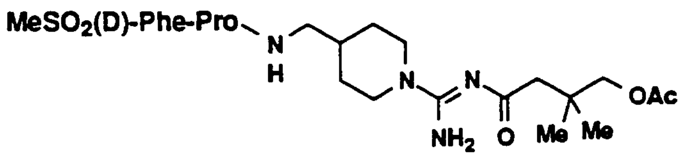

N-[[1-[[[(4-(Acetyloxy]-3,3-dimethyl-1-oxobutyl]imino]aminomethyl]-4-piperidinyl]methyl]-1-[N-(methylsulfonyl)-D-phenylalanyl]-L-prolinamide

$[\alpha]_D$ = -56.2° (c=0.50, $CH_3OH$)

| Elemental analysis for $C_{30}H_{46}N_6O_7S \cdot 1.66\ H_2O \cdot 0.05\ CF_3COOH$: | | | | |
|---|---|---|---|---|
| | C | H | N | S |
| calc'd | 53.93 | 7.42 | 12.54 | 4.78 |
| found | 53.93 | 7.09 | 12.52 | 4.57 |

Example 51

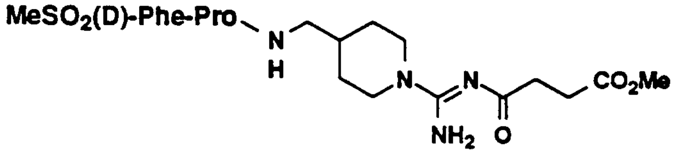

N-[[1-[Amino[(4-methoxy-1,4-dioxobutyl)imino]methyl]-4-piperidinyl]methyl]-1-[N-(methylsulfonyl)-D-phenylalanyl]-L-prolinamide

$[\alpha]_D$ = -68.6° (c=0.29, $CH_3OH$)

| Elemental analysis for $C_{27}H_{40}N_6O_7S \cdot 0.88\ H_2O \cdot 0.7\ CH_2Cl_2$: | | | | |
|---|---|---|---|---|
| | **C** | **H** | **N** | **S** |
| **calc'd** | 49.80 | 6.51 | 12.58 | 4.80 |
| **found** | 49.80 | 6.13 | 12.49 | 4.65 |

Example 52

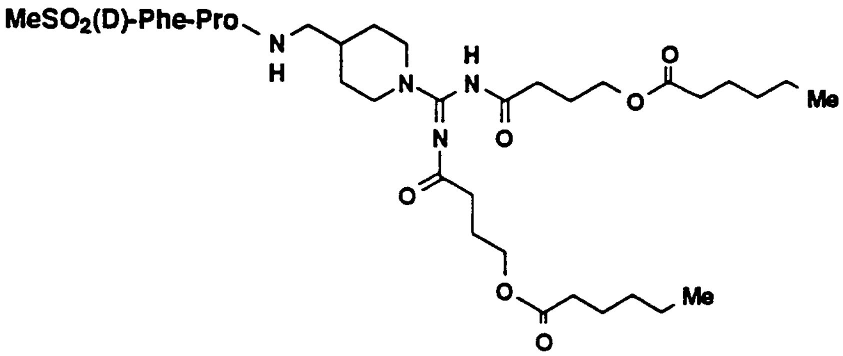

1-[N-(Methylsulfonyl)-D-phenylalanyl]-N-[[1-[[[1-oxo-4-(1-oxohexyloxy)butyl]amino][[1-oxo-4-(1-oxohexyloxy)butyl]imino]methyl]-4-piperidinyl]methyl]-L-prolinamide

$[\alpha]_D$ = -54.5° (c=0.314, $CH_3OH$)

| Elemental analysis for $C_{42}H_{66}N_6O_{10}S \cdot 1.06\ H_2O \cdot 0.45$ hexane: | | | | |
|---|---|---|---|---|
| | **C** | **H** | **N** | **S** |
| **calc'd** | 59.33 | 8.29 | 9.29 | 3.54 |
| **found** | 59.33 | 7.93 | 8.93 | 3.85 |

Example 53

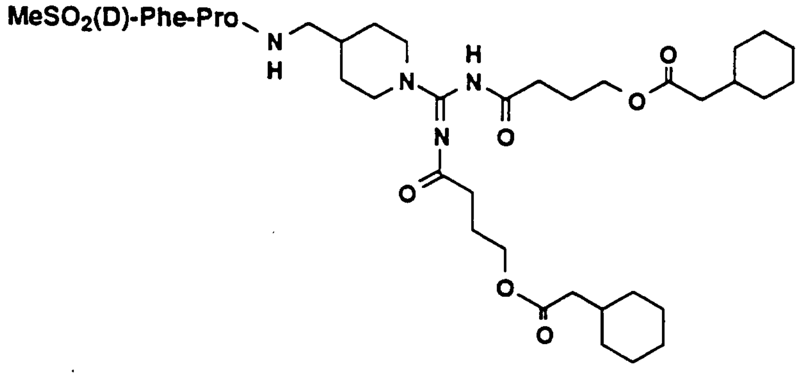

N-[[1-[[[4-[(Cyclohexylacetyl)oxy]-1-oxobutyl]amino]-[[4-[(cyclohexylacetyl)oxy]-1-oxobutyl]imino]methyl]-4-piperidinyl]methyl]-1-[N-(methylsulfonyl)-D-phenylalanyl]-L-prolinamide

$[\alpha]_D$ = -51.4° (c=0.58, $CH_3OH$)

| Elemental analysis for $C_{46}H_{70}N_6O_{10}S$: | | | | |
|---|---|---|---|---|
| | C | H | N | S |
| calc'd | 61.45 | 7.85 | 9.35 | 3.57 |
| found | 61.26 | 7.96 | 9.24 | 3.52 |

Example 54

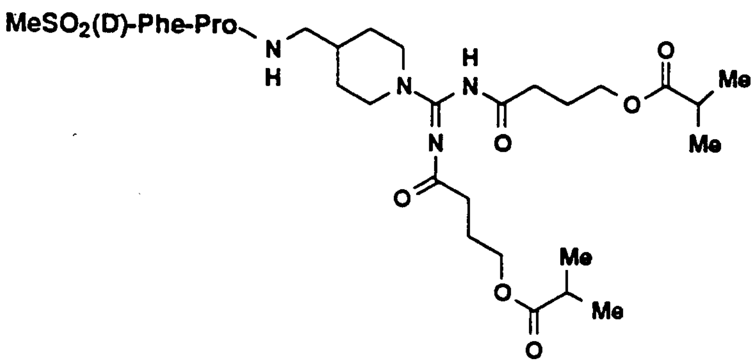

N-[[1-[[[4-(2-Methyl-1-oxopropoxy)-1-oxobutyl]amino]-[[4-(2-methyl-1-oxopropoxy)-1-oxobutyl]imino]methyl]-4-piperidinyl]methyl]-1-[N-(methylsulfonyl)-D-phenylalanyl]-L-prolinamide

$[\alpha]_D$ = -61° (c=0.26, $CH_3OH$)

| Elemental analysis for $C_{38}H_{58}N_6O_{10}S \cdot 0.43\ H_2O$: | | | | |
|---|---|---|---|---|
| | C | H | N | S |
| **calc'd** | 57.14 | 7.43 | 10.52 | 4.01 |
| **found** | 57.21 | 7.50 | 10.45 | 3.86 |

Example 55

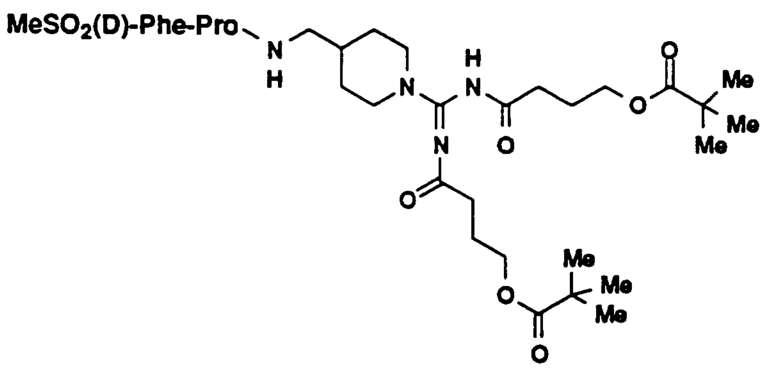

N-[[1-[[[4-(2,2-Dimethyl-1-oxopropory)-1-oxobutyl]amino][[4-(2,2-dimethyl-1-oxopropory)-1-oxobutyl]imino]methyl]-4-piperidinyl]methyl]-1-[N-(methylsulfonyl)-D-phenylalanyl]-L-prolinamide

$[\alpha]_D$ = -57° (c=0.24, $CH_3OH$)

| Elemental analysis for $C_{40}H_{62}N_6O_{10}S \cdot 0.21\ H_2O$: | | | | |
|---|---|---|---|---|
| | C | H | N | S |
| **calc'd** | 58.39 | 7.65 | 10.21 | 3.90 |
| **found** | 58.44 | 7.74 | 10.16 | 3.91 |

Example 56

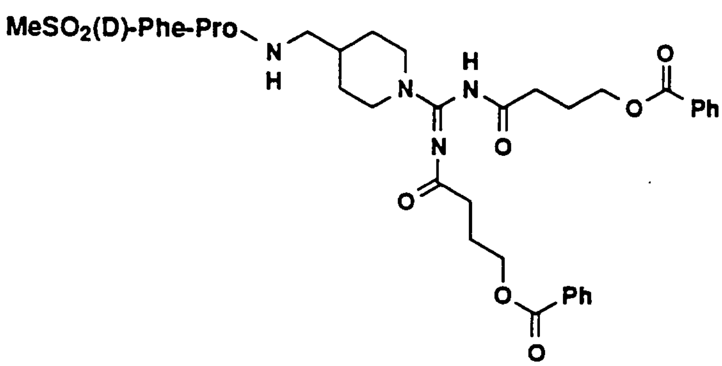

N-[[1-[[[4-(Benzoyloxy)-1-oxobutyl]amino][[4-(benzoyloxy)-1-oxobutyl]imino]methyl]-4-piperidinyl]methyl]-1-[N-(methylsulfonyl)-D-phenylalanyl]-L-prolinamide

$[\alpha]_D$ = -54° (c=0.26, $CH_3OH$)

| Elemental analysis for $C_{44}H_{54}N_6O_{10}S$ • 0.47 $H_2O$ • 0.18 hexane: | | | | |
|---|---|---|---|---|
| | C | H | N | S |
| calc'd | 61.32 | 6.56 | 9.52 | 3.63 |
| found | 61.32 | 6.42 | 9.38 | 3.55 |

Example 57

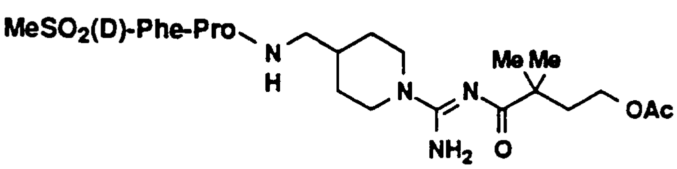

N-[[1-[[[4-(Acetyloxy)-2,2-dimethyl-1-oxobutyl]imino]aminomethyl]-4-piperidinyl]methyl]-1-[N-(methylsulfonyl)-D-phenylalanyl]-L-prolinamide

$[\alpha]_D$ = -61.4° (c=1.12, $CH_3OH$)

| Elemental analysis $C_{30}H_{46}N_6O_7S$ • 1.7 $H_2O$ • 0.5 hexane: | | | | |
|---|---|---|---|---|
| | **C** | **H** | **N** | **S** |
| **calc'd** | 55.94 | 8.02 | 11.86 | 4.53 |
| **found** | 56.28 | 7.61 | 11.53 | 4.57 |

Example 58

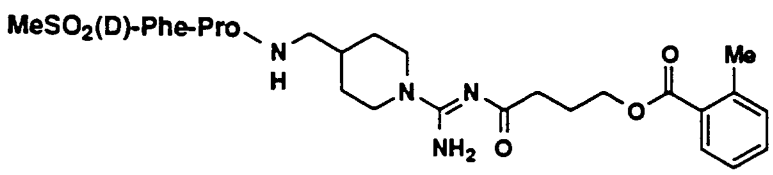

N-[[1-[Amino[[4-(2-methylbenzoyl)oxy]-1-oxobutyl]imino]methyl]-4-piperidinyl]methyl]-1-[N-(methylsulfonyl)-D-phenylalanyl]-L-prolinamide

$[\alpha]_D$ = -82° (c=0.18, $CH_3OH$)

| Elemental analysis for $C_{34}H_{46}N_6O_7S$ • 0.49 $H_2O$ • 0.24 hexane: | | | | |
|---|---|---|---|---|
| | **C** | **H** | **N** | **S** |
| **calc'd** | 59.75 | 7.12 | 11.80 | 3.63 |
| **found** | 59.75 | 7.05 | 11.73 | 4.50 |

Example 59

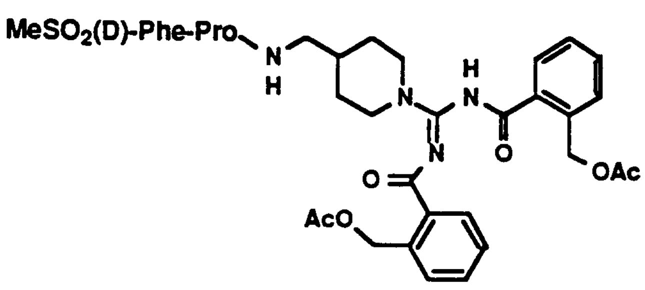

N-[[1-[[[2-[(Acetyloxy)methyl]benzoyl]amino][[2-[(acetyloxy)methyl]benzoyl]imino]methyl]-4-piperidinyl]methyl]-1-[N-(methylsulfonyl)-D-phenylalanyl]-L-prolinamide

$[\alpha]_D$ = -52.6° (c=0.34, $CH_3OH$)

| Elemental analysis for $C_{42}H_{51}N_6O_{10}S \cdot 0.68\ H_2O$: | | | | |
|---|---|---|---|---|
| | C | H | N | S |
| calc'd | 59.76 | 6.25 | 9.95 | 3.80 |
| found | 59.77 | 6.04 | 9.94 | 3.79 |

Example 60

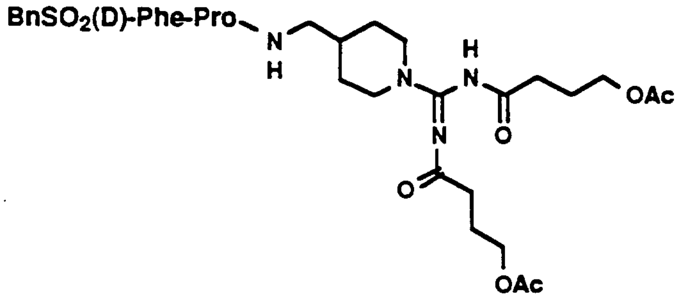

N-[[1-[[[4-(Acetyloxy)-1-oxobutyl]amino][[4-(acetyloxy)-1-oxobutyl]imino]methyl]-4-piperidinyl]methyl]-1-[N-(benzylsulfonyl)-D-phenylalanyl]-L-prolinamide

$[\alpha]_D$ = -38.7° (c=0.46, $CH_3OH$)

| Elemental analysis for $C_{40}H_{54}N_6O_{10}S \cdot 0.7\ H_2O$: | | | | |
|---|---|---|---|---|
| | C | H | N | S |
| calc'd | 58.34 | 6.78 | 10.20 | 3.89 |
| found | 58.56 | 6.72 | 9.98 | 3.53 |

Example 61

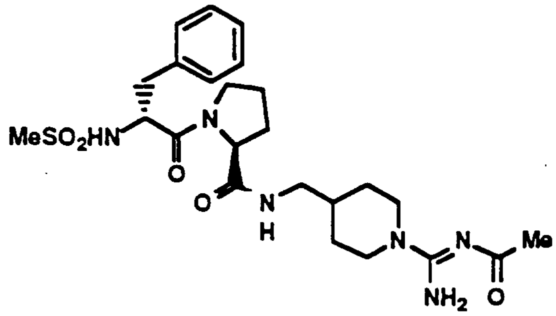

N-[[1-[(Acetylimino)]aminomethyl]-4-piperidinyl]methyl]-1-N-(methylsulfonyl)-D-phenylalanyl]-L-prolinamide

$[\alpha]_D$ = -85.0° (c=0.5, $CH_3OH$)

| Elemental analysis for $C_{24}H_{36}N_6O_5S$ • 1.02 $H_2O$: | | | | |
|---|---|---|---|---|
| | C | H | N | S |
| calc'd | 53.49 | 7.11 | 15.59 | 5.95 |
| found | 53.54 | 6.93 | 15.54 | 5.74 |

Example 62

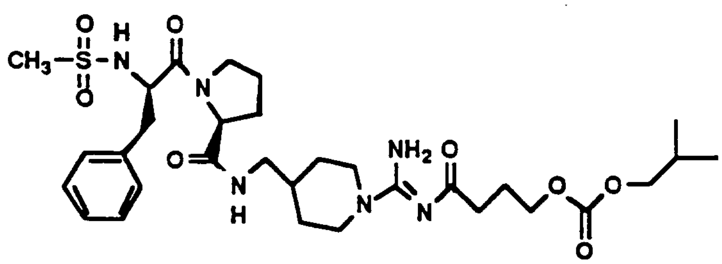

N-[[1-[Amino[[4-[[(2-methylpropoxy)carbonyl]oxy]-1-oxobutyl]imino]methyl]-4-piperidinyl]methyl]-1-[N-(methylsulfonyl)-D-phenylalanyl]-L-prolinamide

$[\alpha]_D$ = -60° (c=0.12, $CH_3OH$)

| Elemental analysis for $C_{31}H_{48}N_6O_8S$ • 0.57 $H_2O$: | | | | |
|---|---|---|---|---|
| | C | H | N | S |
| calc'd | 55.16 | 7.34 | 12.45 | 4.75 |
| found | 55.40 | 7.42 | 12.21 | 4.74 |

Example 63

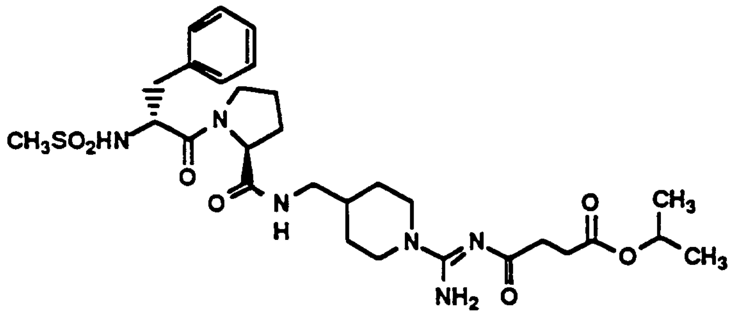

N-[[1-[Amino[[4-(1-methylethoxy)-1,4-dioxobutyl]imino]methyl]-4-piperidinyl]-methyl]-1-[N-(methylsulfonyl)-D-phenylalanyl]-L-prolinamide

$[\alpha]_D$ = -66° (c=0.50, $CH_3OH$)

| Elemental analysis for $C_{29}H_{44}N_6O_7S \cdot 0.56\ H_2O$: | | | | |
|---|---|---|---|---|
| | **C** | **H** | **N** | **S** |
| **calc'd** | 55.22 | 7.21 | 13.32 | 5.08 |
| **found** | 55.27 | 7.19 | 13.27 | 4.78 |

Example 64

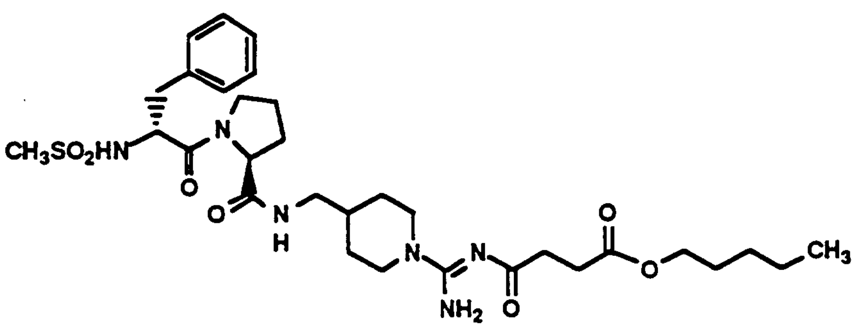

N-[[1-[Amino[[1,4-dioxo-4-(pentyloxy)butyl]imino]methyl]-4-piperidinyl]methyl]-1-[N-(methylsulfonyl)-D-phenylalanyl]-L-prolinamide

$[\alpha]_D$ = -64° (c=0.50, $CH_3OH$)

| Elemental analysis for $C_{31}H_{48}N_6O_7S$ • 1.43 $H_2O$: | | | | |
|---|---|---|---|---|
| | C | H | N | S |
| **calc'd** | 55.20 | 7.60 | 12.46 | 4.75 |
| **found** | 55.44 | 7.30 | 12.23 | 4.38 |

Example 65

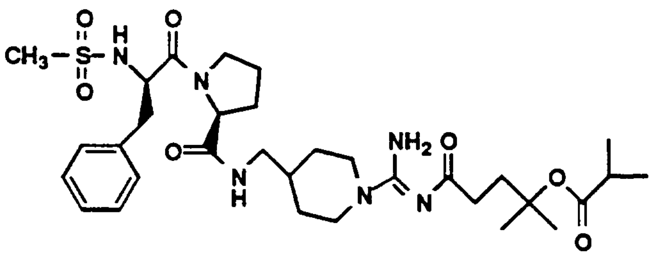

N-[[1-Amino[[[4-methyl-4-(2-methyl-1-oxopropoxy)-1-oxopentyl]imino]methyl]-4-piperidinyl]methyl]-1-[N-(methylsulfonyl)-D-phenylalanyl]-L-prolinamide

$[\alpha]_D$ = -76° (c=0.12, $CH_3OH$)

| Elemental analysis for $C_{32}H_{50}N_6O_7S$ • 0.46 $H_2O$: | | | | |
|---|---|---|---|---|
| | C | H | N | S |
| **calc'd** | 57.27 | 7.65 | 12.52 | 4.78 |
| **found** | 57.47 | 7.66 | 12.32 | 4.43 |

Example 66

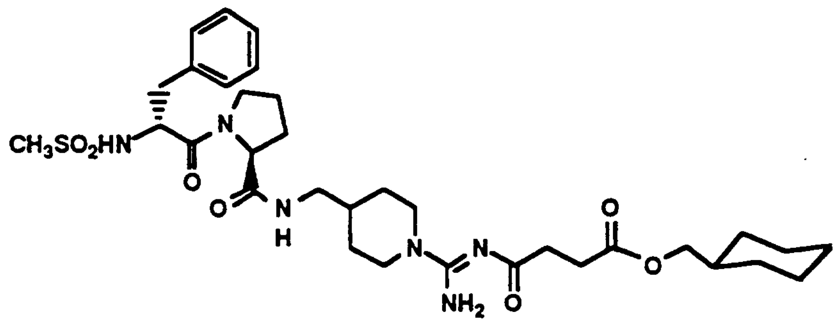

N-[[1-[Amino[[4-(cyclohexylmethoxy)-1,4-dioxobutyl]imino]methyl]-4-piperidinyl]methyl]-1-[N-(methylsulfonyl)-D-phenylalanyl]-L-porolinamide

$[\alpha]_D$ = -59° (c=0.50, $CH_3OH$)

| Elemental analysis for $C_{33}H_{50}N_6O_7S$ • 0.78 $H_2O$: | | | | |
|---|---|---|---|---|
| | C | H | N | S |
| calc'd | 57.54 | 7.54 | 12.20 | 4.65 |
| found | 57.66 | 7.28 | 12.08 | 4.57 |

Example 67

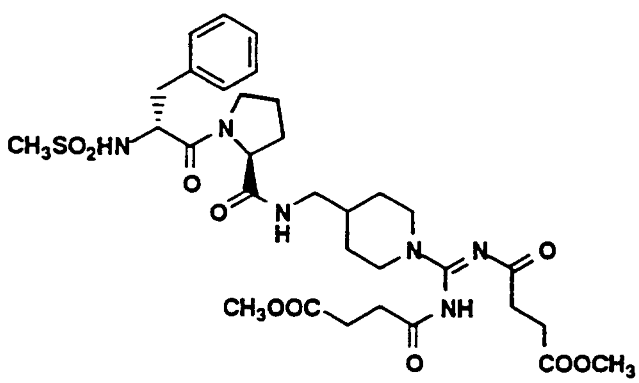

N-[[1-[[(4-Methoxy-1,4-dioxobutyl)amino][(4-methoxy-1,4-dioxobutyl)imino]-methyl]-4-piperidinyl]methyl]-1-[N-(methylsulfonyl)-D-phenylalanyl]-L-prolinamide

$[\alpha]_D$ = -65° (c=0.50, $CH_3OH$)

| Elemental analysis for $C_{32}H_{46}N_6O_{10}S$ • 0.30 $H_2O$: | | | | |
|---|---|---|---|---|
| | C | H | N | S |
| calc'd | 53.96 | 6.60 | 11.80 | 4.50 |
| found | 54.09 | 6.42 | 11.67 | 4.36 |

Example 68

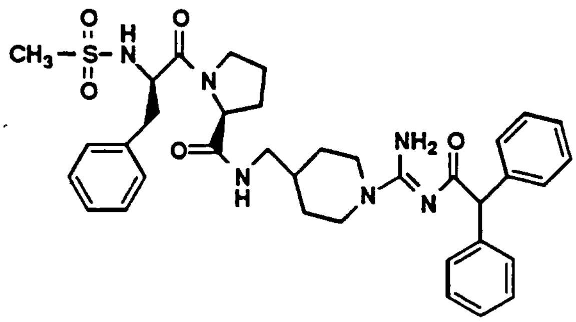

N-[[1-Amino[[(diphenylacetyl)imino]methyl]-4-piperidinyl]methyl]-1-[N-(methylsulfonyl)-D-phenylalanyl]-L-prolinamide

$[\alpha]_D$ = -74° (c=0.13, $CH_3OH$)

| Elemental analysis for $C_{36}H_{44}N_6O_7S$ • 0.70 $H_2O$ • 0.20 hexane: | | | | |
|---|---|---|---|---|
| | C | H | N | S |
| calc'd | 63.59 | 6.91 | 11.96 | 4.56 |
| found | 63.86 | 6.89 | 11.69 | 4.52 |

Example 69

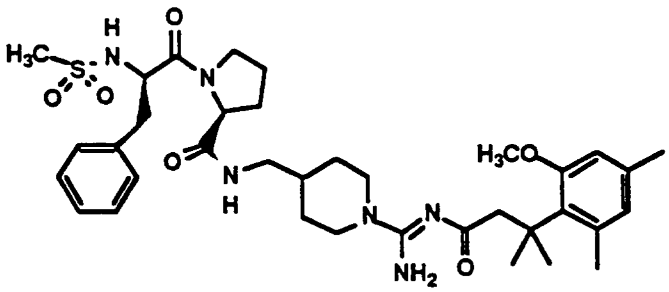

N-[[1-[Amino[[3-(2-methoxy-4,6-dimethylphenyl)-3-methyl-1-oxobutyl]-imino]methyl]-4-piperidinyl]methyl]-1-[N-(methylsulfonyl)-D-phenylalanyl]-L-prolinamide, trifluoroacetate

$[\alpha]_D$ = -50° (c=1.0, $CH_3OH$)

| Elemental analysis for $C_{36}H_{52}N_6O_6S$ • 0.33 $H_2O$ • 1.05 $CF_3COOH$: | | | | | |
|---|---|---|---|---|---|
| | C | H | N | S | F |
| calc'd | 55.63 | 6.58 | 10.22 | 3.90 | 7.28 |
| found | 55.70 | 6.62 | 10.14 | 3.95 | 7.30 |

Example 70

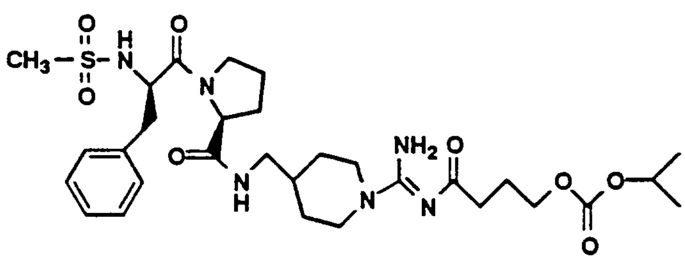

N-[[1-Amino[[4-[[(1-methylethoxy)carbonyl]oxy]-1-oxo-butyl]imino]methyl]-4-piperidinyl]methyl]-1-[N-(methylsulfonyl)-D-phenylalanyl]-L-prolinamide

$[\alpha]_D$ = -59° (c=0.25, $CH_3OH$)

| Elemental analysis for $C_{30}H_{46}N_6O_8S$ • 0.83 $H_2O$ • 0.32 hexane: | | | | |
|---|---|---|---|---|
| | **C** | **H** | **N** | **S** |
| **calc'd** | 55.30 | 7.58 | 12.12 | 4.62 |
| **found** | 55.30 | 7.34 | 11.88 | 4.52 |

Example 71

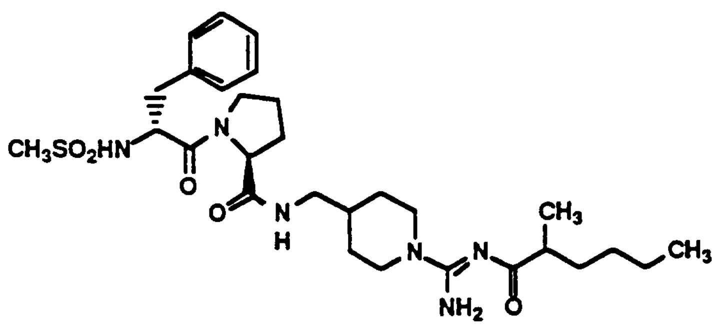

N-[[1-[Amino[(2-methyl-1-oxohexyl)imino]methyl]-4-piperidinyl]methyl]-1-[N-(methylsulfonyl)-D-phenylalanyl]-L-prolinamide

$[\alpha]_D$ = -72° (c=0.50, $CH_3OH$)

| Elemental analysis for $C_{29}H_{46}N_6O_5S$ • 0.47 $H_2O$: | | | | |
|---|---|---|---|---|
| | **C** | **H** | **N** | **S** |
| **calc'd** | 58.13 | 7.89 | 14.03 | 5.35 |
| **found** | 58.42 | 7.93 | 13.74 | 5.27 |

Example 72

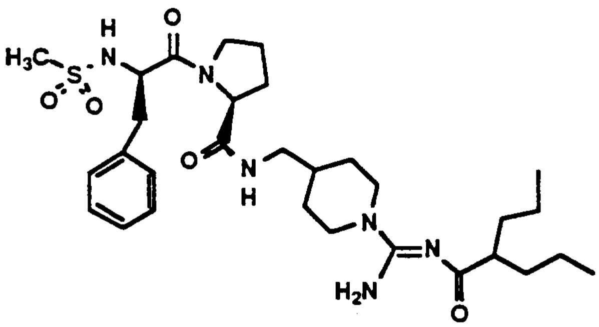

N-[[1-[Amino[(1-oxo-2-propylpentyl)imino]methyl]-4-piperidinyl]methyl]-1-[N-(methylsulfonyl)-D-phenylalanyl]-L-prolinamide

$[\alpha]_D$ = -66° (c=0.76, $CH_3OH$)

| Elemental analysis for $C_{30}H_{48}N_6O_5S$: | | | | |
|---|---|---|---|---|
| | C | H | N | S |
| calc'd | 59.58 | 8.00 | 13.90 | 5.30 |
| found | 59.73 | 8.16 | 14.11 | 4.97 |

Example 73

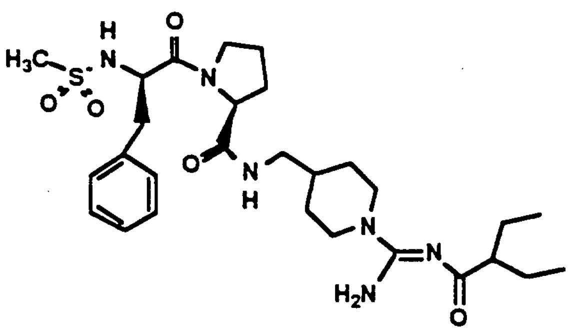

N-[[1-[Amino[(2-ethyl-1-oxobutyl)imino]methyl]-4-piperidinyl]methyl]-1-[N-(methylsulfonyl)-D-phenylalanyl]-L-prolinamide

$[\alpha]_D$ = -72° (c=0.94, $CH_3OH$)

| Elemental analysis for $C_{28}H_{44}N_6O_5S$: | | | | |
|---|---|---|---|---|
| | C | H | N | S |
| **calc'd** | 58.31 | 7.69 | 14.57 | 5.56 |
| **found** | 57.94 | 7.75 | 14.64 | 5.53 |

Example 74

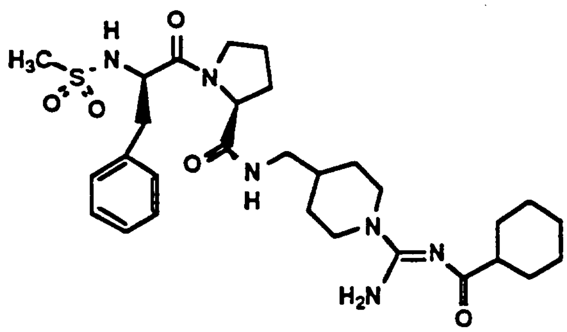

N-[[1-[Amino[(cyclohexylcarbonyl)imino]methyl]-4-piperidinyl]methyl]-1-[N-(methylsulfonyl)-D-phenylalanyl]-L-prolinamide

$[\alpha]_D$ = -66° (c=0.60, $CH_3OH$)

| Elemental analysis for $C_{29}H_{44}N_6O_5S$ • 0.20 hexane: | | | | |
|---|---|---|---|---|
| | C | H | N | S |
| **calc'd** | 59.86 | 7.78 | 13.87 | 5.29 |
| **found** | 59.58 | 7.92 | 13.55 | 5.08 |

Example 75

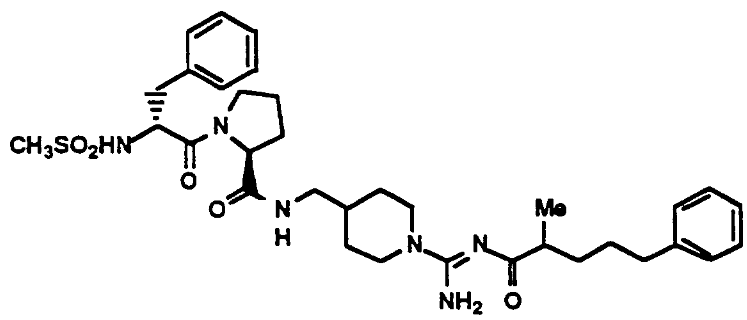

N-[[1-[Amino[(2-methyl-1-oxo-5-phenylpentyl)imino]methyl]-4-piperidinyl]methyl]-1-[N-(methylsulfonyl)-D-phenylalanyl]-L-prolinamide

$[\alpha]_D$ = -70° (c=0.29, $CH_3OH$)

| Elemental analysis for $C_{34}H_{48}N_6O_5S$ • 0.48 $H_2O$ • 0.15 EtOAc: | | | | |
|---|---|---|---|---|
| | C | H | N | S |
| calc'd | 61.59 | 7.49 | 12.46 | 4.75 |
| found | 61.55 | 7.39 | 12.18 | 4.31 |

Example 76

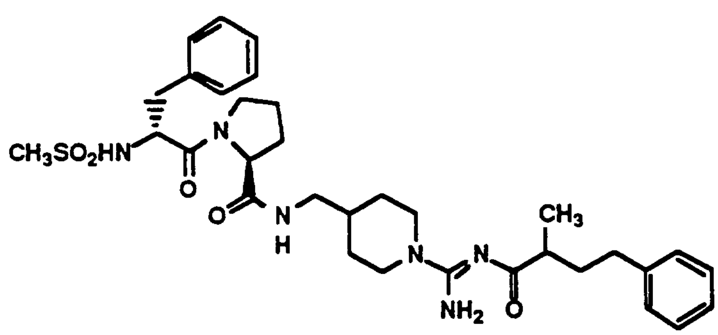

N-[[1-[Amino[(2-methyl-1-oxo-4-phenylbutyl)imino]methyl]-4-piperidinyl]methyl]-1-[N-(methylsulfonyl)-D-phenylalanyl]-L-prolinamide

$[\alpha]_D$ = -61° (c=0.50, $CH_3OH$)

| Elemental analysis for $C_{33}H_{46}N_6O_5S \cdot 0.80\ H_2O$: | | | | |
|---|---|---|---|---|
| | C | H | N | S |
| calc'd | 60.68 | 7.34 | 12.87 | 4.91 |
| found | 60.74 | 7.06 | 12.81 | 4.67 |

Example 77

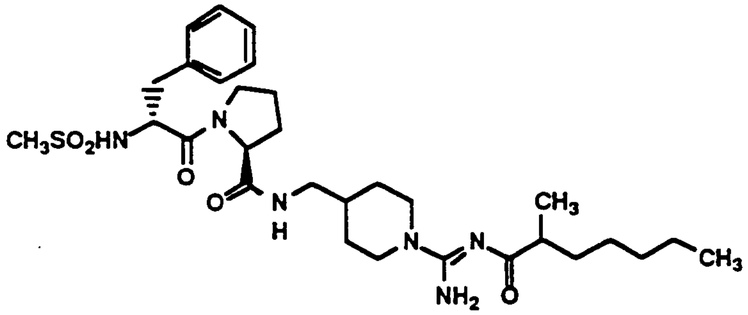

N-[[1-[Amino[(2-methyl-1-oxoheptyl)imino]methyl]-4-piperidinyl]methyl]-1-[N-(methylsulfonyl)-D-phenylalanyl]-L-prolinamide

$[\alpha]_D$ = -68° (c=0.50, $CH_3OH$)

| Elemental analysis for $C_{30}H_{48}N_6O_5S \cdot 0.36\ H_2O$: | | | | |
|---|---|---|---|---|
| | C | H | N | S |
| calc'd | 58.95 | 8.03 | 13.75 | 5.25 |
| found | 59.03 | 7.98 | 13.67 | 5.21 |

Example 78

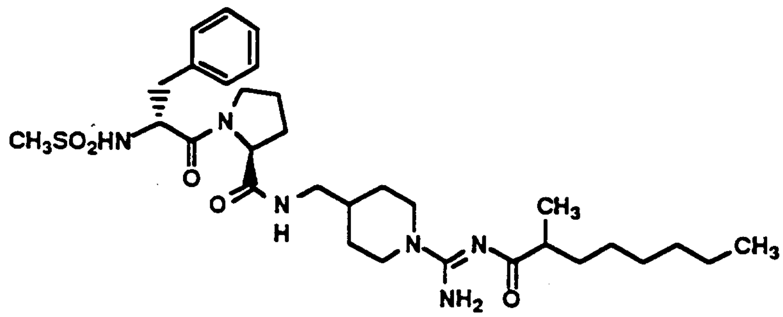

N-[[1-[Amino[(2-methyl-1-oxooctyl)imino]methyl]-4-piperidinyl]methyl]-1-[N-(methylsulfonyl)-D-phenylalanyl]-L-prolinamide

$[\alpha]_D$ = -65° (c=0.50, $CH_3OH$)

| Elemental analysis for $C_{31}H_{50}N_6O_5S$ • 0.26 $H_2O$: | | | | |
|---|---|---|---|---|
| | C | H | N | S |
| calc'd | 59.71 | 8.17 | 13.48 | 5.14 |
| found | 59.78 | 8.07 | 13.41 | 4.86 |

Example 79

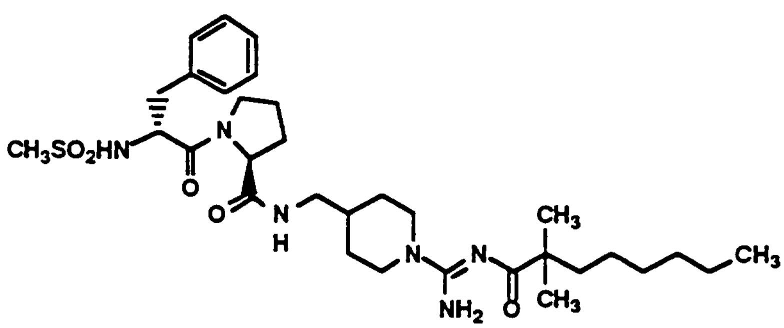

N-[[1-[Amino[(2,2-dimethyl-1-oxooctyl)imino]methyl]-4-piperidinyl]methyl]-1-[N-(methylsulfonyl)-D-phenylalanyl]-L-prolinamide

$[\alpha]_D$ = -71° (c=0.50, $CH_3OH$)

| Elemental analysis for $C_{32}H_{52}N_6O_5S \cdot 0.02\ H_2O$: | | | | |
|---|---|---|---|---|
| | C | H | N | S |
| calc'd | 60.71 | 8.28 | 13.27 | 5.06 |
| found | 60.89 | 8.32 | 13.10 | 4.77 |

Example 80

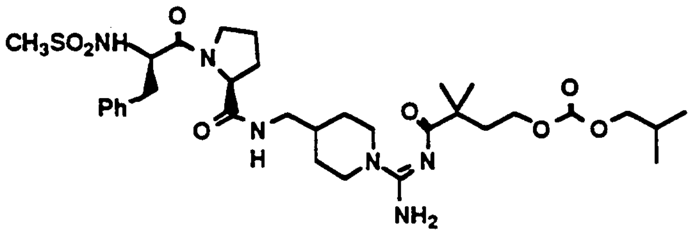

N-[[1-[Amino[[4-[[(2-methylpropoxy)carbonyl]oxy]-2,2-dimethyl-1-oxobutyl]imino]methyl]-4-piperidinyl]methyl]-1-[N-(methylsulfonyl)-D-phenylalanyl]-L-prolinamide

$[\alpha]_D$ = -66° (c=0.24, $CH_3OH$)

| Elemental analysis for $C_{33}H_{52}N_6O_8S$: | | | | |
|---|---|---|---|---|
| | C | H | N | S |
| calc'd | 57.21 | 7.56 | 12.13 | 4.63 |
| found | 57.31 | 7.48 | 12.42 | 4.55 |

Example 81

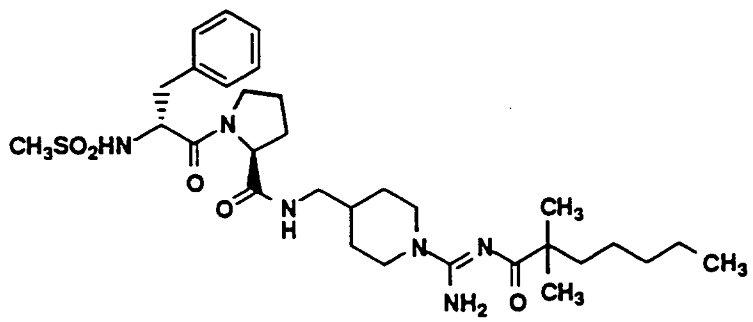

N-[[1-[Amino[(2,2-dimethyl-1-oxoheptyl)imino]methyl]-4-piperidinyl]methyl]-1-[N-(methylsulfonyl)-D-phenylalanyl]-L-prolinamide

$[\alpha]_D$ = -60° (c=0.50, $CH_3OH$)

| Elemental analysis for $C_{31}H_{50}N_6O_5S \cdot 0.45\ H_2O$: | | | | |
|---|---|---|---|---|
| | C | H | N | S |
| calc'd | 59.39 | 8.18 | 13.40 | 5.11 |
| found | 59.39 | 7.93 | 13.20 | 4.67 |

Example 82

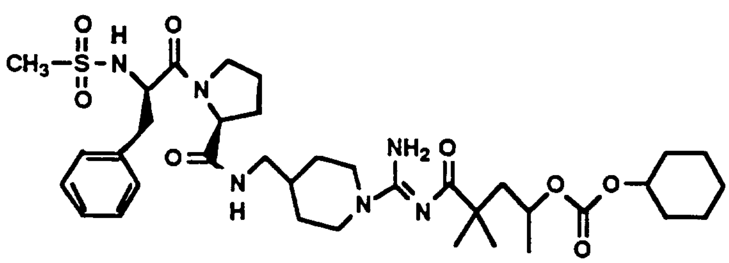

N-[[1-Amino[[4-[[(cyclohexyloxy)carbonyl]oxy]-2,2-dimethyl-1-oxopentyl]imino]methyl]-4-piperidinyl]methyl]-1-[N-(methylsulfonyl)-D-phenylalanyl]-L-prolinamide, monohydrochloride

$[\alpha]_D$ = -55° (c=0.18, $CH_3OH$)

| Elemental analysis for $C_{36}H_{56}N_6O_8S$ • 0.68 $H_2O$ • 1.0 HCl • 0.63 hexane: | | | | | |
|---|---|---|---|---|---|
| | C | H | N | S | Cl |
| calc'd | 57.16 | 8.10 | 10.05 | 3.84 | 4.24 |
| found | 57.16 | 8.15 | 9.96 | 3.83 | 4.34 |

Example 83

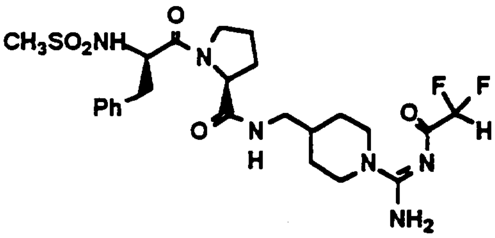

N-[[1-[Amino[(difluoroacetyl)imino]methyl]-4-piperidinyl]methyl]-1-[N-(methylsulfonyl)-D-phenylalanyl]-L-prolinamide

$[\alpha]_D$ = -76° (c=0.74, $CH_3OH$)

| Elemental analysis for $C_{24}H_{34}F_2N_6O_5S$ • 0.33 $H_2O$ • 0.30 hexane: | | | | | |
|---|---|---|---|---|---|
| | C | H | N | S | F |
| calc'd | 52.66 | 6.66 | 14.28 | 6.46 | 5.45 |
| found | 52.66 | 6.56 | 14.38 | 6.46 | 5.26 |

Example 84

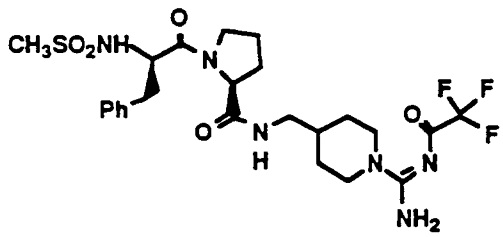

N-[[1-[Amino[(trifluoroacetyl)imino]methyl]-4-piperidinyl]methyl]-1-[N-(methylsulfonyl)-D-phenylalanyl]-L-prolinamide

$[\alpha]_D$ = -73° (c=0.85, $CH_3OH$)

| Elemental analysis for $C_{24}H_{33}F_3N_6O_5S$: | | | | | |
|---|---|---|---|---|---|
| | C | H | N | S | F |
| calc'd | 51.24 | 6.32 | 13.83 | 5.28 | 9.38 |
| found | 51.24 | 6.10 | 13.69 | 4.95 | 9.66 |

Example 85

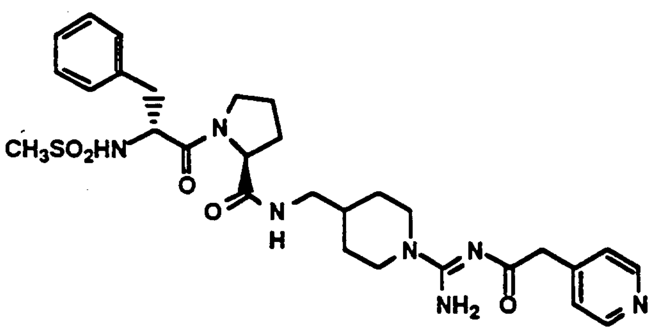

N-[[1-[Amino[(4-pyridinylacetyl)imino]methyl]-4-piperidinyl]methyl]-1-[N-(methylsulfonyl)-D-phenylalanyl]-L-prolinamide, trifluoroacetate

$[\alpha]_D$ = -44° (c=0.50, $CH_3OH$)

| Elemental analysis for $C_{29}H_{39}N_6O_5S \cdot 0.47\ H_2O \cdot 3.05\ CF_3COOH$: | | | | | |
|---|---|---|---|---|---|
| | C | H | N | S | F |
| calc'd | 44.02 | 4.57 | 10.24 | 3.35 | 18.15 |
| found | 44.02 | 4.45 | 10.14 | 3.12 | 18.18 |

Exmaple 86

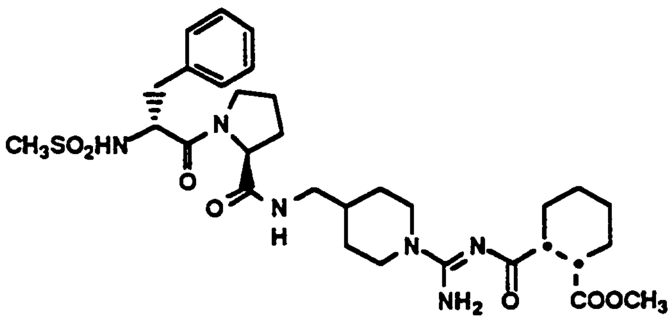

cis-2-[[[Amino[4-[[[1-[N-(methylsulfonyl)-D-phenylalanyl]-L-prolyl]amino]methyl]-1-piperidinyl]methyl]imino]carbonyl]cyclohexanecarboxylic acid, methyl ester, hydrochloride

$[\alpha]_D$ = -59° (c=0.50, $CH_3OH$)

| Elemental analysis for $C_{31}H_{46}N_6O_7S \cdot 0.65\ H_2O \cdot 1.44\ HCl$: | | | | | |
|---|---|---|---|---|---|
| | C | H | N | S | Cl |
| calc'd | 52.44 | 6.78 | 11.84 | 4.52 | 7.19 |
| found | 52.44 | 6.82 | 11.54 | 4.20 | 7.23 |

Example 87

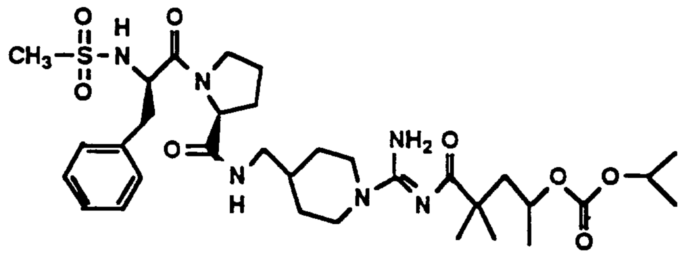

N-[[1-Amino[[[2,2-dimethyl-4-[[(1-methylethoxy)carbonyl]-oxy]-1-oxopentyl]imino]methyl]-4-piperidinyl]methyl]-1-[N-(methylsulfonyl)-D-phenylalanyl]-L-prolinamide, monohydrochloride

$[\alpha]_D$ = -55° (c=0.19, $CH_3OH$)

| Elemental analysis for $C_{33}H_{52}N_6O_8S$ • 0.96 $H_2O$ • 1.20 HCl • 0.58 hexane: | | | | | |
|---|---|---|---|---|---|
| | C | H | N | S | Cl |
| calc'd | 54.50 | 7.93 | 10.45 | 3.99 | 5.29 |
| found | 54.50 | 7.73 | 10.69 | 3.75 | 5.28 |

Example 88

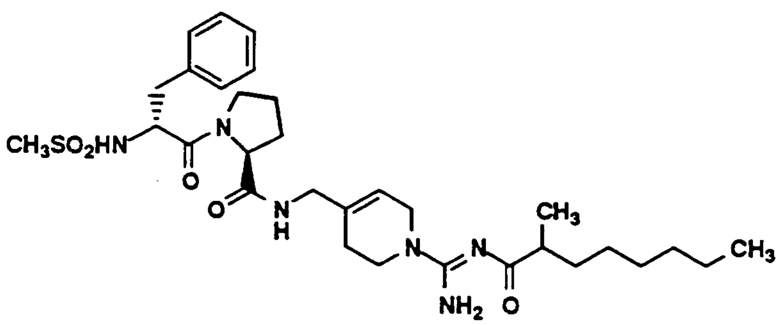

N-[[1-[Amino[(2-methyl-1-oxooctyl)imino]methyl]-1,2,3,6-tetrahydro-4-pyridinyl]methyl]-1-[N-(methylsulfonyl)-D-phenylalanyl]-L-prolinamide

$[\alpha]_D$ = -48.8° (c=0.50, $CH_3OH$)

| Elemental analysis for $C_{31}H_{48}N_6O_5S \cdot 0.35\ H_2O$: | | | | |
|---|---|---|---|---|
| | C | H | N | S |
| calc'd | 59.76 | 7.88 | 13.49 | 5.15 |
| found | 59.82 | 7.96 | 13.43 | 4.74 |

Example 89

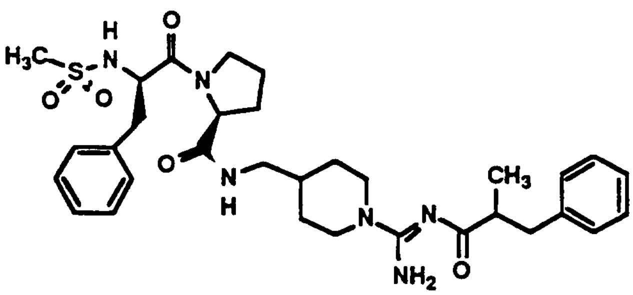

N-[[1-[Amino[(2-methyl-1-oxo-3-phenylpropyl)imino]methyl]-4-piperidinyl]methyl]-1-[N-(methylsulfonyl)-D-phenylalanyl]-L-prolinamide, monohydrochloride

$[\alpha]_D$ = -60.2° (c=0.74, $CH_3OH$)

| Elemental analysis for $C_{32}H_{44}N_6O_5S \cdot 1.05\ H_2O \cdot 1.00\ HCl$: | | | | | |
|---|---|---|---|---|---|
| | C | H | N | S | Cl |
| calc'd | 56.51 | 6.98 | 12.36 | 4.71 | 5.21 |
| found | 56.63 | 6.95 | 12.24 | 4.59 | 4.95 |

Example 90

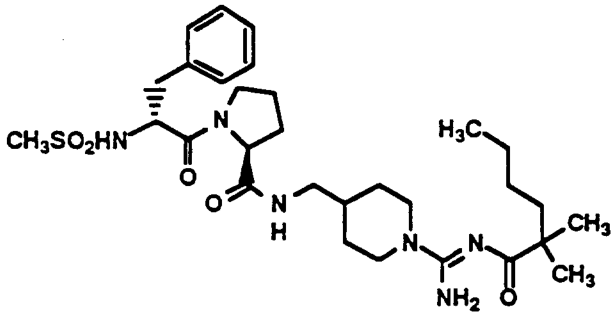

N-[[1-[Amino[(2,2-dimethyl-1-oxohexyl)imino]methyl]-4-piperidinyl]methyl]-1-[N-(methylsulfonyl)-D-phenylalanyl]-L-prolinamide, monohydrochloride

$[\alpha]_D$ = -62.4° (c=0.50, $CH_3OH$)

| Elemental analysis for $C_{30}H_{48}N_6O_5S \cdot 1.26\ H_2O \cdot 1.12\ HCl$: | | | | | |
|---|---|---|---|---|---|
| | C | H | N | S | Cl |
| calc'd | 53.99 | 7.65 | 12.59 | 4.80 | 5.95 |
| found | 54.03 | 7.72 | 12.55 | 4.88 | 6.01 |

Example 91

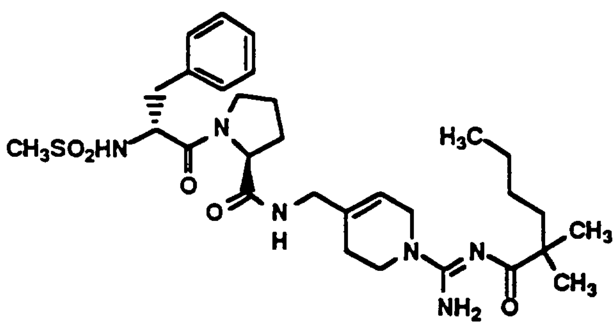

N-[[1-[Amino[(2,2-dimethyl-1-oxohexyl)imino]methyl]-1,2,3,6-tetrahydro-4-pyridinyl]methyl]-1-[N-(methylsulfonyl)-D-phenylalanyl]-L-prolinamide

$[\alpha]_D$ = -47.6° (c=0.50, $CH_3OH$)

| Elemental analysis for $C_{30}H_{46}N_6O_5S$ • 0.25 $H_2O$: | | | | |
|---|---|---|---|---|
| | **C** | **H** | **N** | **S** |
| **calc'd** | 59.33 | 7.72 | 13.84 | 5.28 |
| **found** | 59.41 | 7.82 | 13.78 | 4.84 |

Example 92

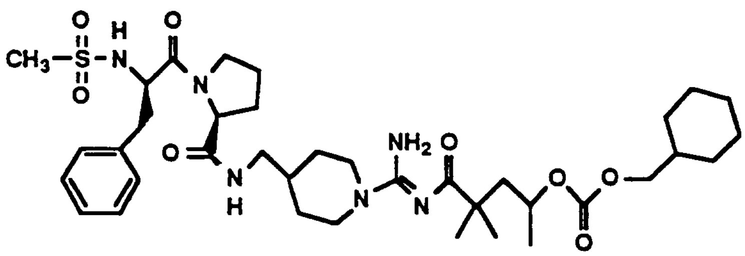

N-[[1-Amino[[[4-[[(Cyclohexylmethoxy)carbonyl]oxy]-2,2-dimethyl-1-oxopentyl]imino]methyl]-4-piperidinyl]-methyl]-1-[N-(methylsulfonyl)-D-phenylalanyl]-L-prolinamide, monohydrochloride

$[\alpha]_D$ = -52.1° (c=0.28, $CH_3OH$)

| Elemental analysis for $C_{37}H_{57}N_6O_8S$ • 1.34 $H_2O$ • 1.20 HCl • 0.40 hexane: | | | | | |
|---|---|---|---|---|---|
| | **C** | **H** | **N** | **S** | **Cl** |
| **calc'd** | 55.78 | 7.90 | 9.91 | 3.78 | 5.01 |
| **found** | 55.78 | 7.80 | 9.81 | 3.84 | 5.03 |

Example 93

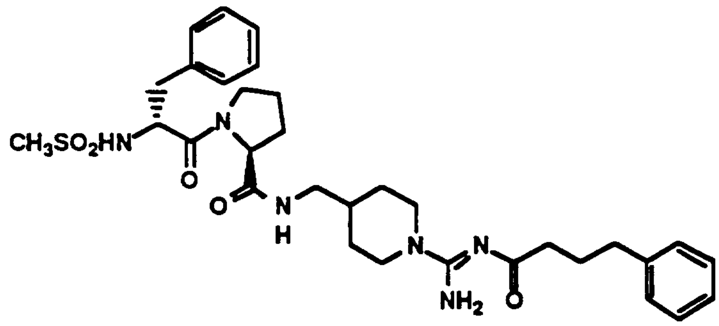

N-[[1-[Amino[(1-oxo-4-phenylbutyl)imino]methyl]-4-piperidinyl]methyl]-1-[N-(methylsulfonyl)-D-phenylalanyl]-L-prolinamide, monohydrochloride

$[\alpha]_D$ = -62.4° (c=0.29, $CH_3OH$)

| Elemental analysis for $C_{32}H_{44}N_6O_5S$ • 1.02 $H_2O$ • 1.0 HCl: | | | | | |
|---|---|---|---|---|---|
| | C | H | N | S | Cl |
| calc'd | 56.56 | 6.98 | 12.37 | 4.72 | 5.22 |
| found | 56.27 | 6.87 | 12.38 | 4.64 | 5.52 |

Example 94

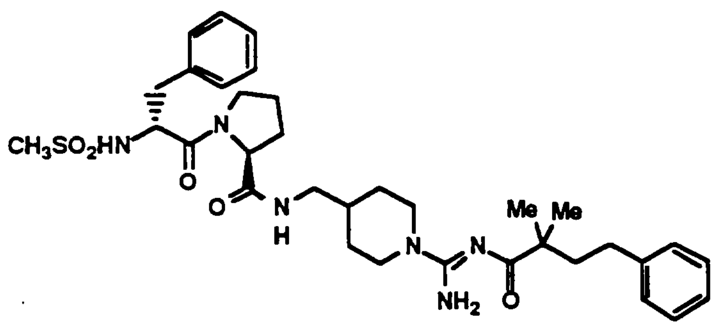

N-[[1-[Amino[(2,2-dimethyl-1-oxo-4-phenylbutyl)imino]methyl]-4-piperidinyl]methyl]-1-[N-(methylsulfonyl)-D-phenylalanyl]-L-prolinamide, monohydrochloride

$[\alpha]_D$ = -62.3° (c=0.26, $CH_3OH$)

| Elemental analysis for $C_{34}H_{48}N_6O_5S$ • 1.51 $H_2O$ • 1.0 HCl: | | | | | |
|---|---|---|---|---|---|
| | C | H | N | S | Cl |
| calc'd | 56.99 | 7.32 | 11.73 | 4.47 | 4.95 |
| found | 57.09 | 7.12 | 11.60 | 4.10 | 4.79 |

Example 95

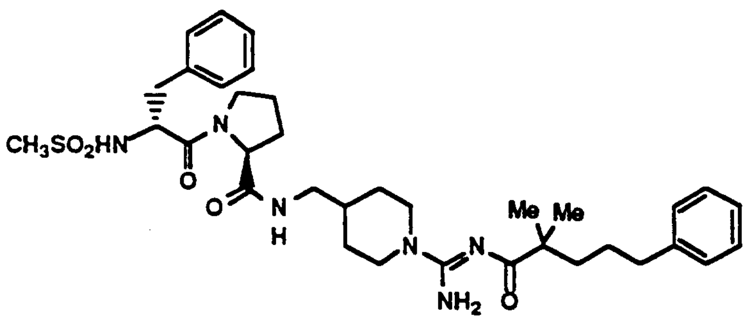

N-[[1-[Amino[(2,2-dimethyl-1-oxo-5-phenylpentyl)imino]methyl]-4-piperidinyl]methyl]-1-[N-(methylsulfonyl)-D-phenylalanyl]-L-prolinamide, monohydrochloride

$[\alpha]_D$ = -58.5° (c=0.27, $CH_3OH$)

| Elemental analysis for $C_{35}H_{50}N_6O_5S$ • 1.42 $H_2O$ • 1.0 HCl: | | | | | |
|---|---|---|---|---|---|
| | C | H | N | S | Cl |
| calc'd | 57.68 | 7.44 | 11.53 | 4.40 | 4.86 |
| found | 57.76 | 7.53 | 11.44 | 4.20 | 5.04 |

Example 96

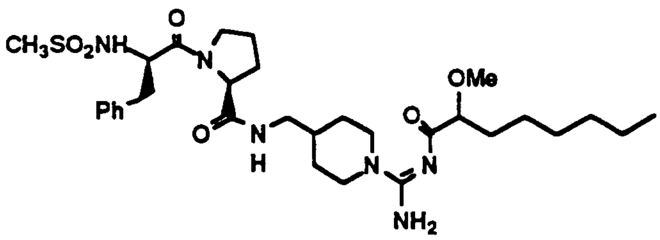

N-[[1-[Amino[(2-methoxy-1-oxooctyl)imino]methyl]-4-piperidinyl]methyl]-1-[N-(methylsulfonyl)-D-phenylalanyl]-L-prolinamide, monohydrochloride

$[\alpha]_D$ = -70.7° (c=0.70, $CH_3OH$)

| Elemental analysis for $C_{31}H_{50}N_6O_6S$ • 0.28 $H_2O$ • 1.08 HCl: | | | | | |
|---|---|---|---|---|---|
| | C | H | N | S | Cl |
| **calc'd** | 54.82 | 7.66 | 12.37 | 5.64 | 4.72 |
| **found** | 54.82 | 7.68 | 12.20 | 5.62 | 4.75 |

Example 97

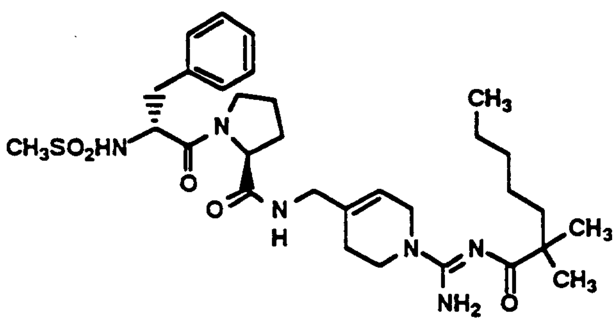

N-[[1-[Amino[(2,2-dimethyl-1-oxohepyl)imino]methyl]-1,2,3,6-tetrahydro-4-pyridinyl]methyl]-1-[N-(methylsulfonyl)-D-phenylalanyl]-L-prolinamide

$[\alpha]_D = -56.6^\circ$ (c=0.50, $CH_3OH$)

| Elemental analysis for $C_{31}H_{48}N_6O_5S \cdot 0.54\ H_2O$: | | | | |
|---|---|---|---|---|
| | C | H | N | S |
| **calc'd** | 59.23 | 8.19 | 13.37 | 5.10 |
| **found** | 59.48 | 7.97 | 13.12 | 5.25 |

Example 98

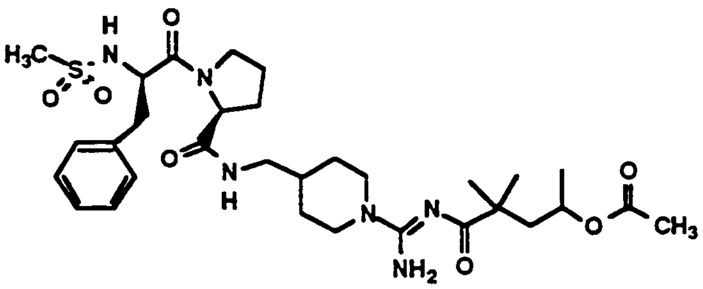

N-[[1-[[[4-(Acetyloxy)-2,2-dimethyl-1-oxopentyl]imino]aminomethyl]-4-piperidinyl]methyl]-1-[N-(methylsulfon-yl)-D-phenylalanyl]-L-prolinamide, monohydrochloride

$[\alpha]_D = -62.5^\circ$ (c=0.60, $CH_3OH$)

| Elemental analysis for $C_{31}H_{48}N_6O_7S \cdot 0.61\ H_2O \cdot 1.00\ HCl$: | | | | | |
|---|---|---|---|---|---|
| | C | H | N | S | Cl |
| **calc'd** | 53.48 | 7.27 | 12.07 | 4.60 | 5.09 |
| **found** | 53.48 | 7.33 | 11.87 | 4.52 | 5.10 |

Example 99

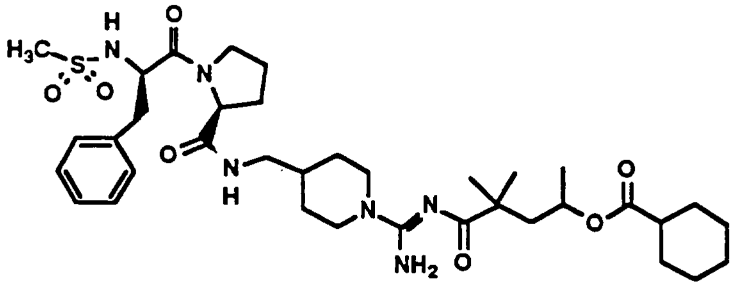

N-[[1-[Amino[[4-[(cyclohexylcarbonyl)oxy]-2,2-dimethyl-1-oxopentyl]imino]-methyl]-4-piperidinyl]methyl]-1-[N-(methylsulfonyl)-D-phenylalanyl]-L-prolinamide, monohydrochloride

$[\alpha]_D$ = -62.1° (c=0.64, $CH_3OH$)

| Elemental analysis for $C_{36}H_{56}N_6O_7S$ • 0.39 $H_2O$ • 1.24 HCl: | | | | | |
|---|---|---|---|---|---|
| | C | H | N | S | Cl |
| calc'd | 56.22 | 7.60 | 10.93 | 4.17 | 5.72 |
| found | 56.22 | 7.62 | 10.94 | 3.98 | 5.69 |

Example 100

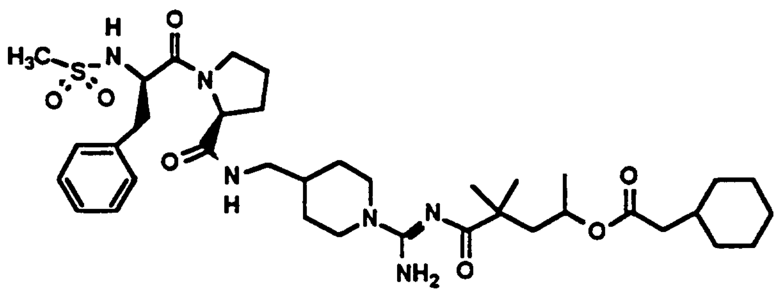

N-[[1-[Amino[[4-[(cyclohexylacetyl)oxy]-2,2-diethyl-1-oxopentyl]imino]-methyl]-4-piperidinyl]methyl]-1-[N-(methylsulfonyl)-D-phenylalanyl]-L-prolinamide, monohydrochloride

$[\alpha]_D$ = -54.6° (c=0.83, $CH_3OH$)

| Elemental analysis for $C_{37}H_{58}N_6O_7S$ • 0.40 $H_2O$ • 1.00 HCl: | | | | | |
|---|---|---|---|---|---|
| | C | H | N | S | Cl |
| calc'd | 57.37 | 7.78 | 10.85 | 4.14 | 4.58 |
| found | 57.01 | 7.56 | 10.71 | 3.85 | 4.98 |

Example 101

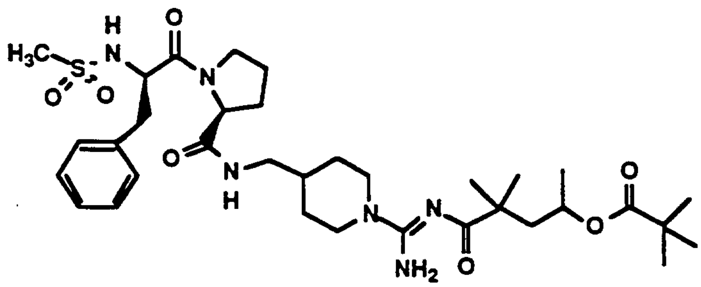

N-[[1-[Amino[[2,2-dimethyl-4-(2,2-dimethyl-1-oxopropoxy)-1-oxopentyl]-imino]methyl]-4-piperidinyl]methyl]-1-[N-(methylsulfonyl)-D-phenylalanyl]-L-prolinamide, monohydrochloride

$[\alpha]_D$ = -56.7° (c=0.88, $CH_3OH$)

| Elemental analysis for $C_{34}H_{54}N_6O_7S$ • 0.45 $H_2O$ • 1.11 HCl: | | | | | |
|---|---|---|---|---|---|
| | C | H | N | S | Cl |
| calc'd | 55.22 | 7.63 | 11.36 | 4.34 | 5.32 |
| found | 55.18 | 7.64 | 11.16 | 4.19 | 5.31 |

Example 102

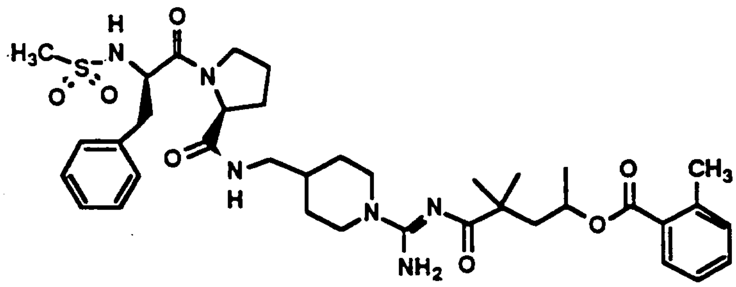

N-[[1-[Amino[[2,2-dimethyl-4-(2-methylbenzoyloxy)-1-oxopentyl]imino]methyl]-4-piperidinyl]methyl]-1-[N-(methylsulfonyl)-D-phenylalanyl]-L-prolinamide, monohydrochloride

$[\alpha]_D$ = -61.8° (c=0.73, $CH_3OH$)

| Elemental analysis for $C_{37}H_{52}N_6O_7S$ • 0.70 $H_2O$ • 1.00 HCl: | | | | | |
|---|---|---|---|---|---|
| | C | H | N | S | Cl |
| calc'd | 57.42 | 7.08 | 10.86 | 4.14 | 4.58 |
| found | 57.28 | 7.09 | 10.75 | 4.13 | 4.90 |

Example 103

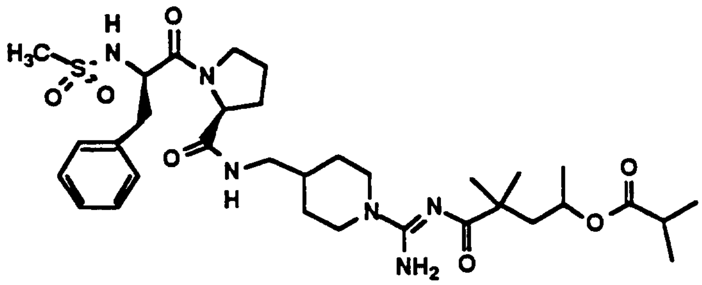

N-[[1-[Amino[[2,2-dimethyl-4-(2-methyl-1-oxopropoxy)-1-oxopentyl]imino]-methyl]-4-piperidinyl]methyl]-1-[N-(methylsulfonyl)-D-phenylalanyl]-L-prolinamide, monohydrochloride

$[\alpha]_D$ = -58.4° (c=1.01, $CH_3OH$)

| Elemental analysis for $C_{33}H_{52}N_6O_7S$ • 0.75 $H_2O$ • 1.00 HCl: | | | | | |
|---|---|---|---|---|---|
| | C | H | N | S | Cl |
| calc'd | 54.53 | 7.56 | 11.56 | 4.41 | 4.88 |
| found | 54.70 | 7.56 | 11.19 | 4.17 | 4.96 |

Example 104

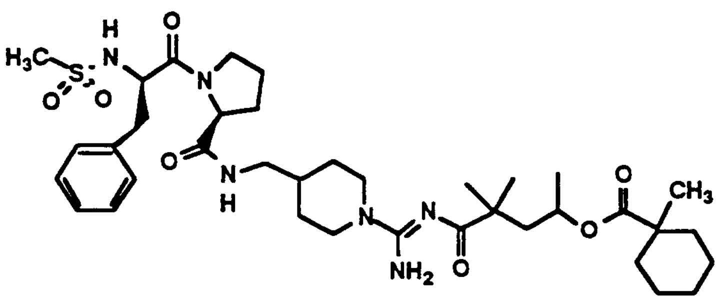

N-[[1-[Amino[[2,2-dimethyl-4-[[(1-methyloyclohexyl)carbonyl]oxy]-1-oxopentyl]-imino]methyl]-4-piperidinyl]methyl]-1-[N-(methylsulfonyl)-D-phenylalanyl]-L-prolinamide, monohydrochloride

$[\alpha]_D$ = -54.3° (c=0.70, $CH_3OH$)

| Elemental analysis for $C_{37}H_{58}N_6O_7S$ • 0.28 $H_2O$ • 1.13 HCl: | | | | | |
|---|---|---|---|---|---|
| | C | H | N | S | Cl |
| calc'd | 57.18 | 7.74 | 10.81 | 4.13 | 5.15 |
| found | 57.18 | 7.85 | 10.64 | 3.86 | 5.16 |

Example 105

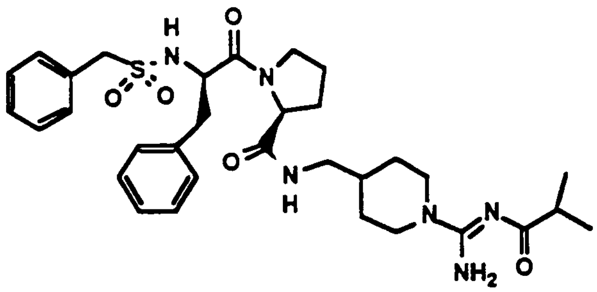

N-[[1-[Amino[(2-methyl-1-oxopropyl)imino]methyl]-4-piperidinyl]methyl]-1-[N-[(phenylmethyl)sulfonyl]-D-phenylalanyl]-L-prolinamide, monohydrochloride

$[\alpha]_D$ = -55.4° (c=0.63, $CH_3OH$)

| Elemental analysis for $C_{32}H_{44}N_6O_5S$ • 0.32 $H_2O$ • 1.00 HCl: | | | | | |
|---|---|---|---|---|---|
| | C | H | N | S | Cl |
| calc'd | 57.62 | 6.90 | 12.60 | 4.81 | 5.32 |
| found | 57.82 | 6.85 | 12.40 | 5.00 | 5.33 |

Example 106

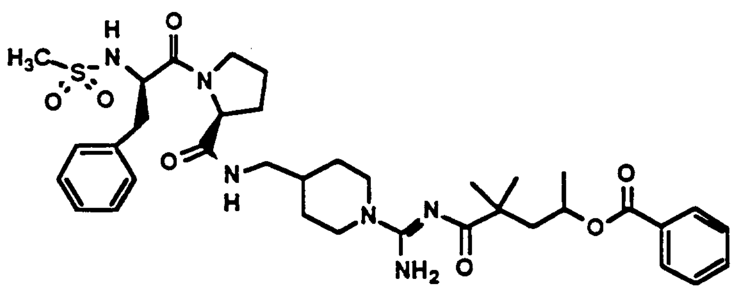

N-[[1-[Amino[[4-(benzoyloxy)-2,2-dimethyl-1-oxopentyl]imino]methyl]-4-piperidinyl]methyl]-1-[N-(methylsulfonyl)-D-phenylalanyl]-L-prolinamide, monohydrochloride

$[\alpha]_D$ = -55.9° (c=0.79, $CH_3OH$)

| Elemental analysis for $C_{36}H_{50}N_6O_7S$ • 0.81 $H_2O$ • 1.00 HCl: | | | | | |
|---|---|---|---|---|---|
| | C | H | N | S | Cl |
| calc'd | 56.75 | 6.96 | 11.03 | 4.21 | 4.65 |
| found | 56.75 | 7.07 | 11.03 | 4.41 | 4.31 |

Example 107

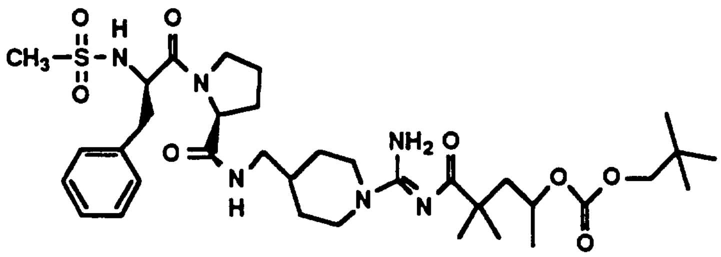

N-[[1-Amino[[[2,2-Dimethyl-4-[[(2,2-dimethylpropoxy)carbonyl]oxy]-1-oxopentyl]imino]methyl]-4-piperidinyl]methyl]-1-[N-(methylsulfonyl)-D-phenylalanyl]-L-prolinamide, monohydrochloride

$[\alpha]_D$ = -50° (c=0.21, $CH_3OH$)

| Elemental analysis for $C_{35}H_{56}N_6O_8S$ • 0.64 $H_2O$ • 1.0 HCl • 0.45 hexane: | | | | | |
|---|---|---|---|---|---|
| | C | H | N | S | Cl |
| calc'd | 56.06 | 8.06 | 10.40 | 3.97 | 4.39 |
| found | 56.06 | 8.06 | 10.26 | 3.71 | 4.39 |

Example 108

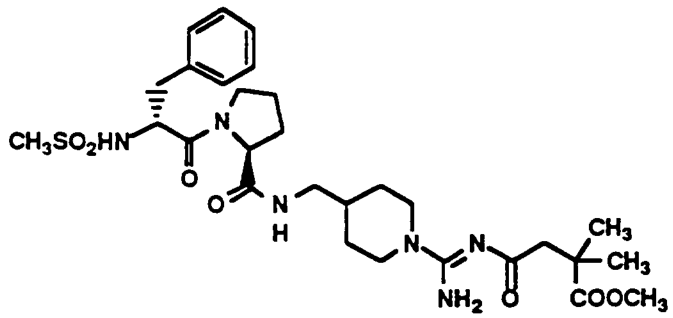

N-[[1-[Amino[(4-methoxy-3,3-dimethyl-1,4-dioxobutyl)imino]methyl]-4-piperidinyl]methyl]-1-[N-(methylsulfonyl)-D-phenylalanyl]-L-prolinamide, monohydrochloride

$[\alpha]_D$ = -56.4° (c=0.50, $CH_3OH$)

| Elemental analysis for $C_{29}H_{44}N_6O_7S$ • 1.0 $H_2O$ • 1.1 HCl: | | | | | |
|---|---|---|---|---|---|
| | C | H | N | S | Cl |
| calc'd | 51.31 | 6.99 | 12.38 | 4.72 | 5.74 |
| found | 51.53 | 6.96 | 12.28 | 4.56 | 5.75 |

Example 109

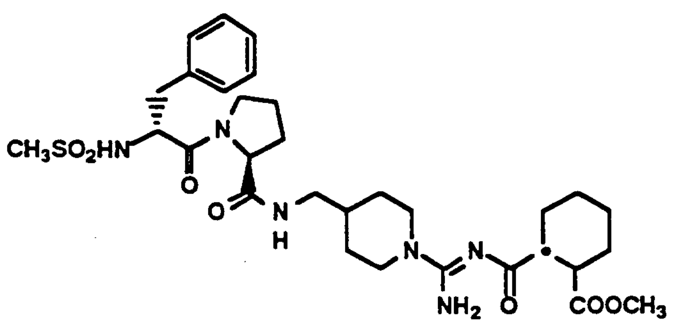

2-[[[Amino[4-[[[1-[N-(methylsulfonyl)-D-phenylalanyl]-L-prolyl]amino]methyl]-1-piperidinyl]methyl]imino]carbonyl]cyclohexanecarboxylic acid, methyl ester, monohydrochloride

$[\alpha]_D$ = -57.4° (c=0.50, $CH_3OH$)

| Elemental analysis for $C_{31}H_{46}N_6O_7S$ • 1.0 $H_2O$ • 1.25 HCl: | | | | | |
|---|---|---|---|---|---|
| | **C** | **H** | **N** | **S** | **Cl** |
| **calc'd** | 52.41 | 6.99 | 11.83 | 4.51 | 6.40 |
| **found** | 52.45 | 7.00 | 11.69 | 4.16 | 6.36 |

Example 110

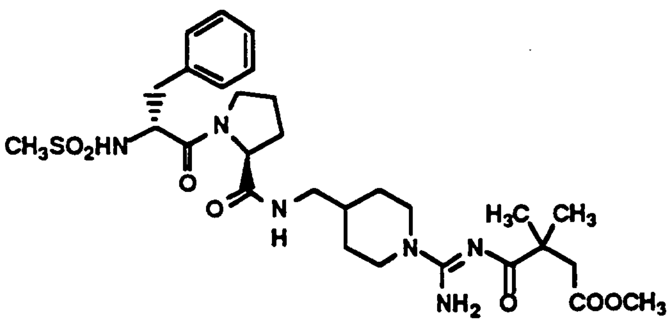

N-[[1-[Amino[(4-methoxy-2,2-dimethyl-1,4-dioxobutyl)imino]methyl]-4-piperidinyl]methyl]-1-[N-(methylsulfonyl)-D-phenylalanyl]-L-prolinamide, monohydrochloride

$[\alpha]_D$ = -59.4° (c=0.50, $CH_3OH$)

| Elemental analysis for $C_{29}H_{44}N_6O_7S$ • 1.0 $H_2O$ • 1.0 HCl: | | | | | |
|---|---|---|---|---|---|
| | **C** | **H** | **N** | **S** | **Cl** |
| **calc'd** | 51.58 | 7.02 | 12.45 | 4.75 | 5.25 |
| **found** | 51.58 | 6.73 | 12.16 | 4.77 | 5.56 |

Example 111

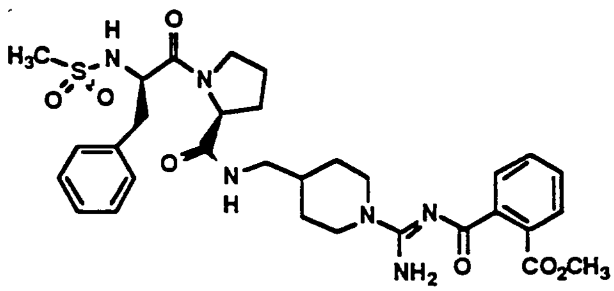

N-[[1-[Amino[[2-(methoxycarbonyl)benzoyl]imino]methyl]-4-piperidinyl]methyl]-1-[N-(methylsulfonyl)-D-phenylalanyl]-L-prolinamide, monohydrochloride

$[\alpha]_D$ = -58.4° (c=0.92, $CH_3OH$)

| Elemental analysis for $C_{31}H_{40}N_6O_7S$ • 0.80 $H_2O$ • 1.20 HCl • 0.20 $C_4H_{10}O$: | | | | | |
|---|---|---|---|---|---|
| | **C** | **H** | **N** | **S** | **Cl** |
| **calc'd** | 53.51 | 6.33 | 11.77 | 4.49 | 5.96 |
| **found** | 53.20 | 6.22 | 11.81 | 4.10 | 6.02 |

Example 112

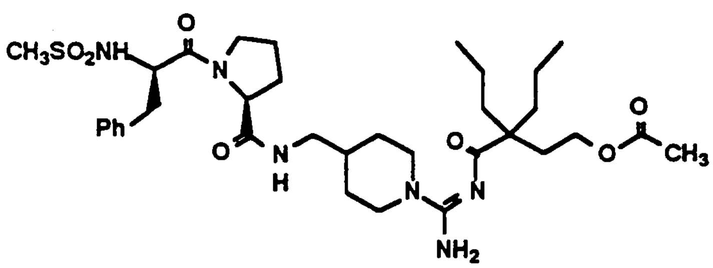

N-[[1-Amino[[[4-(acetyloxy)-2,2-dipropyl-1-oxobutyl]imino]methyl]-4-piperidinyl]methyl]-1-[N-(methylsulfonyl)-D-phenylalanyl]-L-prolinamide, hydrochloride

$[\alpha]_D$ = -60.6° (c=0.74, $CH_3OH$)

| Elemental analysis for $C_{34}H_{54}N_6O_7S \cdot 0.36\ H_2O \cdot 1.0\ HCl$: | | | | | |
|---|---|---|---|---|---|
| | C | H | N | S | Cl |
| calc'd | 55.65 | 7.65 | 11.45 | 4.83 | 4.37 |
| found | 55.85 | 7.75 | 11.32 | 4.56 | 4.12 |

Example 113

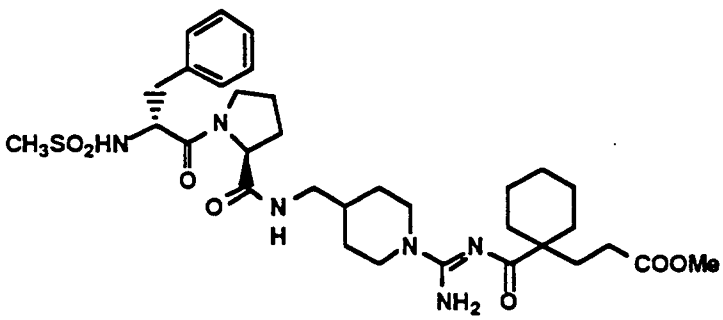

1-[[[Amino[4-[[[1-[N-(methylsulfonyl)-D-phenylalanyl]-L-prolyl]amino]methyl]-1-piperidinyl]methyl]imino]carbonyl]cyclohexanepropanoic acid, methyl ester, monohydrochloride

$[\alpha]_D$ = -63.7° (c=0.27, $CH_3OH$)

| Elemental analysis for $C_{33}H_{50}N_6O_7S \cdot 1.0\ H_2O \cdot 1.0\ HCl$: | | | | | |
|---|---|---|---|---|---|
| | C | H | N | S | Cl |
| calc'd | 54.35 | 7.33 | 11.52 | 4.40 | 4.86 |
| found | 54.40 | 7.40 | 11.48 | 4.37 | 5.05 |

Example 114

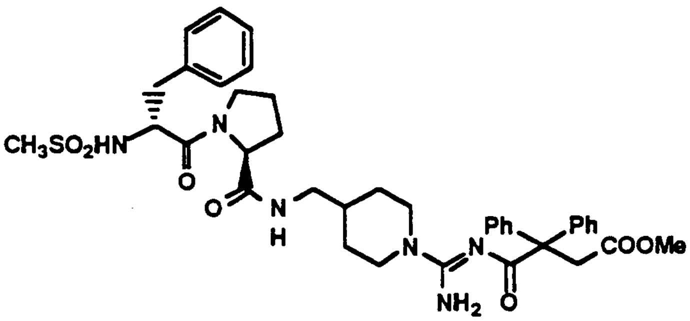

β-[[Amino[4-[[[1-[N-(methylsulfonyl)-D-phenylalanyl]-L-prolyl]amino]methyl]-1-piperidinyl]methyl]imino]carbonyl]-β-phenylbenzenepropanoic acid, methyl ester, monohydrochloride

$[\alpha]_D$ = -52.8° (c=0.25, $CH_3OH$)

| Elemental analysis for $C_{39}H_{48}N_6O_7S$ • 1.1 $H_2O$ • 1.1 HCl: | | | | | |
|---|---|---|---|---|---|
| | C | H | N | S | Cl |
| calc'd | 58.20 | 6.42 | 10.44 | 3.98 | 4.85 |
| found | 57.98 | 6.30 | 10.33 | 4.04 | 4.82 |

Example 115

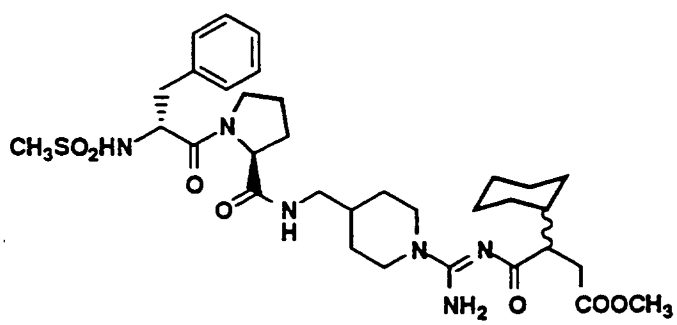

N-[[1-[Amino[(2-cyclohexyl-4-methoxy-1,4-dioxobutyl)imino]methyl]-4-piperidinyl]methyl]-1-[N-(methylsulfonyl)-D-phenylalanyl]-L-prolinamide, monohydrochloride

$[\alpha]_D = -60.8^\circ$ (c=0.50, $CH_3OH$)

| Elemental analysis for $C_{33}H_{50}N_6O_7S \cdot 1.0\ H_2O \cdot 1.0\ HCl$: | | | | | |
|---|---|---|---|---|---|
| | C | H | N | S | Cl |
| calc'd | 54.35 | 7.33 | 11.52 | 4.40 | 4.86 |
| found | 54.53 | 7.23 | 11.40 | 4.35 | 5.00 |

Example 116

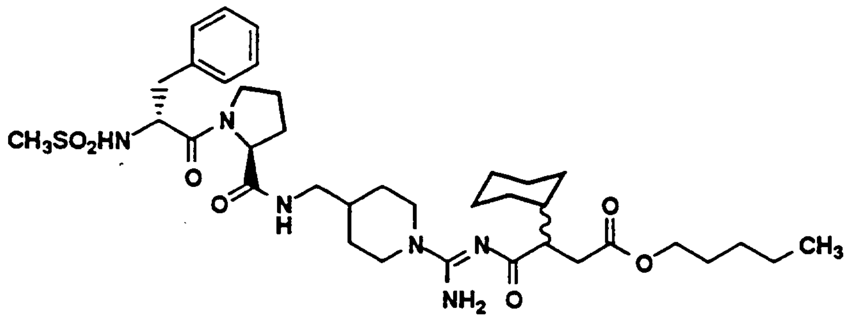

N-[[1-[Amino[[2-cyclohexyl-1,4-dioxo-4-(pentyloxy)butyl]imino]methyl]-4-piperidinyl]methyl]-1-[N-(methylsulfonyl)-D-phenylalanyl]-L-prolinamide, hydrochloride

$[\alpha]_D = -55.2^\circ$ (c=0.50, $CH_3OH$)

| Elemental analysis for $C_{37}H_{58}N_6O_7S \cdot 1.0\ H_2O \cdot 0.75\ HCl$: | | | | | |
|---|---|---|---|---|---|
| | C | H | N | S | Cl |
| calc'd | 56.91 | 7.81 | 10.76 | 4.11 | 4.54 |
| found | 56.92 | 7.61 | 10.61 | 4.19 | 4.62 |

Example 117

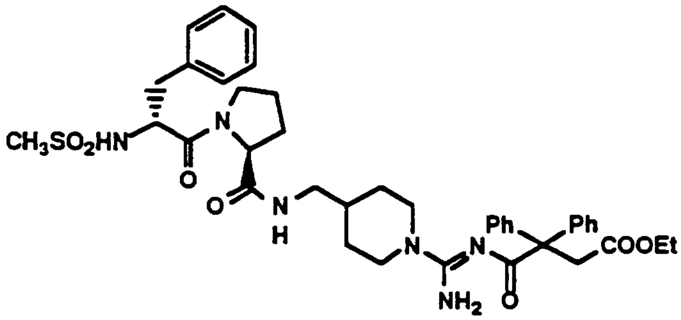

β-[[[Amino[4-[[[1-[N-(methylsulfonyl)-D-phenylalanyl]-L-prolyl]amino]methyl]-1-piperidinyl]methyl]imino]carbonyl]-β-phenylbenzenepropanoic acid, ethyl ester, monohydrochloride

$[\alpha]_D$ = -56.0° (c=0.25, $CH_3OH$)

| Elemental analysis for $C_{40}H_{50}N_6O_7S \cdot 0.90\ H_2O \cdot 1.0\ HCl$: | | | | | |
|---|---|---|---|---|---|
| | C | H | N | S | Cl |
| calc'd | 59.20 | 6.56 | 10.35 | 3.95 | 4.37 |
| found | 58.97 | 6.38 | 10.74 | 4.07 | 3.99 |

Example 118

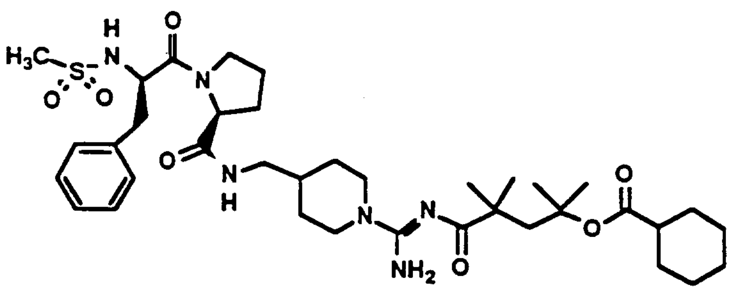

N-[[1-[Amino[[4-[(cyclohexylcarbonyl)oxy]-2,2,4-trimethyl-1-oxopentyl]imino]-methyl]-4-piperidinyl]methyl]-1-[N-(methylsulfonyl)-D-phenylalanyl]-L-prolinamide, monohydrochloride

$[\alpha]_D$ = -53.4° (c=0.44, $CH_3OH$)

| Elemental analysis for $C_{37}H_{58}N_6O_7S$ • 0.55 $H_2O$ • 1.00 HCl: | | | | | |
|---|---|---|---|---|---|
| | C | H | N | S | Cl |
| calc'd | 57.17 | 7.79 | 10.81 | 4.12 | 4.56 |
| found | 57.17 | 7.76 | 10.66 | 3.90 | 4.45 |

Example 119

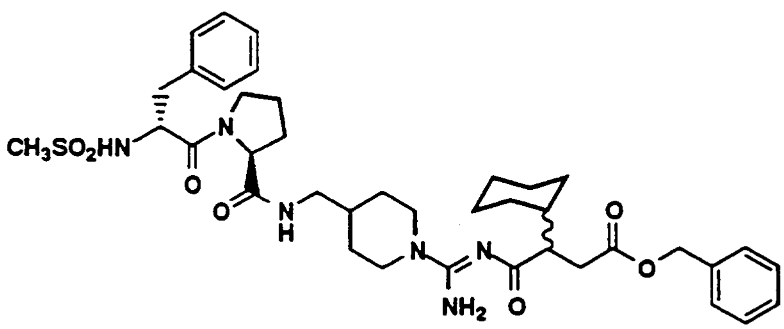

N-[[1-[Amino[[2-cyclohexyl-1,4-dioxo-4-(phenylmethoxy)butyl]imino]methyl]-4-piperidinyl]methyl]-1-[N-(methylsulfonyl)-D-phenylalanyl]-L-prolinamide, monohydrochloride

$[\alpha]_D$ = -61.6° (c=0.50, $CH_3OH$)

| Elemental analysis for $C_{39}H_{54}N_6O_7S$ • 0.85 $H_2O$ • 1.0 HCl: | | | | | |
|---|---|---|---|---|---|
| | C | H | N | S | Cl |
| calc'd | 58.35 | 7.04 | 10.67 | 4.07 | 4.50 |
| found | 58.32 | 6.94 | 10.57 | 3.91 | 4.59 |

It will be appreciated that following the procedures of Examples I to 11 and Reaction schemes I, II or III, prodrugs of structure I which include a Z substructure Z(2), Z(3), Z(4), Z(5) or Z(6) or any of the other Z substructures disclosed hereinbefore may be prepared.

Preferred compounds of formula I are:

- the compound of formula I wherein Acyl is

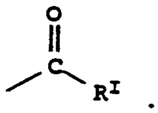

- the compound of formula I wherein Acyl is

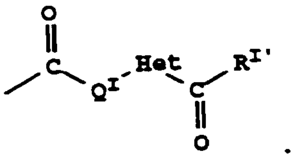

- the compound of formula I wherein Acyl is

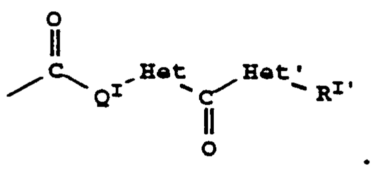

- The compound of formula I wherein Acyl is

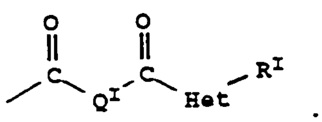

- The compound of formula I wherein Ax is an Acyl and A'x is Acyl which may be the same or different from Ax.
- The compound of formula I wherein A'x or Ax is Acyl.
- The compound of formula I wherein one of Ax or A'x is Acyl and the other is H.
- The compound of formula I wherein Ax is

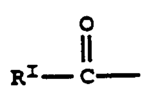

and A'x is

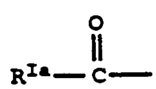

or H wherein $R^I$ is an alkyl group and $R^{Ia}$ is an alkyl group.

- The compound of formula I wherein Ax is

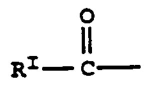

and A'x is

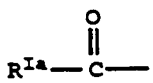

wherein $R^I$ is an alkyl group and $R^{Ia}$ is an alkyl group.

- The compound of formula I wherein one of Ax or A'x is

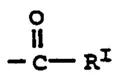

and the other is H.

- The compound of formula I wherein one of Ax or A'x is

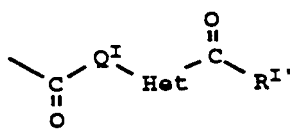

and the other is H.

- The compound of formula I wherein one of Ax or A'x is

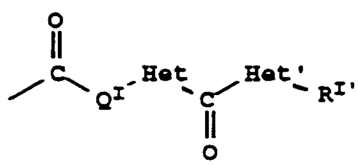

and the other is H.

- The compound of formula I wherein one of Ax or A'x is

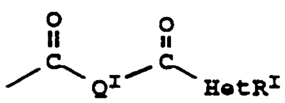

and the other is H.

Preferred compounds of formula Ix are:

- The compound of formula Ix wherein A is aryl or cycloalkyl.
- The compound of formula Ix wherein Q is a single bond and q is 0 or I.

- The compound of formula Ix wherein A is

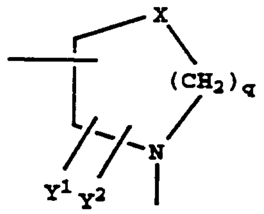

.

- The compound of formula Ix wherein X is $CH_2$ or NH.
- The compound of formula Ix where one of Ax or A'x is an Acyl and the other is H or the same or different Acyl.
- The compound of formula Ix wherein n is 0 or I and m is 2.
- The compound of formula Ix wherein $R^3$ is 2-naphthyl or lower alkyl.
- The compound of formula Ix wherein R is hydroxyalkyl or aralkyl.

Further preferred compounds of formula I have the structure I(A):

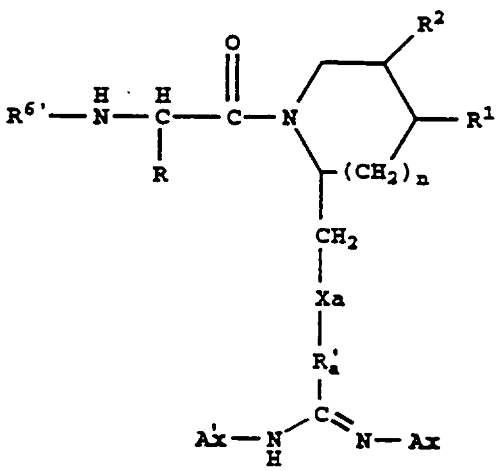

including all stereoisomers, wherein n is 0, I or 2;

Xa is S, SO, $SO_2$ or O;

$R'_a$ is $-A^1-$ or $-A^1-NH-$, where $A^1$ is an alkyl, alkenyl, or alkynyl chain; with the proviso that there is at least one carbon between any S or O and an alkenyl or alkynyl moiety; or

$R'_a$ is $-(CH_2)_p-A^2-$ where $A^2$ is an azacycloalkyl ring A of 4 to 8 ring members, or an azacycloalkenyl ring A of 5 to 9 ring members, or an azaheterocycloalkyl ring A of 6 to 8 ring members,

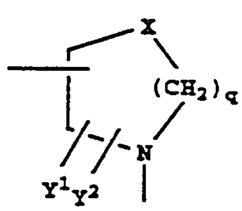

where X is $CH_2$, -CH=CH-, O, S, NH; and provided that

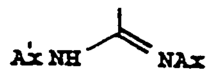

group is attached to the nitrogen atom in the ring as indicated below

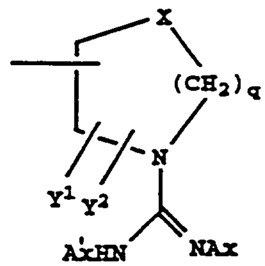

;

q is 0, l, 2, 3 or 4 if X is $CH_2$ or -CH=CH-;

q is 2, 3 or 4 if X is O, S or NH;

$Y^1$ and $Y^2$ are independently H, lower alkyl, oxo or halo;

provided that where X is a hetero atom, that is, $A^2$ is azaheterocycloalkyl, then there must be at least a 2-carbon chain between X and any heteroatom in the ring A or outside ring A; or

$R_a'$ is $-(CH_2)_p-A^3-$ or $-(CH_2)_p-A^3-NH-$, where $A^3$ is aryl or cycloalkyl;

$R^1$ and $R^2$ are independently hydrogen, lower alkyl, cycloalkyl, aryl, hydroxy, alkoxy, keto, thioketal, thioalkyl, thioaryl, amino or alkylamino, or $R^1$ and $R^2$ together with the carbons to which they are attached form a cycloalkyl, aryl or heteroaryl ring;

R is hydrogen, hydroxyalkyl, aminoalkyl, alkyl, cycloalkyl, cycloalkylalkyl, aryl, arylalkyl, alkenyl, alkynyl, amidoalkyl, arylalkoxyalkyl or an amino acid side chain, either protected or unprotected;

$R^{6'}$ is hydrogen,

$$-\overset{O}{\overset{\|}{C}}-R^7 ,$$

$-SO_2R^3$ or $-CO_2R^7$ wherein $R^7$ is lower alkyl, aryl, arylalkyl, cycloheteroalkyl or heteroaryl; and

$R^3$ is alkyl, arylalkyl, aryl, heteroaryl, quinolinyl, tetrahydroquinolinyl, l0-camphoryl, pentamethylchromanyl, pentaalkylphenyl, pentahalo-

Preferred compounds of formula I(A) are:

- The compound of formula I(A) wherein $R_a'$ is $-A^1-$ or $-A^1-NH-$.
- The compound of formula I(A) wherein $R_a'$ is $-(CH_2)_p-A^2-$.
- The compound of formula I(A) wherein $R_a'$ is $-(CH_2)_p-A^3-$ or $-(CH_2)_p-A^3-NH-$.
- The compound of formula I(A) wherein $R_a'$ is $-(CH_2)_pA^2-$ and p is l or 2.
- The compound of formula I(A) wherein n is O.
- The compound of formula I(A) wherein $R^1$ and $R^2$ are H.
- The compound of formula I(A) wherein $R^{6'}$ is $-SO_2R^3$.

Further preferred compounds have the structure I(B):

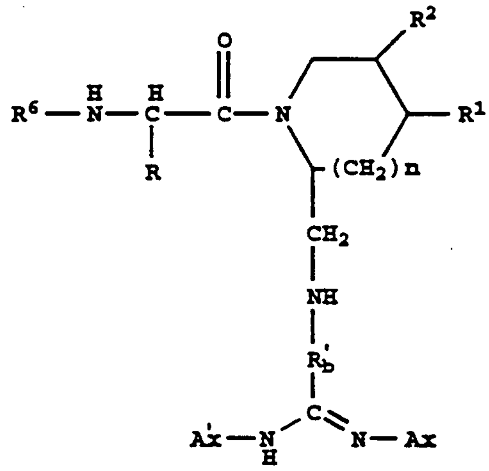

including all stereoisomers, wherein n is 0, I or 2;

$R'_b$ is $-A^1-$, $-CO-A^1-$, $-SO_2-A^1-$, $-A^1-NH-$, $-CO-A^1-NH-$, or $-SO_2A^1-NH-$; wherein $A^1$ is an alkyl, alkenyl or alkynyl chain; with the proviso that there is at least one carbon between any NH, S or O, and an alkenyl or alkynyl moiety; or

$R'_b$ is $-(CH_2)_p-A^2-$, $-(CH_2)_p-CO-A^2-$ or $-(CH_2)_p-SO_2A^2-$ where p is 0, I or 2, and

$A^2$ is an azacycloalkyl ring A of 4 to 8 ring members, or an azacycloalkenyl ring A of 5 to 9 ring members, or an azaheterocycloalkyl ring A of 6 to 8 ring members,

A

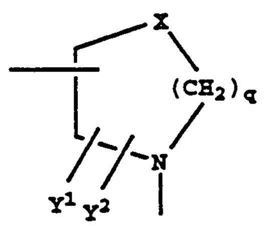

where X is $CH_2$, -CH=CH-, O, S or NH; and the

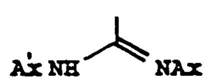

group is attached to the nitrogen atom in the ring as indicated below

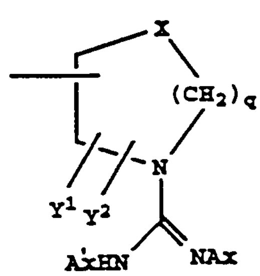
;

q is 0, I, 2, 3 or 4 if X is $CH_2$ or -CH=CH-;

q is 2, 3 or 4 if X is O, S or NH;

$Y^1$ and $Y^2$ are independently H, lower alkyl, oxo or halo;

provided that where X is a hetero atom, that is, A is azaheteroalkyl, then there must be at least a 2-carbon chain between X and any N atom in the ring A or outside ring A; or

$R_b'$ is $-(CH_2)_p-A^3-$, $-(CH_2)_p-CO-A^3-$, or $-(CH_2)_p-SO_2-A^3-$, $-(CH_2)_p-A^3-NH-$, $-(CH_2)_p-CO-A^3-NH$, $-(CH_2)_p-SO_2-A^3-NH$; wherein $A^3$ is aryl or cycloalkyl;

$R^1$ and $R^2$ are independently hydrogen, lower alkyl, cycloalkyl, aryl, hydroxy, alkoxy, keto, thioketal, thioalkyl, thioaryl, amino or alkylamino; or $R^1$ and $R^2$ together with the carbons to which they are attached form a cycloalkyl, aryl or heteroaryl ring;

R is hydrogen, hydroxyalkyl, aminoalkyl, alkyl, cycloalkyl, cycloalkylalkyl, aryl, arylalkyl, alkenyl, alkynyl, amidoalkyl, arylalkoxyalkyl or an amino acid side chain, either protected or unprotected; and

$R^6$ is hydrogen,

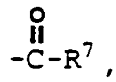,

$-SO_2R^3$ or $-CO_2R^7$,

wherein $R^7$ is lower alkyl, aryl, arylalkyl, cycloheteroalkyl or heteroaryl; and

$R^3$ is alkyl, arylalkyl, aryl, heteroaryl, quinolinyl, tetrahydroquinolinyl, l0-camphoryl, pentamethylchromanyl, pentaalkylphenyl, pentahalophenyl, trialkylphenyl, 3-carboxyphenyl, 3-trifluoromethylphenyl or 4-carboxyphenyl;

including pharmaceutically acceptable salts thereof.

Preferred compounds of formula I(B) are:

- The compound of formula I(B) wherein $R_b'$ is $-A^1-$, or $-A_1-NH-$.
- The compound of formula I(B) wherein $R_b'$ is

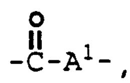, or 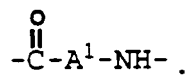.

- The compound of formula I(B) wherein $R_b'$ is $-SO_2-A^1-$, or $-SO_2-A^1-NH-$.
- The compound of formula I(B) wherein $R_b'$ is $-(CH_2)_p-A^2-$.
- The compound of formula I(B) wherein $R_b'$ is

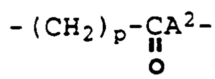

- The compound of formula I(B) wherein $R_b'$ is $-(CH_2)_p-SO_2A^2-$.
- The compound of formula I(B) wherein $R_b'$ is $-(CH_2)_p-A^3-$, or $-(CH_2)_p-A^3-NH-$.
- The compound of formula I(B) wherein $R_b'$ is $-(CH_2)_p-CO-A^3-$, or $-(CH_2)_p-CO-A^3-NH-$.
- The compound of formula I(B) wherein $R_b'$ is $-(CH_2)_p-SO_2-A^3-$, or $-(CH_2)_p-SO_2-A^3-NH-$.
- The compound of formula I(B) wherein n is O.

## Claims

1. A compound having the structure

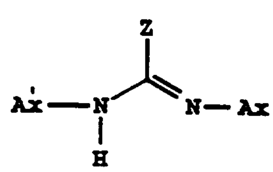

wherein Z is a substructure which when linked to

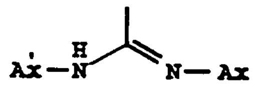

forms a prodrug of a pharmaceutically active compound; with the proviso that Z is exclusive of boron containing moieties, and Ax and A'x may be the same or different and are independently selected from Acyl, H or alkyl, with the proviso that (I) at least one of Ax and A'x is Acyl; and wherein (2) Acyl includes the moiety

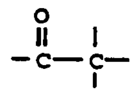

wherein

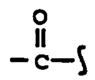

is linked to a nitrogen atom and (3) Acyl is exclusive of a group which includes the moiety

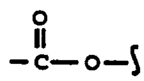

wherein

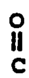

is linked to a nitrogen atom; including all stereoisomers thereof, and pharmaceutically acceptable salts thereof.

**2.** The compound as defined in Claim I wherein
Acyl is

(1) 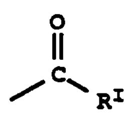 ;

(2) 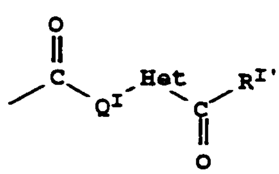 ;

(3) 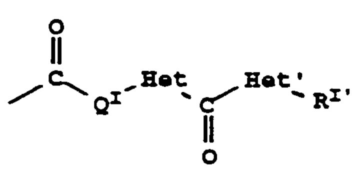 or

(4) 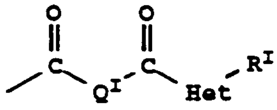 ;

wherein $R^I$ is H, alkyl, cycloalkyl, aryl, heterocycloalkyl, heteroaryl, substituted alkyl or substituted aryl; and

$R^{I'}$ is alkyl, cycloalkyl, aryl, heterocycloalkyl, heteroaryl, substituted alkyl, or substituted aryl;

Het and Het' are independently O, NH, N-loweralkyl or S;

$Q^I$ is alkyl, cycloalkyl, aryl, heterocycloalkyl, substituted alkyl, substituted aryl or heteroaryl.

**3.** The compound as defined in Claim 2 wherein one of Ax or A'x is

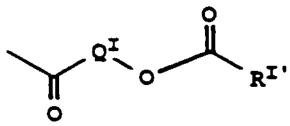

and the other is H.

**4.** The compound as defined in Claim 2 wherein one of Ax or A'x is

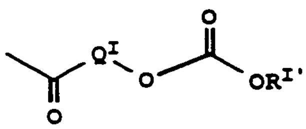

and the other is H.

**5.** The compound as defined in Claim 2 wherein one of Ax or A'x is

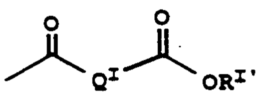

and the other is H.

**6.** The compound as defined in Claim I wherein one of Ax or **A'x** is

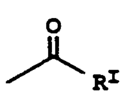 or

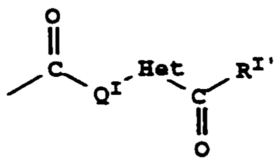

where Het is O, $R^I$ is alkyl or arylalkyl, $R^{I'}$ is alkyl or arylalkyl and $Q^I$ is alkyl or cycloalkyl, and the other of Ax or **A'x** is H.

**7.** The compound as defined in Claim I
wherein Z when linked to

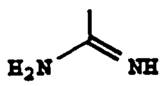

has activity as a thrombin inhibitor, a platelet aggregation inhibitor, a fibrinogen receptor antagonist, a GPIIb/IIIa receptor blocker, an antihypertensive, an antidepressant, an antibiotic, a viricide, an immunostimulant, an anti-inflammatory agent, a peptide hydrolase inhibitor, a Factor Xa inhibitor, an antianaphylatic or an antiulcer agent.

**8.** The compound as defined in Claim I
wherein Z when linked to

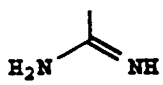

is a thrombin inhibitor.

**9.** A prodrug of a guanidine, thioguanidine or amidine-containing pharmaceutically active compound, the prodrug moiety being linked to the guanidine, thioguanidine or amidine portion of the pharmaceutically active compound as shown in the formula

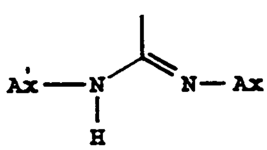

with the proviso that the pharmaceutically active compound is exclusive of boron containing moieties, and Ax and A'x may be the same or different and are independently selected from Acyl, H or alkyl, with the proviso that (I) at least one of Ax and A'x is Acyl and wherein (2) Acyl includes the moiety

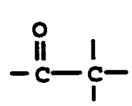

wherein

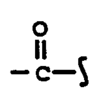

is linked to a nitrogen atom and (3) Acyl is exclusive of a group which includes the moiety

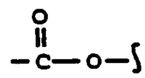

wherein

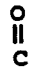

is linked to a nitrogen atom; including all stereoisomers thereof, and pharmaceutically acceptable salts thereof.

**10.** The compound as defined in Claim 9
wherein Acyl is

(1) 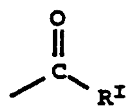 ;

(2) 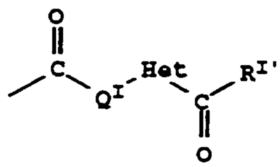 ;

(3) 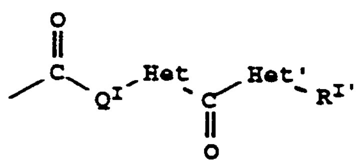 or

(4) 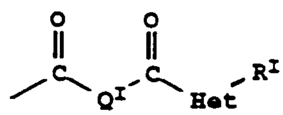 ;

wherein $R^I$ is H, alkyl, cycloalkyl, aryl, heterocycloalkyl, heteroaryl, substituted alkyl or substituted aryl; and
$R^{I'}$ is alkyl, cycloalkyl, aryl, heterocycloalkyl, heteroaryl, substituted alkyl, or substituted aryl;
Het and Het' are independently O, NH, N-loweralkyl or S;
$Q^I$ is alkyl, cycloalkyl, aryl, heterocycloalkyl, substituted alkyl, substituted aryl or heteroaryl.

**11.** A compound having the structure

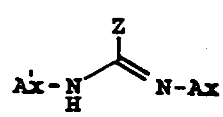

wherein Ax and A'x may be the same or different and are independently selected from Acyl, H or alkyl, with the proviso that (I) at least one of Ax and A'x is Acyl; and wherein (2) Acyl includes the moiety

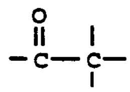

wherein

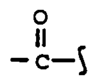

is linked to a nitrogen atom and (3) Acyl is exclusive of a group which includes the moiety

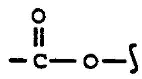

wherein

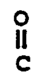

is linked to a nitrogen atom; including all stereoisomers thereof, and pharmaceutically acceptable salts thereof, and wherein Z is

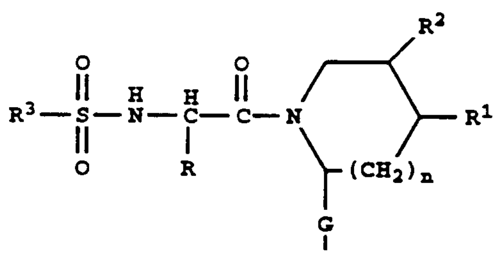

including all stereoisomers, wherein n is 0, l or 2;

G is an amido moiety which optionally includes a cyclic member;

R is hydrogen, hydroxyalkyl, aminoalkyl, alkyl, cycloalkyl, cycloalkylalkyl, aryl, arylalkyl, alkenyl, alkynyl, amidoalkyl, arylalkoxyalkyl or an amino acid side chain, either protected or unprotected;

$R^1$ and $R^2$ are independently hydrogen, lower alkyl, cycloalkyl, aryl, hydroxy, alkoxy, oxo, thioxo, thioketal, thioalkyl, thioaryl, amino or alkylamino; or $R^1$ and $R^2$ together with the carbons to which they are attached form a cycloalkyl, aryl or heteroaryl ring;

$R^3$ is alkyl, arylalkyl, aryl, heteroaryl, quinolinyl, tetrahydroquinolinyl, l0-camphoryl or pentamethylchromanyl, pentaalkylphenyl, pentahalophenyl, trialkylphenyl, 3-carboxyphenyl, 3-trifluoromethylphenyl or 4-carboxyphenyl;

wherein G is

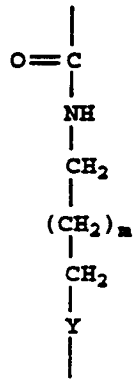

or

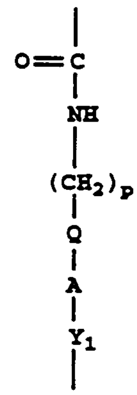

m is 0, l, 2 or 3;
Y is NH or S;
p is 0, l or 2;
Q is a single bond or

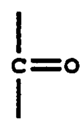

;

$Y_1$ is a bond or -NH-;
A is aryl or cycloalkyl, or an azacycloalkyl ring A of 4 to 8 ring members, or an azacycloalkenyl ring A of 5 to 9 ring members, or an azaheterocycloalkyl ring A of 6 to 8 ring members,

A

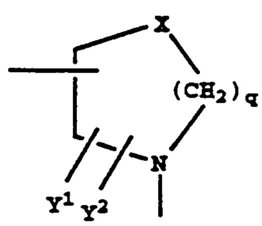

where X is $CH_2$, -CH=CH-, O, S or NH;
q is 0, l, 2, 3 or 4 if X is $CH_2$ or CH=CH;
q is 2, 3 or 4 if X is O, S or NH;
$Y^1$ and $Y^2$ are independently H, lower alkyl, oxo or halo;
if A is an azacycloalkyl, azacycloalkenyl, or azaheterocycloalkyl ring A, then $Y_1$ is a bond and the acylamidine group

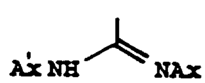

is attached to the nitrogen atom in the ring as indicated below

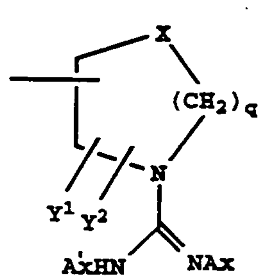

;

including pharmaceutically acceptable salts thereof; or
wherein Z is

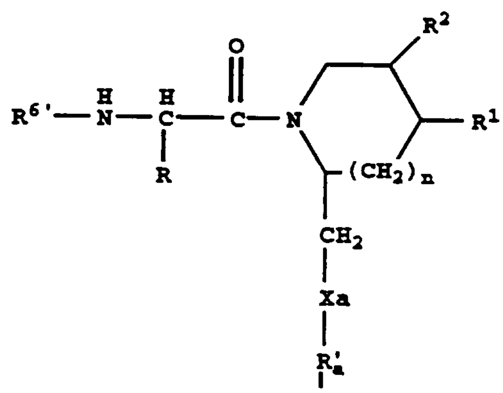

including all stereoisomers, wherein n is 0, I or 2;

Xa is S, SO, $SO_2$ or O;

$R_a'$ is $-A^1-$, or $A^1$-NH-, where $A^1$ is an alkyl, alkenyl, or alkynyl chain; with the proviso that there is at least one carbon between any S or O and an alkenyl or alkynyl moiety; or

$R_a'$ is $-(CH_2)_p-A^2-$ where $A^2$ is an azacycloalkyl ring A of 4 to 8 ring members, or an azacycloalkenyl ring A of 5 to 9 ring members, or an azaheterocycloalkyl ring A of 6 to 8 ring members,

A 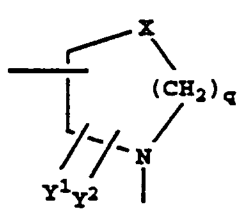

where X is $CH_2$, -CH=CH-, O, S or NH; p is 0, I or 2; and provided that the

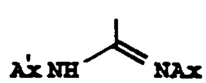

group is attached to the nitrogen atom in the ring as indicated below

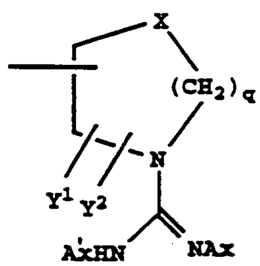 ;

q is 0, l, 2, 3 or 4 if X is $CH_2$ or CH=CH;

q is 2, 3 or 4 if X is O, S or NH;

$Y^1$ and $Y^2$ are independently H, lower alkyl, oxo or halo;

provided that where X is a hetero atom, that is, $A^2$ is azaheteroalkyl, then there must be at least a 2-carbon chain between X and any heteroatom in the ring A or outside ring A; or

$R_a'$ is $-(CH_2)_p-A^3-NH-$ or $-(CH_2)_p-A^3-$, wherein $A^3$ is aryl or cycloalkyl;

R is hydrogen, hydroxyalkyl, aminoalkyl, alkyl, cycloalkyl, cycloalkylalkyl, aryl, arylalkyl, alkenyl, alkynyl, amidoalkyl, arylalkoxyalkyl or an amino acid side chain, either protected or unprotected;

$R^1$ and $R^2$ are independently hydrogen, lower alkyl, cycloalkyl, aryl, hydroxy, alkoxy, oxo, thioxo, thioketal, thioalkyl, thioaryl, amino or alkylamino; or $R^1$ and $R^2$ together with the carbons to which they are attached form a cycloalkyl, aryl, or heteroaryl ring;

$R^{6'}$ is hydrogen,

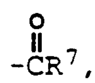

$-SO_2R^3$ or $-CO_2R^7$ wherein $R^7$ is lower alkyl, aryl, arylalkyl, cycloheteroalkyl or heteroaryl; and

$R^3$ is alkyl, arylalkyl, aryl, heteroaryl, quinolinyl, tetrahydroquinolinyl, l0-camphoryl, pentamethylchromanyl, pentaalkylphenyl, pentahalophenyl, trialkylphenyl, 3-carboxyphenyl, 3-trifluoromethylphenyl or 4-carboxyhenyl;

including pharmaceutically acceptable salts thereof; or

wherein Z is

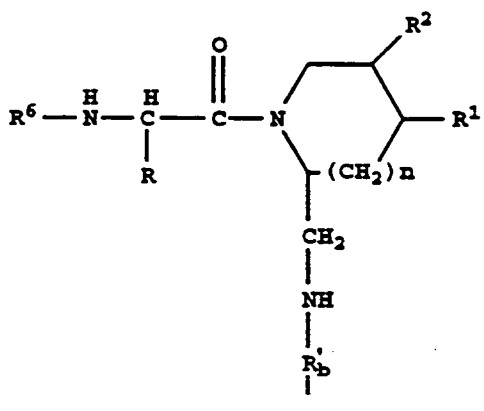

including all stereoisomers, wherein n is 0, l or 2;

$R_b'$ is $-A^1-$, $-CO-A^1-$ or $-SO_2-A^1-$, $-A^1-NH-$, $-CO-A^1-NH-$ or $-SO_2A^1-NH$, wherein $A^1$ is an alkyl, alkenyl or alkynyl chain; with the proviso that there is at least one carbon between any NH, S or O, and an alkenyl or alkynyl moiety; or

$R_b'$ is $-(CH_2)_p-A^2-$, $-(CH_2)_p-CO-A^2-$ or $-(CH_2)_p-SO_2-A^2-$ where p is 0, l or 2; and

$A^2$ is an azacycloalkyl ring A of 4 to 8 ring members, or an azacycloalkenyl ring A of 5 to 9 ring members, or an azaheterocycloalkyl ring A of 6 to 8 ring members,

A

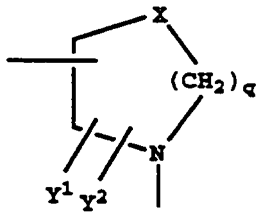

where X is $CH_2$, -CH=CH-, O, S or NH; and the

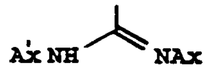

group is attached to the nitrogen atom in the ring as indicated below

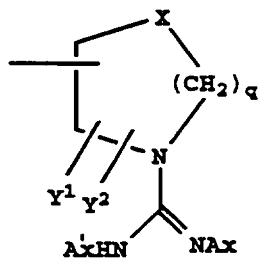

;

q is 0 l, 2, 3 or 4 if X is $CH_2$ or -CH=CH-;

q is 2, 3 or 4 if X is O, S or NH;

$Y^1$ and $Y^2$ are independently H, lower alkyl, oxo or halo;

provided that where X is a hetero atom, that is, A is azaheteroalkyl, then there must be at least a 2-carbon chain between X and any N atom in the ring A or outside ring A.

$R_b$ is $-(CH_2)_p-A^3-$, $-(CH_2)_p-CO-A^3-$, or $-(CH_2)_p-SO_2-A^3-$, $-(CH_2)_p-A^3-NH-$, $-(CH_2)_p-CO-A^3-NH-$ or $-(CH_2)_p-SO_2-A^3-NH$, wherein $A^3$ is aryl or cycloalkyl;

R is hydrogen, hydroxyalkyl, aminoalkyl, alkyl, cycloalkyl, cycloalkylalkyl, aryl, arylalkyl, alkenyl, alkynyl, amidoalkyl, arylalkoxyalkyl or an amino acid side chain, either protected or unprotected;

$R^1$ and $R^2$ are independently hydrogen, lower alkyl, cycloalkyl, aryl, hydroxy, alkoxy, oxo, thioxo, thioketal, thioalkyl, thioaryl, amino or alkylamino; or $R^1$ and $R^2$ together with the carbons to which they are attached form a cycloalkyl, aryl, or heteroaryl ring;

$R^6$ is hydrogen,

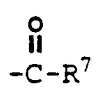

,

$-SO_2R^3$ or $-CO_2R^7$,

wherein $R^7$ is lower alkyl, aryl, arylalkyl, cycloheteroalkyl or heteroaryl; and

$R^3$ is alkyl, arylalkyl, aryl, heteroaryl, quinolinyl, tetrahydroquinolinyl, l0-camphoryl, pentamethylchromanyl, pentaalkylphenyl, pentahalophenyl, trialkylphenyl, 3-carboxyphenyl, 3-trifluoromethylphenyl or 4-carboxyphenyl;

including pharmaceutically acceptable salts thereof; or

where Z is

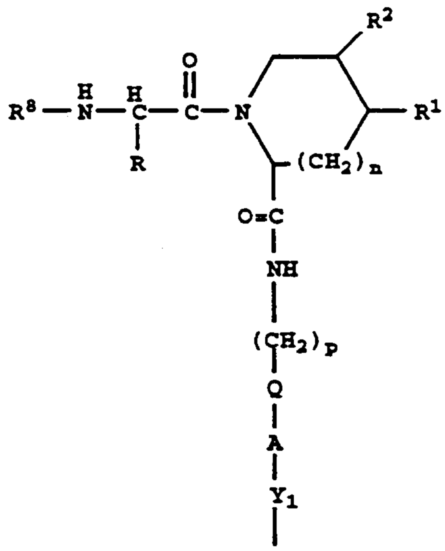

including all stereoisomers, wherein n is 0, I or 2;

$R^8$ is H,

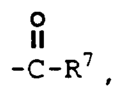

or $-CO_2R^7$ wherein $R^7$ is lower alkyl, aryl, arylalkyl, cycloheteroalkyl or heteroaryl;

R is hydrogen, hydroxyalkyl, aminoalkyl, alkyl, cycloalkyl, cycloalkylalkyl, aryl, arylalkyl, alkenyl, alkynyl, amidoalkyl, arylalkoxyalkyl or an amino acid side chain, either protected or unprotected;

$R^1$ and $R^2$ are independently hydrogen, lower alkyl, cycloalkyl, aryl, hydroxy, alkoxy, oxo, thioxo, thioketal, thioalkyl, thioaryl, amino or alkylamino; or $R^1$ and $R^2$ together with the carbons to which they are attached form a cycloalkyl, aryl, or heteroaryl ring;

p is 0, I or 2;

Q is a single bond or C=O;

$Y_1$ is a bond or -NH-;

A is aryl or cycloalkyl, or an azacycloalkyl ring A of 4 to 8 ring members, or an azacycloalkenyl ring A of 5 to 9 ring members, or an azaheterocycloalkyl ring A of 6 to 8 ring members,

A

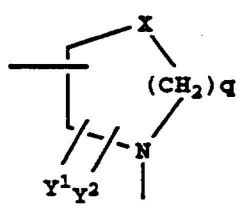

wherein X is $CH_2$, -CH=CH-, O, S or NH;

if A is an azacycloalkyl, azacycloalkenyl, or azaheterocycloalkyl ring A, then $Y_1$ is a bond and the acylamidine group

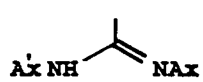

is attached to the nitrogen atom in the ring as indicated below

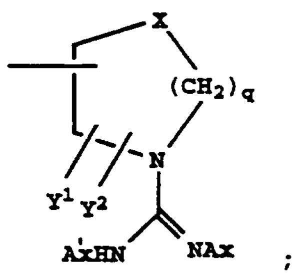

q is 0, l, 2, 3 or 4 if X is $CH_2$ or -CH=CH-;

q is 2, 3 or 4 if X is O, S or NH;

$Y^1$ and $Y^2$ are independently H, lower alkyl, oxo or halo;

provided that where X is a hetero atom, that is, A is azaheterocycloalkyl, then there must be at least a 2-carbon chain between X and any N atom in the ring A or outside ring A; or

wherein Z is

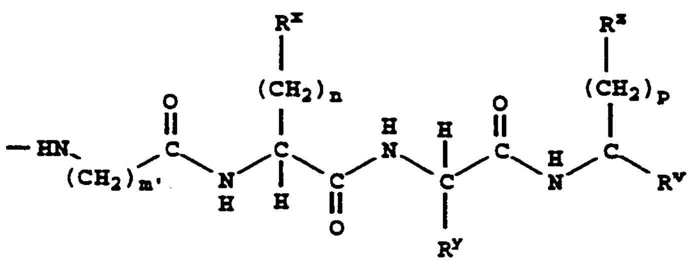

including all stereoisomers thereof wherein m' is 2, 3, 4 or 5; n is 0, l or 2; p is 0, l or 2;

$R^x$ is cycloalkyl, heteroaryl, $CO_2H$, $CONR^sR^t$ (where $R^s$ and $R^t$ are independently selected from H, alkyl, cycloalkyl, cycloheteroalkyl, aryl, or heteroaryl), or aryl optionally substituted with $NO_2$, OH, alkoxy, acyloxy,

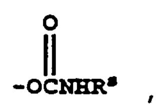

halogen, alkyl, aryl, $CO_2$alkyl, CONHalkyl, alkylthio, arylthio, NHalkyl or NHcycloalkyl;

$R^y$ is an amino acid sidechain;

$R^z$ is H, alkyl, cycloalkyl, aryl, cycloheteroalkyl or heteroaryl;

$R^v$ is H, alkyl, $CO_2R^u$ or $CONR^sR^t$;

wherein $R^u$ is H or alkyl;

including pharmaceutically acceptable salts thereof; or

wherein Z is

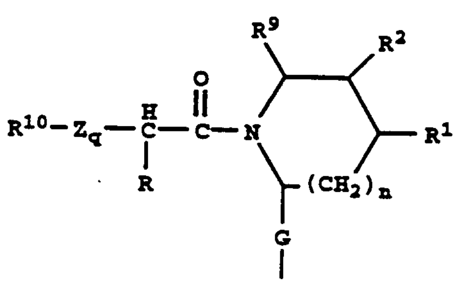

including all stereoisomers, wherein n is 0, l or 2;

Zq is $NR^{11}$ or O, where $R^{11}$ is H, lower alkyl, aryl or arylalkyl;

G is an amido moiety which is

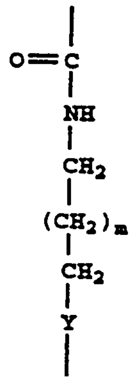 or 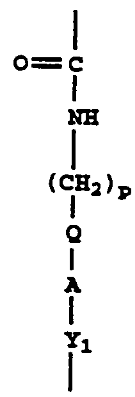

R is hydrogen, hydroxyalkyl, hydroxyalkyl (alkyl), aminoalkyl, lower alkyl, cycloalkyl, cycloalkylalkyl, arylalkyl, alkenyl, alkynyl, amidoalkyl, arylalkoxyalkyl or an amino acid side chain, either protected or unprotected;

$R^9$ is lower alkyl, cycloalkyl, aryl, or arylalkyl; or $R^9$ and $R^2$ together with the carbons to which they are attached form a cycloalkyl, aryl or heteroaryl ring;

$R^2$ and $R^1$ are independently hydrogen, lower alkyl, cycloalkyl, aryl, arylalkyl, hydroxy, alkoxy, oxo, thioketal, thioalkyl, thioaryl, amino or alkylamino; or $R^2$ and $R^1$ together with the carbons to which they are attached form a cycloalkyl, aryl or heteroaryl ring;

$R^{10}$ is H, lower alkyl, arylalkyl, aryl,

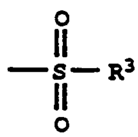 , 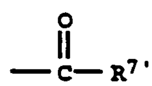

or $-CO_2R^{7'}$, where $R^{7'}$ is lower alkyl, aryl, arylalkyl, cycloheteroalkyl, heteroaryl, quinolinyl or tetrahydroquinolinyl; and

$R^3$ is alkyl, arylalkyl, aryl, heteroaryl, quinolinyl, tetrahydroquinolinyl, l0-camphoryl, pentamethylchromanyl, pentaalkylphenyl, pentahalophenyl, trialkylphenyl, 3-carboxyphenyl, 3-trifluoromethylphenyl or 4-carboxyphenyl;

n is 0, l or 2;

m is 0, l, 2 or 3;

p is 0, l or 2;

Q is a single bond or

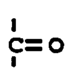 ;

Y is NH or S;

$Y_1$ is a bond or -NH-;

A is aryl or cycloalkyl, or an azacycloalkyl ring A of 4 to 8 ring members, or an azacycloalkenyl ring A of 4 to 8 ring members, or an azaheterocycloalkyl ring A of 6 to 8 ring members,

A 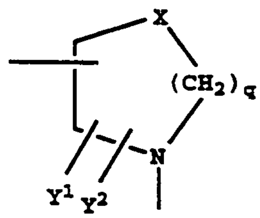

where X is $CH_2$, -CH=CH-, O, S or NH;

if A is an azacycloalkyl, azacycloalkenyl or azaheterocycloalkyl ring A, then $Y_1$ is a bond and the acylamidine group

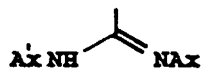

is attached to the nitrogen atom in the ring as indicated below

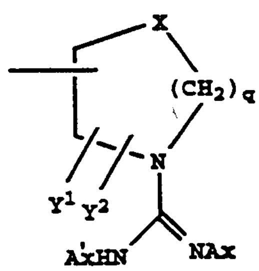 ;

q is 0, l, 2, 3 or 4 if X is $CH_2$ or -CH=CH-;

q is 2, 3 or 4 if X is O, S or NH;

$Y^1$ and $Y^2$ are independently H, lower alkyl, oxo or halo;

provided that where X is a hetero atom, that is, A is azaheteroalkyl, then there must be at least a 2-carbon chain between X and any N atom in the ring A or outside ring A; and

$R^{10}$ is hydrogen,

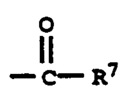

or $-CO_2R^7$, wherein $R^7$ is lower alkyl, aryl, arylalkyl or cycloheteroalkyl;

including pharmaceutically acceptable salts thereof.

**12.** A compound having the structure

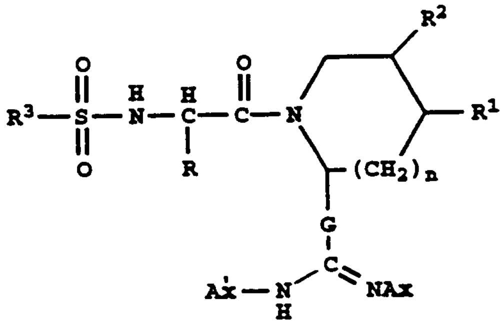

including all stereoisomers, wherein n is 0, l or 2;

G is an amido moiety which optionally includes a cyclic member;

R is hydrogen, hydroxyalkyl, aminoalkyl, alkyl, cycloalkyl, cycloalkylalkyl, aryl, arylalkyl, alkenyl, alkynyl, amidoalkyl, arylalkoxyalkyl or an amino acid side chain, either protected or unprotected;

$R^1$ and $R^2$ are independently hydrogen, lower alkyl, cycloalkyl, aryl, hydroxy, alkoxy, oxo, thioxo, thioketal, thioalkyl, thioaryl, amino or alkylamino; or $R^1$ and $R^2$ together with the carbons to which they are attached form a cycloalkyl, aryl or heteroaryl ring;

$R^3$ is alkyl, arylalkyl, aryl, heteroaryl, quinolinyl, tetrahydroquinolinyl; l0-camphoryl, pentamethylchromanyl, pentaalkylphenyl, pentahalophenyl, trialkylphenyl, 3-carboxyphenyl, 3-trifluoromethylphenyl or 4-carboxyphenyl;

and Ax and A'x may be the same or different and are independently selected from Acyl, H or alkyl, with the proviso that (l) at least one of Ax and A'x is Acyl; (2) Acyl includes the moiety

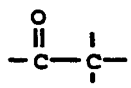

wherein

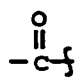

is linked to a nitrogen atom; and (3) Acyl is exclusive of a group which includes the moiety

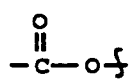

wherein

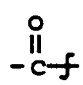

is linked to a nitrogen atom;

including all stereoisomers thereof and pharmaceutically acceptable salts thereof.

**13.** The compound as defined in Claim 12
wherein G is

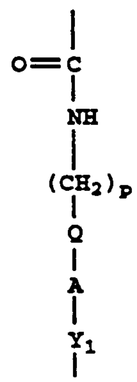

wherein p is 0, I or 2;

Q is a single bond or

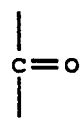

;

$Y_1$ is a bond or -NH-;

A is aryl or cycloalkyl, or an azacycloalkyl ring A of 4 to 8 ring members, or an azacycloalkenyl ring A of 5 to 9 ring members, or an azaheterocycloalkyl ring A of 6 to 8 ring members, of the structure

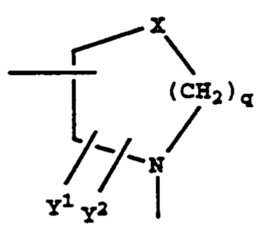

where X is $CH_2$, -CH=CH-, O, S or NH;

if A is an azacycloalkyl, azacycloalkenyl or azaheterocycloalkyl ring, then $Y_1$ is a bond and the

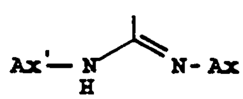

is attached to the nitrogen atom in the ring;

q is 0, I, 2, 3 or 4, provided that

q is 0, I, 2, 3 or 4 if X is $CH_2$ or -CH=CH-;

q is 2, 3 or 4 if X is O, S or NH;

$Y^1$ and $Y^2$ are independently H, lower alkyl, oxo or halo;

with the proviso that where A is azaheteroalkyl, then there must be at least a 2-carbon chain between X and any N atom in the ring or outside the ring.

**14.** The compound as defined in Claim 12 wherein G is

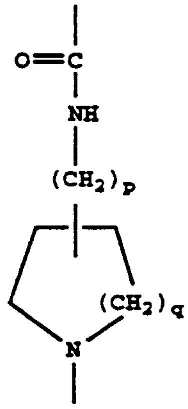

q is 1 or 2, p is l or 2,
$R^1$ and $R^2$ are each H, $R^3$ is alkyl, arylalkyl or aryl, n is 0 or l and R is aralkyl or hydroxyalkyl.

**15.** The compound as defined in Claim l4 having the structure

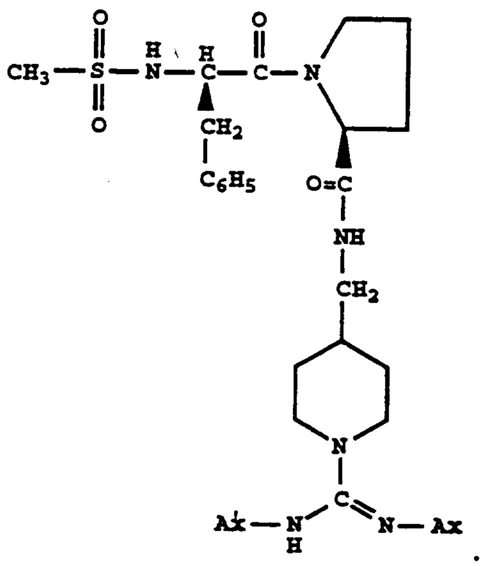

**16.** The compound as defined in Claim l2 wherein n is 0 or l, m is 2, $R^3$ is aryl, arylalkyl or alkyl, R is arylalkyl or hydroxyalkyl, $R^1$ is H or alkyl, $R^2$ is H and Y is -NH-.

**17.** Use of a compound as defined in claim l for manufacturing a medicament for inhibiting or preventing formation of blood clots.

**18.** A pharmaceutical composition comprising a compound as defined in Claim l and a pharmaceutically acceptable carrier therefor.

**19.** The compound as defined in Claim II having the structure

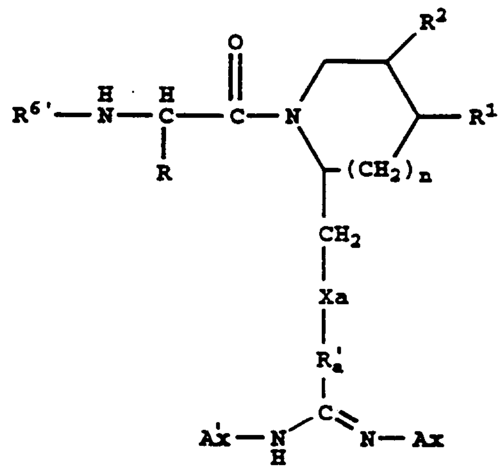

including all stereoisomers, wherein n is 0, I or 2;

Xa is S, SO, $SO_2$ or O;

$R_a'$ is $-A^1-$ or $-A^1-NH-$, where $A^1$ is an alkyl, alkenyl, or alkynyl chain; with the proviso that there is at least one carbon between any S or O and an alkenyl or alkynyl moiety; or

$R_a'$ is $-(CH_2)_p-A^2-$ where $A^2$ is an azacycloalkyl ring A of 4 to 8 ring members, or an azacycloalkenyl ring A of 5 to 9 ring members, or an azaheterocycloalkyl ring A of 6 to 8 ring members,

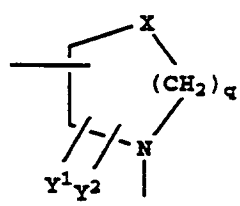

where X is $CH_2$, -CH=CH-, O, S, NH; and provided that

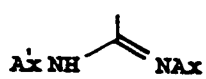

group is attached to the nitrogen atom in the ring as indicated below

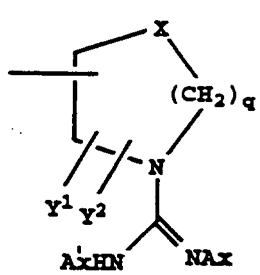

;

q is 0, I, 2, 3 or 4 if X is $CH_2$ or -CH=CH-;

q is 2, 3 or 4 if X is O, S or NH;

$Y^1$ and $Y^2$ are independently H, lower alkyl, oxo or halo;

provided that where X is a hetero atom, that is, $A^2$ is azaheterocycloalkyl, then there must be at least a 2-carbon chain between X and any heteroatom in the ring A or outside ring A; or

$R_a'$ is $-(CH_2)_p-A^3-$ or $-(CH_2)_p-A^3-NH-$, where $A^3$ is aryl or cycloalkyl;

$R^1$ and $R^2$ are independently hydrogen, lower alkyl, cycloalkyl, aryl, hydroxy, alkoxy, keto, thioketal, thioalkyl, thioaryl, amino or alkylamino, or $R^1$ and $R^2$ together with the carbons to which they are attached form a cycloalkyl, aryl or heteroaryl ring;

R is hydrogen, hydroxyalkyl, aminoalkyl, alkyl, cycloalkyl, cycloalkylalkyl, aryl, arylalkyl, alkenyl, alkynyl, amidoalkyl, arylalkoxyalkyl or an amino acid side chain, either protected or unprotected;

$R^{6'}$ is hydrogen,

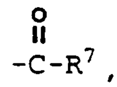

$-SO_2R^3$ or $-CO_2R^7$ wherein $R^7$ is lower alkyl, aryl, arylalkyl, cycloheteroalkyl or heteroaryl; and

$R^3$ is alkyl, arylalkyl, aryl, heteroaryl, quinolinyl, tetrahydroquinolinyl, l0-camphoryl, pentamethylchromanyl, pentaalkylphenyl, pentahalophenyl, trialkylphenyl, 3-carboxyphenyl, 3-trifluoromethylphenyl or 4-carboxyphenyl;

including pharmaceutically acceptable salts thereof.

**20.** The compound as defined in Claim l9 wherei n

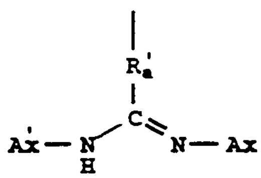

is

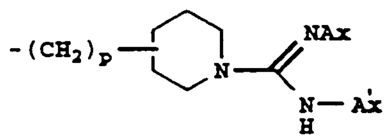

where p is 1 or 2.

**21.** The compound as defined in Claim l9 wherein $R^{6'}$ is

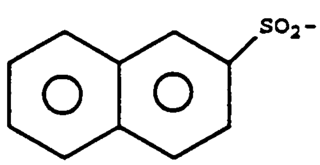

**22.** The compound as defined in Claim l9 wherein R is $-CH_2OH$ or H.

**23.** The compound as defined in Claim l9 wherein n is O, Xa is S or $SO_2$,

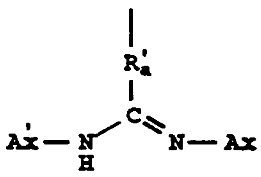

is

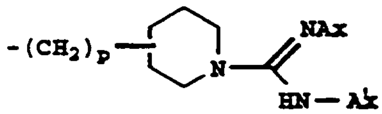

p is l or 2;

$R^1$ and $R^2$ are each H, R is $CH_2OH$ or H and $R^{6'}$ is

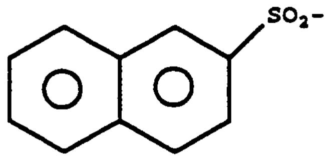

.

**24.** The compound as defined in Claim II having the structure

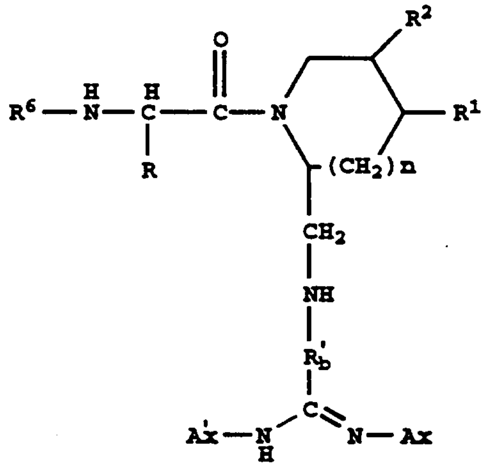

including all stereoisomers, wherein n is 0, I or 2;

$R_b'$ is $-A^1-$, $-CO-A^1-$, $-SO_2-A^1-$, $-A^1-NH-$, $-CO-A^1-NH-$, or $-SO_2A^1-NH-$; wherein $A^1$ is an alkyl, alkenyl or alkynyl chain; with the proviso that there is at least one carbon between any NH, S or O, and an alkenyl or alkynyl moiety; or

$R_b'$ is $-(CH_2)_p-A^2-$, $-(CH_2)_p-CO-A^2-$ or $-(CH_2)_p-SO_2-A^2-$ where p is 0, I or 2, and

$A^2$ is an azacycloalkyl ring A of 4 to 8 ring members, or an azacycloalkenyl ring A of 5 to 9 ring members, or an azaheterocycloalkyl ring A of 6 to 8 ring members,

A

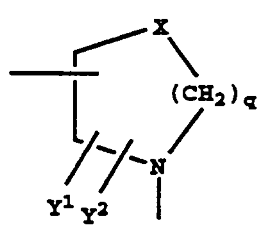

where X is $CH_2$, -CH=CH-, O, S or NH; and the

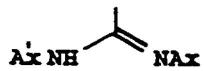

group is attached to the nitrogen atom in the ring as indicated below

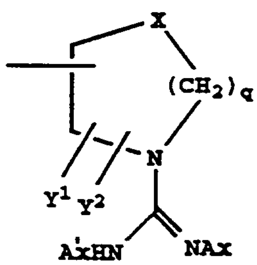

;

q is 0, l, 2, 3 or 4 if X is $CH_2$ or -CH=CH-;

q is 2, 3 or 4 if X is O, S or NH;

$Y^1$ and $Y^2$ are independently H, lower alkyl, oxo or halo;

provided that where X is a hetero atom, that is, A is azaheteroalkyl, then there must be at least a 2-carbon chain between X and any N atom in the ring A or outside ring A; or

$R_b'$ is $-(CH_2)_p-A^3-$, $-(CH_2)_p-CO-A^3-$, or $-(CH_2)_p-SO_2-A^3-$, $-(CH_2)_p-A^3-NH-$, $-(CH_2)_p-CO-A^3-NH$, $-(CH_2)_p-SO_2-A^3-NH$; wherein $A^3$ is aryl or cycloalkyl;

$R^1$ and $R^2$ are independently hydrogen, lower alkyl, cycloalkyl, aryl, hydroxy, alkoxy, keto, thioketal, thioalkyl, thioaryl, amino or alkylamino; or $R^1$ and $R^2$ together with the carbons to which they are attached form a cycloalkyl, aryl or heteroaryl ring;

R is hydrogen, hydroxyalkyl, aminoalkyl, alkyl, cycloalkyl, cycloalkylalkyl, aryl, arylalkyl, alkenyl, alkynyl, amidoalkyl, arylalkoxyalkyl or an amino acid side chain, either protected or unprotected; and

$R^6$ is hydrogen,

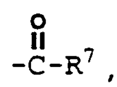

,

$-SO_2R^3$ or $-CO_2R^7$,

wherein $R^7$ is lower alkyl, aryl, arylalkyl, cycloheteroalkyl or heteroaryl; and

$R^3$ is alkyl, arylalkyl, aryl, heteroaryl, quinolinyl, tetrahydroquinolinyl, l0-camphoryl, pentamethylchromanyl, pentaalkylphenyl, pentahalophenyl, trialkylphenyl, 3-carboxyphenyl, 3-trifluoromethylphenyl or 4-carboxyphenyl;

including pharmaceutically acceptable salts thereof.

**25.** The compound as defined in Claim 24
wherein $R_b'$ is $-A^1-$, or $-A_1-NH-$.

**26.** The compound as defined in Claim 24
wherein $R^6$ is 2-naphthylsulfonyl, H, benzyloxycarbonyl, t-butoxycarbonyl or methylsulfonyl.

**27.** The compound as defined in Claim 24
wherein n is O, $R^6$ is 2-naphthylsulfonyl, H, methylsulfonyl, benzyloxycarbonyl or t-butoxycarbonyl, R is arylalkyl, hydroxyalkyl or benzyloxyalkyl, $R^1$ is H, $R^2$ is H and

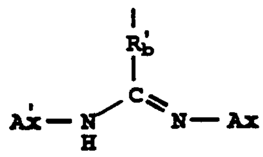 is

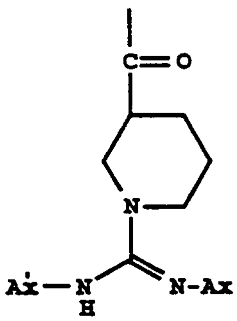 , 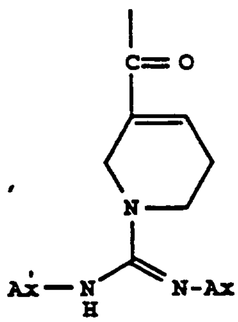 , 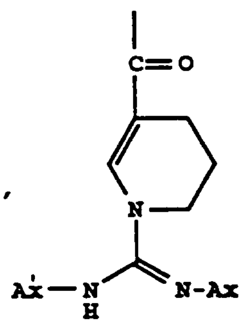 ,

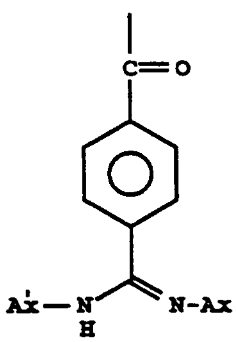 or 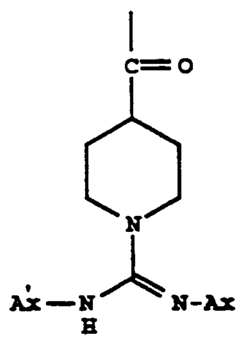 .

28. The compound as defined in Claim 24 having the structure

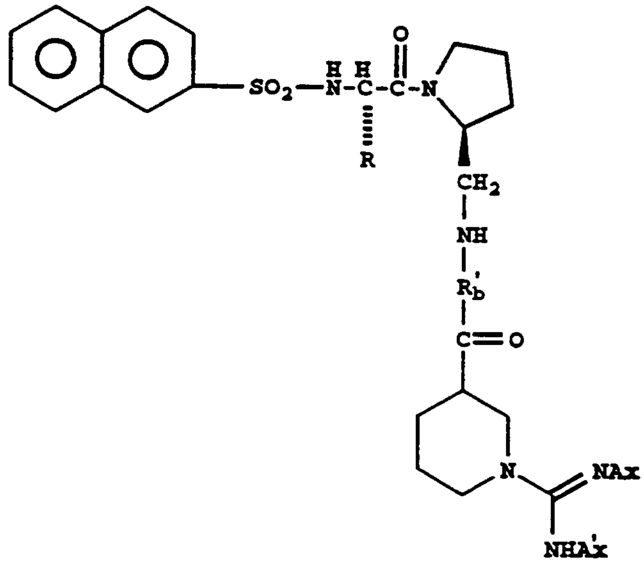 .

**29.** The compound as defined in Claim II having the following structure or name:

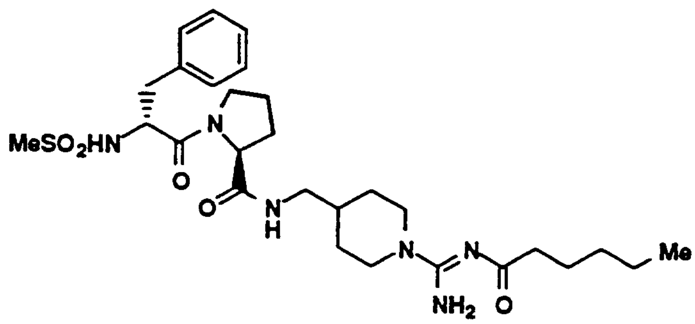

N-[[1-[amino[(1-oxohexyl)imino]methyl]-4-piperidinyl]methyl]-1-[N-(methylsulfonyl)-D-phenylalanyl]-L-prolinamide;

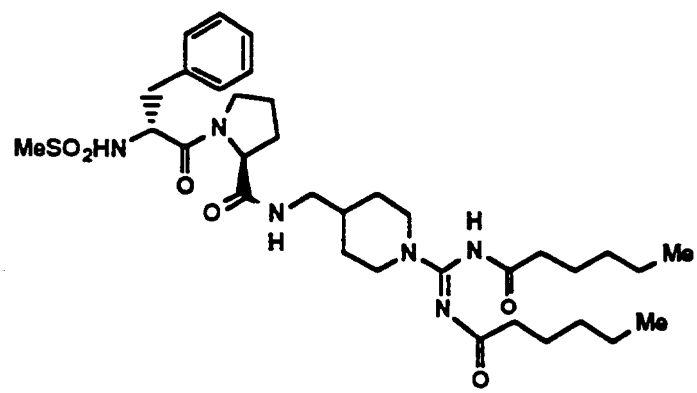

N-[[1-[[(1-oxohexyl)amino][(1-oxohexyl)imino]methyl]-4-piperidinyl]methyl]-1-N-[(methylsulfonyl)-D-phenylalanyl]-L-prolinamide;

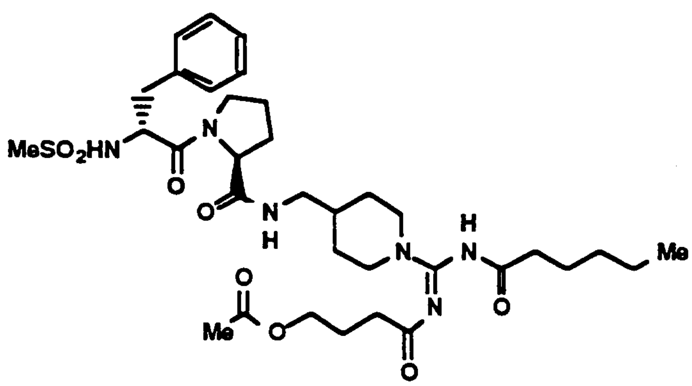

N-[[1-[[[4-(acetyloxy)-1-oxobutyl]imino][(1-oxohexyl]amino]methyl]-4-piperidinyl]methyl]-1-[N-(methylsulfonyl)-D-phenylalanyl]-L-prolinamide;

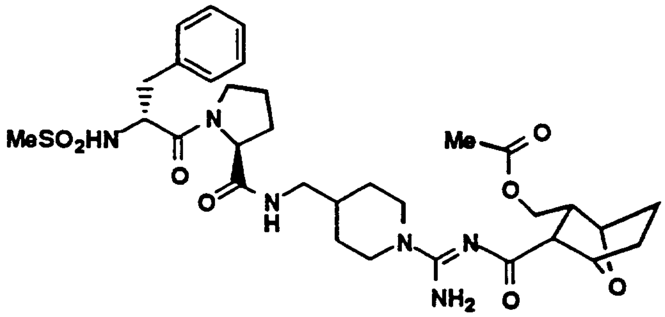

[1S-(exo,exo)]-N[[1-[[[[3-[(acetyloxy)methyl]-7-oxabicyclo[2.2.1]hept-2-yl]carbonyl]amino]iminomethyl]-4-piperidinyl]methyl-1-[N-(methylsulfonyl)-D-phenylalanyl]-L-prolinamide, trifluoroacetate;

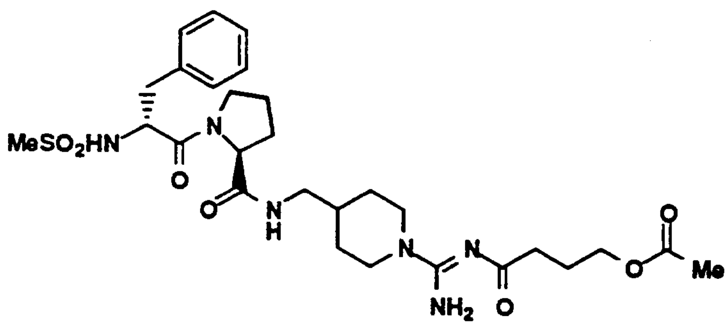

N-[[1-[[[[4-(acetyloxy)-1-oxobutyl]amino]iminomethyl]-4-piperidinyl]methyl]-1-[N-(methylsulfonyl)-D-phenylalanyl]-L-prolinamide;

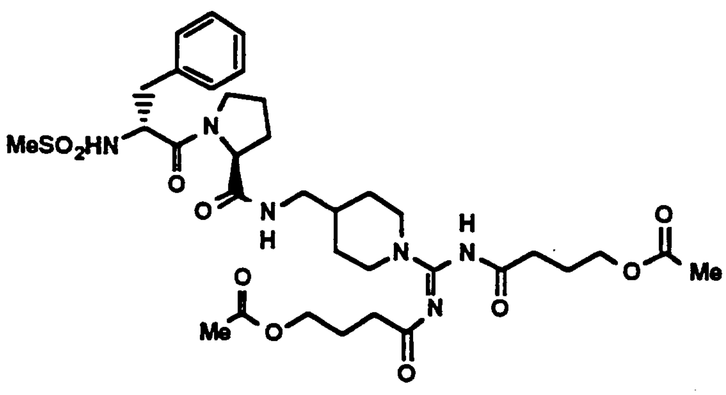

N-[[1-[[[4-(acetyloxy)-1-oxobutyl]amino][4-(acetyloxy)-1-oxobutyl]imino]methyl]-4-piperidinyl]methyl]-1-[N-(methylsulfonyl)-D-phenylalanyl]-L-prolinamide;

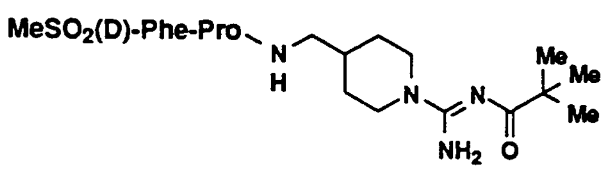

N-[[1-[amino[(2,2-dimethyl-1-oxopropyl)imino]methyl]-4-piperidinyl]methyl]-1-[N-(methylsulfonyl)-D-phenylalanyl]-L-prolinamide;

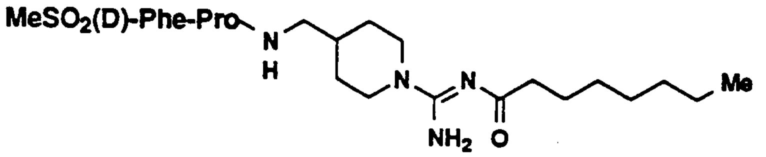

N-[[1-[amino[(1-oxooctyl)imino]methyl]-4-piperidinyl]methyl]-1-[N-(methylsulfonyl)-D-phenylalanyl]-L-prolinamide;

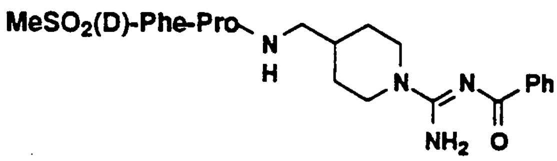

N-[[1-[amino(benzoylimino)methyl]-4-piperidinyl]methyl]-1-[N-(methylsulfonyl)-D-phenylalanyl]-L-prolinamide;

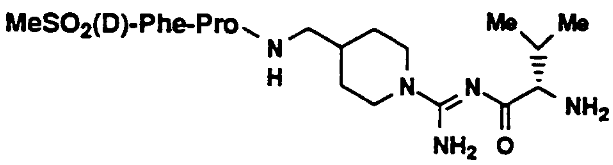

N-[[1-[amino(L-valylimino)methyl]-4-piperidinyl]methyl]-1-[N-(methylsulfonyl)-D-phenylalanyl]-L-prolinamide;

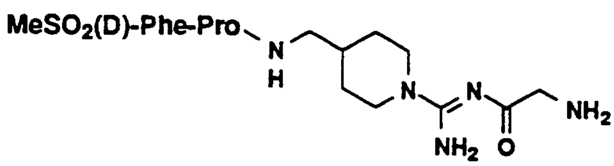

N-[[1-[amino[(aminoacetyl)imino]methyl]-4-piperidinyl]methyl]-1-[N-(methylsulfonyl)-D-phenylalanyl]-L-prolinamide;

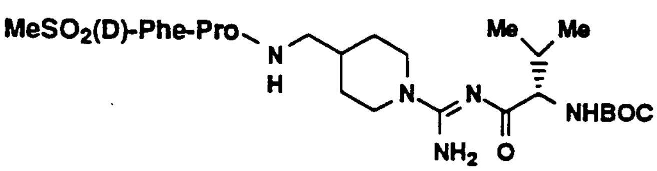

N-[[1-[amino[[N-[[(1,1-dimethylethoxy)carbonyl]amino]-L-valyl]imino]methyl]-4-piperidinyl]methyl]-1-[N-(methylsulfonyl)-D-phenylalanyl]-L-prolinamide;

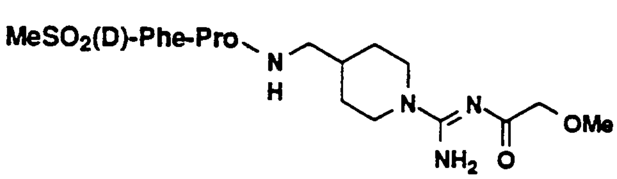

N-[[1-[amino[(methoxyacetyl)imino]methyl]-4-piperidinyl]methyl]-1-[N-(methylsulfonyl)-D-phenylalanyl]-L-prolina-

mide;

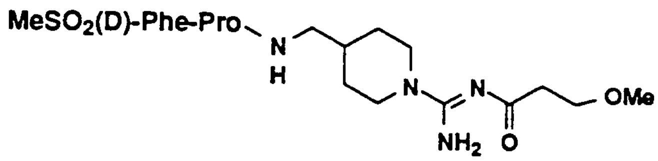

N-[[1-[aminol[(3-methoxy-1-oxopropyl)imino]methyl]-4-piperidinyl]methyl]-1-[N-(methylsulfonyl)-D-phenylalanyl]-L-prolinamide;

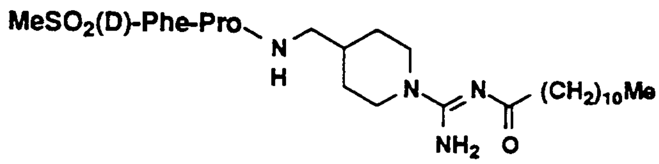

N-[[1-[amino[(1-oxododecyl)imino]methyl]-4-piperidinyl]methyl]-1-[N-(methylsulfonyl)-D-phenylalanyl]-L-prolinamide;

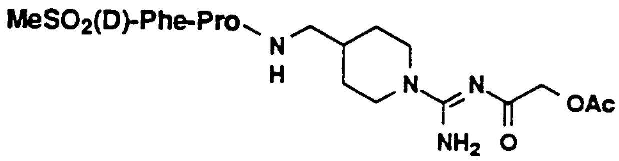

N-[[1-[[[(acetyloxy)acetyl]imino]aminomethyl]-4-piperidinyl]methyl]-1-[N-(methylsulfonyl)-D-phenylalanyl]-L-prolinamide;

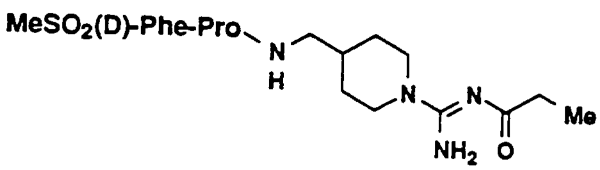

N-[[1-[amino[(1-oxopropyl)imino]methyl]-4-piperidinyl]methyl]-1-[N-(methylsulfonyl)-D-phenylalanyl]-L-prolinamide;

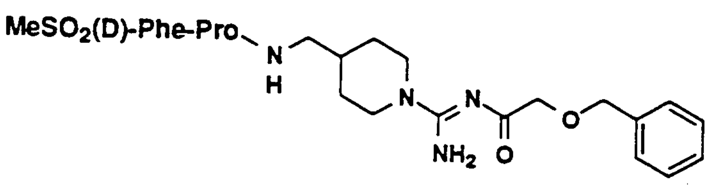

N-[[1-[amino[[(phenylmethoxy)acetyl]imino]methyl]-4-piperidinyl]methyl]-1-[N-(methylsulfonyl)-D-phenylalanyl]-L-prolinamide;

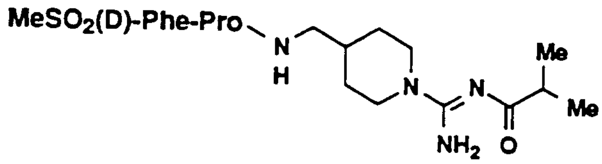

N-[[1-[amino[(2-methyl-1-oxopropyl)imino]methyl]-4-piperidinyl]methyl]-1-[N-(methylsulfonyl)-D-phenylalanyl]-L-prolinamide;

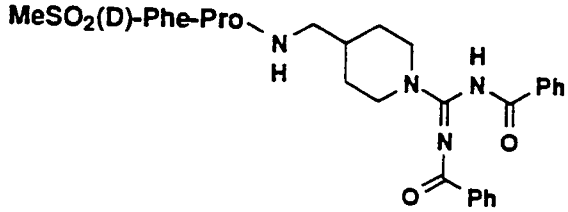

N-[[1-[(benzoylamino)(benzoylimino)methyl]-4-piperidinyl]methyl]-1-[N-(methylsulfonyl)-D-phenylalanyl]-L-prolinamide;

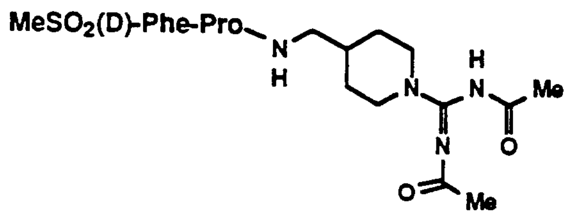

N-[[1-[(acetylamino)(acetylimino)methyl]-4-piperidinyl]methyl]-1-[N-(methylsulfonyl)-D-phenylalanyl]-L-prolinamide;

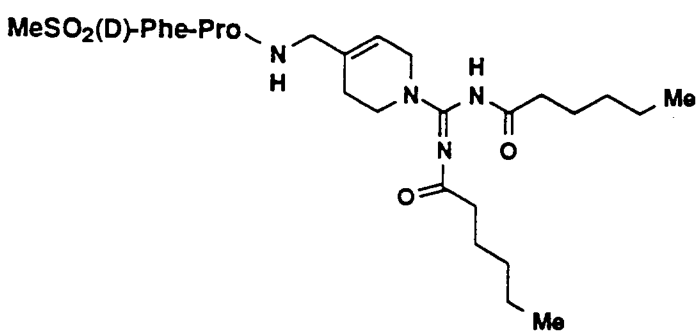

1-[N-(methylsulfonyl)-D-phenylalanyl]-N-[[1,2,3,6-tetrahydro-1-[[(1-oxohexyl)amino][(1-oxohexyl)imino]methyl]-4-pyridinyl]methyl]-L-prolinamide;

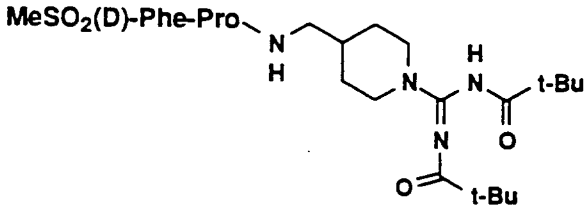

N-[[1-[[(2,2-dimethyl-1-oxopropyl)amino][(2,2-dimethyl-1-oxopropyl)imino]methyl]-4-piperidinyl]methyl]-1-[N-(methylsulfonyl)-D-phenylalanyl]-L-prolinamide;

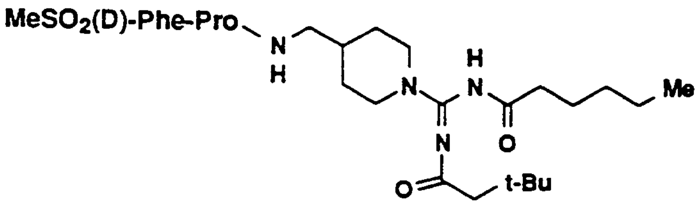

N-[[1-[[(3,3-dimethyl-1-oxobutyl)imino][(1-oxohexyl)amino]methyl]-4-piperidinyl]methyl]-1-[N-(methylsulfonyl)-D-phenylalanyl]-L-prolinamide;

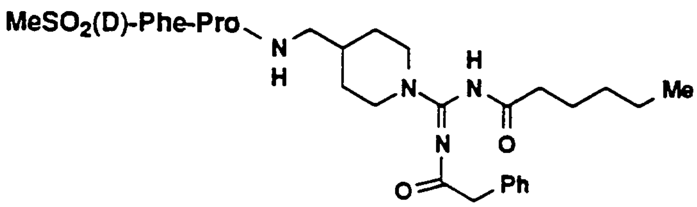

N-[[1-[[(1-oxohexyl)amino][(phenylacetyl)imino]methyl]-4-piperidinyl]methyl]-1-[N-(methylsulfonyl)-D-phenylalanyl]-L-prolinamide;

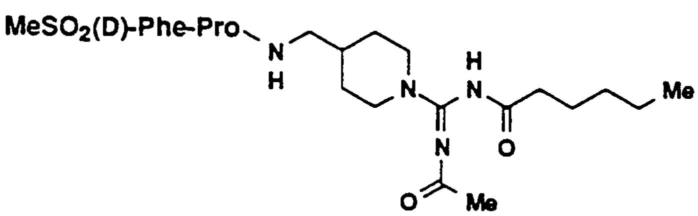

N-[[1-[(acetylimino)[(1-oxohexyl)amino]methyl]-4-piperidinyl]methyl]-1-[N-(methylsulfonyl)-D-phenylalanyl]-L-prolinamide;

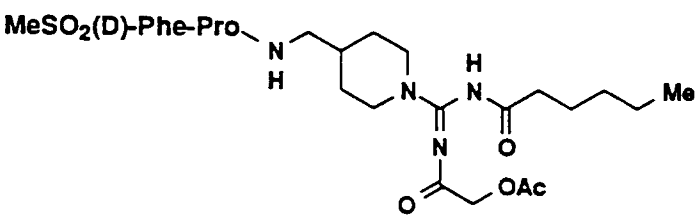

N-[[1-[[[(acetyloxy)acetyl]imino][(1-oxohexyl)amino]methyl]-4-piperidinyl]methyl]-1-[N-(methylsulfonyl)-D-phenyla-

lanyl]-L-prolinamide;

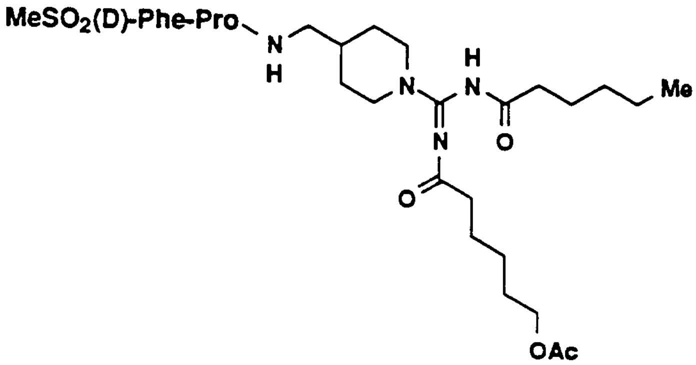

N-[[1-[[[6-(acetyloxy)-1-oxohexyl]imino][(1-oxohexyl)amino]methyl]-4-piperidinyl]methyl]-1-[N-(methylsulfonyl)-D-phenylalanyl]-L-prolinamide;

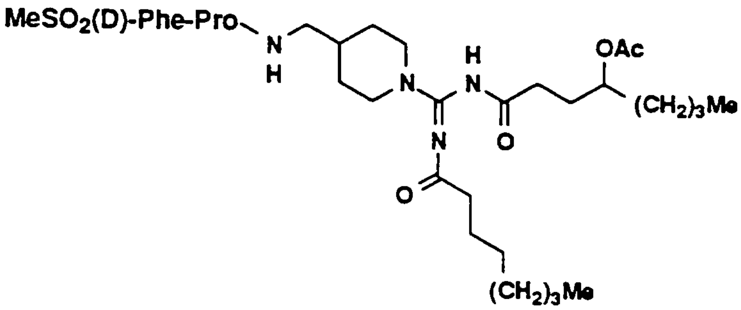

N-[[1-[[[(4-(acetyloxy)-1-oxooctyl)amino][(1-oxooctyl)imino]methyl]-4-piperidinyl]methyl]-1-[N-(methylsulfonyl)-D-phenylalanyl]-L-prolinamide;

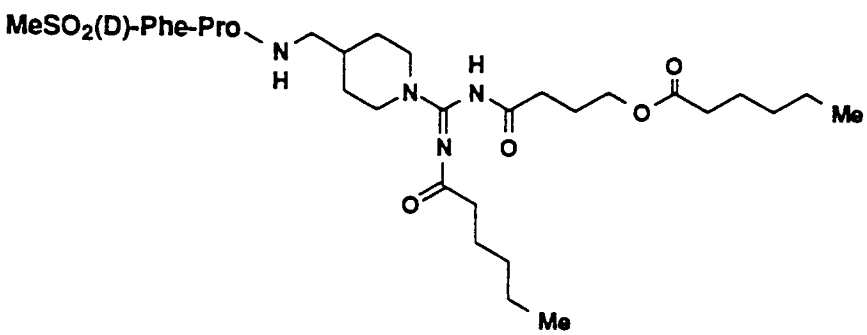

1-[N-(methylsulfonyl)-D-phenylalanyl]-N-[[1-[[(1-oxohexyl)imino][[1-oxo-4-(1-oxohexyloxy)butyl]amino]methyl]-4-piperidinyl]methyl]-L-prolinamide;

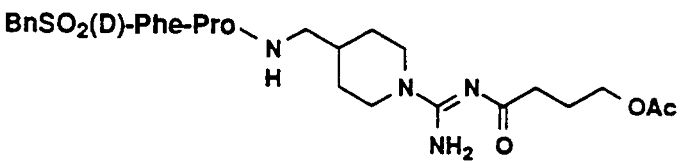

N-[[1-[[[4-(acetyloxy)-1-oxobutyl]imino]aminomethyl]-4-piperidinyl]methyl]-1-[N-(benzylsulfonyl)-D-phenylalanyl]-L-prolinamide;

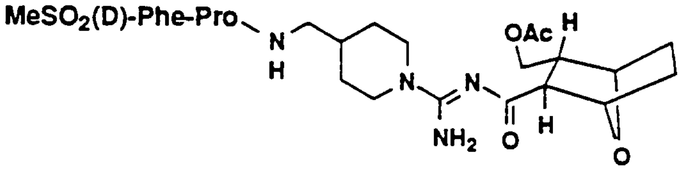

(*exo,exo*)-N-[[1-[[[[3-[(acetyloxy)methyl]-7-oxabicyclo[2.2.1]hept-2-yl]carbonyl]amino]iminomethyl]-4-piperidinyl]methyl]-1-[N-(methylsulfonyl)-D-phenylalanyl]-L-prolinamide;

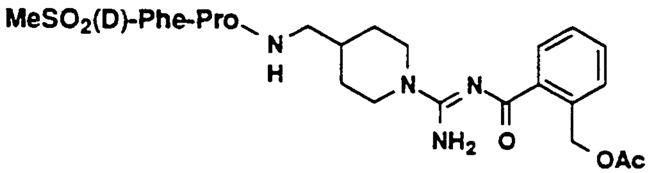

N-[[1-[[[2-[(acetyloxy)methyl]benzoyl]imino]aminomethyl]-4-piperidinyl]methyl]-1-[N-(methylsulfonyl)-D-phenylalanyl]-L-prolinamide;

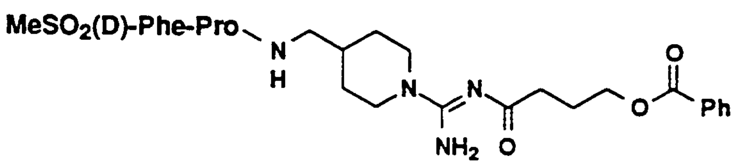

N-[[1-[amino[[4-(benzoyloxy)-1-oxobutyl]imino]methyl]-4-piperidinyl]methyl]-1-[N-(methylsulfonyl)-D-phenylalanyl]-L-prolinamide;

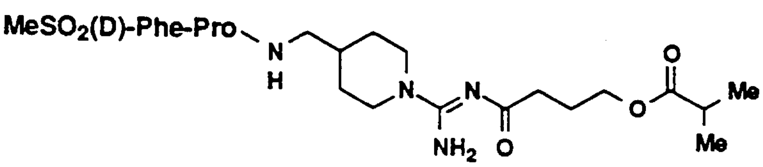

N-[[1-[amino[[4-(2-methyl-1-oxopropoxy)-1-oxobutyl]imino]methyl]-4-piperidinyl]methyl]-1-[N-(methylsulfonyl)-D-phenylalanyl]-L-prolinamide;

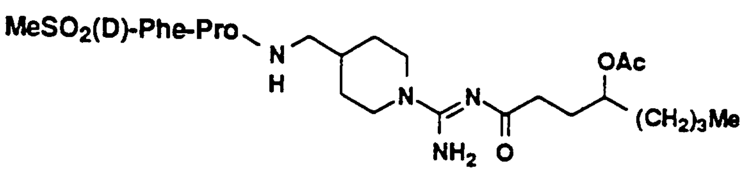

N-[[1-[[[4-(acetyloxy)-1-oxooctyl]imino]aminomethyl]-4-piperidinyl]methyl]-1-[N-(methylsulfonyl)-D-phenylalanyl]-L-prolinamide;

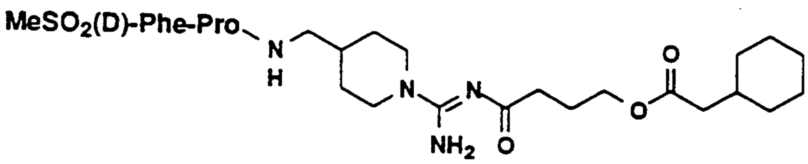

N-[[1-[amino[[4-[(cyclohexylacetyl)oxy]-1-oxobutyl]imino]methyl]-4-piperidinyl]methyl]-1-[N-(methylsulfonyl)-D-phenylalanyl]-L-prolinamide;

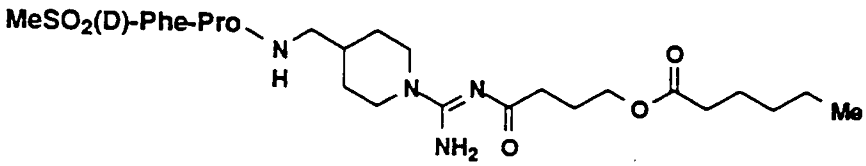

N-[[1-[amino[[1-oxo-4-(1-oxohexyloxy)butyl]imino]methyl]-4-piperidinyl]methyl]-1-[N-(methylsulfonyl)-D-phenylalanyl]-L-prolinamide;

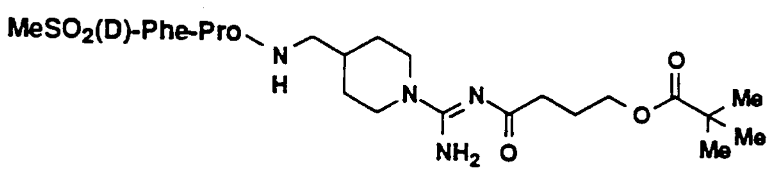

N-[[1-[amino[[4-(2,2-dimethyl-1-oxopropoxy)-1-oxobutyl]imino]methyl]-4-piperidinyl]methyl]-1-[N-(methylsulfonyl)-D-phenylalanyl]-L-prolinamide;

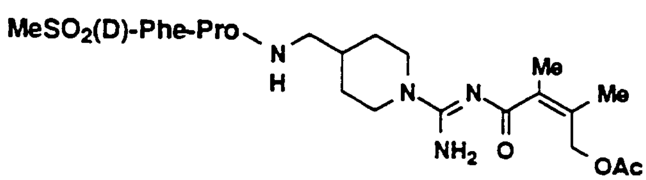

(2Z)-N-[[1-[[[4-(acetyloxy)-2,3-dimethyl-1-oxo-2-butenyl]imino]aminomethyl]-4-piperidinyl]methyl]-1-[N-(methylsulfonyl)-D-phenylalanyl]-L-prolinamide;

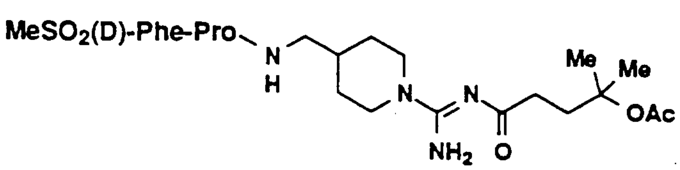

N-[[1-[[[4-(acetyloxy)-4-methyl-1-oxopentyl]imino]aminomethyl]-4-piperidinyl]methyl]-1-[N-(methylsulfonyl)-D-phenylalanyl]-L-prolinamide;

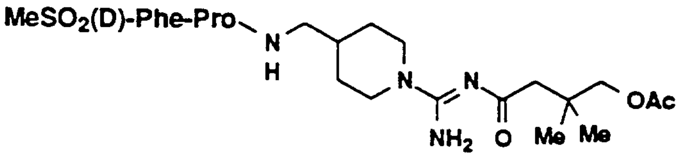

N-[[1-[[[4-(acetyloxy)-3,3-dimethyl-1-oxobutyl]imino]aminomethyl]-4-piperidinyl]methyl]-1-[N-(methylsulfonyl)-D-phenylalanyl]-L-prolinamide;

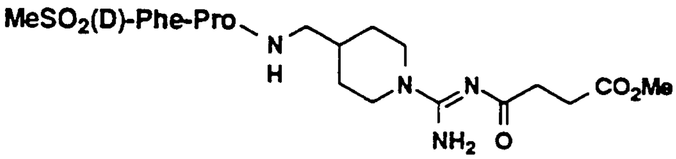

N-[[1-[amino[(4-methoxy-1,4-dioxobutyl)imino]methyl]-4-piperidinyl]methyl]-1-[N-(methylsulfonyl)-D-phenylalanyl]-L-prolinamide;

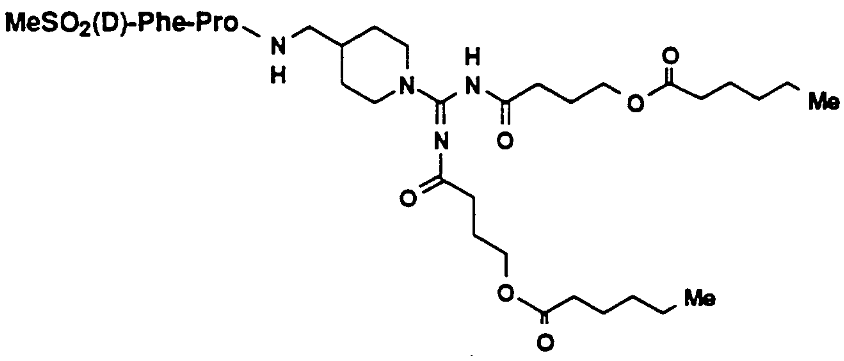

1-[N-(methylsulfonyl)-D-phenylalanyl]-N-[[1-[[[1-oxo-4-(1-oxohexyloxy)butyl]amino][[1-oxo-4-(1-oxohexyloxy)butyl]imino]methyl]-4-piperidinyl]methyl]-L-prolinamide;

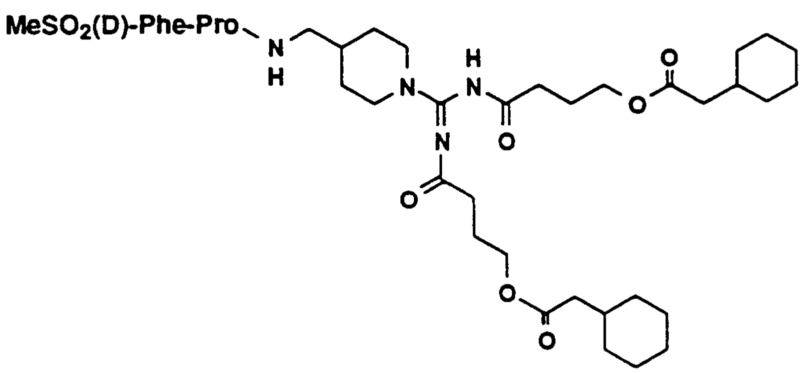

N-[[1-[[[4-[(cyclohexylacetyl)oxy]-1-oxobutyl]amino][[4-[(cyclohexylacetyl)oxy]-1-oxobutyl]imino]methyl]-4-piperidinyl]methyl]-1-[N-(methylsulfonyl)-D-phenylalanyl]-L-prolinamide;

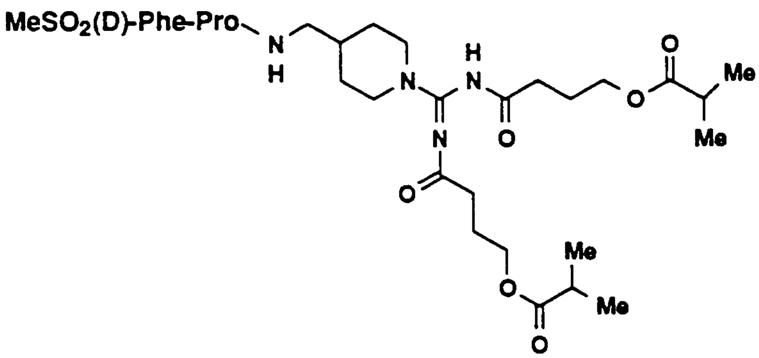

N-[[1-[[[4-(2-methyl-1-oxopropoxy)-1-oxobutyl]amino][[4-(2-methyl-1-oxopropoxy)-1-oxobutyl]imino]methyl]-4-piperidinyl]methyl]-1-[N-(methylsulfonyl)-D-phenylalanyl]-L-prolinamide;

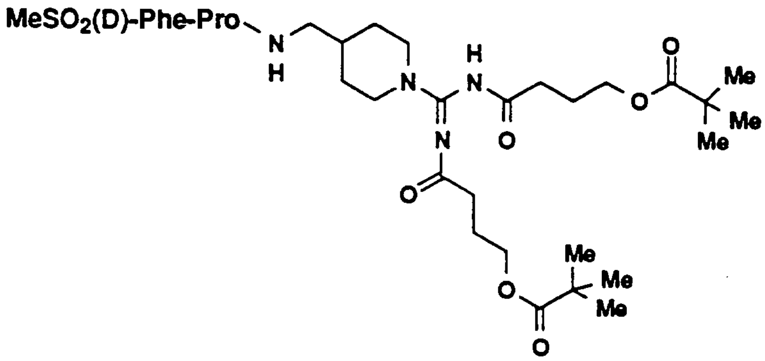

N-[[1-[[[4-(2,2-dimethyl-1-oxopropoxy)-1-oxobutyl]amino][[4-(2,2-dimethyl-1-oxopropoxy)-1-oxobutyl]imino]methyl]-4-piperidinyl]methyl]-1-[N-(methylsulfonyl)-D-phenylalanyl]-L-prolinamide;

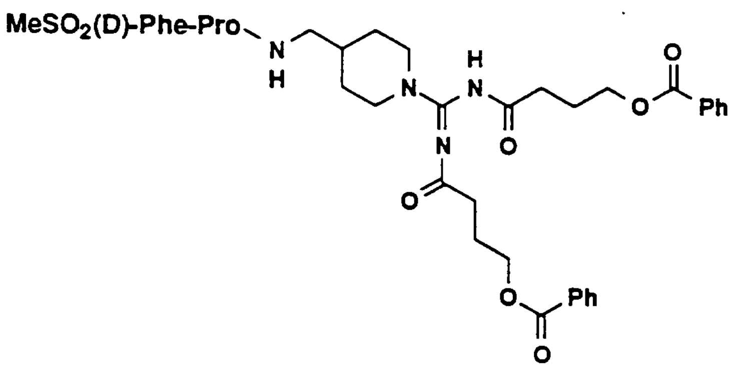

N-[[1-[[[4-(benzoyloxy)-1-oxobutyl]amino][[4-(benzoyloxy)-1-oxobutyl]imino]methyl]-4-piperidinyl]methyl]-1-[N-(methylsulfonyl)-D-phenylalanyl]-L-prolinamide;

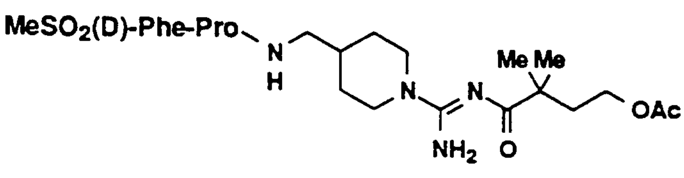

N-[[1-[[[4-(acetyloxy)-2,2-dimethyl-1-oxobutyl]imino]aminomethyl]-4-piperidinyl]methyl]-1-[N-(methylsulfonyl)-D-

phenylalanyl]-L-prolinamide;

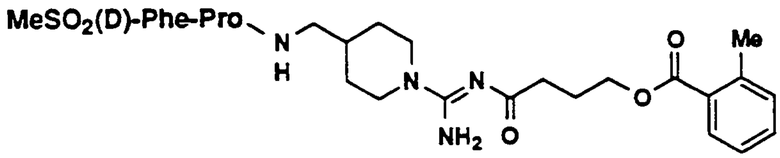

N-[[1-[amino[[4-(2-methylbenzoyl)oxy]-1-oxobutyl]imino]methyl]-4-piperidinyl]methyl]-1-[N-(methylsulfonyl)-D-phenylalanyl]-L-prolinamide;

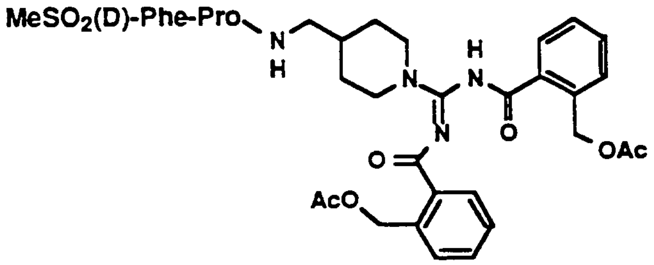

N-[[1-[[[2-[(acetyloxy)methyl]benzoyl]amino][[2-[(acetyloxy)methyl]benzoyl]imino]methyl]-4-piperidinyl]methyl]-1-[N-(methylsulfonyl)-D-phenylalanyl]-L-prolinamide;

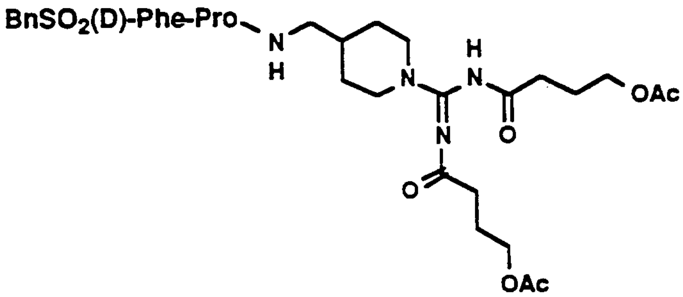

N-[[1-[[[4-(acetyloxy)-1-oxobutyl]amino][[4-(acetyloxy)-1-oxobutyl]imino]methyl]-4-piperidinyl]methyl]-1-[N-(benzylsulfonyl)-D-phenylalanyl]-L-prolinamide;

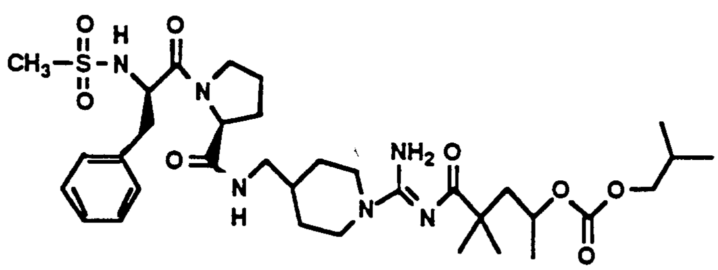

N-[[1-amino[[[2,2-dimethyl-4-[[(2-methylpropoxy)carbonyl]oxy]-1-oxopentyl]imino]methyl]-4-piperidinyl]-methyl]-1-[N-(methylsulfonyl)-D-phenylalanyl]-L-prolinamide, monohydrochloride;

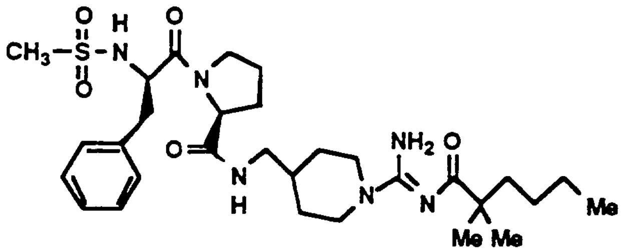

N-[[1-[amino[(2,2-dimethyl-1-oxohexyl)imino]methyl]-4-piperidinyl]methyl]-1-[N-(methylsulfonyl)-D-phenylalanyl]-L-prolinamide, monohydrochloride;

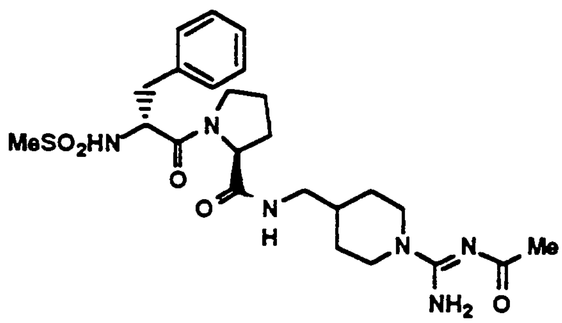

N-[[1-[(acetylimino)]aminomethy]-4-piperidinyl]methyl]-1-N-(methylsulfonyl)-D-phenylalanyl]-L-prolinamide;

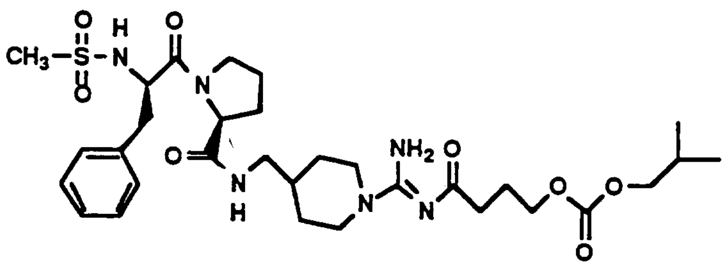

N-[[1-[amino[[4-[[(2-methylpropoxy)carbonyl]oxy]-1-oxobutyl]imino]methyl]-4-piperidinyl]methyl]-1-[N-(methylsulfonyl)-D-phenylalanyl]-L-prolinamide;

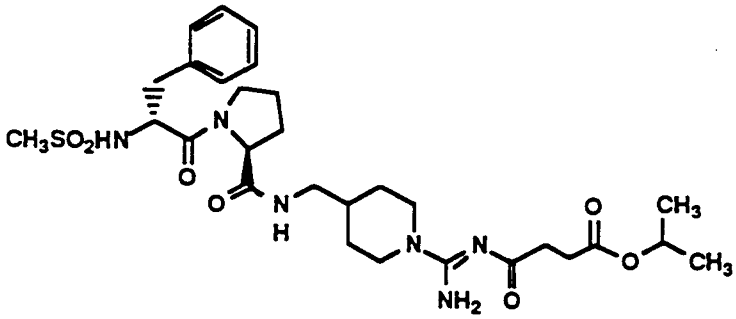

N-[[1-[amino[[4-(1-methylethoxy)-1,4-dioxobutyl]imino]methyl]-4-piperidinyl]-methyl]-1-[N-(methylsulfonyl)-D-phenylalanyl]-L-prolinamide;

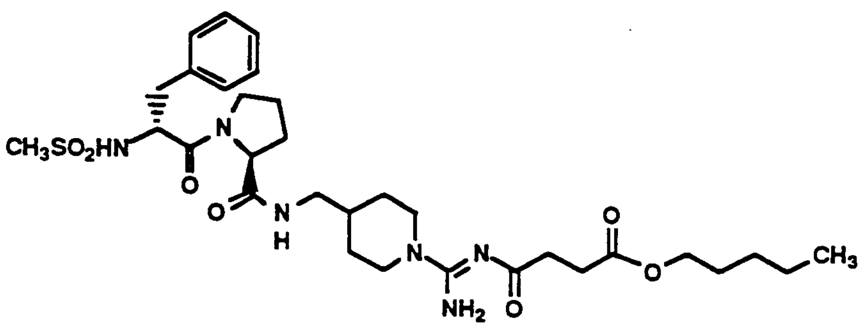

N-[[1-[amino[[1,4-dioxo-4-(pentyloxy)butyl]imino]methyl]-4-piperidinyl]methyl]-1-[N-(methylsulfonyl)-D-phenylalanyl]-L-prolinamide;

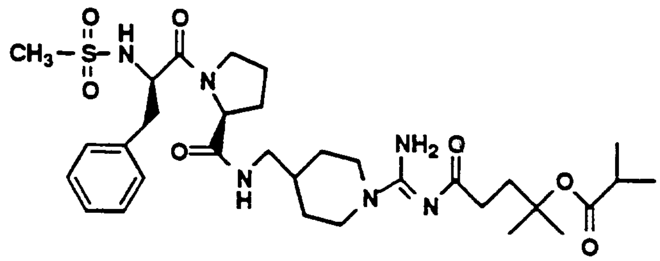

N-[[1-amino[[[4-methyl-4-(2-methyl-1-oxopropoxy)-1-oxopentyl]imino]methyl]-4-piperidinyl]methyl]-1-[N-(methylsulfonyl)-D-phenylalanyl]-L-prolinamide;

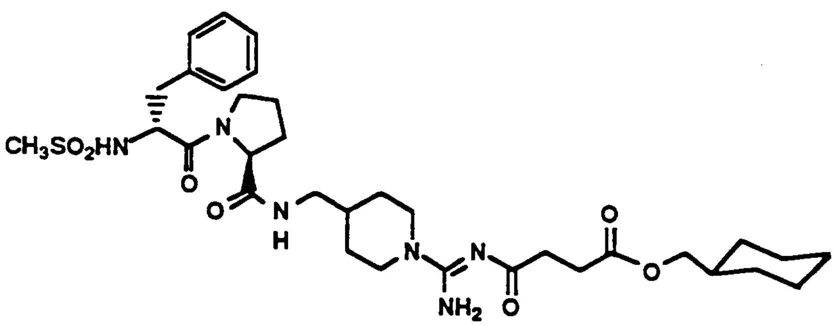

N-[[1-[amino[[4-(cyclohexylmethoxy)-1,4-dioxobutyl]imino]methyl]-4-piperidinyl]methyl]-1-[N-(methylsulfonyl)-D-phenylalanyl]-L-prolinamide;

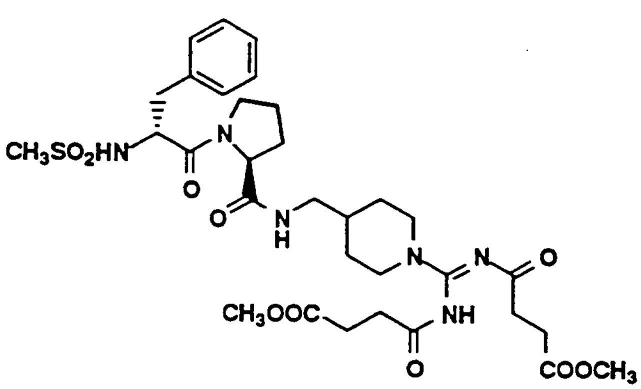

N-[[1-[[(4-methoxy-1,4-dioxobutyl)amino][(4-methoxy-1,4-dioxobutyl)imino]-methyl]-4-piperidinyl]methyl]-1-[N-(methylsulfonyl)-D-phenylalanyl]-L-prolinamide;

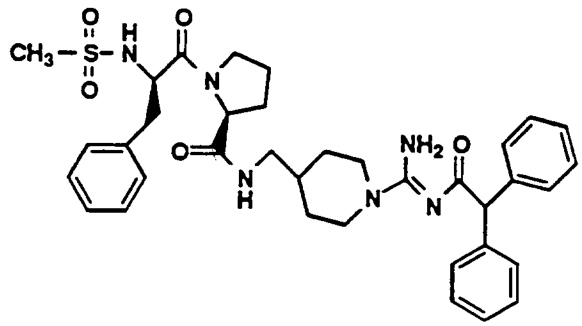

N-[[1-amino[[(diphenylacetyl)imino]methyl]-4-piperidinyl]methyl]-1-[N-(methylsulfonyl)-D-phenylalanyl]-L-prolinamide;

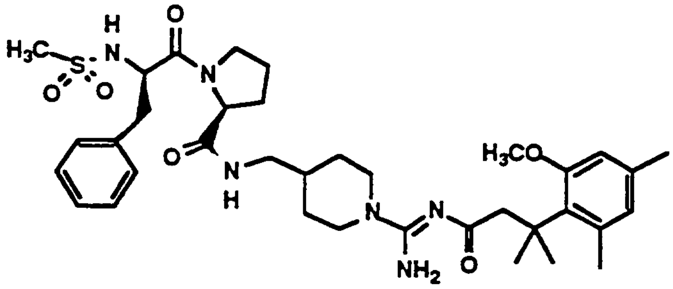

N-[[1-[amino[[3-(2-methoxy-4,6-dimethylphenyl)-3-methyl-1-oxobutyl]-imino]methyl]-4-piperidinyl]methyl]-1-[N-(methylsulfonyl)-D-phenylalanyl]-L-prolinamide, trifluoroacetate;

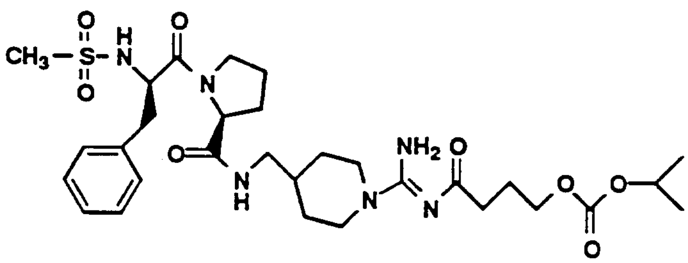

N-[[1-amino[[[4-[[(1-methylethoxy)carbonyl]oxy]-1-oxo-butyl]imino]methyl]-4-piperidinyl]methyl]-1-[N-(methylsulfonyl)-D-phenylalanyl]-L-prolinamide;

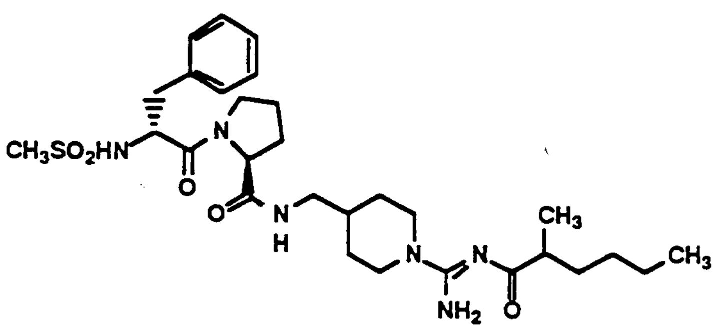

N-[[1-[amino[(2-methyl-1-oxohexyl)imino]methyl]-4-piperidinyl]methyl]-1-[N-(methylsulfonyl)-D-phenylalanyl]-L-prolinamide;

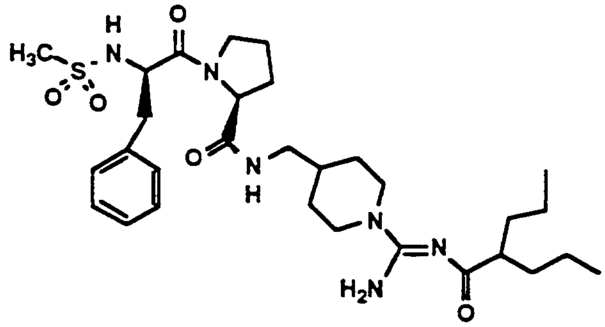

N-[[1-[amino[(1-oxo-2-propylpentyl)imino]methyl]-4-piperidinyl]methyl]-1-[N-(methylsulfonyl)-D-phenylalanyl]-L-prolinamide;

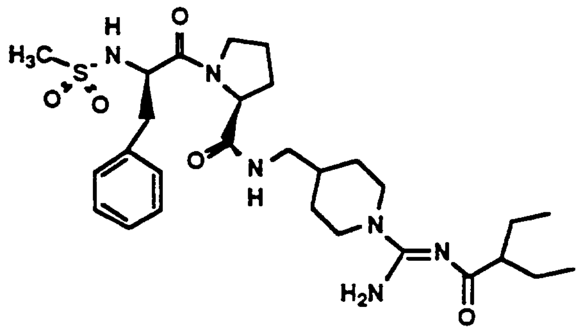

N-[[1-[amino[(2-ethyl-1-oxobutyl)imino]methyl]-4-piperidinyl]methyl]-1-[N-(methylsulfonyl)-D-phenylalanyl]-L-prolinamide;

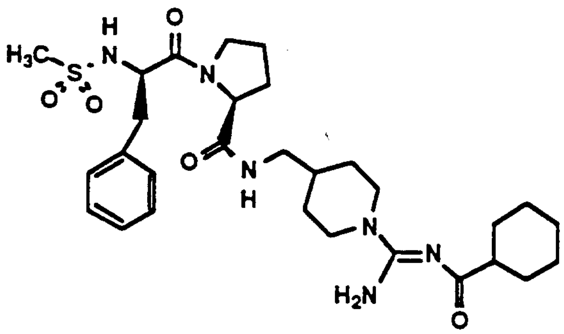

N-[[1-[amino[(cyclohexylcarbonyl)imino]methyl]-4-piperidinyl]methyl]-1-[N-(methylsulfonyl)-D-phenylalanyl]-L-prolinamide;

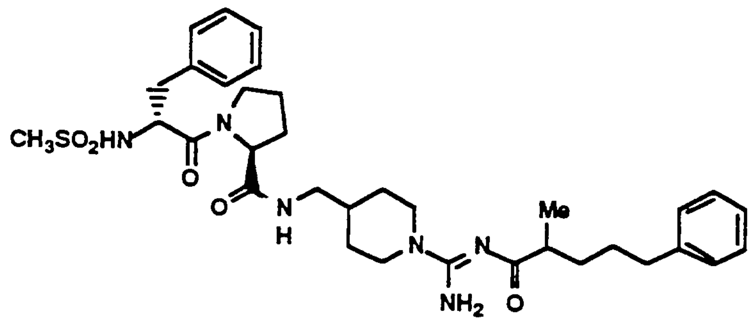

N-[[1-[amino[(2-methyl-1-oxo-5-phenylpentyl)imino]methyl]-4-piperidinyl]methyl]-1-[N-(methylsulfonyl)-D-phenylalanyl]-L-prolinamide;

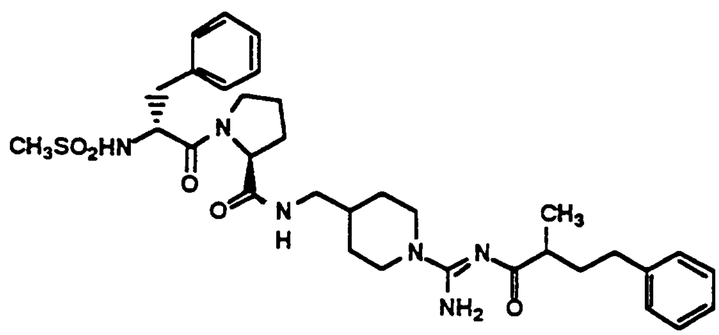

N-[[1-[amino[(2-methyl-1-oxo-4-phenylbutyl)imino]methyl]-4-piperidinyl]methyl]-1-[N-(methylsulfonyl)-D-phenylalanyl]-L-prolinamide;

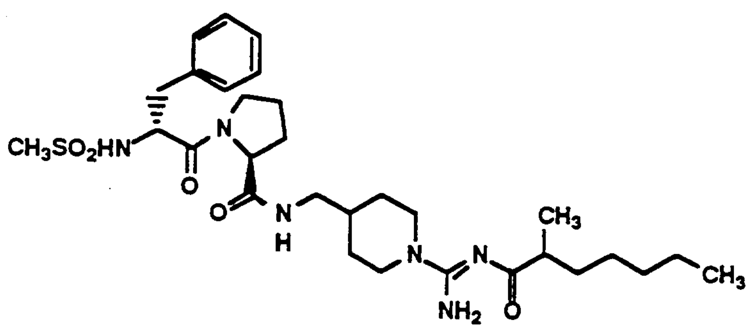

N-[[1-[amino[(2-methyl-1-oxoheptyl)imino]methyl]-4-piperidinyl]methyl]-1-[N-(methylsulfonyl)-D-phenylalanyl]-L-prolinamide;

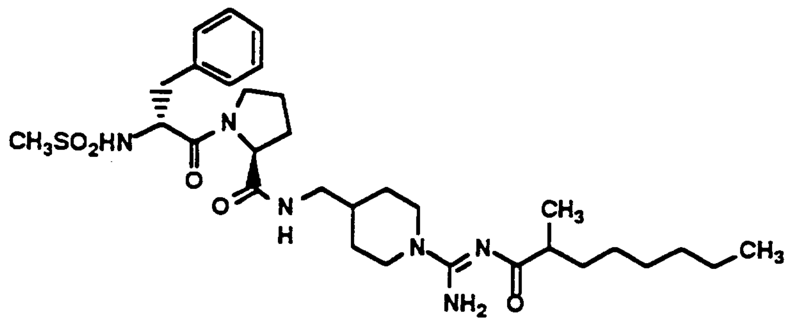

N-[[1-[amino[(2-methyl-1-oxooctyl)imino]methyl]-4-piperidinyl]methyl]-1-[N-(methylsulfonyl)-D-phenylalanyl]-L-prolinamide;

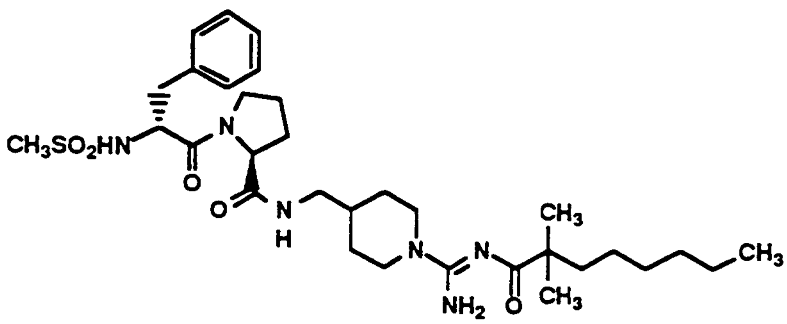

N-[[1-[amino[(2,2-dimethyl-1-oxooctyl)imino]methyl]-4-piperidinyl]methyl]-1-[N-(methylsulfonyl)-D-phenylalanyl]-L-prolinamide;

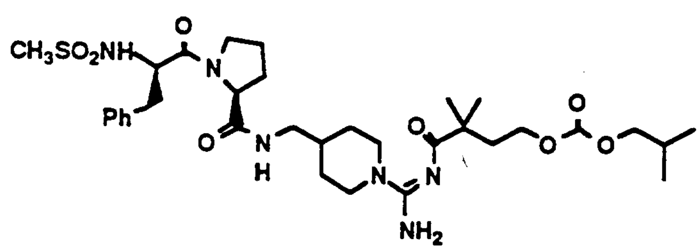

N-[[1-[amino[[4-[[(2-methylpropoxy)carbonyl]oxy]-2,2-dimethyl-1-oxobutyl]imino]methyl]-4-piperidinyl]methyl]-1-[N-(methylsulfonyl)-D-phenylalanyl]-L-prolinamide;

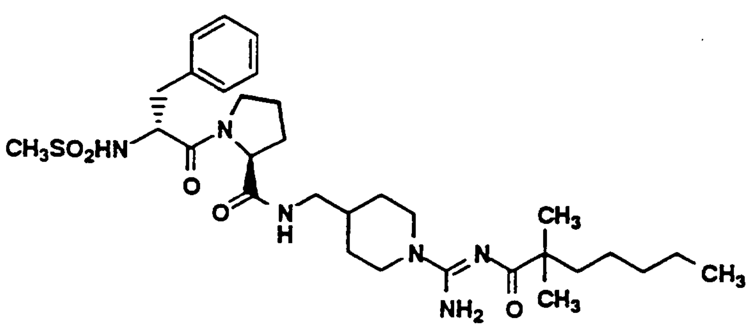

N-[[1-[amino[(2,2-dimethyl-1-oxoheptyl)imino]methyl]-4-piperidinyl]methyl]-1-[N-(methylsulfonyl)-D-phenylalanyl]-L-prolinamide;

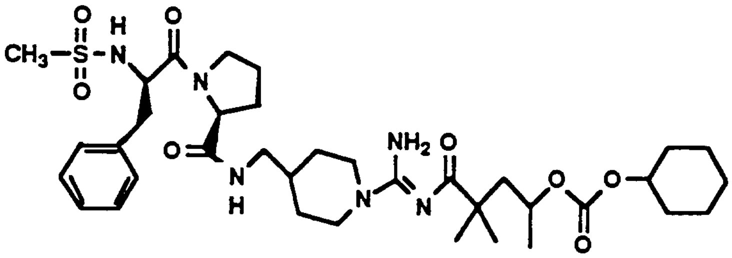

N-[[1-amino[[[4-[[(cyclohexyloxy)carbonyl]oxy]-2,2-dimethyl-1-oxopentyl]imino]methyl]-4-piperidinyl]methyl]-1-[N-(methylsulfonyl)-D-phenylalanyl]-L-prolinamide, monohydrochloride;

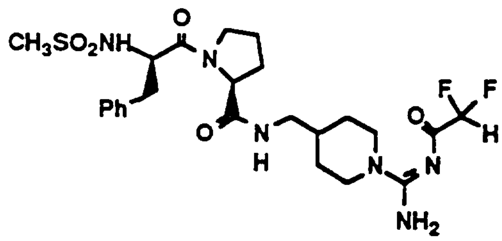

N-[[1-[amino[(difluoroacetyl)imino]methyl]-4-piperidinyl]methyl]-1-[N-(methylsulfonyl)-D-phenylalanyl]-L-prolinamide;

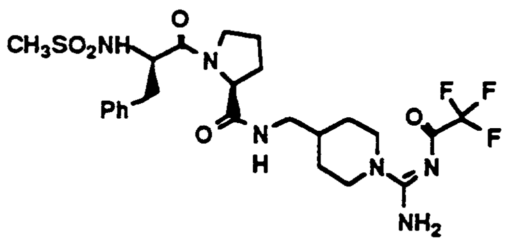

N-[[1-[amino[(trifluoroacetyl)imino]methyl]-4-piperidinyl]methyl]-1-[N-(methylsulfonyl)-D-phenylalanyl]-L-prolinamide;

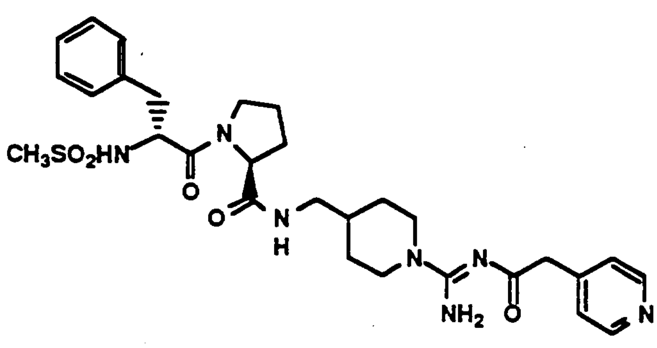

N-[[1-[amino[(4-pyridinylacetyl)imino]methyl]-4-piperidinyl]methyl]-1-[N-(methylsulfonyl)-D-phenylalanyl]-L-prolinamide, trifluoroacetate;

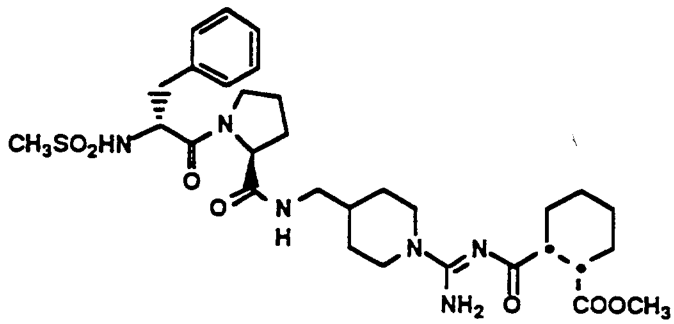

cis-2-[[[amino[4-[[[1-[N-(methylsulfonyl)-D-phenylalanyl]-L-prolyl]amino]methyl]-1-piperidinyl]methyl]imino]carbonyl]cyclohexanecarboxylic acid, methyl ester, hydrochloride;

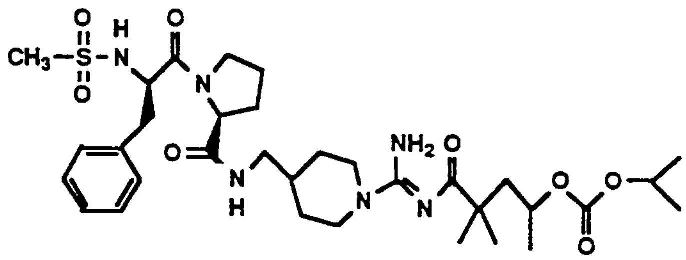

N-[[1-amino[[[2,2-dimethyl-4-[[(1-methylethoxy)carbonyl]-oxy]-1-oxopentyl]imino]methyl]-4-piperidinyl]methyl]-1-[N-(methylsulfonyl)-D-phenylalanyl]-L-prolinamide, monohydrochloride;

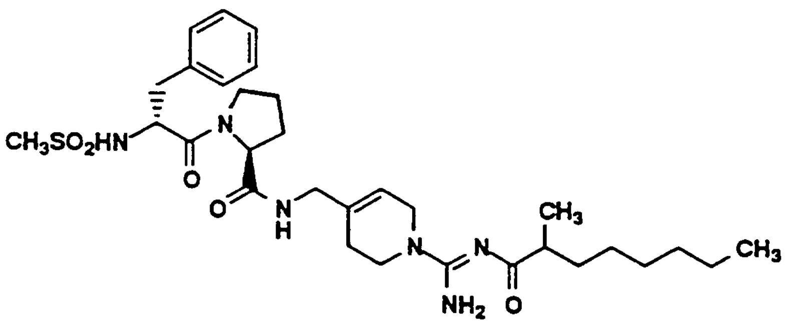

N-[[1-[amino[(2-methyl-1-oxooctyl)imino]methyl]-1,2,3,6-tetrahydro-4-pyridinyl]methyl]-1-[N-(methylsulfonyl)-D-phenylalanyl]-L-prolinamide;

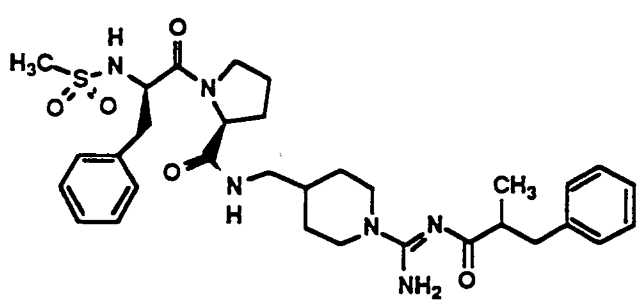

N-[[1-[amino[(2-methyl-1-oxo-3-phenylpropyl)imino]methyl]-4-piperidinyl]methyl]-1-[N-(methylsulfonyl)-D-phenyla-

lanyl]-L-prolinamide, monohydrochloride;

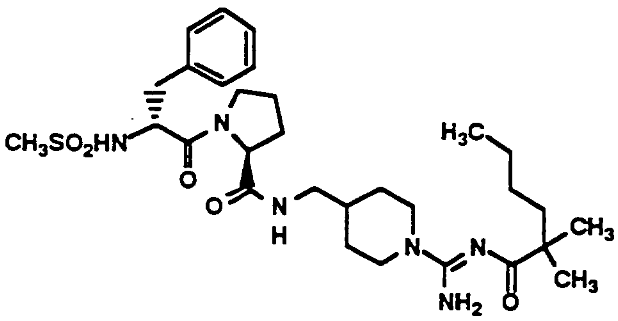

N-[[1-[mino[(2,2-dimethyl-1-oxohexyl)imino]methyl]-4-piperidinyl]methyl]-1-[N-(methylsulfonyl)-D-phenylalanyl]-L-prolinamide, monohydrochloride;

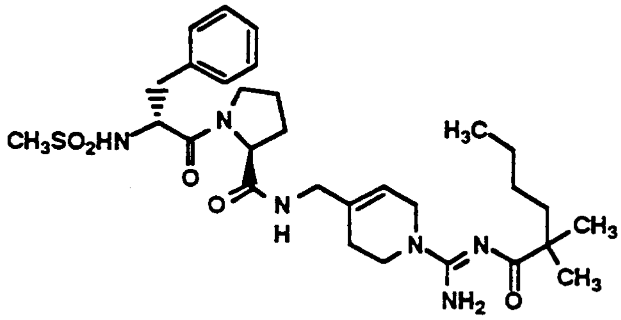

N-[[1-[amino[(2,2-dimethyl-1-oxohexyl)imino]methyl]-1,2,3,6-tetrahydro-4-pyridinyl]methyl]-1-[N-(methylsulfonyl)-D-phenylalanyl]-L-prolinamide;

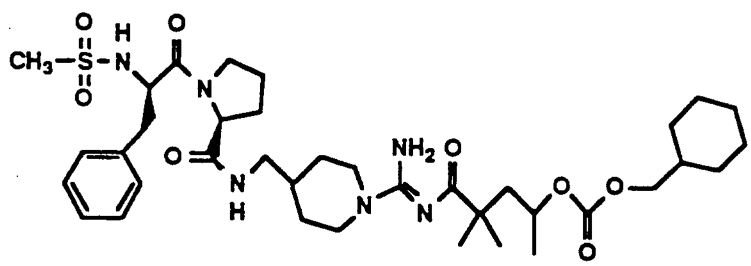

N-[[1-amino[[[4-[[(Cyclohexylmethoxy)carbonyl]oxy]-2,2-dimethyl-1-oxopentyl]imino]methyl]-4-piperidinyl]-methyl]-1-[N-(methylsulfonyl)-D-phenylalanyl]-L-prolinamide, monohydrochloride;

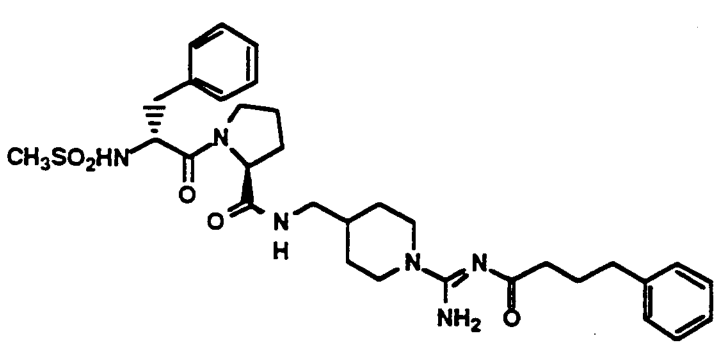

N-[[1-[amino[(1-oxo-4-phenylbutyl)imino]methyl]-4-piperidinyl]methyl]-1-[N-(methylsulfonyl)-D-phenylalanyl]-L-prolinamide, monohydrochloride;

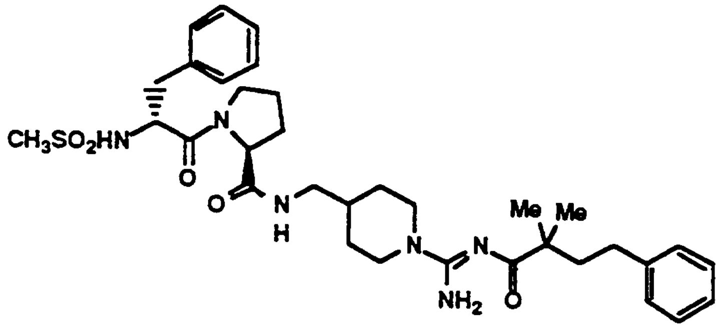

N-[[1-[amino[(2,2-dimethyl-1-oxo-4-phenylbutyl)imino]methyl]-4-piperidinyl]methyl]-1-[N-(methylsulfonyl)-D-phenylalanyl]-L-prolinamide, monohydrochloride;

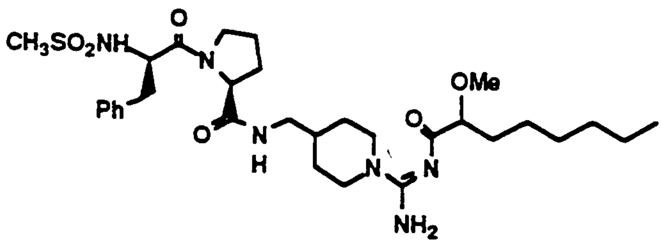

N-[[1-[aminol(2-methoxy-1-oxooctyl)imino]methyl]-4-piperidinyl]methyl]-1-[N-(methylsulfonyl)-D-phenylalanyl]-L-prolinamide, monohydrochloride;

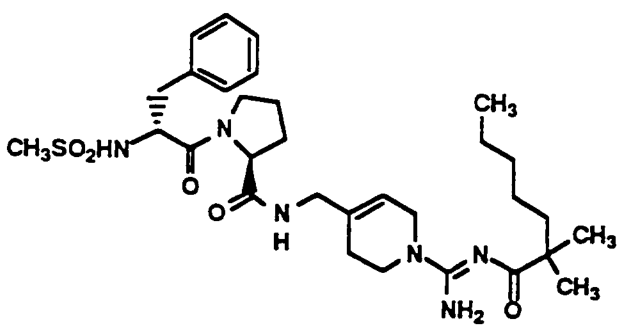

N-[[1-[amino[(2,2-dimethyl-1-oxohepyl)imino]methyl]-1,2,3,6-tetrahydro-4-pyridinyl]methyl]-1-[N-(methylsulfonyl)-D-phenylalanyl]-L-prolinamide;

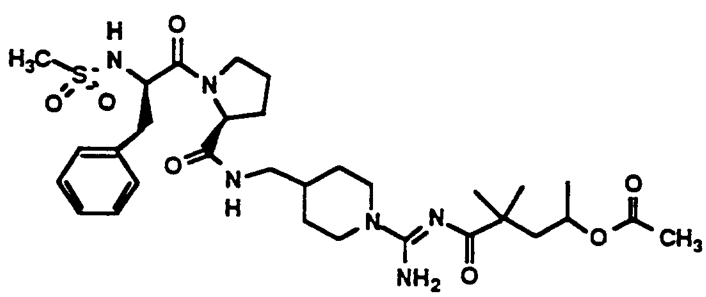

N-[[1-[[[4-(acetyloxy)-2,2-dimethy-1-oxopentyl]imino]aminomethyl]-4-piperidinyl]methyl]-1-[N-(methylsulfon-yl)-D-phenylalanyl]-L-prolinamide, monohydrochloride;

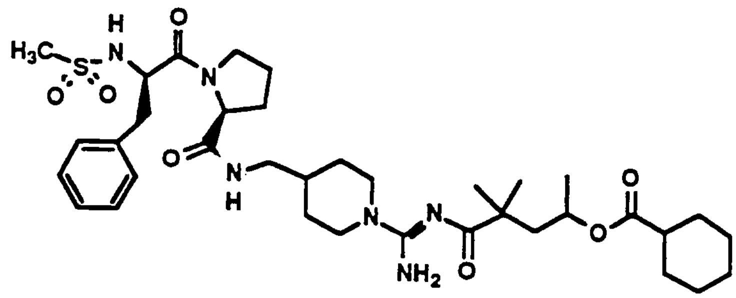

N-[[1-[amino[[4-[(cyclohexylcarbonyl)oxy]-2,2-dimethyl-1-oxopentyl]imino]-methyl]-4-piperidinyl]methyl]-1-[N-(methylsulfonyl)-D-phenylalanyl]-L-prolinamide, monohydrochloride;

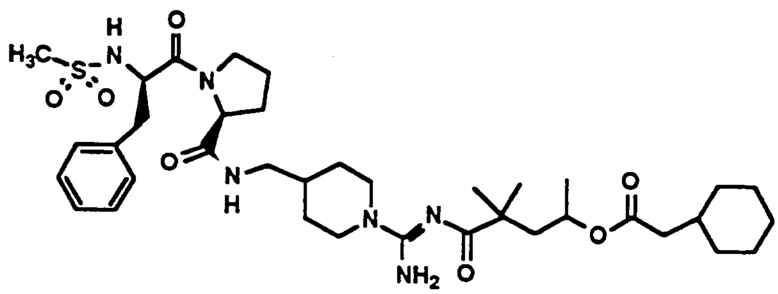

N-[[1-[amino[[4-[(cyclohexylacetyl)oxy]-2,2-diethyl-1-oxopentyl]imino]-methyl]-4-piperidinyl]methyl]-1-[N-(methylsulfonyl)-D-phenylalanyl]-L-prolinamide, monohydrochloride;

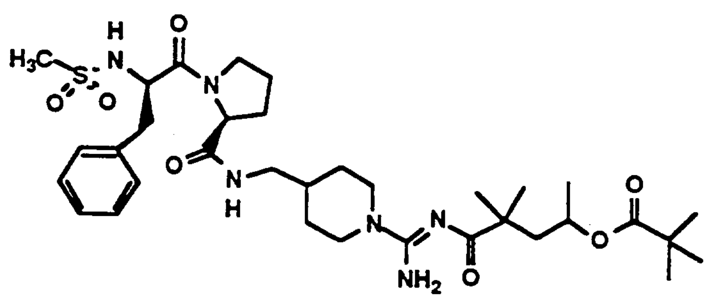

N-[[1-[amino[[2,2-dimethyl-4-(2,2-dimethyl-1-oxopropoxy)-1-oxopentyl]-imino]methyl]-4-piperidinyl]methyl]-1-[N-(methylsulfonyl)-D-phenylalanyl]-L-prolinamide, monohydrochloride;

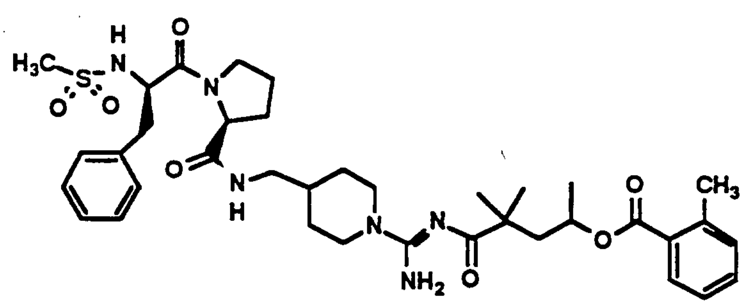

N-[[1-[amino[[2,2-dimethyl-4-(2-methylbenzoyloxy)-1-oxopentyl]imino]methyl]-4-piperidinyl]methyl]-1-[N-(methylsulfonyl)-D-phenylalanyl]-L-prolinamide, monohydrochloride;

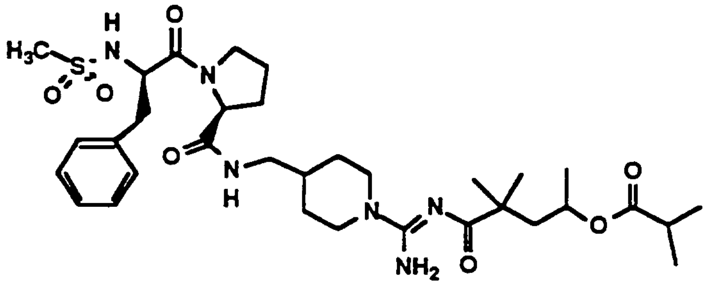

N-[[1-[amino[[2,2-dimethyl-4-(2-methyl-1-oxopropoxy)-1-oxopentyl]imino]-methyl]-4-piperidinyl]methyl]-1-[N-(methylsulfonyl)-D-phenylalanyl]-L-prolinamide, monohydrochloride;

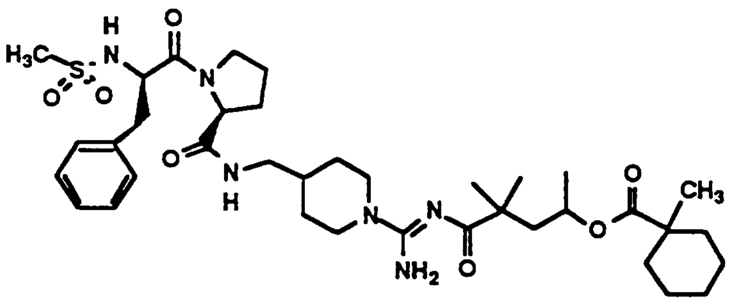

N-[[1-[amino[[2,2-dimethyl-4-[[(1-methylcyclohexyl)carbonyl]oxy]-1-oxopentyl]-imino]methyl]-4-piperidinyl]methyl]-1-[N-(methylsulfonyl)-D-phenylalanyl]-L-prolinamide, monohydrochloride;

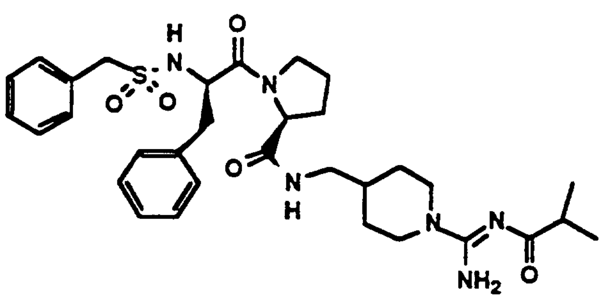

N-[[1-[amino[(2-methyl-1-oxopropyl)imino]methyl]-4-piperidinyl]methyl]-1-[N-[(phenylmethyl)sulfonyl]-D-phenylalanyl]-L-prolinamide, monohydrochloride;

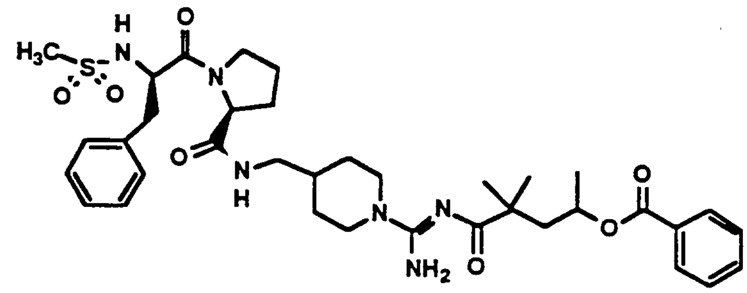

N-[[1-[amino[[4-(benzoyloxy)-2,2-dimethyl-1-oxopentyl]imino]methyl]-4-piperidinyl]methyl]-1-[N-(methylsulfonyl)-D-phenylalanyl]-L-prolinamide, monohydrochloride;

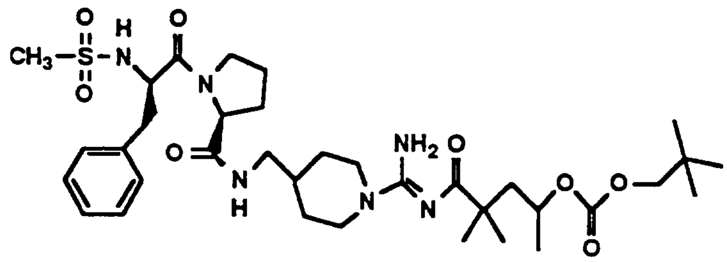

N-[[1-amino[[[2,2-Dimethyl-4-[[(2,2-dimethylpropoxy)carbonyl]oxy]-1-oxopentyl]imino]methyl]-4-piperidinyl]methyl]-1-[N-(methylsulfonyl)-D-phenylalanyl]-L-prolinamide, monohydrochloride;

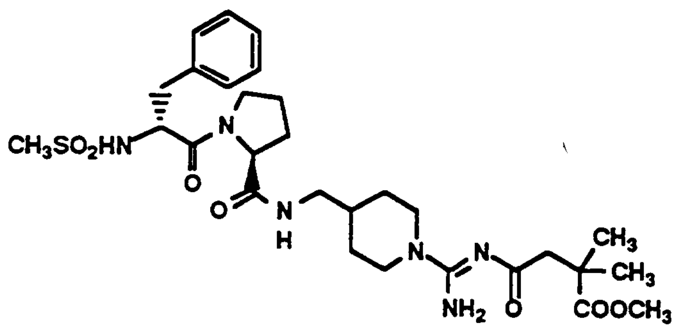

N-[[1-[amino[(4-methoxy-3,3-dimethyl-1,4-dioxobutyl)imino]methyl]-4-piperidinyl]methyl]-1-[N-(methylsulfonyl)-D-phenylalanyl]-L-prolinamide, monohydrochloride;

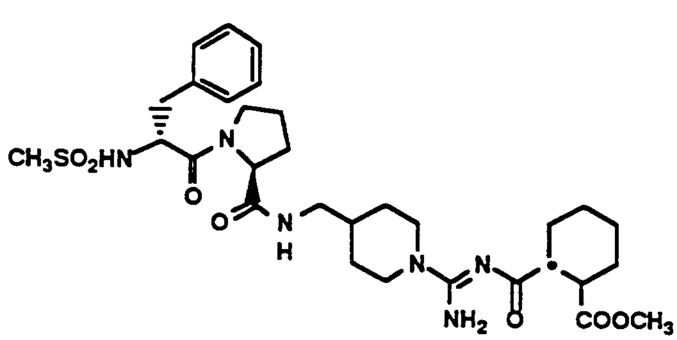

2-[[[amino[4-[[[1-[N-(methylsulfonyl)-D-phenylalanyl]-L-prolyl]amino]methyl]-1-piperidinyl]methyl]imino]carbonyl]cyclohexanecarborylic acid, methyl ester, monohydrochloride;

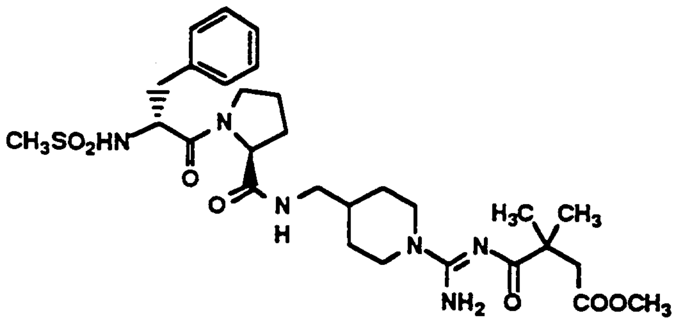

N-[[1-[amino[(4-methoxy-2,2-dimethyl-1,4-dioxobutyl)imino]methyl]-4-piperidinyl]methyl]-1-[N-(methylsulfonyl)-D-phenylalanyl]-L-prolinamide, monohydrochloride;

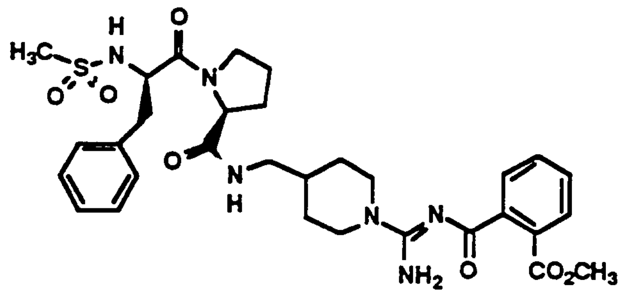

N-[[1-[amino[[2-(methoxycarbonyl)benzoyl]imino]methyl]-4-piperidinyl]methyl]-1-[N-(methylsulfonyl)-D-phenylalanyl]-L-prolinamide, monohydrochloride;

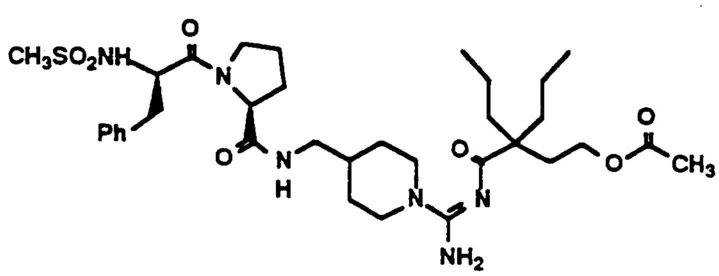

N-[[1-amino[[[4-(acetyloxy)-2,2-dipropyl-1-oxobutyl]imino]methyl]-4-piperidinyl]methyl]-1-[N-(methylsulfonyl)-D-phenylalanyl]-L-prolinamide, hydrochloride;

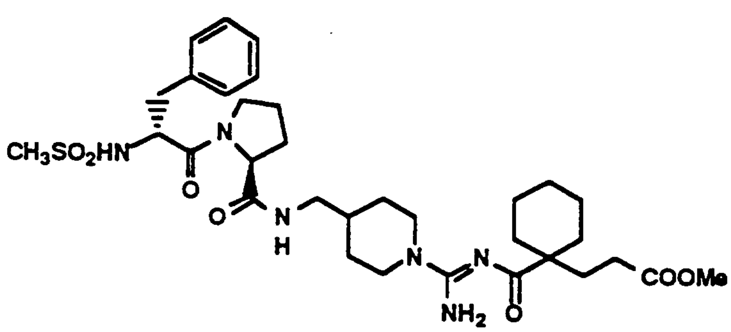

1-[[[amino[4-[[[1-[N-(methylsulfonyl)-D-phenylalanyl]-L-prolyl]amino]methyl]-1-piperidinyl]methyl]imino]carbonyl]cyclohexanepropanoic acid, methyl ester, monohydrochloride;

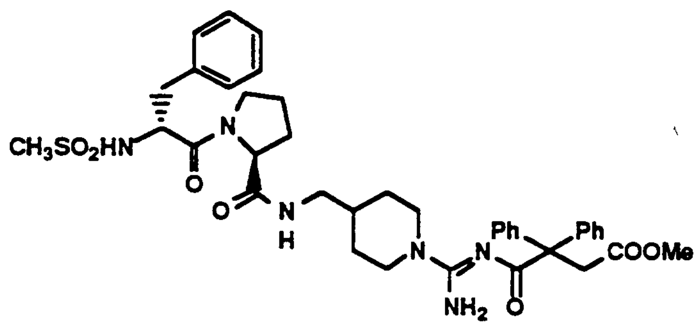

β-[[[amino[4-[[[1-[N-(methylsulfonyl)-D-phenylalanyl]-L-prolyl]amino]methyl]-1-piperidinyl]methyl]imino]carbonyl]-β-phenylbenzenepropanoic acid, methyl ester, monohydrochloride;

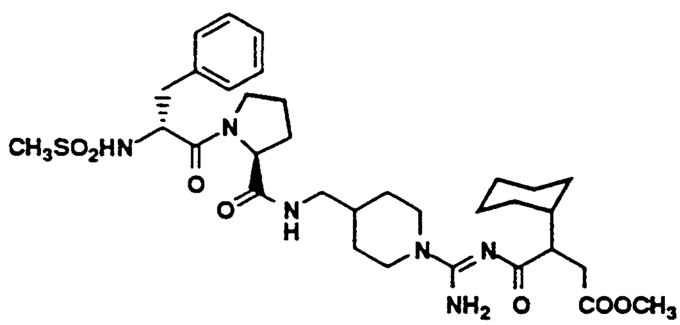

N-[[1-[amino[(2-cyclohexyl-4-methoxy-1,4-dioxobutyl)imino]methyl]-4-piperidinyl]methyl]-1-[N-(methylsulfonyl)-D-phenylalanyl]-L-prolinamide, monohydrochloride;

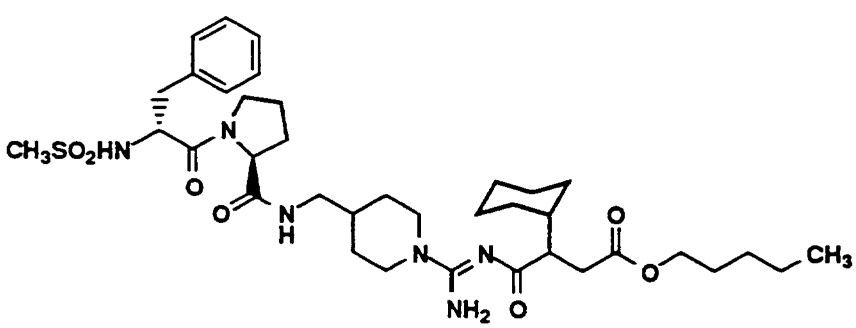

N-[[1-[amino[[2-cyclohexyl-1,4-dioxo-4-(pentyloxy)butyl]imino]methyl]-4-piperidinyl]methyl]-1-[N-(methylsulfonyl)-D-phenylalanyl]-L-prolinamide, hydrochloride;

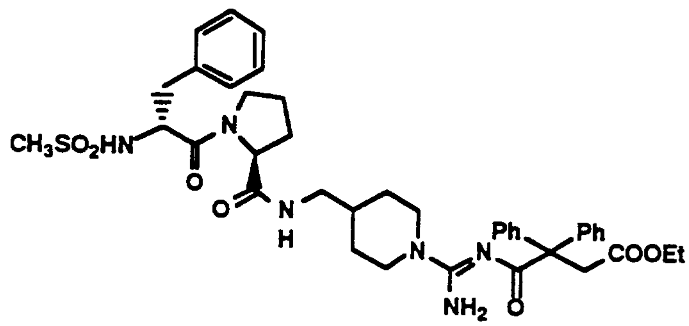

β-[[[amino[4-[[[1-[N-(methylsulfonyl)-D-phenylalanyl]-L-prolyl]amino]methyl]-1-piperidinyl]methyl]imino]carbonyl]-β-phenylbenzenepropanoic acid, ethyl ester, monohydrochloride;

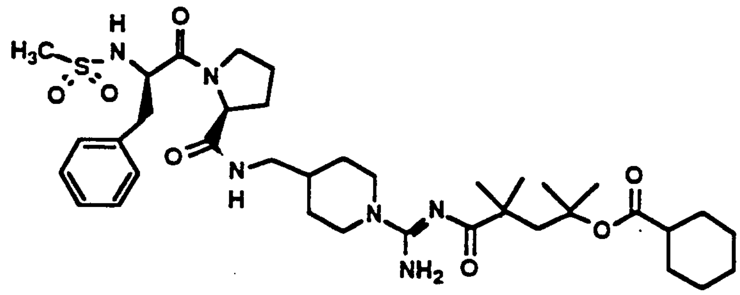

N-[[1-[amino[[4-[(cyclohexylcarbonyl)oxy]-2,2,4-trimethyl-1-oxopentyl]imino]-methyl]-4-piperidinyl]methyl]-1-[N-(methylsulfonyl)-D-phenylalanyl]-L-prolinamide,monohydrochloride;

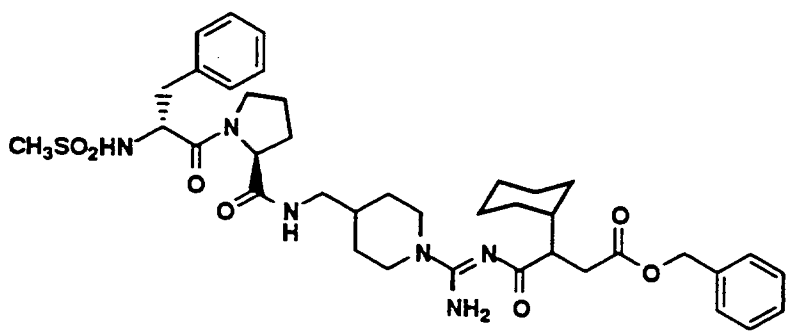

N-[[1-[amino[[2-cyclohexyl-1,4-dioxo-4-(phenylmethoxy)butyl]imino]methyl]-4-piperidinyl]methyl]-1-[N-(methylsulfonyl)-D-phenylalanyl]-L-prolinamide, monohydrochloride;

**30.** A method for preparing a monoacyl guanidine-containing compound as defined in Claim I of the structure

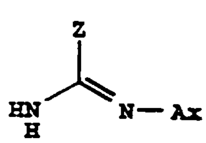

wherein Z represents a thrombin inhibitor substructure and Ax is Acyl, which comprises reacting a protected monoacylguanylpyrazole of the structure

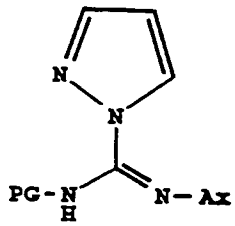

where PG is a protecting group, with a compound of the formula ZH, where Z is an amino-containing substructure, to form a protected monoacyl guanidine containing compound of the structure

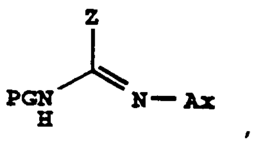

and removing the protecting group to form the monoacyl guanidine-containing compound.

**31.** The method as defined in Claim 30 wherein the reaction of the monoacylpyrazole with ZH is carried out in the presence of an amine base.

**32.** A method for preparing a monoacyl guanidine-containing compound as defined in Claim I of the structure

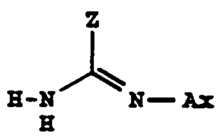

wherein Ax is Acyl and Z represents a thrombin inhibitor substructure, which comprises reacting a monoacyl guanidine pyrazole of the structure

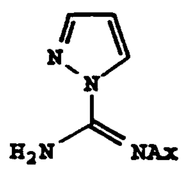

with a compound of the structure ZH wherein Z is an amino-containing thrombin inhibitor substructure to form the monoacyl guanidine-containing compound.

**33.** The method as defined in Claim 32 wherein the reaction is carried out in the presence of a base.

**34.** The method as defined in Claim 32 wherein the base is DBU.

**35.** The method as defined in Claim 32 wherein the monoacyl guanidine pyrazole is prepared by reacting IH-pyrazole-I-carboxamidine hydrochloride with an acid $R^ICOOH$ and a carbodiimide, or BOP reagent or with a succinimide ester $R^ICOOSu$, Su representing a succinimide, or with an acid anhydride $R^ICO_2OCR^I$ or an acid chloride $R^ICOCl$ in the presence of a base wherein $R^I$ is H, alkyl, cycloalkyl, aryl, substituted alkyl or substituted aryl.

**36.** The method as defined in Claim 32 wherein the protected monoacyl guanidine pyrazole is prepared by reacting (N-BOC) IH-pyrazole-I-carboxamidine with an acid $R^ICOOH$ and a carbodiimide, or BOP reagent or with a succinimide ester $R^ICOOSu$, Su representing a succinimide, or with an acid anhydride $R^ICO_2OCR^I$ or an acid chloride $R^ICOCl$

in the presence of a base wherein $R^I$ is H, alkyl, cycloalkyl, aryl, substituted alkyl or substituted aryl, and where the base is NaH, LHMDS or KOtBu.